# EUROPEAN PATENT APPLICATION

(11) **EP 0 774 460 A1**
(43) Date of publication of application: **21.05.1997**
(21) Application number: 95913390.1
(22) Date of filing: 30.03.1995
(51) Int. Cl.: C07D 207/09, C07D 207/34, C07D 209/48, C07D 211/26, C07D 401/12, C07D 403/06, C07D 403/12, C07D 405/12, C07D 409/12, C07D 413/12, C07D 207/08, C07D 205/04, C07D 207/12, C07D 419/06, C07D 223/04

(54) **BENZOIC ACID COMPOUND AND USE THEREOF AS MEDICINE**

(30) Priority: 30.03.1994 JP 60941/94; 05.07.1994 JP 153686/94; 20.01.1995 JP 749294/95
(71) Applicant: YOSHITOMI PHARMACEUTICAL INDUSTRIES, LTD., Osaka-shi Osaka 541 (JP)
(72) Inventor: KAWAKITA, Takeshi Yoshitomi Pharmaceutical Indus, Yoshitomimachi Chikujo-gun (JP); KUROITA, Takanobu Yoshitomi Pharmaceutical Indus, Yoshitomimachi Chikujo-gun (JP); MUROZONO, Takahiro Yoshitomi Pharmaceutical Indus, Yoshitomimachi Chikujo-gun Fukuoka 871 (JP); HAKIRA, Hidetoshi Yoshitomi Pharmaceutical Indus, Osaka-shi, Osaka 541 (JP); HAGA, Keiichiro Yoshitomi Pharmaceutical Indus, Yoshitomimachi Chikujo-gun Fukuoka 871 (JP)
(74) Representative: von Kreisler, Alek, Dipl.-Chem.
(86) International application number: JP9500616
(87) International publication number: WO9526953

(57) **Abstract**

Benzoic acid compounds of the formula 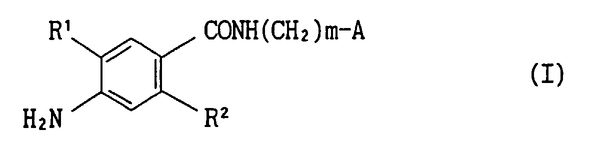 wherein each symbol is as defined in the specification, optical isomers thereof and pharmaceutically acceptable salts thereof; pharmaceutical composition comprising this compound and pharmaceutically acceptable additive; and serotonin 4 receptor agonists, gastrointestinal prokinetic agents and therapeutic agents for various gastrointestinal diseases, which comprise this compound as active ingredient.

The compounds of the present invention selectively activate serotonin 4 receptor, and are useful medications for the prophylaxis and treatment of various gastrointestinal diseases (e.g., delayed gastric emptying, indigestion, meteorism, reflux esophagitis, abdominal indefinite complaint, intestinal pseudoileus, constipation, acute or chronic gastritis, gastric or duodenal ulcer, Crohn's disease, non-ulcer dyspepsia, ulcerative colitis, postgastrectomy syndrome, postoperative digestive function failure, gastrointestinal injury due to gastric neurosis, gastroptosis, diabetes, etc., and irritable bowel syndrome), central nervous disorders (e.g., schizophrenia, depression, anxiety, disturbance of memory and dementia), cardiac function disorders (e.g., cardiac failure and myocardial ischemia), urinary diseases (e.g., dysuria caused by urinary obstruction, ureterolith or prostatomegaly) and the like.

## Description

### Technical Field

The present invention relates to novel benzoic acid compounds. More particularly, the present invention relates to novel benzoic acid compounds which selectively act on serotonin 4 (hereinafter referred to as 5-HT₄) receptors, and which are useful for the prophylaxis and therapy of various gastrointestinal diseases, central nervous disorders, cardiac function disorders, urinary diseases and the like, optical isomers thereof, pharmaceutically acceptable salts thereof, and use thereof as medicaments.

### Background Art

When control mechanism of gastrointestinal motility fails and prokinetic function declines, atonic constipation and digestive symptoms such as abdominal distention, anorexia and heartburn emerge. Declined gastrointestinal motility is found with aging, stress or diseases such as chronic gastritis, non-ulcer dyspepsia, reflux esophagitis, peptic ulcer, diabetes and the like, and gastrointestinal prokinetic agents have been used for treatments.

Ever since metoclopramide (The Merck Index, vol. 11, 6063) was developed as a gastrointestinal prokinetic agent, various substituted benzamide derivatives have been synthesized. At present, cisapride (The Merck Index, vol. 11, 2318) and others have been clinically used as gastrointestinal prokinetic agents besides metoclopramide. While benzamide derivatives such as metoclopramide and cisapride have been speculated to show effects via certain receptors in the digestive organs, the actual function of the receptors involved in the promotion of gastrointestinal motility has long been unclarified. Recently, however, 5-HT₄ receptor has been identified to be a new serotonin receptor subtype which stimulates adenylate cyclase activity, and benzamide derivatives have been found to promote gastrointestinal motility by activating 5-HT₄ receptor in the digestive organs (The Journal of Pharmacology and Experimental Therapeutics, vol. 252, pp. 1378-1386, European Journal of Pharmacology, vol. 196, pp. 149-155). Having found the action of metoclopramide and cisapride as 5-HT₄ receptor agonists, many attempts have been made to use 5-HT₄ receptor agonists as gastrointestinal prokinetic agents. The Journal of Pharmacology and Experimental Therapeutics, vol. 264, pp. 240-248 reports that substituted benzamide (SC53116) which is a 5-HT₄ receptor agonist stimulated gastrointestinal motility. As 5-HT₄ receptor agonists, Japanese Patent Unexamined Publication No. 157518/1994 discloses oxadiazole derivatives; Japanese Patent Unexamined Publication No. 10881/1995 discloses oxazabicyclo derivatives; and WO 94/12497 discloses endo-N-(8-methyl-8-azabicyclo[3.2.1]oct-3-yl)-1-isopropyl-2(1H)-quinolone-3-carboxamide and acid addition salts thereof.

The 5-HT₄ receptor has been found to be also present in brain, heart, endocrine system and urinary system [British Journal of Pharmacology, vol. 109, pp. 618-624 and Naunyn-Schmiedeberg's Archives of Pharmacology, vol. 344, pp. 150-159, Trends in Pharmacological sciences, vol. 13, pp. 141-145 (1992), Pharmacological Reviews, vol. 46, pp. 182-185 (1994)].

In view of such disclosures, a compound having affinity for 5-HT₄ receptor is considered to be clinically applicable to digestive organs, brain, heart and urinary system. In other words, a 5-HT₄ receptor agonist should be useful as a medication for the prophylaxis and treatment of various gastrointestinal diseases (e.g., delayed gastric emptying, indigestion, meteorism, reflux esophagitis, abdominal indefinite complaint, intestinal pseudoileus, constipation, acute or chronic gastritis, gastric or duodenal ulcer, Crohn's disease, non-ulcer dyspepsia, ulcerative colitis, postgastrectomy syndrome, postoperative digestive function failure, gastrointestinal injury due to gastric neurosis, gastroptosis, diabetes, etc., and irritable bowel syndrome), central nervous disorders (e.g., schizophrenia, depression, anxiety, disturbance of memory and dementia), cardiac function disorders (e.g., cardiac failure and myocardial ischemia), urinary diseases (e.g., dysuria caused by urinary obstruction, ureterolith or prostatomegaly) and the like.

While there have been documented some compounds which show selective agonistic effect on 5-HT₄ receptors (British Journal of Pharmacology, vol. 110, pp. 119-126, Journal of Medicinal Chemistry, vol. 36, pp. 4121-4123), a compound has not been known at all, which has amide, urea or a heterocycle having an amide or urea bond in the ring, at an alkyl moiety bound to nitrogen atom of cyclic amine.

The above-mentioned substituted benzamide derivatives, such as metoclopramide, have, besides 5-HT₄ receptor agonistic effect, dopamine 2 (hereinafter referred to as D₂) receptor antagonism or serotonin 3 (hereinafter referred to as 5-HT₃) receptor antagonism, and are not entirely satisfactory in terms of efficacy and safety, since they cause side effects such as extrapyramidal disorders due to D₂ receptor antagonism, and side effects such as constipation due to 5-HT₃ receptor antagonism (Drugs, vol. 41, pp. 574-595), and are associated with such problems to be solved.

As gastrointestinal prokinetic agents, Japanese Patent Unexamined Publication Nos. 262724/1993, 50883/1989 and 211685/1992 disclose compounds having 5-HT₃ receptor antagonism. These compounds again cause side effects such as constipation, as mentioned above, and are not satisfactory. Since 5-HT₄ receptors are reportedly distributed widely in the entirety of digestive organs, a medication which selectively activates 5-HT₄ receptors is expected to make a superior gastrointestinal prokinetic agent.

Accordingly, the present invention aims at providing a compound which has selective affinity for 5-HT₄ receptors in various tissues, and which is useful as a medication for the prophylaxis and treatment of various gastrointestinal diseases (e.g., delayed gastric emptying, indigestion, meteorism, reflux esophagitis, abdominal indefinite complaint, intestinal pseudoileus, constipation, acute or chronic gastritis, gastric or duodenal ulcer, Crohn's disease, non-ulcer dyspepsia, ulcerative colitis, postgastrectomy syndrome, postoperative digestive function failure, gastrointestinal injury due to gastric neurosis, gastroptosis, diabetes, etc., and irritable bowel syndrome), central nervous disorders (e.g., schizophrenia, depression, anxiety, disturbance of memory and dementia), cardiac function disorders (e.g., cardiac failure and myocardial ischemia), urinary diseases (e.g., dysuria caused by urinary obstruction, ureterolith or prostatomegaly) and the like.

In view of the above-mentioned situations, the present inventors have conducted intensive studies with the aim of finding a compound with selective agonistic effects on 5-HT₄ receptors and found that an inventive new benzoic acid compound, namely, a benzoic acid compound which has, at an alkyl moiety bound to nitrogen atom of cyclic amine of the following formulas (II-a) - (II-f) and (III), amide, urea or a heterocycle having an amide or urea bond in the ring, shows superior selective activation of 5-HT₄ receptors, which resulted in the completion of the present invention.

### Disclosure of the Invention

Thus, the present invention provides the following.
(1) Benzoic acid compounds of the formula 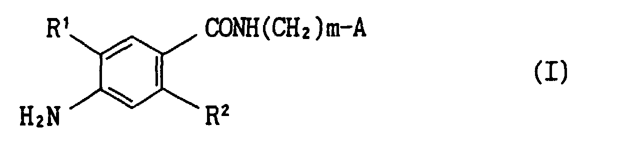 wherein
   - R¹: is a halogen;
   - R²: is a lower alkoxy, a substituted lower alkoxy, a cycloalkyloxy or a cycloalkylalkoxy;
   - m: is 1 or 2; and
   - A: is 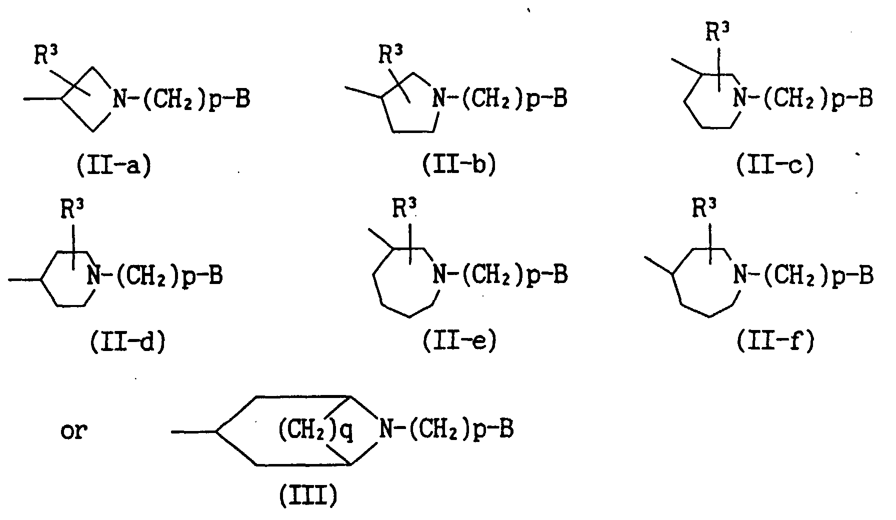
   wherein
   R³ is hydrogen, hydroxy, lower alkyl or lower alkoxy, p is an integer of 1-6, q is 2 or 3, and B is a group of the formula
   -N(R⁴)-X¹-R⁵,
   -N(R⁴)-X²-N(R⁶)(R⁷),
   -X¹-N(R⁸)(R⁹) or
   -Het
   wherein
   X¹ is CO, CS or SO₂, X² is CO or CS, R⁴ is hydrogen, lower alkyl, phenyl, substituted phenyl, aralkyl or substituted aralkyl, R⁵ is lower alkyl, cycloalkyl, crosslinked cycloalkyl, aryl, substituted aryl, aralkyl, substituted aralkyl, heteroaryl, substituted heteroaryl, heteroarylalkyl, substituted heteroarylalkyl or 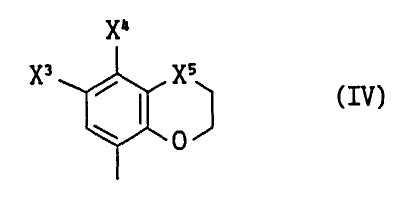 wherein X³ is halogen, X⁴ is hydrogen or amino, and X⁵ is a direct bond, methylene, oxygen atom, NH or N-CH₃,
   R⁶ and R⁷ are the same or different and each is hydrogen, lower alkyl, cycloalkyl, aryl, substituted aryl, aralkyl or substituted aralkyl, or R⁶ and R⁷ optionally form a ring together with the adjacent nitrogen atom, R⁸ and R⁹ are the same or different and each is hydrogen, lower alkyl, cycloalkyl, aryl, substituted aryl, aralkyl or substituted aralkyl or R⁸ and R⁹ optionally form a ring together with the adjacent nitrogen atom, and Het is a 5- or 6-membered mono- or bicyclic heterocycle having amide or urea in the ring and having 1 to 5 hetero atom(s) selected from the group consisting of oxygen atom, sulfur atom and nitrogen atom,
   optical isomers thereof and pharmaceutically acceptable salts thereof.
(2) Benzoic acid compounds of the formula 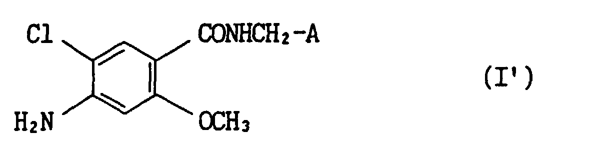 wherein A is as defined above, optical isomers thereof and pharmaceutically acceptable salts thereof.
(3) Benzoic acid compounds of the formula 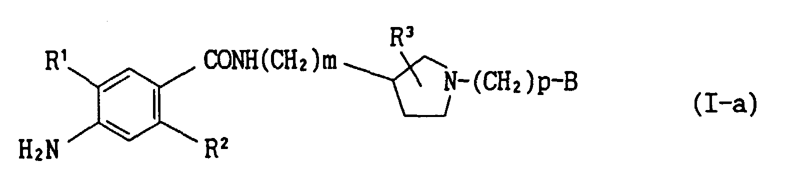 wherein R¹, R², R³, m, p and B are as defined above, optical isomers thereof and pharmaceutically acceptable salts thereof.
(4) Benzoic acid compounds of the formula 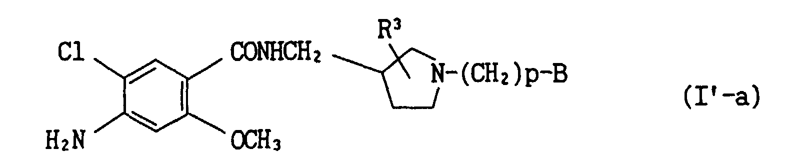 wherein R³, p and B are as defined above, optical isomers thereof and pharmaceutically acceptable salts thereof.
(5) Benzoic acid compounds of the formula 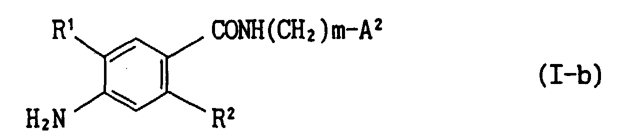 wherein R¹, R² and m are as defined above, and A² is 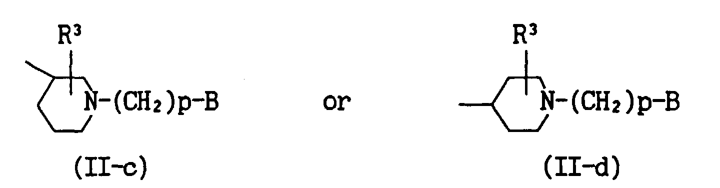 wherein R³, p and B are as defined above, optical isomers thereof and pharmaceutically acceptable salts thereof.
(6) Benzoic acid compounds of the formula 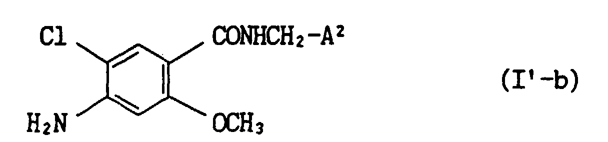 wherein A² is as defined above, optical isomers thereof and pharmaceutically acceptable salts thereof.
(7) Benzoic acid compounds of above (1) or (2), wherein B is a group of the formula
   -N(R⁴)-CO-R⁵,
   -N(R⁴)-CO-N(R⁶)(R⁷),
   -CO-N(R⁸)(R⁹) or 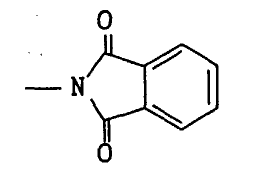 wherein R⁴, R⁵, R⁶, R⁷, R⁸ and R⁹ are as defined above, optical isomers thereof and pharmaceutically acceptable salts thereof.
   (8) Benzoic acid compounds of above (3) or (4), wherein B is a group of the formula
   -N(R⁴)-CO-R⁵,
   -N(R⁴)-CO-N(R⁶)(R⁷),
   -CO-N(R⁸)(R⁹) or 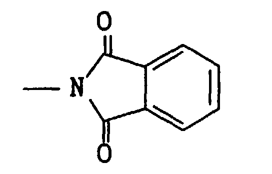 wherein R⁴, R⁵, R⁶, R⁷, R⁸ and R⁹ are as defined above, optical isomers thereof and pharmaceutically acceptable salts thereof.
(9) Benzoic acid compounds of above (5) or (6), wherein B is a group of the formula
   -N(R⁴)-CO-R⁵,
   -N(R⁴)-CO-N(R⁶)(R⁷),
   -CO-N(R⁸)(R⁹) or 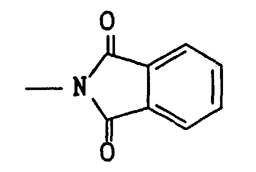 wherein R⁴, R⁵, R⁶, R⁷, R⁸ and R⁹ are as defined above, optical isomers thereof and pharmaceutically acceptable salts thereof.
(10) Benzoic acid compounds of any one of above (1) to (9), wherein B is a group of the formula
   -N(R⁴)-CO-R⁵ or
   -N(R⁴)-CO-N(R⁶)(R⁷)
   wherein R⁴, R⁵, R⁶ and R⁷ are as defined above, optical isomers thereof and pharmaceutically acceptable salts thereof.
(11) Benzoic acid compounds of any one of above (1) to (10), wherein B is a group of the formula
   -NHCOR⁵
   wherein R⁵ is as defined above, optical isomers thereof and pharmaceutically acceptable salts thereof.
(12) Benzoic acid compounds of any one of above (1) to (10), wherein B is a group of the formula
   -NHCONHR^{6a}
   wherein R^{6a} is hydrogen, lower alkyl, cycloalkyl, aryl, substituted aryl, aralkyl or substituted aralkyl, optical isomers thereof and pharmaceutically acceptable salts thereof.
(13) Benzoic acid compounds of any one of above (1) to (9), wherein B is a group of the formula
   -CONHR^{8a}
   wherein R^{8a} is hydrogen, lower alkyl, cycloalkyl, aryl, substituted aryl, aralkyl or substituted aralkyl, optical isomers thereof and pharmaceutically acceptable salts thereof.
(14) Benzoic acid compounds of any one of above (1) to (9), wherein B is a group of the formula 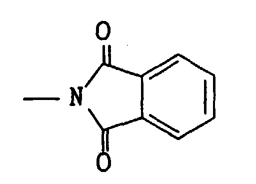 optical isomers thereof and pharmaceutically acceptable salts thereof.
(15) Benzoic acid compounds of any one of above (1) to (11), wherein R⁵ is aryl, substituted aryl, aralkyl, heteroaryl or substituted heteroaryl, optical isomers thereof and pharmaceutically acceptable salts thereof.
(16) Benzoic acid compounds of any one of above (1) to (10) and (12), wherein R⁶ is lower alkyl, aryl or substituted aryl, optical isomers thereof and pharmaceutically acceptable salts thereof.
(17) Benzoic acid compounds of any one of above (1) to (9) and (13), wherein R⁸ is aryl or substituted aryl, optical isomers thereof and pharmaceutically acceptable salts thereof.
(18) Benzoic acid compounds of any one of above (1) to (11), wherein R⁵ is 1-methyl-3-indolyl, 1-isopropyl-3-indolyl, 1-benzyl-3-indolyl, 1-naphthyl, 2-naphthyl, phenyl, 2-chlorophenyl, 3-chlorophenyl, 4-chlorophenyl, 4-methylphenyl, 4-nitrophenyl, 4-amino-5-chloro-2-methoxyphenyl, 2-thienyl or 3-phenylpropyl, optical isomers thereof and pharmaceutically acceptable salts thereof.
(19) Benzoic acid compounds of any one of above (1) to (10) and (12), wherein R⁶ is ethyl, propyl, phenyl or 4-chlorophenyl, optical isomers thereof and pharmaceutically acceptable salts thereof.
(20) Benzoic acid compounds of any one of above (1) to (9) and (13), wherein R⁸ is phenyl, optical isomers thereof and pharmaceutically acceptable salts thereof.
(21) Benzoic acid compounds of any one of above (1) to (20), wherein p is an integer of 3-6, optical isomers thereof and pharmaceutically acceptable salts thereof.
(22) Benzoic acid compounds of any one of above (1) to (20), wherein p is 4 or 5, optical isomers thereof and pharmaceutically acceptable salts thereof.
(23) Benzoic acid compounds of (4), wherein R³ is hydrogen, p is an integer of 2-5, and B is a group of the formula
   -NHCOR^{5a},
   -NHCONHR^{6b},
   -CONHR^{8b} or 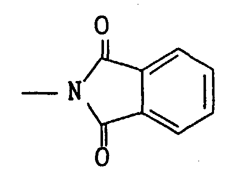 wherein R^{5a} is aryl, substituted aryl, aralkyl, heteroaryl or substituted heteroaryl, R^{6b} is lower alkyl, aryl or substituted aryl, and R^{8b} is aryl or substituted aryl, optical isomers thereof and pharmaceutically acceptable salts thereof.
(24) Benzoic acid compounds of any one of (1)-(4), (7), (8), (10), (11), (15), (18), (21), (22) and (23), which are selected from
   4-amino-N-((3R)-1-(3-benzoylaminopropyl)pyrrolidin-3-ylmethyl)-5-chloro-2-methoxybenzamide,
   4-amino-5-chloro-2-methoxy-N-((3R)-1-(4-(1-naphthoylamino)butyl)pyrrolidin-3-ylmethyl)benzamide,
   4-amino-N-((3R)-1-(5-benzoylaminopentyl)pyrrolidin-3-ylmethyl)-5-chloro-2-methoxybenzamide,
   4-amino-N-((3R)-1-(5-(4-amino-5-chloro-2-methoxybenzoylamino)pentyl)pyrrolidin-3-ylmethyl)-5-chloro-2-methoxybenzemide,
   N-(4-((3R)-3-(4-amino-5-chloro-2-methoxybenzoylaminomethyl)pyrrolidin-1-yl)butyl)-1-methyl-1H-indole-3-carboxamide,
   N-(5-((3R)-3-(4-amino-5-chloro-2-methoxybenzoylaminomethyl)pyrrolidin-1-yl)pentyl)-1-methyl-1H-indole-3-carboxamide,
   N-(4-((3R)-3-(4-amino-5-chloro-2-methoxybenzoylaminomethyl)pyrrolidin-1-yl)butyl)-1-isopropyl-1H-indole-3-carboxamide,
   N-(4-((3R)-3-(4-amino-5-chloro-2-methoxybenzoylaminomethyl)pyrrolidin-1-yl)butyl)-1-benzyl-1H-indole-3-carboxamide,
   4-amino-5-chloro-2-methoxy-N-((3R)-1-(4-(2-naphthoylamino)butyl)pyrrolidin-3-ylmethyl)benzamide,
   4-amino-5-chloro-N-((3R)-1-(5-(4-chlorobenzoylamino)pentyl)pyrrolidin-3-ylmethyl)-2-methoxybenzamide,
   4-amino-5-chloro-N-(1-(3-(3-chlorobenzoylamino)propyl)pyrrolidin-3-ylmethyl)-2-methoxybenzamide,
   4-amino-5-chloro-N-(1-(3-(2-chlorobenzoylamino)propyl)pyrrolidin-3-ylmethyl)-2-methoxybenzamide,
   4-amino-5-chloro-2-methoxy-N-(1-(3-(4-nitrobenzoylamino)propyl)pyrrolidin-3-ylmethyl)benzamide,
   4-amino-5-chloro-2-methoxy-N-((3R)-1-(2-(4-phenylbutyrylamino)ethyl)pyrrolidin-3-ylmethyl)benzamide,
   4-amino-5-chloro-N-(1-(3-(4-chlorobenzoylamino)propyl)pyrrolidin-3-ylmethyl)-2-methoxybenzamide,
   4-amino-5-chloro-2-methoxy-N-(1-(3-(4-methylbenzoylamino)propyl)pyrrolidin-3-ylmethyl)benzamide and
   4-amino-5-chloro-2-methoxy-N-(1-(3-(2-thiophenecarbonylamino)propyl)pyrrolidin-3-ylmethyl)benzamide,
      optical isomers thereof and pharmaceutically acceptable salts thereof.
(25) Benzoic acid compounds of any one of (1)-(4), (7), (8), (10), (12), (16), (19), (21), (22) and (23), which are selected from
   4-amino-5-chloro-2-methoxy-N-((3R)-1-(5-(3-n-propylureido)pentyl)pyrrolidin-3-ylmethyl)benzamide,
   4-amino-5-chloro-2-methoxy-N-((3R)-1-(5-(3-phenylureido)pentyl)pyrrolidin-3-ylmethyl)benzamide and
   4-amino-5-chloro-N-((3R)-1-(5-(3-ethylureido)pentyl)pyrrolidin-3-ylmethyl)-2-methoxybenzamide,
      optical isomers thereof and pharmaceutically acceptable salts thereof.
(26) Benzoic acid compounds of any one of (1)-(4), (7), (8), (13), (17), (20), (21) and (23), which is 4-amino-5-chloro-2-methoxy-N-(1-(3-phenylcarbamoylpropyl)pyrrolidin-3-ylmethyl)benzamide, optical isomers thereof and pharmaceutically acceptable salts thereof.
(27) Benzoic acid compounds of any one of (1)-(4), (7), (8), (14), (21), (22) and (23), which is 4-amino-5-chloro-N-(1-(5-(2,3-dihydro-1,3-dioxo-1H-isoindol-2-yl)pentyl)pyrrolidin-3-ylmethyl)-2-methoxybenzamide, optical isomers thereof and pharmaceutically acceptable salts thereof.
(28) Benzoic acid compounds of above (6), wherein A² is a group of the formula 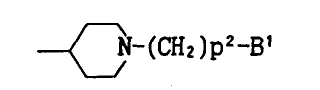 wherein p² is 4 or 5, and B¹ is a group of the formula
   -NHCOR^{5a} or
   -NHCONHR^{6b}
   wherein R^{5a} is aryl, substituted aryl, heteroaryl or substituted heteroaryl, and R^{6b} is lower alkyl, aryl or substituted aryl, and pharmaceutically acceptable salts thereof.
(29) Benzoic acid compounds of any one of (1), (2), (5)-(7), (9), (10), (11), (15), (18), (21), (22) and (28), which are selected from
   N-(4-(4-(4-amino-5-chloro-2-methoxybenzoylaminomethyl)piperidin-1-yl)butyl)-1-methyl-1H-indole-3-carboxamide,
   4-amino-5-chloro-2-methoxy-N-(1-(4-(1-naphthoylamino)butyl)piperidin-4-ylmethyl)benzamide,
   4-amino-5-chloro-2-methoxy-N-(1-(4-(2-naphthoylamino)butyl)piperidin-4-ylmethyl)benzamide,
   4-amino-N-(1-(5-benzoylaminopentyl)piperidin-4-ylmethyl)-5-chloro-2-methoxybenzamide,
   4-amino-5-chloro-N-(1-(5-(3-chlorobenzoylamino)pentyl)piperidin-4-ylmethyl)-2-methoxybenzamide,
   4-amino-5-chloro-N-(1-(5-(4-methylbenzoylamino)pentyl)piperidin-4-ylmethyl)-2-methoxybenzamide and
   N-(5-(4-(4-amino-5-chloro-2-methoxybenzoylaminomethyl)piperidin-1-yl)pentyl)-1-methyl-1H-indole-3-carboxamide, and pharmaceutically acceptable salts thereof.
(30) Benzoic acid compounds of any one of (1), (2), (5)-(7), (9), (10), (12), (16), (19), (21), (22) and (28), which are selected from
   4-amino-5-chloro-2-methoxy-N-(1-(4-(3-n-propylureido)butyl)piperidin-4-ylmethyl)benzamide,
   4-amino-5-chloro-2-methoxy-N-(1-(5-(3-n-propylureido)pentyl)piperidin-4-ylmethyl)benzamide, and
   4-amino-5-chloro-N-(1-(5-(3-(4-chlorophenyl)ureido)pentyl)piperidin-4-ylmethyl)-2-methoxybenzamide, and pharmaceutically acceptable salts thereof.
(31) Pharmaceutical compositions comprising benzoic acid compounds of any one of (1 )-(30) or optical isomers thereof or pharmaceutically acceptable salts thereof, and pharmaceutically acceptable additives.
(32) Serotonin 4 receptor agonists comprising benzoic acid compounds of any one of (1)-(30) or optical isomers thereof or pharmaceutically acceptable salts thereof as active ingredients.
(33) Gastrointestinal prokinetic agents comprising benzoic acid compounds of any one of (1)-(30) or optical isomers thereof or pharmaceutically acceptable salts thereof as active ingredients.
(34) Therapeutic agents for various gastrointestinal diseases selected from the group consisting of delayed gastric emptying, indigestion, meteorism, reflux esophagitis, abdominal indefinite complaint, intestinal pseudoileus, constipation, acute or chronic gastritis, gastric or duodenal ulcer, Crohn's disease, non-ulcer dyspepsia, ulcerative colitis, postgastrectomy syndrome, postoperative digestive function failure, gastrointestinal injury due to gastric neurosis, gastroptosis, diabetes, etc., and irritable bowel syndrome, which comprises benzoic acid compounds of any one of (1)-(30) or optical isomers thereof or pharmaceutically acceptable salts thereof as active ingredients.

The compound of the present invention is characterized by substitution of amide, urea or heterocycle having an amide or urea bond in the ring, at the alkyl moiety bound to nitrogen atom of cyclic amine of the formulas (II-a) - (II-f) and (III), and, with regard to the cyclic amine of the formulas (II-a) - (II-f), a bond between the binding site of the formula (I), namely,
-CONH(CH₂)ₘ-
and carbon atom other than that adjacent to the intercyclic nitrogen atom. Such characteristic chemical structure leads to 5-HT₄ receptor activation with much superior selectivity by the compound of the present invention. Naturally, the above-mentioned amide includes thioamide, sulfonamide, carbamoyl and sulfamoyl, and urea includes thiourea.

Of the above-mentioned symbols, halogen at R¹ is exemplified by, for example, fluorine, chlorine, bromine and iodine, with particular preference given to chlorine.

Lower alkoxy at R² is a linear or branched alkoxy having 1 to 4 carbon atoms, and exemplified by, for example, methoxy, ethoxy, propoxy, isopropoxy, butoxy, isobutoxy and tert-butoxy, with particular preference given to methoxy.

Substituted lower alkoxy at R² is substituted by, for example, fluorine, alkoxy (e.g., methoxy, ethoxy and isopropoxy), acyl (e.g., acetyl and propionyl) and cyano. Specific examples of substituted lower alkoxy include, for example, fluoromethoxy, 2- fluoroethoxy, 3-fluoropropoxy, methoxymethoxy, ethoxymethoxy, 2-methoxyethoxy, 2-ethoxyethoxy, 3-methoxypropoxy, 3-ethoxypropoxy, 2-oxopropoxy, 2-oxobutoxy, 3-oxobutoxy, 3-oxopentyloxy, 4-oxopentyloxy, 4-oxohexyloxy, cyanomethoxy, 2-cyanoethoxy and 3-cyanopropoxy.

Cycloalkyloxy at R² is exemplified by cyclopropyloxy, cyclobutyloxy, cyclopentyloxy, cyclohexyloxy and the like.

Cycloalkylalkoxy at R² is exemplified by cyclopropylmethoxy, 1-cyclopropylethoxy, 2-cyclopropylethoxy, cyclobutylmethoxy, cyclopentylmethoxy, cyclohexylmethoxy and the like, with particular preference given to cyclopropylmethoxy.

Lower alkyl at R³ is a linear or branched alkyl having 1 to 6 carbon atoms, and exemplified by, for example, methyl, ethyl, propyl, isopropyl, butyl, isobutyl, tert-butyl, pentyl and hexyl.

Lower alkoxy at R³ is the same as that at R².

Lower alkyl at R⁴ is the same as that at R³, with preference given to methyl.

Examples of substituents for substituted phenyl at R⁴ are, for example, halogen (e.g., fluorine, chlorine and bromine), lower alkyl having 1 to 4 carbon atoms such as methyl, ethyl, propyl, isopropyl, butyl, isobutyl and tert-butyl, lower alkoxy having 1 to 4 carbon atoms such as methoxy, ethoxy, propoxy, isopropoxy, butoxy, isobutoxy and tert-butoxy, aralkyl such as benzyl, hydroxy, nitro and amino.

Aralkyl at R⁴ is linear or branched alkyl having 1 to 4 carbon atoms (e.g., methyl, ethyl, propyl, isopropyl, butyl, isobutyl and tert-butyl) which has been substituted by phenyl, and is exemplified by, for example, benzyl, 2-phenethyl, 3-phenylpropyl and 4-phenylbutyl.

Substituents for substituted aralkyl at R⁴ are the same as those for substituted phenyl at R⁴.

Lower alkyl at R⁵ is the same as that at R³, and particularly preferred is methyl.

Cycloalkyl at R⁵ is that having 3 to 6 carbon atoms, such as cyclopropyl, cyclobutyl, cyclopentyl, cyclohexyl and the like, with particular preference given to cyclohexyl.

Crosslinked cycloalkyl at R⁵ is exemplified by adamantyl, noradamantyl and the like, with preference given to adamantyl.

Aryl at R⁵ and R^{5a} includes, for example, phenyl, 1-naphthyl and 2-naphthyl.

Substituents for substituted aryl at R⁵ and R^{5a} are the same as those for substituted phenyl at R⁴. Preferred are halogen, lower alkyl, lower alkoxy, amino and nitro, and particularly preferred are chlorine, methyl, methoxy, amino and nitro. Specific examples of substituted aryl include 4-chlorophenyl, 3-chlorophenyl, 2-chlorophenyl, 4-methylphenyl, 4-methoxyphenyl, 4-amino-5-chloro-2-methoxyphenyl, 4-nitrophenyl and the like, and preferred are 4-chlorophenyl, 3-chlorophenyl, 2-chlorophenyl, 4-amino-5-chloro-2-methoxyphenyl, 4-methylphenyl and 4-nitrophenyl.

Aralkyl at R⁵ and R^{5a} is the same as that at R⁴, and preferred is 3-phenylpropyl.

Substituents for substituted aralkyl at R⁵ are the same as those for substituted phenyl at R⁴.

Heteroaryl at R⁵ and R^{5a} is exemplified by, for example, pyrrolyl, pyridyl, indolyl, indazolyl, thienyl, furyl, benzofuranyl, thionaphthenyl and the like, with preference given to 4-pyridyl, 2-thienyl, 2-indolyl and 3-indolyl.

Substituents for substituted heteroaryl at R⁵ and R^{5a} are the same as those for substituted phenyl at R⁴. Preferred are lower alkyl and aralkyl. Specific examples of substituted heteroaryl include 1-methyl-3-indolyl, 1-ethyl-3-indolyl, 1-propyl-3-indolyl, 1-isopropyl-3-indolyl, 1-butyl-3-indolyl, 1-benzyl-3-indolyl, 1-(2-phenethyl)-3-indolyl, 1-(3-phenylpropyl)-3-indolyl, 1-(4-phenylbutyl)-3-indolyl, 1-methyl-3-indazolyl and the like. Preferred are 1-methyl-3-indolyl, 1-isopropyl-3-indolyl, 1-benzyl-3-indolyl and 1-methyl-3-indazolyl.

Heteroarylalkyl at R⁵ is exemplified by, for example, 2-thienylmethyl, 3-thienylmethyl, 2-(2-thienyl)ethyl, 3-(2-thienyl)propyl, 2-(2-furyl)ethyl, 2-(3-furyl)ethyl, 2-pyridylmethyl, 3-pyridylmethyl, 4-pyridylmethyl, 3-indolylmethyl, 2-(3-indolyl)ethyl and 3-(3-indolyl)propyl.

Substituted heteroarylalkyl at R⁵ has, as substituent, lower alkyl (e.g., methyl, ethyl, propyl, isopropyl, butyl and the like), aralkyl (e.g., benzyl and the like), and the like, and is specifically (1-methyl-3-indolyl)methyl, (1-isopropyl-3-indolyl)methyl, (1-benzyl-3-indolyl)methyl, 2-(1-methyl-3-indolyl)ethyl, 3-(1-methyl-3-indolyl)propyl and the like.

Halogen at X³ is the same as that at R¹, with particular preference given to chlorine. Examples of preferable group of the formula (IV) include 6-chloro-4-methyl-3,4-dihydro-2H-1,4-benzoxazin-8-yl and the like.

Lower alkyl at R⁶, R^{6a}, R^{6b} and R⁷ is the same as that at R³, with preference given to methyl, ethyl, propyl, isopropyl and n-butyl, and with particular preference given to ethyl and propyl.

Cycloalkyl at R⁶, R^{6a} and R⁷ is the same at that at R⁵.

Aryl at R⁶, R^{6a}, R^{6b} and R⁷ is phenyl, 1-naphthyl or 2-naphthyl, with preference given to phenyl.

Substituents for substituted aryl at R⁶, R^{6a}, R^{6b} and R⁷ are the same as those for substituted phenyl at R⁴. Preferred is halogen, particularly chlorine. Specific examples of substituted aryl include, for example, 2-chlorophenyl, 3-chlorophenyl, 4-chlorophenyl, 2-bromophenyl, 3-bromophenyl, 4-bromophenyl, 2-fluorophenyl, 3-fluorophenyl and 4-fluorophenyl, with particular preference given to 4-chlorophenyl.

Aralkyl at R⁶, R^{6a} and R⁷ is the same as that at R⁴.

Substituents for substituted aralkyl at R⁶, R^{6a} and R⁷ are the same as those for substituted phenyl at R⁴.

The ring formed by R⁶ and R⁷ together with the adjacent nitrogen atom may have, in the ring, hetero atom such as sulfur atom, oxygen atom and nitrogen atom. Further, it may have, in the ring, lower alkyl such as methyl and ethyl or lower alkoxy such as methoxy and ethoxy. Specific examples of the ring include pyrrolidine, piperidine, morpholine, thiomorpholine, N-methylpiperazine and the like, with particular preference given to morpholine.

Lower alkyl at R⁸, R^{8a} and R⁹ is the same as that at R³.

Cycloalkyl at R⁸, R^{8a} and R⁹ is the same as that at R⁵.

Aryl at R⁸, R^{8a}, R^{8b} and R⁹ is the same as that at R⁶ and R⁷, with preference given to phenyl.

Substituents for substituted aryl at R⁸, R^{8a}, R^{8b} and R⁹ are the same as those for substituted phenyl at R⁴. Examples of preferable substituents include, for example, halogen and lower alkyl, particularly chlorine and methyl. Specific examples of substituted aryl include 2-chlorophenyl, 3-chlorophenyl, 4-chlorophenyl, 2-bromophenyl, 3-bromophenyl, 4-bromophenyl, 2-fluorophenyl, 3-fluorophenyl, 4-fluorophenyl, 2-methylphenyl, 3-methylphanyl and 4-methylphenyl, with preference given to 3-chlorophenyl and 4-methylphenyl.

Aralkyl at R⁸, R^{8a} and R⁹ is the same as that at R⁴.

Substituents for substituted aralkyl at R⁸, R^{8a} and R⁹ are the same as those for substituted phenyl at R⁴.

The ring formed by R⁸ and R⁹ together with the adjacent nitrogen atom may have, in the ring, hetero atom such as sulfur atom, oxygen atom and nitrogen atom. Further, it may have, in the ring, lower alkyl such as methyl and ethyl or lower alkoxy such as methoxy and ethoxy. Specific examples of the ring include pyrrolidine, piperidine, morpholine, thiomorpholine and N-methylpiperazine.

Het at B in the case of monocyclic heterocycle can be expressed by the following formulas 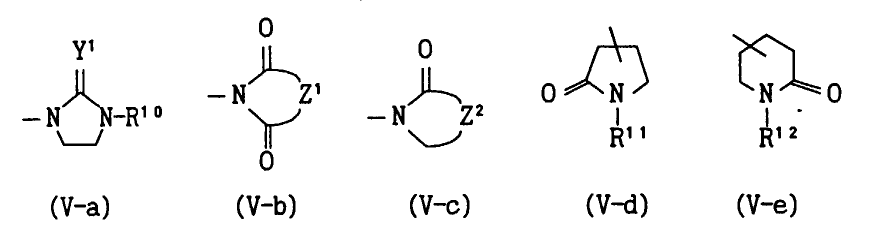 wherein Y¹ is oxygen atom or sulfur atom, Z¹ is
-(CH₂)₂- or -CH=CH-
Z² is
-(CH₂)₂- or -(CH₂)₃-
and R¹⁰, R¹¹ and R¹² are each hydrogen, or linear or branched alkyl having 1 to 4 carbon atoms or aralkyl.

Het at B in the case of bicyclic heterocycle can be expressed by the following formulas 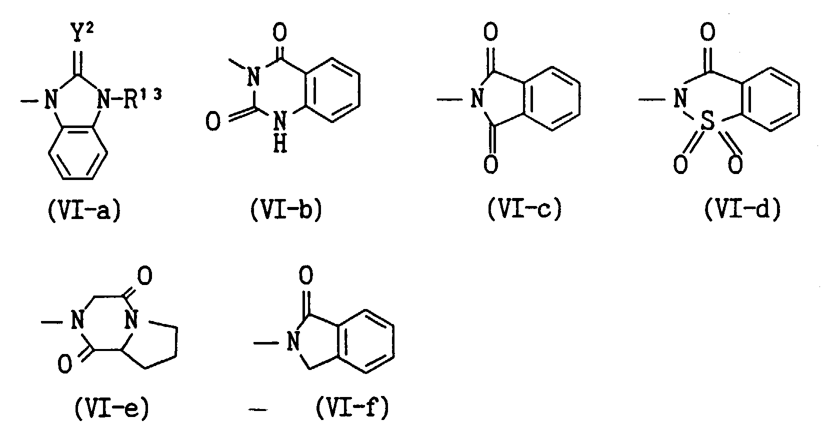 wherein Y² is oxygen atom or sulfur atom, and R¹³ is hydrogen, or linear or branched alkyl having 1 to 4 carbon atoms or aralkyl.

Of the above-mentioned symbols, linear or branched alkyl having 1 to 4 carbon atoms at R¹⁰, R¹¹, R¹² and R¹³ is exemplified by, for example, methyl, ethyl, propyl, isopropyl, butyl, isobutyl, tert-butyl and the like, with preference given to methyl. Aralkyl at R¹⁰, R¹¹, R¹² and R¹³ is the same as that at R⁴, wherein preferred is benzyl. Of the above-mentioned substituents, the group of the formula (VI-c) is preferable.

Specific examples of cyclic amine of the formula (II-b) include, for example, 1-(2-acetylaminoethyl)pyrrolidin-3-yl, 1-(4-acetylminobutyl)pyrrolidin-3-yl, 1-(5-acetylaminopentyl)pyrrolidin-3-yl, 1-(2-cyclohexanecarbonylaminoethyl)pyrrolidin-3-yl, 1-(3-cyclohexanecarbonylaminopropyl)pyrrolidin-3-yl, 1-(4-cyclohexanecarbonylaminobutyl)pyrrolidin-3-yl, 1-(2-benzoylaminoethyl)pyrrolidin-3-yl, 1-(3-benzoylaminopropyl)pyrrolidin-3-yl, 1-(3-(4-chlorobenzoylamino)propyl)pyrrolidin-3-yl, 1-(3-(4-methylbenzoylmino)propyl)pyrrolidin-3-yl, 1-(3-(4-methoxybenzoylamino)propyl)pyrrolidin-3-yl, 1-(3-(2-thiophenecarbonylmino)propyl)pyrrolidin-3-yl, 1-(4-benzoylaminobutyl)pyrrolidin-3-yl, 1-(2-(3-methylureido)ethyl)pyrrolidin-3-yl, 1-(3-(3-methylureido)propyl)pyrrolidin-3-yl, 1-(4-(3-methylureido)butyl)pyrrolidin-3-yl, 1-(5-(3-methylureido)pentyl)pyrrolidin-3-yl, 1-(2-(3-n-propylureido)ethyl)pyrrolidin-3-yl, 1-(3-(3-n-propylureido)propyl)pyrrolidin-3-yl, 1-(4-(3-n-propylureido)butyl)pyrrolidin-3-yl, 1-(2-(3-phenylureido)ethyl)pyrrolidin-3-yl, 1-(3-(3-phenylureido)propyl)pyrrolidin-3-yl, 1-(4-(3-phenylureido)butyl)pyrrolidin-3-yl, 1-(5-(3-phenylureido)pentyl)pyrrolidin-3-yl, 1-(2-(3-methylthioureido)ethyl)pyrrolidin-3-yl, 1-(3-(3-methylthioureido)propyl)pyrrolidin-3-yl, 1-(4-(3-methylthioureido)butyl)pyrrolidin-3-yl, 1-(2-(3-phenylthioureido)ethyl)pyrrolidin-3-yl, 1-(4-(3-phenylthioureido)butyl)pyrrolidin-3-yl, 1-(4-(2,3-dihydro-1,3-dioxo-1H-isoindol-2-yl)butyl)pyrrolidin-3-yl, 1-(2-(2,3-dihydro-1,3-dioxo-1H-isoindol-2yl)ethyl)pyrrolidin-3-yl, 1-(3-(2,3-dihydro-1,3-dioxo-1H-isoindol-2-yl)propyl)pyrrolidin-3-yl, 1-(5-(2,3-dihydro-1,3-dioxo-1H-isoindol-2-yl)pentyl)pyrrolidin-3-yl, 1-(2-(4-amino-5-chloro-2-methoxybenzoylamino)ethyl)pyrrolidin-3-yl, 1-(3-(4-amino-5-chloro-2-methoxybenzoylamino)propyl)pyrrolidin-3-yl, 1-(3-(4-pyridinecarbonylamino)propyl)pyrrolidin-3-yl, 1-(3-((6-chloro-4-methyl-3,4-dihydro-2H-1,4-benzoxazin-8-yl)carbonylamino)propyl)pyrrolidin-3-yl, 1-(4-((6-chloro-4-methyl-3,4-dihydro-2H-1,4-benzoxazin-8-yl)carbonylamino)butyl)pyrrolidin-3-yl, 1-(2-((1-methyl-1H-indol-3-yl)carbonylamino)ethyl)pyrrolidin-3-yl, 1-(3-((1-methyl-1H-indol-3-yl)carbonylamino)propyl)pyrrolidin-3-yl, 1-(4-((1-methyl-1H-indol-3-yl)carbonylamino)butyl)pyrrolidin-3-yl, 1-(2-methylsulfonylrninoethyl)pyrrolidin-3-yl, 1-(3-(1,1,3-trioxo-2,3-dihydro-1,2-benzisothiazol-2-yl)propyl)pyrrolidin-3-yl, 1-(3-(2,3-dihydro-2-oxobenzimidazol-1-yl)propyl)pyrrolidin-3-yl, (R)-1-(3-benzoylaminopropyl)pyrrolidin-3-yl, (S)-1-(3-benzoylaminopropyl)pyrrolidin-3-yl, (R)-1-(4-(3-n-propylureido)butyl)pyrrolidin-3-yl, (S)-1-(4-(3-n-propylureido)butyl)pyrrolidin-3-yl, (R)-1-(5-(3-n-propylureido)pentyl)pyrrolidin-3-yl, (S)-1-(5-(3-n-propylureido)pentyl)pyrrolidin-3-yl, (R)-1-(6-(3-n-propylureido)hexyl)pyrrolidin-3-yl, (S)-1-(6-(3-n-propylureido)hexyl)pyrrolidin-3-yl, (R)-1-(5-(3-phenylureido)pentyl)pyrrolidin-3-yl, (S)-1-(5-(3-phenylureido)pentyl)pyrrolidin-3-yl, (R)-1-(6-(3-phenylureido)hexyl)pyrrolidin-3-yl, (S)-1-(6-(3-phenylureido)hexyl)pyrrolidin-3-yl, 1-(4-(4-amino-5-chloro-2-methoxybenzoylamino)butyl)pyrrolidin-3-yl, (R)-1-(4-(4-amino-5-chloro-2-methoxybenzoylamino)butyl)pyrrolidin-3-yl, (R)-1-(5-(4-amino-5-chloro-2-methoxybenzoylamino)pentyl)pyrrolidin-3-yl, (R)-1-(4-((1-methyl-1H-indol-3-yl)carbonylamino)butyl)pyrrolidin-3-yl, (S)-1-(4-((1-methyl-1H-indol-3-yl)carbonylamino)butyl)pyrrolidin-3-yl, 1-(5-((1-methyl-1H-indol-3-yl)carbonylamino)pentyl)pyrrolidin-3-yl, (R)-1-(5-((1-methyl-1H-indol-3-yl)carbonylamino)pentyl)pyrrolidin-3-yl, (S)-1-(5-((1-methyl-1H-indol-3-yl)carbonylamino)pentyl)pyrrolidin-3-yl, (R)-1-(6-((1-methyl-1H-indol-3-yl)carbonylamino)hexyl)pyrrolidin-3-yl, (S)-1-(6-((1-methyl-1H-indol-3-yl)carbonylamino)hexyl)pyrrolidin-3-yl, (R)-1-(5-cyclohexanecarbonylaminopentyl)pyrrolidin-3-yl, (R)-1-(4-(1-adamantanecarbonylamino)butyl)pyrrolidin-3-yl, (R)-1-(4-(1-naphthoylamino)butyl)pyrrolidin-3-yl, (R)-1-(4-phenylacetylaminobutyl)pyrrolidin-3-yl, (R)-1-(5-benzoylaminopentyl)pyrrolidin-3-yl, (R)-1-(5-benzenesulfonylaminopentyl)pyrrolidin-3-yl, (R)-1-(4-morpholinocarbonylaminobutyl)pyrrolidin-3-yl, (R)-1-(5-(3-n-butylureido)pentyl)pyrrolidin-3-yl, 1-(3-phenylcarbamoylpropyl)pyrrolidin-3-yl, 1-(3-(4-methylphenylcarbamoyl)propyl)pyrrolidin-3-yl, 1-(3-(3-chlorophenylcarbamoyl)propyl)pyrrolidin-3-yl, (R)-1-(4-((1-methyl-1H-indol-2-yl)carbonylamino)butyl)pyrrolidin-3-yl, (R)-1-(4-((1-isopropyl-1H-indol-3-yl)carbonylamino)butyl)pyrrolidin-3-yl, (R)-1-(4-((1-benzyl-1H-indol-3-yl)carbonylamino)butyl)pyrrolidin-3-yl, 1-(4-(N-benzoyl-N-methylamino)butyl)pyrrolidin-3-yl, (R)-1-(3-(3-phenylpropionylamino)propyl)pyrrolidin-3-yl, (R)-1-(2-(4-phenylbutyrylamino)ethyl)pyrrolidin-3-yl, (R)-1-(4-(2-naphthoylamino)butyl)pyrrolidin-3-yl, (R)-1-(5-(4-chlorobenzoylamino)pentyl)pyrrolidin-3-yl, (R)-1-(5-(3-ethylureido)pentyl)pyrrolidin-3-yl, (R)-1-(5-(3-isopropylureido)pentyl)pyrrolidin-3-yl, 1-(3-(3-chlorobenzoylamino)propyl)pyrrolidin-3-yl, 1-(3-(2-chlorobenzoylamino)propyl)pyrrolidin-3-yl, 1-(3-(4-nitrobenzoylamino)propyl)pyrrolidin-3-yl and the like.

Of these, preferred are (R)-1-(3-benzoylaminopropyl)pyrrolidin-3-yl, (R)-1-(4-(1-naphthoylamino)butyl)pyrrolidin-3-yl, (R)-1-(5-benzoylaminopentyl)pyrrolidin-3-yl, (R)-1-(5-(3-n-propylureido)pentyl)pyrrolidin-3-yl, (R)-1-(5-(3-phenylureido)pentyl)pyrrolidin-3-yl, 1-(5-(2,3-dihydro-1,3-dioxo-1H-isoindol-2-yl)pentyl)pyrrolidin-3-yl,1-(3-phenylcarbamoylpropyl)pyrrolidin-3-yl, (R)-1-(5-(4-amino-5-chloro-2-methoxybenzoylamino)pentyl)pyrrolidin-3-yl, (R)-1-(4-((1-methyl-1H-indol-3-yl)carbonylamino)butyl)pyrrolidin-3-yl, (R)-1-(5-((1-methyl-1H-indol-3-yl)carbonylamino)pentyl)pyrrolidin-3-yl, (R)-1-(4-((1-isopropyl-1H-indol-3-yl)carbonylamino)butyl)pyrrolidin-3-yl, (R)-1-(4-((1-benzyl-1H-indol-3-yl)carbonylamino)butyl)pyrrolidin-3-yl, (R)-1-(2-(4-phenylbutyrylamino)ethyl)pyrrolidin-3-yl, (R)-1-(4-(2-naphthoylamino)butyl)pyrrolidin-3-yl, (R)-1-(5-(4-chlorobenzoylamino)pentyl)pyrrolidin-3-yl, (R)-1-(5-(3-ethylureido)pentyl)pyrrolidin-3-yl, 1-(3-(3-chlorobenzoylamino)propyl)pyrrolidin-3-yl, 1-(3-(2-chlorobenzoylamino)propyl)pyrrolidin-3-yl, 1-(3-(4-nitrobenzoylamino)propyl)pyrrolidin-3-yl, 1-(3-(4-chlorobenzoylamino)propyl)pyrrolidin-3-yl, 1-(3-(4-methylbenzoylamino)propyl)pyrrolidin-3-yl and 1-(3-(2-thiophenecarbonylamino)propyl)pyrrolidin-3-yl. A group of the formula (II-b) wherein the absolute configuration at 3-position of pyrrolidine is R-configuration is more preferable.

Examples of the cyclic amine of the formula (IId) include, for example, 1-(4-((1-methyl-1H-indol-3-yl)carbonylamino)butyl)piperidin-4-yl, 1-(4-(1-naphthoylamino)butyl)piperidin-4-yl, 1-(4-(2-naphthoylamino)butyl)piperidin-4-yl, 1-(4-(3-n-propylureido)butyl)piperidin-4-yl, 1-(4-benzoylaminobutyl)piperidin-4-yl, 1-(4-(3-n-propylureido)butyl)piperidin-4-yl, 1-(5-(3-n-propylureido)pentyl)piperidin-4-yl, 1-(5-benzoylaminopentyl)piperidin-4-yl, 1-(5-(3-chlorobenzoylamino)pentyl)piperidin-4-yl, 1-(5-(4-methylbenzoylamino)pentyl)piperidin-4-yl, 1-(5-(3-phenylureido)pentyl)piperidin-4-yl, 1-(5-(3-(4-chlorophenyl)ureido)pentyl)piperidin-4-yl, 1-(5-((1-methyl-1H-indol-3-yl)carbonylamino)pentyl)piperidin-4-yl, 1-(3-(2,3-dihydro-1,3-dioxo-1H-isoindol-2-yl)propyl)piperidin-4-yl, 1-(5-(2,3-dihydro-1,3-dioxo-1H-isoindol-2-yl)pentyl)piperidin-4-yl, 1-(6-(2,3-dihydro-1,3-dioxo-1H-isoindol-2-yl)hexyl)piperidin-4-yl, 1-(3-benzoylaminopropyl)piperidin-4-yl, 1-(4-benzenesulfonylaminobutyl)piperidin-4-yl, 1-(5-((1-methyl-1H-indazol-3-yl)carbonylamino)pentyl)piperidin-4-yl, 1-(5-(2-chlorobenzoyl)aminopentyl)piperidin-4-yl, 1-(5-(4-chlorobenzoyl)aminopentyl)piperidin-4-yl, 1-(5-benzenesulfonylaminopentyl)piperidin-4-yl, 1-(6-((1-methyl-1H-indol-3-yl)carbonylamino)hexyl)piperidin-4-yl, 1-(6-benzoylaminohexyl)piperidin-4-yl and 1-(6-phenylureido)hexyl)piperidin-4-yl.

Of these, preferred are 1-(4-(1-naphthoylamino)butyl)piperidin-4-yl, 1-(4-(2-naphthoylamino)butyl)piperidin-4-yl, 1-(5-benzoylaminopentyl)piperidin-4-yl, 1-(5-(3-chlorobenzoylamino)pentyl)piperidin-4-yl, 1-(5-(4-methylbenzoylamino)pentyl)piperidin-4-yl, 1-(4-(3-n-propylureido)butyl)piperidin-4-yl, 1-(5-(3-n-propylureido)pentyl)piperidin-4-yl, 1-(5-(3-(4-chlorophenyl)ureido)pentyl)piperidin-4-yl, 1-(4-((1-methyl-1H-indol-3-yl)carbonylamino)butyl)piperidin-4-yl and 1-(5-((1-methyl-1H-indol-3-yl)carbonylamino)pentyl)piperidin-4-yl.

The pharmaceutically acceptable salts of the compound of the present invention are, for example, acid addition salt and quaternary ammonium salt. Examples of the acid addition salt include, for example, hydrochloride, sulfate, hydrobromide, phosphate, nitrate, methanesulfonate, ethanesulfonate, fumarate, maleate, benzoate, citrate, malate, mandelate, p-toluenesulfonate, acetate, succinate, malonate, lactate, salicylate, gallate, picrate, carbonate, accorbate, trifluoroacetate and tartrate. Examples of quaternary ammonium salt include, for example, quaternary ammonium salts with lower alkyl halide (e.g., methyl iodide, methyl bromide, ethyl iodide, ethyl bromide and the like), lower alkylsulfonate (e.g., methyl methanesulfonate, ethyl methanesulfonate and the like), and lower alkyl arylsulfonate (e.g., methyl p-toluenesulfonate and the like). The N-oxide derivative at substituent A of the compound of the formula (I) is also encompassed in the compound of the present invention. The compound of the present invention may be hydrates (e.g., monohydrate, 1/2 hydrate and 3/2 hydrate) or solvates. For crystallization of compound, oxalic acid may be used.

When the compound of the present invention and pharmaceutically acceptable salts thereof have asymmetric carbon, the compound can be present as optical isomers. The present invention also encompasses such optical isomers and mixtures thereof. In addition, geometric isomers of cis-compounds and trans-compounds, as well as mixtures thereof are also encompasseed in the present invention.

The compound of the present invention can be produced by the following methods.
Method 1 : The compound of the formula (I) can be synthesized by the following route. 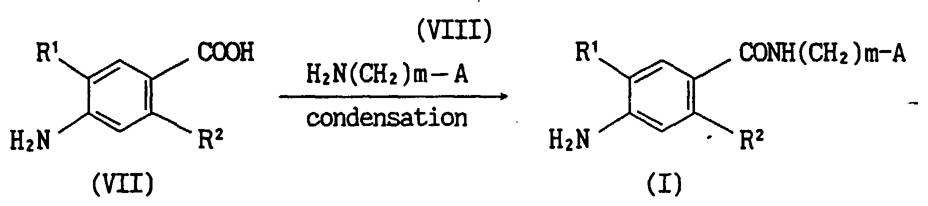 wherein R¹, R², m and A are as defined above.
   That is, the compound is produced by reacting carboxylic acid of compound (VII) or reactive derivative thereof with compound (VIII) in a suitable solvent.
   When compound (VII) is a free carboxylic acid, the reaction is carried out using a routine condensing agent. Examples of the condensing agent include dicyclohexylcarbodiimide, 1-ethyl-3-(3-dimethylaminopropyl)carbodiimide hydrochloride, carbonyldiimidazole, benzotriazol-1-yloxytris(dimethylamino)phosphonium hexafluorophosphate, N-methyl-2-chloropyridinium iodide and the like. The instant reaction is preferably carried out by condensing in the presence of an organic base such as 1-hydroxybenzotriazole and N-methylmorpholine. The solvent to be used may be, for example, dimethylformamide, dimethyl sulfoxide, methylene chloride, tetrahydrofuran, acetonitrile, dioxane, benzene and toluene. While the reaction temperature varies depending on the kind of solvent to be used, it is generally from -20°C to 50°C, and the reaction time which varies depending on the kind of reaction temperature is generally 1-24 hr.
   When compound (VII) is reactive derivative of carboxylic acid such as acid halide, acid anhydride, mixed acid anhydride and ester, the reaction is carried out in an inert solvent in the presence of a base as necessary. The base to be used as necessary is, for example, sodium carbonate, potassium carbonate, sodium hydrogencarbonate, potassium hydrogencarbonate, sodium hydroxide, potassium hydroxide, triethylamine, diisopropylethylamine or pyridine. Examples of the inert solvent to be used include, for example, methylene chloride, chloroform, ethyl acetate, diethyl ether, tetrahydrofuran, dioxane, acetonitrile, benzene, toluene and mixed solvent thereof. While the reaction temperature varies depending on the kind of solvent to be used, it is generally from -30°C to 50°C, and the reaction time which varies depending on reaction temperature is generally 1-24 hr.
Method 2 : A compound (VIII) wherein A is a group of the formula (II-a) - (II-f), B is a group of the formula -N(R⁴)-X¹-R⁵ (B-1) and R⁴ is hydrogen can be synthesized by the following route. 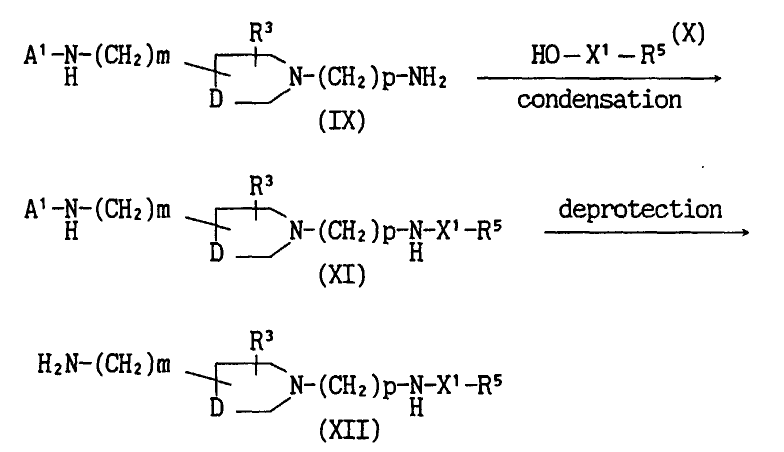 wherein A¹ is an urethane type amino-protecting group such as tert-butoxycarbonyl, 1,1-dimethylethoxycarbonyl and isopropyloxycarbonyl, D is -(CH₂)ₙ- wherein n is an integer of 0-3, and R³, R⁵, m, p and X¹ are as defined above.
   That is, the compound is produced by condensing compound (IX) with compound (X) or reactive derivative thereof in a suitable solvent and then deprotecting the obtained compound (XI).
   The reaction of compound (IX) and compound (X) can be carried out according to the condensation shown in Method 1.
   The compound (XI) can be deprotected by a method generally used for deprotection of amino-protecting group. For example, when A¹ is tert-butoxycarbonyl, deprotection is performed by treating with an acid in a suitable solvent as necessary. The acid to be used for this reaction may be any as long as it does not hydrolyze amide bond, and is exemplified by, for example, hydrogen chloride, sulfuric acid, trifluoroacetic acid and trifluoromethanesulfonic acid. The solvent to be used as necessary may be, for example, methanol, ethanol, propanol, isopropyl alcohol, tetrahydrofuran, acetone, ethyl acetate, methylene chloride, chloroform, dioxane, water and mixed solvents thereof. The reaction temperature is from under ice-cooling to the refluxing temperature of the solvent, preferably at room temperature, and reaction time is from 30 min to 2 hr.
Method 3 : A compound (VIII) wherein A is a group of the formula (II-a) - (II-f), B is a group of the formula -N(R⁴)-X¹-R⁵ (B-1) and R⁴ is lower alkyl, aralkyl or substituted aralkyl can be synthesized by the following route. 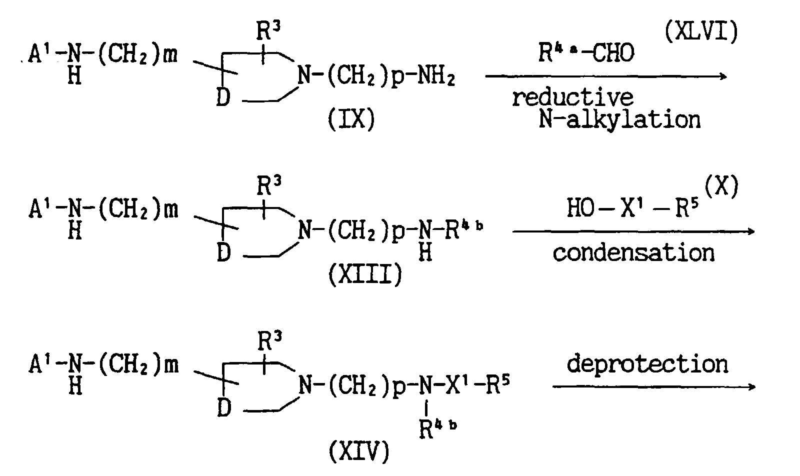 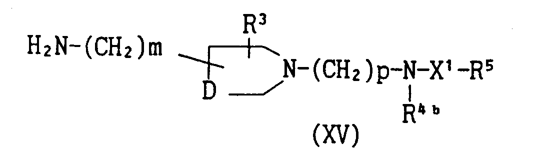 wherein R^{4a} is hydrogen, alkyl having 1 to 5 carbon atoms, aralkyl or substituted aralkyl, R^{4b} is lower alkyl, aralkyl or substituted aralkyl, and A¹, D, R³, R⁵, m, p and X¹ are as defined above.
   That is, the compound (XV) is produced by subjecting compound (IX) and compound (XLVI) to reductive N-alkylation in a suitable solvent to give compound (XIII), reacting the resultant compound with compound (X) according to Method 2 to give compound (XIV) and deprotection.
   The solvent to be used for the reductive N-alkylation includes, for example, methanol, ethanol, propanol, ethyl acetate, tetrahydrofuran, dioxane, dimethylformamide and acetic acid. The reductive N-alkylating agent to be used may be, for example, sodium cyanoborohydride, sodium borohydride, formic acid or sodium formate. While the reaction temperature varies depending on the kind of solvent to be used, it is generally from 0°C to 30°C, and the reaction time which varies depending on the kind of reaction temperature is generally 1-24 hr.
Method 4 : A compound (VIII) wherein A is a group of the formula (II-a) - (II-f), B is a group of the formula -N(R⁴)-X¹-R⁵ (B-1) and R⁴ is lower alkyl, phenyl, substituted phenyl, aralkyl or substituted aralkyl can be synthesized by the following route. 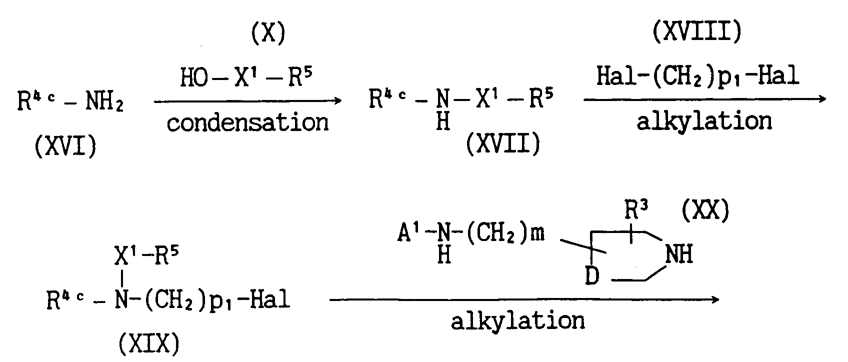 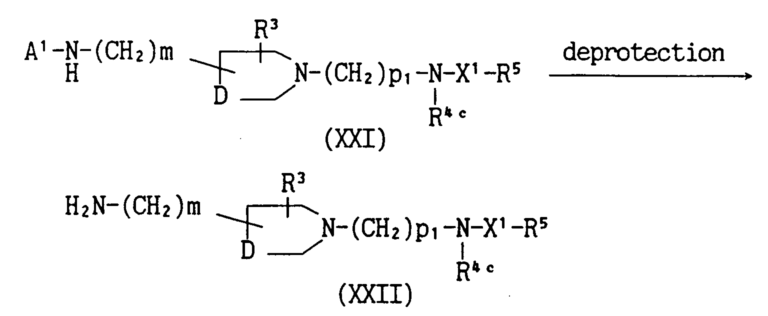 wherein R^{4c} is lower alkyl, phenyl, substituted phenyl, aralkyl or substituted aralkyl, Hal is halogen such as chlorine, bromine, iodine and the like, p₁ is an integer of 2-5, and A¹, D, R³, R⁵, m and X¹ are as defined above.
   That is, the compound (XXII) is produced by subjecting compound (XVI) and compound (X) to condensation of Method 1 to give compound (XVII), reacting the resulting compound with compound (XVIII) in a suitable solvent in the presence of a base to give compound (XIX), subjecting the resulting compound to alkylation with compound (XX) in a suitable solvent in the presence of a base to give compound (XXI) and deprotection according to Method 2.
   The solvent to be used for the reaction of compound (XVII) and compound (XVIII) includes, for example, methanol, ethanol, propanol, ethyl acetate, tetrahydrofuran, dioxane, dimethylformamide, dimethyl sulfoxide and toluene. The base to be used may be, for example, sodium carbonate, potassium carbonate, sodium hydrogencarbonate, potassium hydrogencarbonate, sodium hydroxide, potassium hydroxide, sodium hydride, potassium hydride, triethylamine, diisopropylethylamine or pyridine. While the reaction temperature varies depending on the kind of solvent to be used, it is generally from 0°C to 80°C, and the reaction time which varies depending on the kind of reaction temperature is generally 1-24 hr.
   The solvent to be used for the reaction of compound (XIX) and compound (XX) includes, for example, methanol, ethanol, propanol, ethyl acetate, tetrahydrofuran, dioxane, dimethylformamide, dimethyl sulfoxide, toluene and mixed solvents thereof. The base to be used may be, for example, sodium carbonate, potassium carbonate, sodium hydrogencarbonate, potassium hydrogencarbonate, sodium hydroxide, potassium hydroxide, triethylamine, diisopropylethylamine or pyridine. While the reaction temperature varies depending on the kind of solvent to be used, it is generally from 0°C to 80°C, and the reaction time which varies depending on the kind of reaction temperature is generally 1-24 hr.
Method 5 : A compound (VIII) wherein A is a group of the formula (II-a) - (II-f), B is a group of the formula -N(R⁴)-X²-N(R⁶)(R⁷) (B-2) and R⁷ is hydrogen can be synthesized by the following route. 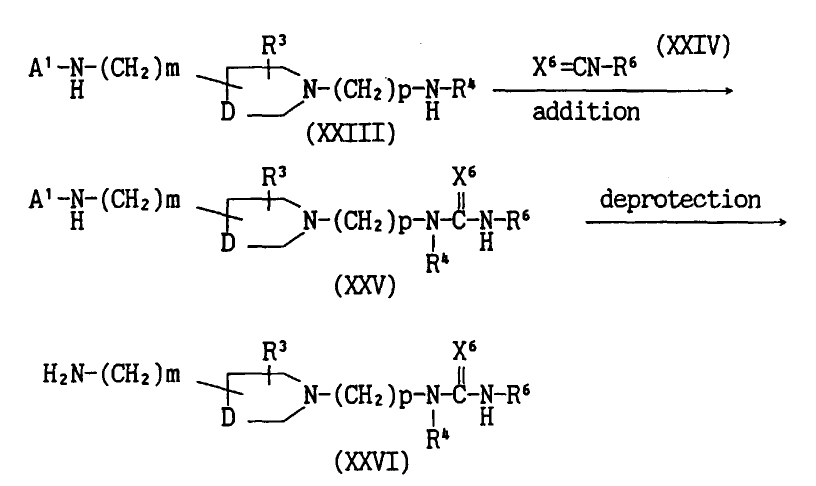 wherein X⁶ is oxygen atom or sulfur atom, and A¹, D, R³, R⁴, R⁶, m and p are as defined above.
   That is, the compound (XXVI) is produced by reacting compound (XXIII) and compound (XXIV) in a suitable solvent to give compound (XXV), and deprotection according to Method 2.
   The solvent to be used for the reaction of compound (XXIII) and compound (XXIV) includes, for example, methylene chloride, chloroform, ethyl acetate, tetrahydrofuran, dioxane, benzene and toluene. The reaction temperature is generally from -30°C to 40°C, and the reaction time is generally 10 min - 5 hr.
Method 6 : A compound (VIII) wherein A is a group of the formula (II-a) - (II-f), and B is a group of the formula -N(R⁴)-X²-N(R⁶)(R⁷) (B-2) can be synthesized by the following route. 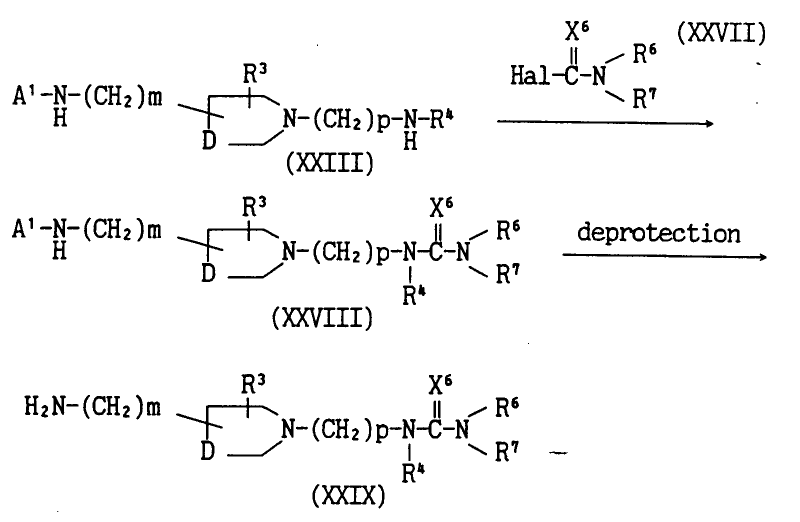 wherein A¹, D, R³, R⁴, R⁶, R⁷, m, p, X⁶ and Hal are as defined above.
   That is, the compound (XXIX) is produced by reacting compound (XXIII) and compound (XXVII) in a suitable solvent in the presence of a base to give compound (XXVIII), and deprotection according to Method 2.
   The solvent to be used for the reaction of compound (XXIII) and compound (XXVII) includes, for example, dimethylformamide, dimethyl sulfoxide, methylene chloride, chloroform, ethyl acetate, tetrahydrofuran, dioxane, benzene and toluene. The base to be used may be, for example, sodium carbonate, potassium carbonate, sodium hydrogencarbonate, potassium hydrogencarbonate, sodium hydroxide, potassium hydroxide, triethylamine, diisopropylethylamine or pyridine. The reaction temperature is generally from -30°C to 40°C, and the reaction time is generally 10 min - 5 hr.
Method 7 : The compound (IX) and compound (XX) which are intermediates shown in Method 2 - Method 4 can be synthesized by the following route. 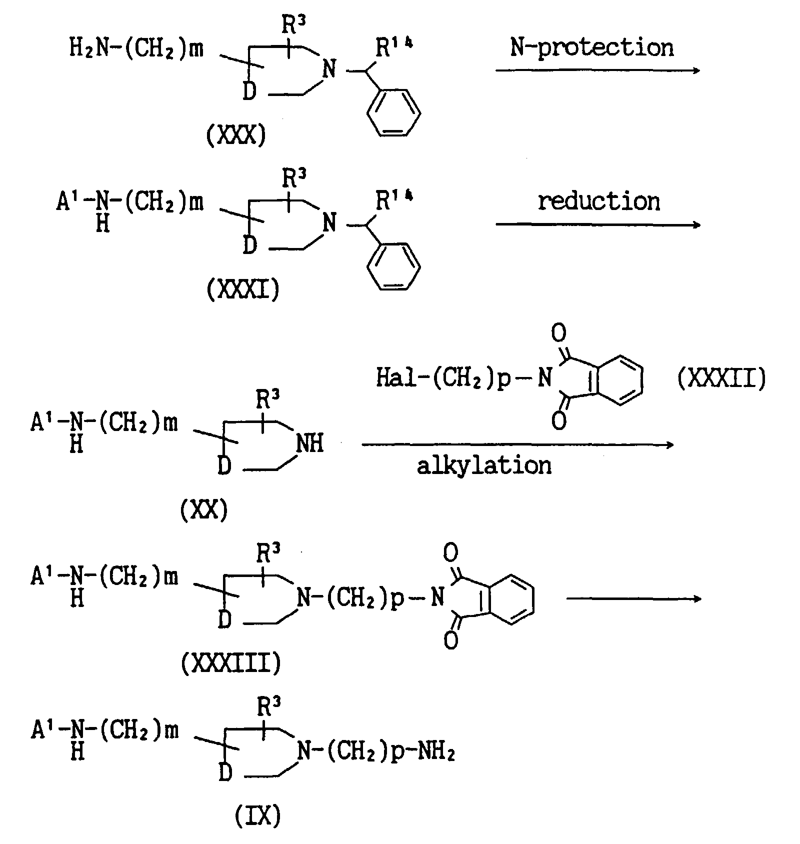 wherein R¹⁴ is hyrogen or lower alkyl, and A¹, D, R³, m, p and Hal are as defined above.
   That is, the compound (XX) is produced by reacting a starting compound (XXX) with an amino-protecting reagent in a suitable solvent to give compound (XXXI), and subjecting the resulting compound to reduction in a suitable solvent in the presence of a catalyst under a hydrogen atmosphere or using a suitable hydrogen source. Then, compound (XX) is subjected to alkylation with compound (XXXII) in a suitable solvent in the presence of a base to give compound (XXXIII), and the resulting compound is reacted in a suitable solvent in the presence of a base to give compound (IX).
   The amino-protecting reagent to be used for the reaction with compound (XXX) includes, for example, di-tert-butyl dicarbonate and 2-tert-butoxycarbonyloxyimino-2-phenylacetonitrile. The solvent to be used includes, for example, tetrahydrofuran, acetone, ethyl acetate, methylene chloride, chloroform, dioxane, water and mixed solvents thereof. While the reaction temperature varies depending on the kind of solvent to be used, it is generally from -20°C to 50°C, and the reaction time which varies depending on the kind of reaction temperature is generally 1-24 hr.
   The solvent to be used for reduction of compound (XXXI) includes, for example, methanol, ethanol, propanol, isopropyl alcohol, butanol, formic acid, acetic acid, water, tetrahydrofuran, dimethyl sulfoxide and mixed solvents thereof. The catalyst to be used includes, for example, palladium-carbon, Raney-nickel and platinum oxide. The hydrogen source to be used includes, for example, hydrazine hydrate, cyclohexene, 1,4-cyclohexadiene, formic acid and ammonium formate. While the reaction temperature varies depending on the kind of solvent to be used, it is generaly from 0°C to the boiling point of the solvent to be used, and the reaction time which varies depending on the kind of reaction temperature is generally 1-6 hr.
   The solvent to be used for alkylation of compound (XX) includes, for example, methylene chloride, 1,2-dichloroethane, chloroform, methanol, ethanol, propanol, isopropyl alcohol, butanol, tetrahydrofuran, diethyl ether, dioxane, dimethylformamide, dimethyl sulfoxide, benzene, toluene, xylene and mixed solvents thereof. The base to be used may be, for example, sodium carbonate, potassium carbonate, sodium hydrogencarbonate, potassium hydrogencarbonate, sodium hydroxide, potassium hydroxide, triethylamine, diisopropylethylamine or pyridine. While the reaction temperature varies depending on the kind of solvent to be used, it is generally from 0°C to 140°C, and the reaction time which varies depending on the kind of reaction temperature is generally 1-24 hr.
   The solvent to be used for reaction of compound (XXXIII) includes, for example, methanol, ethanol, propanol, isopropyl alcohol and butanol. The base to be used may be, for example, hydrazine hydrate, methylhydrazine, phenylhydrazine and methylamine. While the reaction temperature varies depending on the kind of solvent to be used, it is generally from 50°C to the boiling point of the solvent to be used, and the reaction time which varies depending on the kind of reaction temperature is generally 1-10 hr.
Method 8 : The compound (XXXIII) wherein p is 2-5 can be synthesized by the following route. 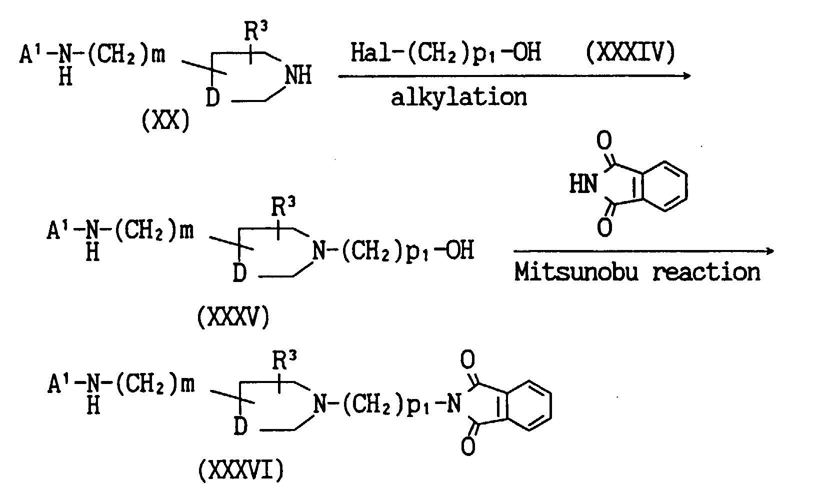 wherein A¹, D, R³, m, p₁ and Hal are as defined above.
   That is, the compound (XX) is subjected to alkylation with compound (XXXIV) in a suitable solvent in the presence of a base to give compound (XXXV) and Mitsunobu reaction [Synthesis, p. 1 (1988)] with phthalimide in a suitable solvent to produce compound (XXXVI).
   The solvent to be used for alkylation includes, for example, methylene chloride, 1,2-dichloroethane, chloroform, methanol, ethanol, propanol, isopropyl alcohol, butanol, tetrahydrofuran, diethyl ether, dioxane, dimethylformamide, dimethyl sulfoxide, benzene, toluene and xylene. The base to be used may be, for example, sodium carbonate, potassium carbonate, sodium hydrogencarbonate, potassium hydrogencarbonate, sodium hydroxide, potassium hydroxide, triethylamine, diisopropylethylamine or pyridine. While the reaction temperature varies depending on the kind of solvent to be used, it is generally from 0°C to 140°C, and the reaction time which varies depending on the kind of reaction temperature is generally 1-24 hr.
   The solvent to be used for Mitsunobu reaction includes, for example, tetrahydrofuran and dioxane. The reaction temperature is generally 0 - 40°C, and the reaction time which varies depending on the kind of reaction temperature is generally 1-24 hr.
Method 9 : A compound (VIII) wherein A is a group of the formula (II-a) - (II-f), and B is a group of the formula -X¹-N(R⁶)(R⁷) (B-3) can be synthesis by the following route. 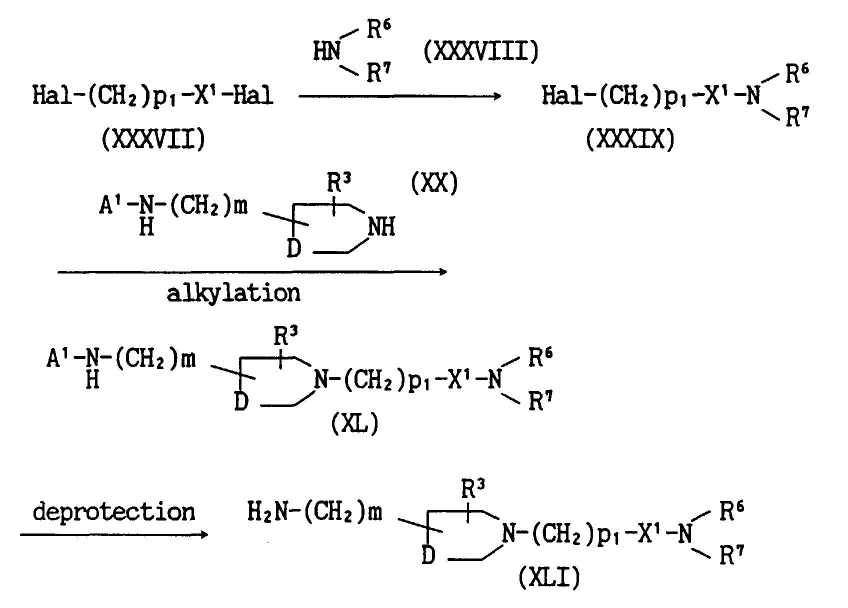 wherein A¹, D, R³, R⁶, R⁷, m, p₁, X¹ and Hal are as defined above.
   That is, the compound (XLI) is produced by reacting compound (XXXVII) and compound (XXXVIII) in a suitable solvent in the presence of a base to give compound (XXXIX), and subjecting the resulting compound to alkylation with compound (XX) in a suitable solvent in the presence of a base to give compound (XL), followed by deprotection according to Method 2.
   The solvent to be used for the reaction of compound (XXXVII) and compound (XXXVIII) includes, for example, methanol, ethanol, propanol, ethyl acetate, tetrahydrofuran, dioxane, dimethylformamide, dimethyl sulfoxide, acetone, methyl ethyl ketone, methylene chloride, chloroform, benzene, toluene and xylene. The base to be used may be, for example, sodium carbonate, potassium carbonate, sodium hydrogencarbonate, potassium hydrogencarbonate, sodium hydroxide, potassium hydroxide, triethylamine, diisopropylethylamine or pyridine. While the reaction temperature varies depending on the kind of solvent to be used, it is generally from -20°C to 30°C, and the reaction time which varies depending on the kind of reaction temperature is generally 20 min - 5 hr.
   The solvent to be used for alkylation of compound (XX) includes, for example, methylene chloride, 1,2-dichloroethane, chloroform, methanol, ethanol, propanol, isopropyl alcohol, butanol, tetrahydrofuran, diethyl ether, dioxane, dimethylformamide, dimethyl sulfoxide, benzene, toluene, xylene and mixed solvents thereof. The base to be used may be, for example, sodium carbonate, potassium carbonate, sodium hydrogencarbonate, potassium hydrogencarbonate, sodium hydroxide, potassium hydroxide, triethylamine, diisopropylethylamine or pyridine. While the reaction temperature varies depending on the kind of solvent to be used, it is generally from 0°C to 140°C, and the reaction time which varies depending on the kind of reaction temperature is generally 1-24 hr.
Method 10: A compound (VIII) wherein A is a group of the formula (II-a) - (II-f), and B is a group of the formula -Het (B-4) can be synthesis by the following route. 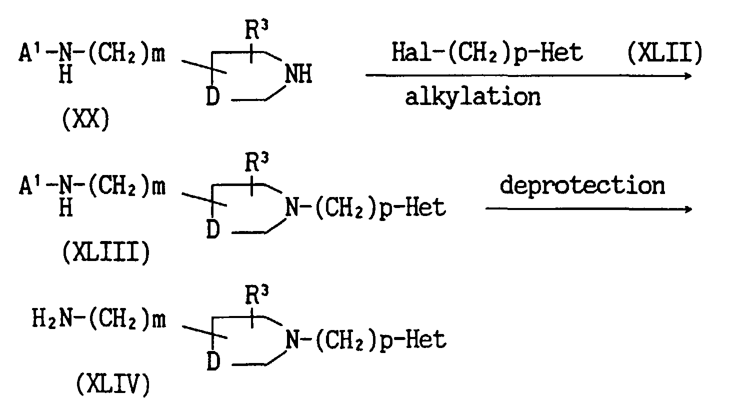 wherein A¹, D, R³, m, p, Hal and Het are as defined above.
   That is, the compound (XLIV) is produced by subjecting the compound (XX) to alkylation with compound (XLII) in a suitable solvent in the presence of a base to give compound (XLIII) and deprotection according to Method 2.
   The solvent to be used for the alkylation includes, for example, methanol, ethanol, propanol, ethyl acetate, tetrahydrofuran, dioxane, dimethylformamide, dimethyl sulfoxide, acetone, methyl ethyl ketone, methylene chloride, chloroform, benzene, toluene, xylene and mixed solvents thereof. The base to be used may be, for example, sodium carbonate, potassium carbonate, sodium hydrogencarbonate, potassium hydrogencarbonate, sodium hydroxide, potassium hydroxide, triethylamine, diisopropylethylamine or pyridine. While the reaction temperature varies depending on the kind of solvent to be used, it is generally from 0°C to 140°C, and the reaction time which varies depending on the kind of reaction temperature is generally 1-24 hr.
Method 11: A compound (VIII) wherein A is a group of the formula (III) can be synthesized in the same manner as in the synthesis of a compound wherein A is expressed by the formula (II-a) - (II-f), by the use of a compound of the formula 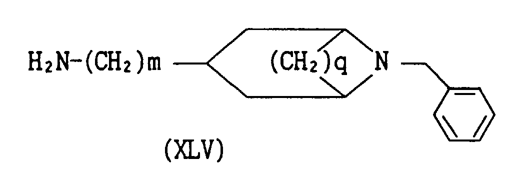 wherein m and q are as defined above, as a starting material.
Method 12: The compound of the formula (I) can be also synthesized by tee following route. 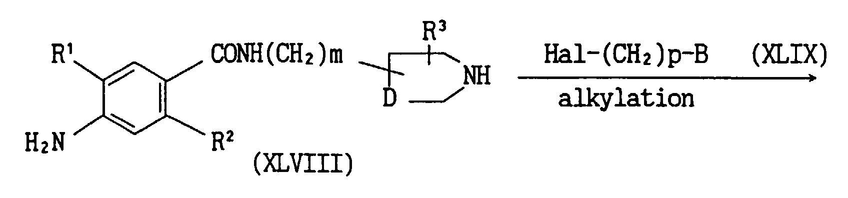 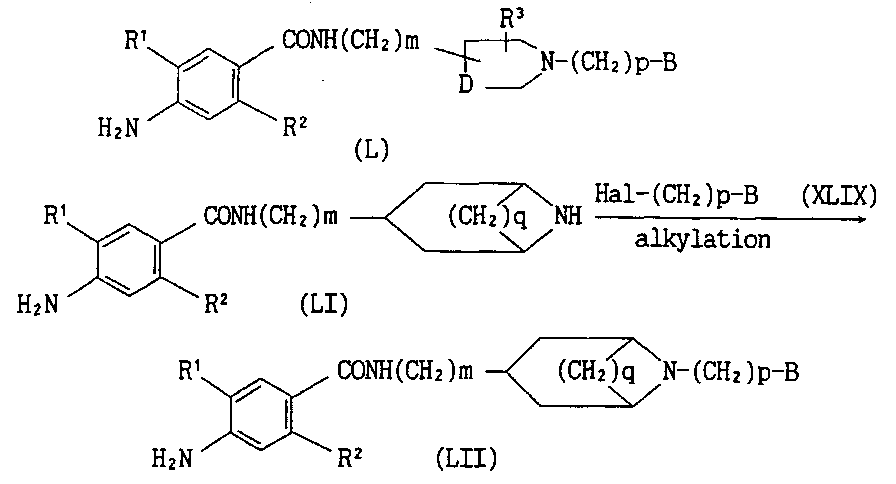 wherein D, R¹, R², R³, m, p, q, B and Hal are as defined above.
   That is, the compound is produced by reacting intermediates of compound (XLVIII) and compound (LI) with compound (XLIX) in a suitable solvent in the presence of a base.
   The solvent to be used for the alkylation includes, for example, methylene chloride, 1,2-dichloroethane, chloroform, methanol, ethanol, propanol, isopropyl alcohol, butanol, tetrahydrofuran, diethyl ether, dioxane, dimethylformamide, dimethyl sulfoxide, benzene, toluene and xylene. The base to be used may be, for example, sodium carbonate, potassium carbonate, sodium hydrogencarbonate, potassium hydrogencarbonate, sodium hydroxide, potassium hydroxide, triethylamine, diisopropylethylamine or pyridine. While the reaction temperature varies depending on the kind of solvent to be used, it is generally from 0°C to 140°C, and the reaction time which varies depending on the kind of reaction temperature is generally 1-24 hr.
   The intermediates of compound (XLVIII) and compound (LI) can be produced according to Japanese Patent Unexamined Publication Nos. 211685/1992, 262724/1993 and others.
Method 13: The compound of the formula (I) can be also synthesized by the following route. 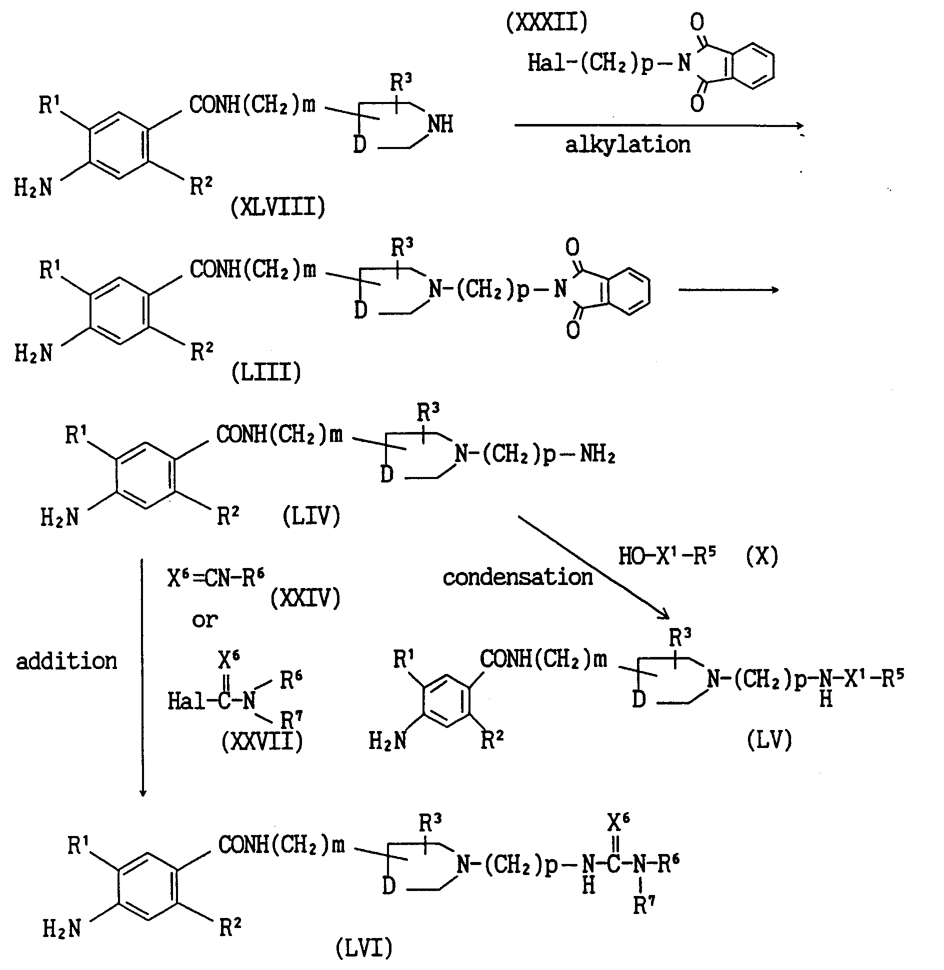 wherein D, R¹, R², R³, R⁵, R⁶, R⁷, X¹, X⁶, m, p and Hal are as defined above.
   That is, compound (XLVIII) is subjected to alkylation with compound (XXXII) in a suitable solvent in the presence of a base to give compound (LIII), and a reaction of the obtained compound in a suitable solvent in the presence of a base gives compound (LIV). Then, the compound is reacted with compound (X), compound (XXIV), compound (XXVII) and the like to give compound (LV) or compound (LVI).
   The solvent to be used for the alkylation of compound (XLVIII) includes, for example, methylene chloride, 1,2-dichloroethane, chloroform, methanol, ethanol, propanol, isopropyl alcohol, butanol, tetrahydrofuran, diethyl ether, dioxane, dimethylformamide, dimethyl sulfoxide, benzene, toluene, xylene and mixed solvents thereof. The base to be used may be, for example, sodium carbonate, potassium carbonate, sodium hydrogencarbonate, potassium hydrogencarbonate, sodium hydroxide, potassium hydroxide, triethylamine, diisopropylethylamine or pyridine. While the reaction temperature varies depending on the kind of solvent to be used, it is generally from 0°C to 140°C, and the reaction time which varies depending on the kind of reaction temperature is generally 1-24 hr.
   The solvent to be used for the reaction from compound (LIII) to compound (LIV) includes, for example, methanol, ethanol, propanol, isopropyl alcohol and butanol. The base to be used may be, for example, hydrazine hydrate, methylhydrazine, phenylhydrazine and methylamine. While the reaction temperature varies depending on the kind of solvent to be used, it is generally from 50°C to the boiling point of the solvent to be used, and the reaction time which varies depending on the kind of reaction temperature is generally 1-10 hr.
   The reaction with compound (X) is carried out according to Method 2, and the reaction with compound (XXIV) or compound (XXVII) is carried out according to Method 5 or Method 6, whereby compound (LV) or compound (LVI) can be produced, respectively.
Method 14: The compound of the formula (I) can be also synthesized by the following route. 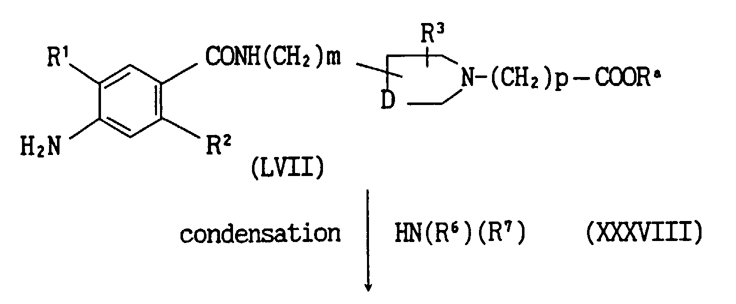 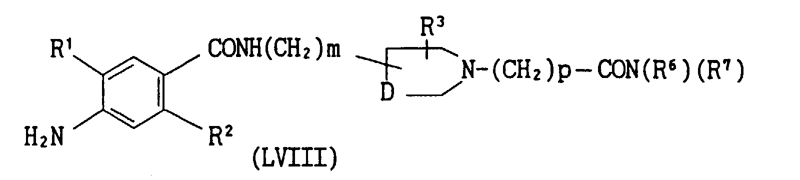 wherein R^{a} is hydrogen or lower alkyl, and D, R¹, R², R³, R⁶, R⁷, m and p are as defined above.
   That is, the compound can be produced by reacting carboxylic acid represented by compound (LVII) or a reactive derivative thereof with compound (XXXVIII) in a suitable solvent.
   When compound (LVII) is a free carboxylic acid, the reaction is carried out using a routine condensing agent. Examples of the condensing agent include dicyclohexylcarbodiimide, 1-ethyl-3-(3-dimethylaminopropyl)carbodiimide hydrochloride, carbonyldiimidazole, benzotriazol-1-yloxytris(dimethylamino)phosphonium hexafluorophosphate, N-methyl-2-chloropyridinium iodide and the like. The instant reaction is preferably carried out by condensing in the presence of an organic base such as 1-hydroxybenzotriazole and N-methylmorpholine. The solvent to be used may be, for example, dimethylformamide, dimethyl sulfoxide, methylene chloride, tetrahydrofuran, acetenitrile, dioxane or benzene. While the reaction temperature varies depending on the kind of solvent to be used, it is generally from -20°C to 50°C, and the reaction time which varies depending on the kind of reaction temperature is generally 1-24 hr.
   When compound (LVII) is reactive derivative of carboxylic acid such as acid halide, acid anhydride, mixed acid anhydride and ester, the reaction is carried out in an inert solvent in the presence of a base as necessary. The base to be used as necessary is, for example, sodium carbonate, potassium carbonate, sodium hydrogencarbonate, potassium hydrogencarbonate, sodium hydroxide, potassium hydroxide, triethylamine, diisopropylethylamine or pyridine. Examples of the inert solvent to be used include, for example, methylene chloride, chloroform, ethyl acetate, diethyl ether, tetrahydrofuran, dioxane, acetonitrile, benzene, toluene and mixed solvent thereof. While the reaction temperature varies depending on the kind of solvent to be used, it is generally from -30°C to 50°C, and the reaction time which varies depending on the kind of reaction temperature is generally 1-24 hr.
   The compound (LVII) can be produced according to Japanese Patent Unexamined Publication No. 262724/1993.
Method 15: A quaternary ammonium salt of compound of the formula (I) can be synthesized by the following route. 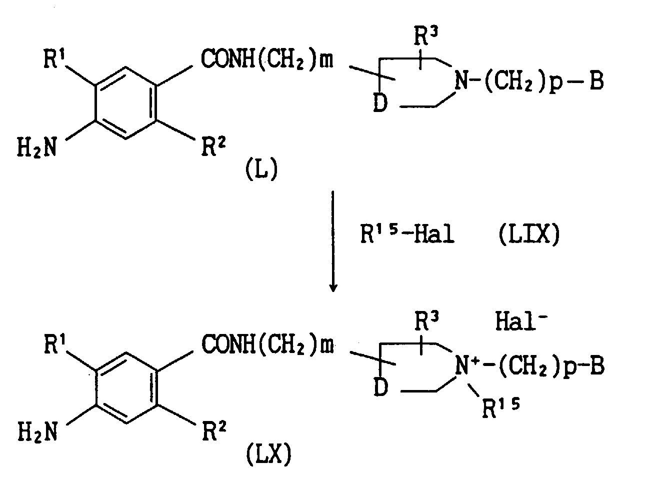 wherein R¹⁵ is lower alkyl, and D, R¹, R², R³, m, p, B and Hal are as defined above.
   That is, the salt can be produced, for example, by reacting compound (L) and compound (LIX) in a suitable solvent.
   The solvent to be used includes, for example, methylene chloride, 1,2-dichloroethane, chloroform, methanol, ethanol, propanol, isopropyl alcohol, butanol, tetrahydrofuran, diethyl ether, dioxane, benzene, toluene and xylene. While the reaction temperature varies depending on the kind of solvent to be used, it is generally 0 - 40°C, and the reaction time which varies depending on the kind of reaction temperature is generally 1-24 hr.
   The compound of the formula (I) thus obtained can be separated and purified from reaction mixture by a method known *per se*, such as recrystallization, column chromatography, and the like.
Method 16: The compound (VII) can be produced according to the method described in Journal of Medicinal Chemistry, vol. 34, pp. 616-624 (1991). 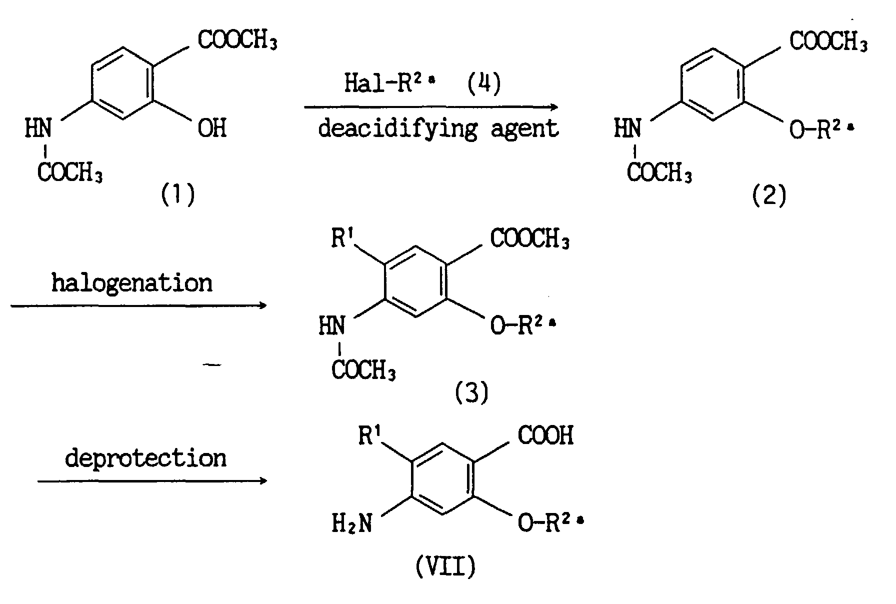 wherein R^{2a} is lower alkyl, substituted lower alkyl, cycloalkyl or cycloalkylalkyl, and R¹ and Hal are as defined above.
   That is, compound (1) is reacted with compound (4) to give compound (2). This compound is halogenated to give compound (3), which is subjected to deprotection to give compound (VII).
   The deacidifying agent to be used for the reaction of compound (1) and compound (4) is exemplified by potassium carbonate, sodium carbonate and the like. The solvent to be used for this reaction may be, for example, dimethylformamide or dimethyl sulfoxide. The reaction temperature is from room temperature to 100°C, and the reaction time is 2-10 hr.
   Examples of halogenating agent to be used for halogenation of compound (2) include, for example, N-bromosuccinimide and N-chlorosuccinimide. The solvent to be used for the instant reaction includes, for example, dimethylformamide, dimethyl sulfoxide, toluene and acetonitrile. The reaction temperature is from room temperature to 100°C, and the reaction time is 1-10 hr.
   The reagent to be used for deprotection of compound (3) may be, for example, aqueous sodium hydroxide solution and aqueous potassium hydroxide solution. This reaction may be carried out in a solvent such as methanol, ethanol, isopropyl alcohol and the like. The reaction temperature is the refluxing temperature of solvent to be used, and the reaction time is 3-10 hr.
   When the compound of the formula (I) of the present invention and pharmaceutically acceptable salts thereof have asymmetric carbon, they are generally produced as racemates which can be optically resolved into optical isomers by a conventional method such as preparative recrystallization and chromatography. Also, the use of optically active starting compound results in optical isomers. When compound has two or more asymmetric carbons, they can be obtained as individual diastereomers or mixtures thereof which can be separated by a conventional method such as preparative recrystallization and chromatography.
   When the cyclic amine of the formula (II) of the compound of the present invention is that of the formula (II-b), its optical isomer(s) can be produced, for example, from optically active carboxylate obtained by the method described in Journal of Medicinal Chemistry, vol. 33, pp. 71-77 (1990) and the like, or by the route shown in the following Method 17.
Method 17 : A compound (VIII) wherein m is 1, A is a group of the formula (II-b) and the absolute configuration at the 3-position of pyrrolidine is R-configuration can be synthesized by the following route. 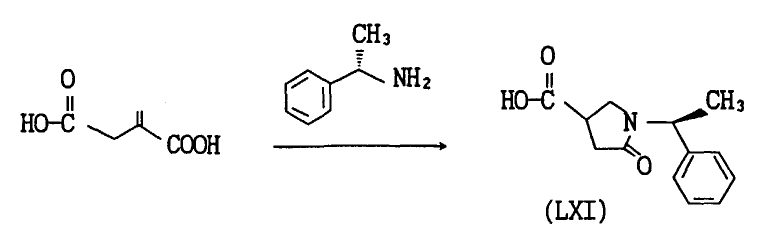 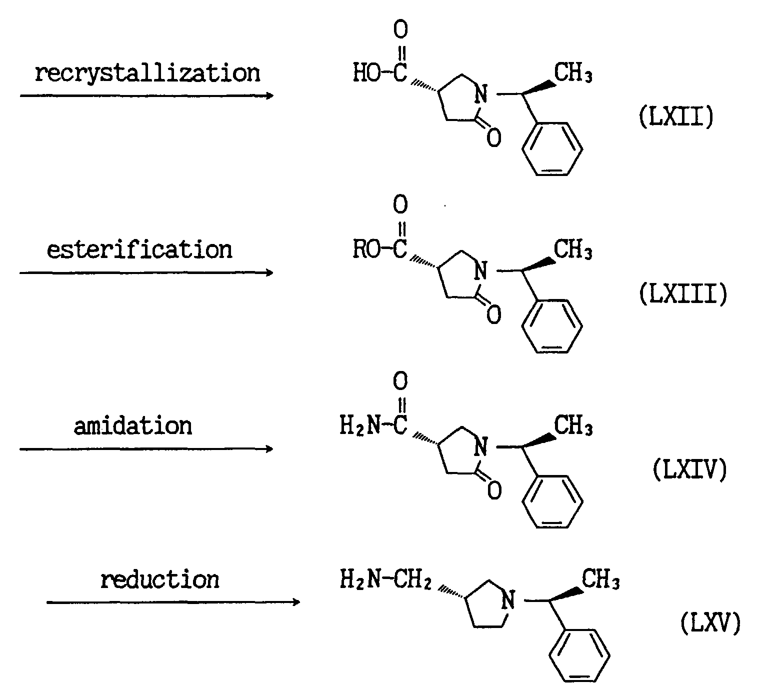 wherein R is lower alkyl.
   That is, itaconic acid is used as a starting compound and reacted with (S)-1-phenylethylamine without solvent or in a suitable solvent to give compound (LXI), followed by repetitive recrystallization in a suitable solvent to give optically pure diastereomer compound (LXII). This compound is reacted in an alcohol solvent in the presence of an acid catalyst to give compound (LXIII), which is subjected to amidation in a suitable solvent in the presence of ammonia gas to give compound (LXIV), followed by reaction with a suitable reducing agent in a suitable solvent to give compound (LXV).
   The solvent to be used for the reaction of itaconic acid and (S)-1-phenylethylamine is exemplified by methanol, ethanol, propanol, acetone, ethyl acetate, benzene, toluene, tetrahydrofuran, dioxane, 1,3-dimethylimidazolidinone, dimethylformamide, dimethyl sulfoxide and the like. While the reaction temperature varies depending on the kind of solvent to be used, it is generally from 30°C to the refluxing temperature of the solvent, and the reaction time which varies depending on the kind of reaction temperature is generally 1-24 hr.
   The solvent to be used for the recrystallization of compound. (LXI) is exemplified by water, methanol, ethanol, propanol, isopropyl alcohol, butanol, acetone, ethyl acetate, benzene, toluene, dioxane, mixed solvents thereof and the like.
   The alcohol solvent to be used for the esterification of compound (LXII) is exemplified by methanol, ethanol, propanol, butanol and the like. The acid catalyst to be used may be, for example, hydrochloric acid, sulfuric acid, p-toluenesulfonic acid or thionyl chloride. The reaction temperature is generally from 0°C to the refluxing temperature of the solvent, and the reaction time which varies depending on the kind of reaction temperature is generally 1-24 hr.
   The solvent to be used for the amidation of compound (LXIII) includes, for example, methanol, ethanol, propanol, isopropyl alcohol, butanol, ethyl acetate, benzene, toluene and dioxane. The reaction temperature is generally from -20°C to 50°C, and the reaction time which varies depending on the kind of reaction temperature is generally 1-24 hr.
   The solvent to be used for the reduction of compound (LXIV) includes, for example, methanol, ethanol, propanol, isopropyl alcohol, benzene, toluene, tetrahydrofuran, dioxane and diethyl ether. The reducing agent to be used includes, for example, lithium aluminum hydride, diborane, diisobutylaluminum hydride, sodium borohydride-sulfuric acid and sodium borohydride-boron trifluoride. The reaction temperature is generally from -30°C to the refluxing temperature of the solvent, and the reaction time which varies depending on the kind of reaction temperature is generally 1-24 hr.
   The compound wherein the absolute configuration at the 3-position of pyrrolidine is S can be synthesized in the same manner as above using itaconic acid and (R)-1-phenylethylamine as starting compounds.
   The optically active compound (VIII) thus obtained can be separated and purified from reaction mixture by a method known *per se*, such as recrystallization, column chromatography, and the like.

The Experimental Examples are shown in the following, to which the present invention is not limited.

### Experimental Example 1 : 5-HT₄ receptor binding test

According to the method of C.J. Grossman et al. (British Journal of Pharmacology, vol. 109, pp. 618-624), a specific 5-HT₄ receptor binding test was run using, as a tracer ligand, 1-methyl-1H-indole-3-carboxylic acid · 1-(2-methylsulfonylaminoethyl)-piperidin-4-ylmethyl ester substituted by tritium (hereinafter to be referred to as [3H]GR113808). Specifically, crude synapse membrane sample was prepared from guinea pig striatum, suspended in 50 mM HEPES buffer (pH 7.4) and used for testing. The test compound having several different concentrations and [3H]GR113808 (final concentration 0.1 nM) were added to this suspension and allowed to react at 37°C. Thirty minutes later, the reaction mixture was filtered by suction with cell harvester. The filter was washed with 50 mM HEPES buffer, and the radioactivity on the filter was counted by liquid scintillation counter. The non-specific binding was determined in the presence of 1 µM GR113808. The concentration of the test compound necessary for 50% inhibition (IC₅₀) and Ki value were determined from graph.

### Experimental Example 2 : 5-HT₃ receptor binding test

According to the method of Peroutka et al (European Journal of Pharmacology, vol. 148, pp. 297-299), a specific 5-HT₃ receptor binding test was run using, as a tracer ligand, endo-1-methyl-N-(9-methyl-9-azabicyclo[3.3.1]non-3-yl)-1H-indazole-3-carboxamide substituted by tritium (hereinafter to be referred to as [3H]Granisetron). Specifically, crude synapse membrane sample was prepared from rat cerebral cortex, suspended in 50 mM HEPES buffer (pH 7.4) and used for testing. The test compound having several different concentrations and [3H]Granisetron (final concentration 1.0 nM) were added to this suspension and allowed to react at 25°C. Thirty minutes later, the reaction mixture was filtered by suction with cell harvester. The filter was washed with 50 mM Tris buffer (pH 7.4), and the radioactivity on the filter was counted using liquid scintillation counter or β-plate. The non-specific binding was determined in the presence of 1 µM Tropisetron. The concentration of the test compound necessary for 50% inhibition (IC₅₀) and Ki value were determined from graph.

The results of the above-mentioned Experimental Examples 1 and 2 are shown in Table A, wherein ∗ shows IC₅₀.

**Table A**

| Example | Affinity for receptor (Ki value, nM) | |
|---|---|---|
| | 5-HT₄ | 5-HT₃ |
| 11 | 6.2 | >1000* |
| 13 | 3.1 | >1000* |
| 17 | 3.1 | >1000* |
| 19 | 4.4 | >1000* |
| 22 | 1.3 | >1000* |
| 24 | 1.6 | >1000* |
| 25 | 1.4 | >1000* |
| 27 | 2.2 | >1000* |
| 29 | 1.7 | >1000* |
| 30 | 0.81 | >1000* |
| 31 | 2.3 | >1000* |
| 45 | 5.5 | >1000* |
| 46 | 1.5 | >1000* |
| 48 | 2.1 | >1000* |
| 56 | 0.65 | >1000* |
| 59 | 1.1 | 190 |
| 70 | 7.4 | >1000* |
| 71 | 7.4 | >1000* |
| 78 | 1.4 | 370 |
| 85 | 0.44 | 260 |
| 88 | 1.2 | 350 |
| 90 | 0.43 | >1000* |
| 94 | 3.5 | >1000* |
| 95 | 1.2 | >1000* |
| 98 | 1.6 | >1000* |
| 100 | 5.3 | >1000* |

### Experimental Example 3 : contraction of isolated guinea pig ascending colon

About 3 cm long ascending colon segment was taken from male Hartley guinea pig and suspended in 10 ml of a Tyrode solution ventilated with a mixed gas of oxygen (95%) and CO₂ (5%) at 37°C with the load of 2 g. The contraction was isotonically measured via isotonic transducer. After an equilibrium period of about 30 min, the test compound was administered non-cumulatively from lower concentrations, and dose-dependent contraction was measured. The test compound was administered at 15 minutes intervals. Then, contraction by the test compound in the presence of a 5-HT₄ receptor antagonist, 4-amino-3-chloro-6-methoxybenzoic acid · 2-diethylaminoethyl ester (0.3 µM, hereinafter to be referred to as SDZ 205-557) was determined, and antagonistic effect on the reaction by SDZ 205-557 was confirmed.

The contraction by the test compound was evaluated based on the ratio of reaction relative to mean contraction induced by methacholine (30 µM) administered before and after each determination of dose-dependent contraction. The contraction was probit-converted using the maximum value of dose-response curve as 100, and expressed by EC₅₀ as determined from linear regression and maximum reaction ratio.

The results of the above-mentioned Experimental Example are shown in Table B.

**Table B**

| Example | Contraction of removed colon (EC₅₀, nM) |
|---|---|
| 22 | 3.3 |
| 24 | 3.9 |
| 25 | 4.6 |
| 27 | 5.3 |
| 29 | 3.7 |
| 30 | 1.6 |
| 46 | 6.9 |
| 48 | 4.8 |
| 56 | 1.0 |
| 70 | 6.7 |
| 85 | 1.2 |
| 88 | 1.4 |
| 90 | 0.9 |
| 98 | 4.0 |

As demonstrated in the above tests, the compound of the present invention and pharmaceutically acceptable salts thereof have selective and high affinity for 5-HT₄ receptors, and are useful as superior gastrointestinal prokinetic agents for the prophylaxis and treatment of various gastrointestinal diseases selected from delayed gastric emptying, indigestion, meteorism, reflux esophagitis, abdominal indefinite complaint, intestinal pseudoileus, constipation, acute or chronic gastritis, gastric and duodenal ulcers, Crohn's disease, non-ulcer dyspepsia, ulcerative colitis, postgastrectomy syndrome, postoperative digestive function failure, gastrointestinal injury due to gastric neurosis, gastroptosis, diabetes, etc., and irritable bowel syndrome. They are also useful for the prophylaxis and treatment of diseases in which 5-HT₄ receptors are involved, such as central nervous disorders (e.g., schizophrenia, depression, anxiety, disturbance of memory and dementia); cardiac function disorders (e.g., cardiac failure and myocardial ischemia); and urinary diseases (e.g., dysuria caused by urinary obstruction, ureterolith or prostatomegaly).

When the compound of the present invention and pharmaceutically acceptable salts thereof are used as medicaments, they can be administered as they are or upon formulation into pharmaceutical compositions such as tablets, buccal, pills, capsules, powders, fine granules, granules, liquids, oral liquids inclusive of syrups, injections, inhalants, suppositories, transdermal liquids, ointments, transdermal plasters, transmucosal plasters (e.g., intraoral plaster), transmucosal liquids (e.g. nasal liquid), and the like, using pharmaceutically acceptable carriers, excipients, extenders, diluents and other additives, orally or parenterally to the patients in need of treatment. Examples of the pharmaceutically acceptable carriers, excipients, extenders, diluents and other additives are solid or liquid nontoxic pharmaceutical substances, such as lactose, magnesium stearate, starch, talc, gelatin, agar, pectin, gum arabic, olive oil, sesame oil, cacao butter, ethylene glycol and other conventional ones. While the clinical dose of the compound of the present invention varies depending on the compound to be selected, disease, symptom, body weight, age, sex and the like of the patients who will undergo treatment, administration route and the like, it is generally 1-2000 mg, preferably 10-300 mg, daily for an adult by oral administration which is given in a sigle dose or 2-4 doses.

### Best Mode for Embodying the Invention

The present invention is specifically described by Preparation Examples, Examples and Comparative Examples, to which the invention is not limited. When the compound to be obtained is an optical isomer, it can be confirmed by its optical rotation and high performance liquid chromatography. When the optical isomer is crystal, the absolute configuration thereof can be confirmed by X-ray diffraction.

### Preparation Example 1

Itaconic acid (650 g) and (S)-1-phenylethylamine (605 g) were suspended in 1,3-dimethylimidazolidinone (650 ml), and the suspension was stirred at 80°C for 1 hr with heating, and further at 120°C for 3 hr with heating. The mixture was cooled to room temperature, and water (4 ℓ) was added. The precipitated crystals were collected by filtration and dried to give 950 g of 1-((S)-1-phenylethyl)-5-oxo-3-pyrrolidinecarboxylic acid. The crystals were recrystallized 3 times with isopropyl alcohol to give 156 g of (3S)-1-((S)-1-phenylethyl)-5-oxo-3-pyrrolidinecarboxylic acid.
Melting point 209-211°C, [α]_{D}²⁵ = -69.1 (c=1.0, dimethylformamide), optical purity: not less than 99%

The compound of the following Preparation Example 2 was produced in the same manner as in Preparation Example 1.

### Preparation Example 2

(3R)-1-((R)-1-phenylethyl)-5-oxo-3-pyrrolidinecarboxylic acid,
Melting point 207-210°C, [α]_{D}²⁵ = +68.4 (c=1.0, dimethylformamide), optical purity: not less than 99%

### Preparation Example 3

(35)-1-((S)-1-Phenylethyl)-5-oxo-3-pyrrolidinecarboxylic acid (666 g, optical purity not less than 99%) was dissolved in methanol (6 ℓ), and conc. sulfuric acid (15 ml) was added at room temperature. The mixture was stirred at refluxing temperature for 7 hr. Methanol was evaporated, and the residue was extracted with ethyl acetate.
The organic layer was washed with sodium hydrogencarbonate, dried over magnesium sulfate and concentrated under reduced pressure to give 738 g of methyl (3S)-1-((S)-1-phenylethyl)-5-oxo-3-pyrrolidinecarboxylate.
¹H-NMR (CDCl₃,ppm) δ : 1.47-1.53(3H,d,J=7.3Hz), 2.72-2.83(2H,m), 3.03-3.23(2H,m), 3.50-3.61(1H,m), 3.74(3H,s), 5.44-5.59(1H,q,J=7.3Hz), 7.20-7.41(5H,m)

In the same manner as in Preparation Example 3, the compound of the following Preparation Example 4 was produced.

### Preparation Example 4

methyl (3R)-1-((R)-1-phenylethyl)-5-oxo-3-pyrrolidinecarboxylate
¹H-NMR (CDCl₃,ppm) δ : 1.48-1.54(3H,d,J=7.3Hz), 2.70-2.83(2H,m), 3.05-3.25(2H,m), 3.61-3.71(1H,m), 3.75(3H,s), 5.43-5.58(1H,q,J=7.3Hz), 7.22-7.41(5H,m), [α]_{D}²⁵ = +55.5 (c=2.1, ethyl acetate)

### Preparation Example 5

Methyl (3S)-1-((S)-1-phenylethyl)-5-oxo-3-pyrrolidinecarboxylate (738 g) was dissolved in methanol (3.5 ℓ). The solution was stirred for 8 hr under ice-cooling while blowing in ammonia gas, and concentrated under reduced pressure to give 537 g of (3S)-1-((S)-1-phenylethyl)-5-oxo-3-pyrrolidinecarboxamide.
Melting point 149∼150°C, [α]_{D}²⁵ = -58.2 (c=1.0, chloroform)

In the same manner as in Preparation Example 5, the compound of the following Preparation Example 6 was produced.

### Preparation Example 6

(3R)-1-((R)-1-phenylethyl)-5-oxo-3-pyrrolidinecarboxamide,
Melting point 151∼153°C

### Preparation Example 7

(3S)-1-((S)-1-Phenylethyl)-5-oxo-3-pyrrolidinecarboxamide (150 g) was dissolved in tetrahydrofuran (2.5 ℓ), and sodium borohydride (138 g) was added with stirring. Conc. sulfuric acid (130 ml) was dropwise added under ice-cooling. The mixture was stirred for 1 hr at room temperature, and further at refluxing temperature for 10 hr. 6N Hydrochloric acid (300 ml) was dropwise added under ice-cooling, and the mixture was stirred at refluxing temperature for 4 hr. The solvent was evaporated under reduced pressure, a 3N sodium hydroxide solution was added to make same alkaline, and the mixture was extracted with chloroform. The extract was dried over magnesium sulfate, concentrated under reduced pressure and distilled under reduced pressure to give 95 g of (3R)-3-aminomethyl-1-((S)-1-phenylethyl)pyrrolidine.
¹H-NMR (CDCl₃,ppm) δ : 1.27-1.42(3H,d,J=6.6Hz), 1.30-1.52(3H,m), 1.85-2.03(1H,m), 2.05-2.30(2H,m), 2.35-2.59(2H,m), 2.60-2.67(2H,d,J=7.2Hz), 2.73-2.83(1H,m), 3.13-3.23(1H,q,J=6.6Hz), 7.18-7.41(5H,m), [α]_{D}²⁵ = -56.6 (c=1.0, chloroform)

In the same manner as in Preparation Example 7, the compound of the following Preparation Example 8 was produced.

### Preparation Example 8

(3S)-3-aminomethyl-1-((R)-1-phenylethyl)pyrrolidine,
1H-NMR (CDCl₃,ppm) δ : 1.27-1.42(3H,d,J=6.6Hz), 1.30-1.52(3H,m), 1.85-2.03(1H,m), 2.05-2.30(2H,m), 2.35-2.59(2H,m), 2.60-2.67(2H,d,J=7.2Hz), 2.73-2.83(1H,m), 3.13-3.23(1H,q,J=6.6Hz), 7.18-7.41(5H,m)

### Preparation Example 9

Methylene chloride (1000 ml) was added to (1-benzylpyrrolidin-3-ylmethyl)amine (112 g), and a solution of di-tert-butyl dicarbonate (141 g) in methylene chloride was dropwise added at 0°C with stirring. The mixture was stirred at room temperature for 8 hr. Aqueous potassium carbonate solution was added and the mixture was extracted with chloroform. The mixture was dried over magnesium sulfate, and concentrated under reduced pressure to give 171 g of 1-benzyl-3-(tert-butoxycarbonylaminomethyl)pyrrolidine.
Melting point 51∼54°C

In the same manner as in Preparation Example 9, the compounds of the following Preparation Examples 10-12 were produced.

### Preparation Example 10

1-benzyl-4-(tert-butoxycarbonylaminomethyl)piperidine
¹H-NMR (CDCl₃,ppm) δ : 1.20-1.35(3H,m), 1.43(9H,s), 1.55-1.73(2H,m), 1.85-1.99(2H,m), 2.80-3.03(4H,m), 3.48(2H,s), 4.53-4.65(1H,br), 7.20-7.35(5H,m)

### Preparation Example 11

(3R) -3-(tert-butoxycarbonylaminomethyl)-1-((S)-1-phenylethyl)pyrrolidine
¹H-NMR (CDCl₃,ppm) δ : 1.35(3H,d,J=6.6Hz), 1.45(9H,s), 1.45-1.50(1H,m), 1.90-2.03(1H,m), 2.18-2.36(3H,m), 2.48-2.57(1H,m), 2.73-2.86(1H,m), 3.03-3.10(2H,m), 3.10-3.18(1H,q,J=6.6Hz), 5.10-5.21(1H,br), 7.18-7.33(5H,m), [α]_{D}²⁵ = -11.9 (c=1.0, chloroform)

### Preparation Example 12

(3S)-3-(tert-butoxycarbonylaminomethyl)-1-((R)-1-phenylethyl)pyrrolidine,
¹H-NMR (CDCl₃,ppm) δ : 1.35(3H,d,J=6.6Hz), 1.45(9H,s), 1.45-1.50(1H,m), 1.90-2.03(1H,m), 2.18-2.36(3H,m), 2.48-2.57(1H,m), 2.73-2.86(1H,m), 3.03-3.10(2H,m), 3.10-3.18(1H,q,J=6.6Hz), 5.10-5.21(1H,br), 7.18-7.33(5H,m), [α]_{D}²⁵ = +11.1 (c=1.0, chloroform)

### Preparation Example 13

Ethanol (1700 ml) and 10% palladium-carbon (34 g, M type) were added to 1-benzyl-3-(tert-butoxycarbonylaminomethyl)pyrrolidine (171 g), and hydrazine hydrate (29 ml) was dropwise added at room temperature with stirring and the mixture was refluxed with stirring at 80°C for 2 hr. 10% Palladium-carbon was filtered off, and the mixture was concentrated under reduced pressure to give 116 g of 3-tert-butoxycarbonylaminomethyl)pyrrolidine.
¹H-NMR (CDCl₃,ppm) δ : 1.35-1.50(10H,m), 1.89-1.97(1H,m), 2.21-2.28(2H,m), 2.58-2.66(1H,m), 2.83-3.12(5H,m), 4.82-4.92(1H,br)

In the same manner as in Preparation Example 13, the compounds of the following Preparation Examples 14-16 were produced.

### Preparation Example 14

4-(tert-butoxycarbonylaminomethyl)piperidine
¹H-NMR (CDCl₃,ppm) δ : 1.20-1.35(3H,m), 1.44(9H,s), 1.55-1.80(2H,m), 2.60-2.73(2H,m), 2.95-3.28(4H,m), 4.73-4.83(1H,br)

### Preparation Example 15

(3S)-3-(tert-butoxycarbonylaminomethyl)pyrrolidine
¹H-NMR (CDCl₃,ppm) δ : 1.35-1.52(10H,m), 1.85-1.97(1H,m), 2.16-2.32(2H,m), 2.56-2.67(1H,m), 2.82-3.15(5H,m), 4.74-4.89(1H,br),
[α]_{D}²⁵ = -9.2 (c=1.0, chloroform)

### Preparation Example 16

(3R)-3-(tert-butoxycarbonylaminomethyl)pyrrolidine
¹H-NMR (CDCl₃,ppm) δ : 1.35-1.52(10H,m), 1.91-1.95(1H,m), 2.07-2.31(2H,m), 2.55-2.67(1H,m), 2.83-3.15(5H,m), 4.72-4.85(1H,br),
[α]_{D}²⁵ = +8.3 (c=1.0, chloroform)

### Preparation Example 17

Dimethylformamide (200 ml) was added to 3-(tert-butoxycarbonylaminomethyl)pyrrolidine (14 g), and potassium carbonate (30 g) and N-(3-bromopropyl)phthalimide (18 g) were added at room temperature with stirring, and the mixture was stirred at room temperature for 8 hr. The reaction mixture was concentrated under reduced pressure, and aqueous potassium carbonate solution was added to the residue. The mixture was extracted with ethyl acetate. The mixture was dried over magnesium sulfate, and concentrated under reduced pressure. The obtained residue was purified by silica gel chromatography to give 17 g of 2-(3-(3-tert-butoxycarbonylaminomethylpyrrolidin-1-yl)propyl)-2,3-dihydro-1H-isoindole-1,3-dione.
¹H-NMR (CDCl₃,ppm) δ : 1.43(9H,s), 1.54-1.73(1H,m), 1.82-2.14(3H,m), 2.25-2.69(7H,m), 3.01-3.09(2H,m), 3.70-3.86(2H,m), 4.90-5.04(1H,br), 7.69-7.73(2H,m), 7.81-7.86(2H,m)

In the same manner as in Preparation Example 17, the compounds of the following Preparation Examples 18-39 were produced.

### Preparation Example 18

2-(2-(3-tert-butoxycarbonylaminomethylpyrrolidin-1-yl)ethyl)-2,3-dihydro-1H-isoindole-1,3-dione oxalate
Melting point 141-143°C

### Preparation Example 19

2-(2-((3R)-3-tert-butoxycarbonylaminomethylpyrrolidin-1-yl)ethyl)-2,3-dihydro-1H-isoindole-1,3-dione
¹H-NMR (CDCl₃,ppm) δ : 1.44-1.52(10H,m), 1.81-2.08(1H,m), 2.15-2.60(7H,m), 2.88-3.11(2H,m), 3.82-3.86(2H,m), 4.95-5.09(1H,br), 7.65-7.74(2H,m), 7.82-7.89(2H,m)

### Preparation Example 20

2-(3-((3R)-3-tert-butoxycarbonylaminomethylpyrrolidin-1-yl)propyl)-2,3-dihydro-1H-isoindole-1,3-dione
¹H-NMR (CDCl₃,ppm) δ : 1.42-1.52(10H,m), 1.81-2.08(3H,m), 2.20-2.65(7H,m), 2.97-3.12(2H,m), 3.82-3.85(2H,m), 4.90-5.04(1H,br), 7.68-7.76(2H,m), 7.81-7.88(2H,m)

### Preparation Example 21

2-(3-((3S)-3-tert-butoxycarbonylaminomethylpyrrolidin-1-yl)propyl)-2,3-dihydro-1H-isoindole-1,3-dione
¹H-NMR (CDCl₃,ppm) δ : 1.42-1.52(10H,m), 1.81-2.08(3H,m), 2.20-2.65(7H,m), 2.97-3.12(2H,m), 3.82-3.85(2H,m), 4.90-5.04(1H,br), 7.68-7.76(2H,m), 7.81-7.88(2H,m)

### Preparation Example 22

2-(4-(3-tert-butoxycarbonylaminomethylpyrrolidin-1-yl)butyl)-2,3-dihydro-1H-isoindole-1,3-dione
¹H-NMR(CDCl₃,ppm) δ : 1.40(9H,s),1.55-1.62(3H,m),1.70-1.81(2H,m), 1.91-2.05(1H,m),2.35-2.44(2H,m),2.45-2.60(3H,m),2.62-2.73(2H,m), 3.06-3.18(2H,m),3.71-3.83(2H,m),4.95-5.01(1H,br),7.68-7.72(2H,m), 7.79-7.83(2H,m)

### Preparation Example 23

2-(4-(4-tert-butoxycarbonylaminomethylpiperidin-1-yl)butyl)-2,3-dihydro-1H-isoindole-1,3-dione
¹H-NMR(CDCl₃,ppm) δ : 1.15-1.35(3H,m), 1.43(9H,s), 1.43-1.73(6H,m), 1.83-1.97(2H,m), 2.27-2.38(2H,m), 2.82-3.06(4H,m), 3.65-3.73(2H,m), 4.53-4.67(1H,br), 7.79-7.89(4H,m)

### Preparation Example 24

2-(4-((3R)-3-tert-butoxycarbonylaminomethylpyrrolidin-1-yl)butyl)-2,3-dihydro-1H-isoindole-1,3-dione
¹H-NMR(CDCl₃,ppm) δ :1.35-1.60(12H,m), 1.62-1.81(2H,m), 1.87-2.05(1H,m), 2.06-2.68(7H,m), 3.00-3.18(2H,m), 3.65-3.79(2H,m), 4.93-5.05(1H,br), 7.67-7.89(4H,m)

### Preparation Example 25

2-(4-((3S)-3-tert-butoxycarbonylaminomethylpyrrolidin-1-yl)butyl)-2,3-dihydro-1H-isoindole-1,3-dione
¹H-NMR(CDCl₃,ppm) δ :1.35-1.60(12H,m), 1.62-1.81(2H,m), 1.87-2.05(1H,m), 2.06-2.68(7H,m), 3.00-3.18(2H,m), 3.65-3.79(2H,m), 4.93-5.05(1H,br), 7.67-7.89(4H,m)

### Preparation Example 26

2-(5-(3-tert-butoxycarbonylaminomethylpyrrolidin-1-yl)pentyl)-2,3-dihydro-1H-isoindole-1,3-dione
¹H-NMR(CDCl₃,ppm) δ :1.31-1.79(7H,m),1.42(9H,s),1.89-2.05(1H,m), 2.30-2.69(7H,m),3.05-3.16(2H,m),3.61-3.75(2H,m),4.92-5.05(1H,br), 7.66-7.75(2H,m),7.79-7.86(2H,m)

### Preparation Example 27

2-(5-((3R)-3-tert-butoxycarbonylaminomethylpyrrolidin-1-yl)pentyl)-2,3-dihydro-1H-isoindole-1,3-dione
¹H-NMR(CDCl₃,ppm) δ :1.32-1.80(7H,m), 1.41(9H,s), 1.90-2.05(1H,m), 2.30-2.70(7H,m), 3.06-3.16(2H,m), 3.65-3.79(2H,m), 4.95-5.06(1H,br), 7.65-7.75(2H,m), 7.78-7.87(2H,m)

### Preparation Exampl 28

2-(5-((3S)-3-tert-butoxycarbonylaminomethylpyrrolidin-1-yl)pentyl)-2,3-dihydro-1H-isoindole-1,3-dione
¹H-NMR(CDCl₃,ppm) δ :1.32-1.80(7H,m), 1.41(9H,s), 1.90-2.05(1H,m), 2.30-2.70(7H,m), 3.06-3.16(2H,m), 3.65-3.79(2H,m), 4.95-5.06(1H,br), 7.65-7.75(2H,m), 7.78-7.87(2H,m)

### Preparation Example 29

2-(6-((3R)-3-tert-butoxycarbonylaminomethylpyrrolidin-1-yl)hexyl)-2,3-dihydro-1H-isoindole-1,3-dione
¹H-NMR(CDCl₃,ppm) δ :1.28-2.40(19H,m), 2.75-3.79(11H,m), 5.66-5.73(1H,br), 7.68-7.92(4H,m)

### Preparation Example 30

2-(6-((3S)-3-tert-butoxycarbonylaminomethylpyrrolidin-1-yl)hexyl)-2,3-dihydro-1H-isoindole-1,3-dione
¹H-NMR(CDCl₃,ppm) δ : 1.28-2.40(19H,m), 2.75-3.79(11H,m), 5.66-5.73(1H,br), 7.68-7.92(4H,m)

### Preparation Example 31

2-(5-(4-tert-butoxycarbonylaminomethylpiperidin-1-yl)pentyl)-2,3-dihydro-1H-isoindole-1,3-dione
¹H-NMR(CDCl₃,ppm) δ : 1.35-1.52(12H,m), 1.55-1.91(8H,m), 2.30-2.51(2H,m), 2.61-2.73(2H,m), 3.03-3.09(2H,m), 3.18-3.33(2H,m), 3.62-3.70(2H,m), 4.75-4.85(1H,br), 7.68-7.88(4H,m)

### Preparation Example 32

4-(3-tert-butoxycarbonylaminomethylpyrrolidin-1-yl)-N-phenylbutylamide
¹H-NMR(CDCl₃,ppm) δ : 1.44(9H,s), 1.82-2.20(5H,m), 2.25-2.81(8H,m), 2.85-3.23(2H,m), 4.98-5.10(1H,br), 6.96-7.70(5H,m), 8.65-8.82(1H,br)

### Preparation Example 33

4-(3-tert-butoxycarbonylaminomethylpyrrolidin-1-yl)-N-(4-methylphenyl)butylamide
¹H-NMR(CDCl₃,ppm) δ : 1.44(9H,s), 1.82-2.10(5H,m), 2.30(3H,s), 2.25-2.80(8H,m), 2.85-3.23(2H,m), 5.05-5.15(1H,br), 7.05-7.60(4H,m), 8.55-8.64(1H,br)

### Preparation Example 34

4-(3-tert-butoxycarbonylaminomethylpyrrolidin-1-yl)-N-(3-chlorophenyl)butylamide
¹H-NMR(CDCl₃,ppm) δ : 1.44(9H,s), 1.83-2.20(5H,m), 2.25-2.80(8H,m), 2.90-3.25(2H,m), 4.95-5.12(1H,br), 7.00-7.70(4H,m), 9.02-9.19(1H,br)

### Preparation Example 35

2-(4-(4-(2-hydroxyethyl)piperidin-1-yl)butyl)-2,3-dihydro-1H-isoindole-1,3-dione
¹H-NMR(CDCl₃,ppm) δ : 1.18-1.95(13H,m), 2.25-2.38(2H,m), 2.80-2.94(2H,m), 3.60-3.78(4H,m), 7.67-7.90(4H,m)

### Preparation Example 36

3-tert-butoxycarbonylaminomethyl-1-(2-methylsulfonylaminoethyl)-pyrrolidine
¹H-NMR(CDCl₃,ppm) δ : 1.44(9H,s), 1.44-1.53(1H,m), 1.90-2.20(1H,m), 2.29-2.78(7H,m), 2.98(3H,s), 3.05-3.26(4H,m), 4.70-4.80(1H,br), 5.00-5.17(1H,br)

### Preparation Example 37

3-tert-butoxycarbonylaminomethyl-1-(3-(1,1,3-trioxo-2,3-dihyro-1,2-benzisothiazol-2-yl)propyl)pyrrolidine
¹H-NMR(CDCl₃,ppm) δ :1.40(9H,s),1.45-1.55(1H,m),1.91-2.20(1H,m), 2.20-2.27(2H,m),2.30-2.73(7H,m),3.04-3.18(2H,m),3.80-3.92(2H,m), 5.02-5.10(1H,br),7.80-7.92(3H,m),8.05-8.09(1H,m)

### Preparation Example 38

3-tert-butoxycarbonylaminomethyl-1-(3-(2,3-dihydro-2-oxobenzimidazol-1-yl)propyl)pyrrolidine
¹H-NMR(CDCl₃,ppm) δ : 1.40(9H,s), 1.45-1.50(3H,m), 1.93-2.08(1H,m), 2.45-2.78(7H,m), 3.47(2H,t,J=6.3Hz), 3.84(3H,s), 3.91(2H,t,J=6.9Hz), 4.40(2H,s), 6.98-7.14(4H,m), 9.10-9.10(1H,br)

### Preparation Example 39

N-(4-(3-tert-butoxycarbonylaminomethylpyrrolidin-1-yl)butyl)-N-methylbenzamide
¹H-NMR(CDCl₃,ppm) δ : 1.43(9H,s), 1.45-1.99(5H,m), 2.01-2.25(1H,m), 2.45-3.33(12H,m), 3.45-3.55(2H,m), 5.01-5.20(1H,br), 7.25-7.50(5H,m)

### Preparation Example 40

Ethanol (200 ml) was added to 2-(3-(3-tert-butoxycarbonylaminomethylpyrrolidin-1-yl)propyl)-2,3-dihydro-1H-isoindole-1,3-dione (17 g) and hydrazine hydrate (4.5 ml) was added at room temperature with stirring and the mixture was refluxed with stirring for 8 hr. The precipitated crystals were filtered off, and the filtrate was concentrated under reduced pressure to give 11 g of 3-(3-tert-butoxycarbonylaminomethylpyrrolidin-1-yl)propylamine.
¹H-NMR(CDCl₃,ppm) δ : 1.44(9H,s),1.53-1.73(3H,m),1.87-2.06(1H,m), 2.30-3.27(13H,m),5.06-5.22(1H,br)

In the same manner as in Preparation Example 40, the compounds of the following Preparation Examples 41-54 were produced.

### Preparation Example 41

2-(3-tert-butoxycarbonylaminomethylpyrrolidin-1-yl)ethylamine
¹H-NMR(CDCl₃,ppm) δ : 1.45(9H,s),1.48-1.50(1H,m),1.80-1.93(1H,m), 2.25-2.61(7H,m),3.06-3.43(4H,m),4.96-5.10(1H,br)

### Preparation Example 42

2-((3R)-3-tert-butoxycarbonylaminomethylpyrrolidin-1-yl)ethylamine
¹H-NMR(CDCl₃,ppm) δ : 1.44(9H,s), 1.48-1.50(1H,m), 1.85-2.02(1H,m), 2.15-2.55(7H,m), 2.88-3.13(2H,m), 3.82-3.86(2H,m), 4.95-5.09(1H,br)

### Preparation Example 43

3-((3R)-3-tert-butoxycarbonylaminomethylpyrrolidin-1-yl)propylamine
¹H-NMR(CDCl₃,ppm) δ : 1.44(9H,s), 1.50-1.70(3H,m), 1.88-2.04(1H,m), 2.20-2.69(7H,m), 2.74-2.83(2H,m), 3.02-3.17(2H,m), 5.06-5.16(1H,br)

### Preparation Example 44

3-((3S)-3-tert-butoxycarbonylaminomethylpyrrolidin-1-yl)propylamine
¹H-NMR(CDCl₃,ppm) δ : 1.44(9H,s), 1.50-1.70(3H,m), 1.88-2.04(1H,m), 2.20-2.69(7H,m), 2.74-2.83(2H,m), 3.02-3.17(2H,m), 5.06-5.16(1H,br)

### Preparation Example 45

4-(3-tert-butoxycarbonylaminomethylpyrrolidin-1-yl)butylamine
¹H-NMR(CDCl₃,ppm) δ : 1.45(9H,s),1.48-1.75(5H,m),1.90-2.06(1H,m), 2.31-2.80(7H,m),3.06-3.17(2H,m),3.22-3.43(2H,m),5.04-5.15(1H,br)

### Preparation Example 46

4-((3R)-3-tert-butoxycarbonylaminomethylpyrrolidin-1-yl)butylamine
¹H-NMR(CDCl₃,ppm) δ : 1.45(9H,s), 1.40-1.57(5H,m), 1.87-2.03(1H,m), 2.28-2.79(7H,m), 3.05-3.13(2H,m), 3.38-3.52(2H,m), 5.12-5.23(1H,br)

### Preparation Example 47

4-((3S)-3-tert-butoxycarbonylaminomethylpyrrolidin-1-yl)butylamine
¹H-NMR(CDCl₃,ppm) δ : 1.45(9H,s), 1.40-1.57(5H,m), 1.87-2.03(1H,m), 2.28-2.79(7H,m), 3.05-3.13(2H,m), 3.38-3.52(2H,m), 5.12-5.23(1H,br)

### Preparation Example 48

5-(3-tert-butoxyoarbonylaminomethylpyrrolidin-1-yl)pentylamine
¹H-NMR(CDCl₃,ppm) δ : 1.45(9H,s), 1.48-1.60(7H,m), 1.95-2.07(1H,m), 2.25-2.78(7H,m), 3.10-3.20(2H,m), 3.25-3.45(2H,m), 5.00-5.09(1H,br)

### Preparation Example 49

5-((3R)-3-tert-butoxycarbonylaminomethylpyrrolidin-1-yl)pentylamine
¹H-NMR(CDCl₃+CD₃OD,ppm) δ : 1.28-1.59(7H,m), 1.44(9H,s), 1.90-2.04(1H,m), 2.22-2.80(7H,m), 3.05-3.13(2H,m), 3.55-3.59(2H,m)

### Preparation Example 50

5-((3S)-3-tert-butoxycarbonylaminomethylpyrrolidin-1-yl)pentylamine
¹H-NMR(CDCl₃+CD₃OD,ppm) δ : 1.28-1.59(7H,m), 1.44(9H,s), 1.90-2.04(1H,m), 2.22-2.80(7H,m), 3.05-3.13(2H,m), 3.55-3.59(2H,m)

### Preparation Example 51

6-((3R)-3-tert-butoxycarbonylaminomethylpyrrolidin-1-yl)hexylamine
¹H-NMR(CDCl₃+CD₃OD,ppm) δ : 1.20-1.78(9H,m), 1.44(9H,s), 1.90-2.05(1H,m), 2.22-2.85(7H,m), 3.20-3.41(2H,m), 3.53-3.59(2H,m)

### Preparation Example 52

6-((3S)-3-tert-butoxycarbonylaminomethylpyrrolidin-1-yl)hexylamine
¹H-NMR(CDCl₃+CD₃OD,ppm) δ : 1.20-1.78(9H,m), 1.44(9H,s), 1.90-2.05(1H,m), 2.22-2.85(7H,m), 3.20-3.41(2H,m), 3.53-3.59(2H,m)

### Preparation Example 53

4-(4-tert-butoxycarbonylaminomethylpiperidin-1-yl)butylamine
¹H-NMR(CDCl₃,ppm) δ : 1.15-1.35(3H,m), 1.43(9H,s), 1.43-1.72(6H,m), 1.80-1.95(2H,m), 2.24-2.38(2H,m), 2.82-3.05(6H,m), 4.70-4.92(1H,br)

### Preparation Example 54

5-(4-tert-butoxycarbonylaminomethylpiperidin-1-yl)pentylamine
¹H-NMR(CDCl₃,ppm) δ : 1.25-1.77(20H,m), 1.95-2.12(2H,m), 2.38-2.45(2H,m), 2.75-2.85(2H,m), 2.95-3.10(2H,m), 4.38-4.55(2H,m), 4.73-4.84(1H,br)

### Preparation Example 55

3-(3-tert-Butoxycarbonylaminomethylpyrrolidin-1-yl)propylamine (5 g) was dissolved in dimethylformamide (50 ml) and 4-amino-5-chloro-2-methoxybenzoic acid (3.9 g) and 1-hydroxybenzotriazole (3.2 g) were added. The mixture was stirred at 0°C for 15 min and 1-ethyl-3-(3-dimethylaminopropyl)carbodiimide (4.5 g) was added, followed by stirring at room temperature for 10 hr. The reaction mixture was concentrated under reduced pressure, and aqueous potassium carbonate solution was added to the residue. The mixture was extracted with ethyl acetate, dried over magnesium sulfate, and concentrated under reduced pressure. The obtained residue was purified by silica gel chromatography to give 3.4 g of 4-amino-N-(3-(3-tert-butoxycarbonylaminomethylpyrrolidin-1-yl)propyl)-5-chloro-2-methoxybenzamide.
¹H-NMR(CDCl₃,ppm) δ : 1.43(9H,s), 1.43-1.62(1H,m), 1.80-2.10(3H,m), 2.36-2.82(7H,m), 3.03-3.18(2H,m), 3.44-3.56(2H,m), 3.90(3H,s), 4.38(2H,s), 5.03-5.15(1H,br), 6.30(1H,s), 7.75-7.87(1H,br), 8.09(1H,s)

In the same manner as in Preparation Example 55, the compounds of the following Preparation Examples 56-80 were produced.

### Preparation Example 56

4-amino-N-(2-(3-tert-butoxycarbonylaminomethylpyrrolidin-1-yl)-ethyl)-5-chloro-2-methoxybenzamide
¹H-NMR (CDCl₃,ppm) δ : 1.43(9H,s), 1.43-1.66(1H,m), 1.92-2.10(1H,m), 2.30-2.82(7H,m), 3.07-3.20(2H,m), 3.50-3.63(2H,m), 3.89(3H,s), 4.35(2H,s), 4.68-4.83(1H,br), 6.28(1H,s), 8.01-8.14(1H,br), 8.10(1H,s)

### Preparation Example 57

4-amino-N-(4-(3-tert-butoxycarbonylaminomethylpyrrolidin-1-yl)-butyl)-5-chloro-2-methoxybenzamide
¹H-NMR (CDCl₃,ppm) δ : 1.43(9H,s), 1.45-1.70(5H,m), 1.90-2.04(1H,m), 2.32-2.72(7H,m), 3.05-3.16(2H,m), 3.36-3.46(2H,m), 3.89(3H,s), 6.30(1H,s), 7.65-7.73(1H,br), 8.10(1H,s)

### Preparation Example 58

4-amino-N-(4-((3R)-3-tert-butoxycarbonylaminomethylpyrrolidin-1-yl)butyl)-5-chloro-2-methoxybenzamide
¹H-NMR (CDCl₃,ppm) δ : 1.43(9H,s), 1.45-1.68(5H,m), 1.90-2.07(1H,m), 2.31-2.68(7H,m), 3.03-3.14(2H,m), 3.36-3.44(2H,m), 3.89(3H,s), 6.29(1H,s), 7.65-7.73(1H,br), 8.09(1H,s)

### Preparation Example 59

4-amino-N-(5-((3R)-3-tert-butoxycarbonylaminomethylpyrrolidin-1-yl)pentyl)-5-chloro-2-methoxybenzamide
¹H-NMR (CDCl₃,ppm) δ : 1.42(9H,s), 1.30-1.80(7H,m), 1.95-2.10(1H,m), 2.20-2.71(7H,m), 3.04-3.15(2H,m), 3.38-3.48(2H,m), 3.89(3H,s), 6.30(1H,s), 7.64-7.70(1H,br), 8.08(1H,s)

### Preparation Example 60

N-(3-(3-tert-butoxycarbonylaminomethylpyrrolidin-1-yl)propyl)-4-pyridinecarboxamide
¹H-NMR (CDCl₃,ppm) δ : 1.35-1.60(10H,m),1.75-2.20(4H,m),2.30-2.45(2H,m), 2.55-2.90(4H,m),3.03-3.18(2H,m),3.51-3.68(2H,m),4.71-4.85(1H,br), 7.58-7.66(2H,m),8.55-8.65(1H,br),8.65-8.79(2H,m)

### Preparation Example 61

N-(2-(3-tert-butoxycarbonylaminomethylpyrrolidin-1-yl)ethyl)-6-chloro-4-methyl-3,4-dihydro-2H-1,4-benzoxazine-8-carboxamide
¹H-NMR (CDCl₃,ppm) δ : 1.42(9H,s), 1.45-1.62(1H,m), 1.92-2.08(1H,m), 2.30-2.83(7H,m), 2.90(3H,s), 3.06-3.18(2H,m), 3.30-3.38(2H,m), 3.52-3.64(2H,m), 4.35-4.41(2H,m), 4.75-4.87(1H,br), 6.66(1H,d,J=2.64Hz), 7.42(1H,d,J=2.64Hz), 8.04-8.16(1H,br)

### Preparation Example 62

N-(3-(3-tert-butoxycarbonylaminomethylpyrrolidin-1-yl)propyl)-6-chloro-4-methyl-3,4-dihydro-2H-1,4-benzoxazine-8-carboxamide
¹H-NMR (CDCl₃,ppm) δ : 1.43(9H,s), 1.43-1.57(1H,m), 1.77-2.10(3H,m), 2.62-2.74(7H,m), 2.90(3H,s), 3.05-3.14(2H,m), 3.32-3.38(2H,m), 3.42-3.56(2H,m), 4.35-4.43(2H,m), 5.00-5.10(1H,br), 6.67(1H,d,J=1.98Hz), 7.40(1H,d,J=1.98Hz), 7.81-7.90(1H,br)

### Preparation Example 63

N-(4-(3-tert-butoxycarbonylaminomethylpyrrolidin-1-yl)butyl)-6-chloro-4-methyl-3,4-dihydro-2H-1,4-benzoxazine-8-carboxamide
¹H-NMR (CDCl₃,ppm) δ :1.40(9H,s),1.45-1.81(5H,m),1.90-2.07(1H,m), 2.31-2.49(2H,m),2.50-2.64(3H,m),2.65-2.78(2H,m),2.89(3H,s), 3.06-3.17(2H,m),3.34-3.41(2H,m),3.41-3.48(2H,m),4.35-4.41(2H,m), 5.03-5.10(1H,br),6.67(1H,d,J=2.64Hz),7.42(1H,d,J=2.64Hz), 7.74-7.83(1H,br)

### Preparation Example 64

N-(2-(3-tert-butoxycarbonylaminomethylpyrrolidin-1-yl)ethy)-1-methyl-1H-indole-3-carboxamide
¹H-NMR (CDCl₃,ppm) δ : 1.43(9H,s), 1.42-1.60(1H,m), 1.90-2.04(1H,m), 2.29-2.80(7H,m), 3.23-3.31(2H,m), 3.66-3.78(2H,m), 3.79(3H,s), 4.95-5.12(1H,br), 7.22-7.41(4H,m), 7.70(1H,s), 8.03-8.07(1H,m)

### Preparation Example 65

N-(3-(3-tert-butoxycarbonylaminomethylpyrrolidin-1-yl)propyl)-1-methyl-1H-indole-3-carboxamide
¹H-NMR (CDCl₃,ppm) δ : 1.42(9H,s), 1.42-1.60(1H,m), 1.80-2.04(3H,m), 2.33-2.47(2H,m), 2.66-2.90(5H,m), 3.00-3.13(2H,m), 3.54-3.65(2H,m), 3.81(3H,s), 4.76-4.87(1H,br), 7.20-7.37(4H,m), 7.70(1H,s), 8.00-8.05(1H,m)

### Preparation Example 66

N-(4-(3-tert-butoxycarbonylaminomethylpyrrolidth-1-yl)butyl)-1-methyl-1H-indole-3-carboxamide
¹H-NMR (CDCl₃,ppm) δ :1.41(9H,s),1.45-1.80(5H,m),1.91-2.05(1H,m), 2.31-2.82(7H,m),3.05-3.16(2H,m),3.45-3.56(2H,m),3.80(3H,s), 4.95-5.03(1H,br),7.20-7.36(4H,m),7.69(1H,s),7.88-7.99(1H,br)

### Preparation Example 67

N-(4-((3R)-3-tert-butoxycarbonylaminomethylpyrrolidin-1-yl)butyl)-1-methyl-1H-indole-3-carboxamide
¹H-NMR(CDCl₃,ppm) δ : 1.41(9H,s), 1.42-1.70(5H,m), 1.96-2.07(1H,m), 2.25-2.73(7H,m), 3.06-3.16(2H,m), 3.45-3.53(2H,m), 3.78(3H,s), 4.91-4.99(1H,br), 7.23-7.38(4H,m), 7.70(1H,s), 7.83-7.96(1H,br)

### Preparation Example 68

N-(4-((3S)-3-tert-butoxycarbonylaminomethylpyrrolidin-1-yl)butyl)-1-methyl-1H-indole-3-carboxamide
¹H-NMR(CDCl₃,ppm) δ :1.41(9H,s), 1.42-1.70(5H,m), 1.96-2.07(1H,m), 2.25-2.73(7H,m), 3.06-3.16(2H,m), 3.45-3.53(2H,m), 3.78(3H,s), 4.91-4.99(1H,br), 7.23-7.38(4H,m), 7.70(1H,s), 7.83-7.96(1H,br)

### Preparation Example 69

N-(5-(3-tert-butoxycarbonylaminomethylpyrrolidin-1-yl)pentyl)-1-methyl-1H-indole-3-carboxamide
¹H-NMR(CDCl₃,ppm) δ :1.15-1.70(7H,m), 1.41(9H,s), 1.90-2.10(1H,m), 2.31-2.875(7H,m), 3.01-3.11(2H,m), 3.40-3.51(2H,m), 3.81(3H,s), 4.95-5.00(1H,br), 7.15-7.31(4H,m), 7.67(1H,s), 7.84-7.95(1H,br)

### Preparation Example 70

N-(5-((3R)-3-tert-butoxycarbonylaminomethylpyrrolidin-1-yl)pentyl)-1-methyl-1H-indole-3-carboxamide
¹H-NMR(CDCl₃,ppm) δ :1.17-1.72(7H,m), 1.42(9H,s), 2.00-2.14(1H,m), 2.45-3.22(9H,m), 3.38-3.54(2H,m), 3.80(3H,s), 5.00-5.10(1H,br), 7.20-7.37(4H,m), 7.75(1H,s), 7.93-7.99(1H,br)

### Preparation Example 71

N-(5-((3S)-3-tert-butoxycarbonylaminomethylpyrrolidin-1-yl)pentyl)-1-methyl-1H-indole-3-carboxamide
¹H-NMR(CDCl₃,ppm) δ :1.17-1.72(7H,m), 1.42(9H,s), 2.00-2.14(1H,m), 2.45-3.22(9H,m), 3.38-3.54(2H,m), 3.80(3H,s), 5.00-5.10(1H,br), 7.20-7.37(4H,m), 7.75(1H,s), 7.93-7.99(1H,br)

### Preparation Example 72

N-(6-((3R)-3-tert-butoxycarbonylaminomethylpyrrolidin-1-yl)hexyl)-1-methyl-1H-indole-3-carboxamide
¹H-NMR(CDCl₃,ppm) δ : 1.17-1.62(18H,m), 1.72-1.85(1H,m), 1.95-2.12(1H,m), 2.45-2.85(4H,m), 2.95-3.20(2H,m), 3.26-3.41(2H,m), 3.47(3H,s), 3.60-3.70(2H,m), 6.45-6.60(1H,br), 7.12-7.35(4H,m), 7.85-7.92(1H,m), 8.08-8.18(1H,m)

### Preparation Example 73

N-(6-((3S)-3-tert-butoxycarbonylaminomethylpyrrolidin-1-yl)hexyl)-1-methyl-1H-indole-3-carboxamide
¹H-NMR(CDCl₃,ppm) δ : 1.17-1.62(18H,m), 1.72-1.85(1H,m), 1.95-2.12(1H,m), 2.45-2.85(4H,m), 2.95-3.20(2H,m), 3.26-3.41(2H,m), 3.47(3H,s), 3.60-3.70(2H,m), 6.45-6.60(1H,br), 7.12-7.35(4H,m), 7.85-7.92(1H,m), 8.08-8.18(1H,m)

### Preparation Example 74

N-(4-((3R)-3-tert-butoxycarbonylaminomethylpyrrolidin-1-yl)butyl)-1-methyl-1H-indole-2-carboxamide
¹H-NMR(CDCl₃,ppm) δ : 1.43(9H,s), 1.42-1.80(5H,m), 1.88-2.07(1H,m), 2.28-2.75(7H,m), 3.03-3.16(2H,m), 3.38-3.53(2H,m), 4.05(3H,s), 4.82-4.96(1H,br), 6.80-6.88(1H,br), 7.08-7.40(4H,m), 7.55-7.66(1H,m)

### Preparation Example 75

N-(4-((3R)-3-tert-butoxycarbonylaminomethylpyrrolidin-1-yl)butyl)-1-isopropyl-1H-indole-3-carboxamide
¹H-NMR(CDCl₃,ppm) δ : 1.42(9H,s), 1.55(6H,d,J=6.6Hz), 1.60-1.92(5H,m), 2.03-2.19(1H,m), 2.54-3.33(9H,m), 3.44-3.59(2H,m), 4.62-4.78(1H,m), 5.08-5.18(1H,br), 6.75-6.88(1H,br), 7.18-7.43(3H,m), 8.08-8.18(2H,m)

### Preparation Example 76

N-(4-((3R)-3-tert-butoxycarbonylaminomethylpyrrolidin-1-yl)butyl)-1-benzyl-1H-indole-3-carboxamide
¹H-NMR(CDCl₃,ppm) δ : 1.42(9H,s), 1.44-1.80(5H,m), 1.88-2.05(1H,m), 2.29-2.85(7H,m), 3.01-3.17(2H,m), 3.40-5.58(2H,m), 4.95-5.08(1H,br), 5.30(2H,s), 6.47-6.62(1H,br), 7.08-7.35(8H,m), 7.73-7.85(1H,s), 8.00-8.18(1H,m)

### Preparation Example 77

N-(4-(4-tert-butoxycarbonylaminomethylpiperidin-1-yl)butyl)-1-methyl-1H-indole-3-carboxamide
¹H-NMR(CDCl₃,ppm) δ : 1.15-1.30(3H,m), 1.44(9H,s), 1.55-1.75(6H,m), 1.83-2.10(3H,m), 2.35-2.45(2H,m), 2.88-3.05(3H,m), 3.44-3.55(2H,m), 3.82(3H,s), 4.53-4.64(1H,br), 6.14-6.25(1H,br), 7.18-7.38(3H,m), 7.65(1H,s), 7.85-7.93(1H,m)

### Preparation Example 78

N-(3-((3R)-3-tert-butoxycarbonylaminomethylpyrrolidin-1-yl)propyl)-3-phenylpropylamide
¹H-NMR(CDCl₃,ppm) δ : 1.44(9H,s), 1.46-1.53(1H,m), 1.59-1.70(2H,m), 1.90-2.04(1H,m), 2.30-2.70(9H,m), 2.90-3.00(2H,m), 3.05-3.14(2H,m), 3.25-3.36(2H,m), 4.88-4.98(1H,br), 6.75-6.84(1H,br), 7.14-7.33(5H,m)

### Preparation Example 79

N-(2-((3R)-3-tert-butoxycarbonylaminomethylpyrrolidin-1-yl)ethyl)-4-phenylbutylamide
¹H-NMR(CDCl₃,ppm) δ : 1.40-1.55(10H,m), 1.88-2.04(3H,m), 2.14-2.78(11H,m), 3.05-3.50(4H,m), 4.78-4.92(1H,br), 6.31-6.45(1H,br), 7.10-7.33(5H,m)

### Preparation Example 80

N-(5-(4-tert-butoxycarbonylaminomethylpiperidin-1-yl)pentyl)-1-methyl-1H-indole-3-carboxamide
¹H-NMR(CDCl₃,ppm) δ : 1.18-1.72(11H,m), 1.43(9H,s), 1.85-2.02(2H,m), 2.30-2.39(2H,m), 2.85-3.05(4H,m), 3.44-3.55(2H,m), 3.79(3H,s), 4.60-4.74(1H,br), 6.05-6.13(1H,br), 7.20-7.38(3H,m), 7.66(1H,s), 7.92-7.98(1H,m)

### Preparation Example 81

4-Aminomethyl-1-tert-butoxycarbonylpiperidine (10.0 g) was dissolved in dimethylformamide and 4-amino-5-chloro-2-methoxybenzoic acid (9.4 g) and 1-hydroxybenzotriazole (6.6 g) were added. After stirring at 0°C for 40 min, 1-ethyl-3-(3-dimethylaminopropyl)carbodiimide hydrochloride (9.4 g) was added, and the mixture was stirred at room temperature for 24 hr. The reaction mixture was concentrated under reduced pressure. Aqueous potassium carbonate solution was added to the residue and the mixture was extracted with ethyl acetate. The extract was dried over magnesium sulfate and concentrated under reduced pressure. The obtained residue was purified by silica gel chromatography to give 19.0 g of 4-amino-5-chloro-N-(1-tert-butoxycarbonylpiperidin-4-ylmethyl)-2-methoxybenzamide.
¹H-NMR(CDCl₃,ppm) δ : 1.05-1.26(1H,m), 1.45(9H,s), 1.65-1.89(2H,m), 2.63-2.89(2H,m), 2.88(2H,s), 2.95(2H,s), 3.28-3.38(2H,m), 3.89(3H,s), 4.05-4.20(2H,brs), 6.32(1H,s), 7.73-7.82(1H,br), 8.09(1H,s)

### Preparation Example 82

4-amino-5-chloro-N-(1-tert-butoxycarbonylpiperidin-4-ylmethyl)-2-methoxybenzamide (18.9 g) was dissolved in 4N hydrochloric acid - dioxane solution (150 ml). The mixture was stood at room temperature for 2 hr and the precipitated crystals were collected by filtration to give 15.3 g of 4-amino-5-chloro-2-methoxy-N-(piperidin-4-ylmethyl)-benzamide hydrochloride.
Melting point 208∼211°C

### Preparation Example 83

4-Amino-5-chloro-2-methoxy-N-(piperidin-4-ylmethyl)benzamide hydrochloride (15.0 g) was dissolved in dimethylformamide (100 ml) and toluene (150 ml), and potassium carbonate (18.6 g) and 4-bromobutylphthalimide (12.7 g) were added. The mixture was stirred at 70°C for 2 hr. The reaction mixture was concentrated under reduced pressure and the residue was extracted with ethyl acetate. The extract was dried over magnesium sulfate and concentrated under reduced pressure. The obtained residue was purified by silica gel chromatography to give 13.6 g of 4-amino-5-chloro-N-(1-(4-(2,3-dihydro-1,3-dioxo-1H-isoindol-2-yl)butyl)piperidin-4-ylmethyl)-2-methoxybenzamide.
¹H-NMR(CDCl₃,ppm) δ : 1.20-1.39(2H,m), 1.55-1.98(9H,m), 2.28-2.41(2H,m), 2.85-2.97(2H,m), 3.25-3.37(2H,m), 3.66-3.78(2H,m), 3.89(3H,s), 4.42(2H,s), 6.29(1H,s), 7.65-7.86(4H,m), 8.09(1H,s)

In the same manner as in Preparation Example 83, the compounds of the following Preparation Examples 84-86 were produced.

### Preparation Example 84

4-amino-5-chloro-N-(1-(3-(2,3-dihydro-1,3-dioxo-1H-isoindol-2-yl)-propyl)piperidin-4-ylmethyl)-2-methoxybenzamide
¹H-NMR (CDCl₃,ppm) δ :1.05-1.22(2H,m), 1.41-1.70(3H,m), 1.75-1.95(4H,m), 2.33-2.45(2H,m), 2.80-2.93(2H,m), 3.18-3.25(2H,m), 3.72-3.80(2H,m), 3.88(3H,s), 4.43(2H,s), 6.30(1H,s), 7.65-7.88(4H,m), 8.08(1H,s)

### Preparation Example 85

4-amino-5-chloro-N-(1-(5-(2,3-dihydro-1,3-dioxo-1H-isoindol-2-yl)-pentyl)piperidin-4-ylmethyl)-2-methoxybenzamide
¹H-NMR (CDCl₃,ppm) δ :1.20-1.98(11H,m), 2.00-2.11(2H,m), 2.30-2.40(2H,m), 2.85-2.97(2H,m), 3.25-3.37(2H,m), 3.66-3.78(2H,m), 3.89(3H,s), 4.42(2H,s), 6.29(1H,s), 7.63-7.85(4H,m), 8.09(1H,s)

### Preparation Example 86

4-amino-5-chloro-N-(1-(6-(2,3-dihydro-1,3-dioxo-1H-isoindol-2-yl)-hexyl)piperidin-4-ylmethyl)-2-methoxybenzamide
¹H-NMR (CDCl₃,ppm) δ :1.30-1.82(13H,m), 1.95-2.08(2H,m), 2.33-2.42(2H,m), 2.95-3.05(2H,m), 3.25-3.38(2H,m), 3.64-3.75(2H,m), 89(3H,s), 4.48(2H,s), 6.32(1H,s), 7.68-7.82(4H,m), 8.08(1H,s)

### Preparation Example 87

4-Amino-5-chloro-N-(1-(4-(2,3-dihydro-1,3-dioxo-1H-isoindol-2-yl)butyl)piperidin-4-ylmethyl)-2-methoxybenzamide (8.3 g) was dissolved in ethanol (100 ml) and hydrazine hydrate (1.1 ml) was added at room temperature with stirring. The mixture was refluxed with stirring for 3 hr. The precipitated crystals were filtered off and the filtrate was concentrated under reduced pressure to give 4.4 g of 4-amino-N-(1-(4-aminobutyl)piperidin-4-ylmethyl)-5-chloro-2-methoxybenzamide.
¹H-NMR(CDCl₃+CD₃OD,ppm) δ :1.25-1.41 (2H,m), 1.45-1.79(7H,m), 1.91-2.07(2H,m), 2.28-2.45(2H,m), 2.73-2.83(2H,m), 2.95-3.03(2H,m), 3.25-3.33(2H,m), 3.89(3H,s), 6.35(1H,s), 7.82-7.90(1H,br), 7.99(1H,s)

In the same manner as in Preparation Example 87, the compounds of the following Preparation Examples 88-90 were produced.

### Preparation Example 88

4-amino-N-(1 -(3-aminopropyl)piperidin-4-ylmethyl)-5-chloro-2-methoxybenzamide
¹H-NMR (CDCl₃+CD₃OD,ppm) δ :1.20-1.41(2H,m), 1.50-2.05(7H,m), 2.38-2.48(2H,m), 2.78-2.85(2H,m), 2.92-3.03(2H,m), 3.25-3.35(2H,m), 3.90(3H,s), 6.40(1H,s), 7.82-7.95(1H,br), 8.00(1H,s)

### Preparation Example 89

4-amino-N-(1-(5-aminopentyl)piperidin-4-ylmethyl)-5-chloro-2-methoxybenzamide
¹H-NMR (CDCl₃+CD₃OD,ppm) δ :1.20-1.85(11H,m), 1.90-2.07(2H,m), 2.28-2.42(2H,m), 2.66-2.78(2H,m), 2.88-3.03(2H,m), 3.25-3.42(2H,m), 3.48(3H,s), 6.36(1H,s), 7.83-7.95(1H,br), 8.01(1H,s)

### Preparation Example 90

4-amino-N-(1-(6-aminohexyl)piperidin-4-ylmethyl)-5-chloro-2-methoxybenzamide
¹H-NMR (CDCl₃+CD₃OD,ppm) δ :1.25-1.88(13H,m), 1.96-2.18(2H,m), 2.32-2.48(2H,m), 2.73-2.85(2H,m), 2.95-3.10(2H,m), 3.25-3.40(2H,m), 3.90(3H,s), 6.36(1H,s), 7.87-7.95(1H,br), 8.00(1H,s)

### Example 1

2-(3-tert-Butoxycarbonylaminomethylpyrrolidin-1-yl)ethylamine (1 g) was dissolved in methylene chloride (10 ml) and triethylamine (0.86 ml) was added. Then, a solution of acetyl chloride (0.29 ml) in methylene chlride was dropwise added under ice-cooling. The mixture was stirred at room temperature for 1 hr, and the reaction mixture was concentrated under reduced pressure to give 1-(2-acetylaminoethyl)-3-tert-butoxycarbonylaminomethylpyrrolidine.

The obtained compound was dissolved in a solution (15 ml) of 4N hydrochloric acid-dioxane and the mixture was stood at room temperature for 30 min. The reaction mixture was concentrated under reduced pressure. Dimethylformamide (15 ml) was added to the residue and the mixture was neutralized with triethylamine. 4-Amino-5-chloro-2-methoxybenzoic acid (0.83 g) and 1-hydroxybenzotriazole (0.61 g) were added, and the mixture was stirred at 0°C for 15 min. Then, 1-ethyl-3-(3-dimethylaminopropyl)carbodiimide hydrochloride (0.86 g) was added, and the mixture was stirred at room temperature for 8 hr. The reaction mixture was concentrated under reduced pressure. Aqueous potassium carbonate solution was added to the residue and the mixture was extracted with ethyl acetate. The extract was dried over magnesium sulfate and concentrated under reduced pressure. The obtained residue was purified by silica gel chromatography to give 0. 56 g of N-(1-(2-acetylaminoethyl)pyrrolidin-3-ylmethyl)-4-amino-5-chloro-2-methoxybenzamide.
¹H-NMR (CDCl₃,ppm) δ : 1.48-1.64(1H,m), 1.98(3H,s),1.94-2.17(1H,m), 2.36-2.78(7H,m), 3.18-3.62(4H,m), 3.90(3H,s), 4.44(2H,s), 6.31(1H,s), 6.30-6.44(1H,br), 7.75-7.88(1H,br), 8.08(1H,s)

### Example 2

4-(3-tert-Butoxycarbonylaminomethylpyrrolidin-1-yl)butylamine (1.05 g) as starting compound was reacted and treated in the same manner as in Example 1 using acetyl chloride (0.30 ml) and 4-amino-5-chloro-2-methoxybenzoic acid (0.78 g) to give N-(1-(4-acetylaminobutyl)pyrrolidin-3-ylmethyl)-4-amino-5-chloro-2-methoxybenzamide.
¹H-NMR (CDCl₃,ppm) δ : 1.48-1.72(5H,m),1.96(3H,s),2.02-2.14(1H,m), 2.41-2.87(7H,m),3.20-3.30(2H,m),3.35-3.57(2H,m),3.92(3H,s),4.45(2H,s), 6.31(1H,s),6.30-6.40(1H,br),7.79-7.90(1H,br),8.10(1H,s)

### Example 3

5-(3-tert-Butoxycarbonylaminomethylpyrrolidin-1-yl)pentylamine (0.99 g) as starting compound was reacted and treated in the same manner as in Example 1 using acetyl chloride (0.25 ml) and 4-amino-5-chloro-2-methoxybenzoic acid (0.71 g) to give N-(1-(5-acetylaminopentyl)pyrrolidin-3-ylmethyl)-4-amino-5-chloro-2-methoxybenzamide.
¹H-NMR (CDCl₃,ppm) δ : 1.30-2.12(8H,m),1.97(3H,s),2.35-2.76(7H,m), 3.18-3.57(4H,m),3.89(3H,s),4.39(2H,s),5.60-5.77(1H,br),6.30(1H,s), 7.76-7.90(1H,br),8.08(1H,s)

### Example 4

2-(3-tert-Butoxycarbonylaminomethylpyrrolidin-1-yl)ethylamine (1.00 g) as starting compound was reacted and treated in the same manner as in Example 1 using cyclohexanecarbonyl chloride (0.64 g) and 4-amino-5-chloro-2-methoxybenzoic acid (0.84 g) to give 4-amino-5-chloro-N-(1-(2-cyclohexanecarbonylaminoethyl)pyrrolidin-3-ylmethyl)-2-methoxybenzamide.
¹H-NMR (CDCl₃,ppm) δ : 1.04-2.18(16H,m),2.34-2.76(6H,m),3.21-3.81(2H,m), 3.88(3H,s),4.39(2H,s),6.06-6.24(1H,br),6.30(1H,s),7.72-7.90(1H,br), 8.10(1H,s)

### Example 5

3-(3-tert-Butoxycarbonylaminomethylpyrrolidin-1-yl)propylamine (0.98g) as starting compound was reacted and treated in the same manner as in Example 1 using cyclohexanecarbonyl chloride (0.54 ml) and 4-amino-5-chloro-2-methoxybenzoic acid (0.77 g) to give 4-amino-5-chloro-N-(1-(3-cyclohexanecarbonylaminopropyl)pyrrolidin-3-yl-methyl)-2-methoxybenzamide.
¹H-NMR (CDCl₃,ppm) δ : 1.07-2.15(18H,m),2.40-2.95(6H,m),3.27-3.57(2H,m), 3.90(3H,s),4.37(2H,s),6.31(1H,s),6.78-6.91(1H,br),7.74-7.90(1H,br), 8.10(1H,s)

### Example 6

4-(3-tert-Butoxycarbonylaminomethylpyrrolidin-1-yl)butylamine (1.00 g) as starting compound was reacted and treated in the same manner as in Example 1 using cyclohexanecarbonyl chloride (0.49 ml) and 4-amino-5-chloro-2-methoxybenzoic acid (0.74 g) to give 4-amino-5-chloro-N-(1-(4-cyclohexanecarbonylaminobutyl)pyrrolidin-3-ylmethyl)-2-methoxybenzamide.
¹H-NMR (CDCl₃,ppm) δ : 1.10-1.89(18H,m),1.97-2.12(2H,m),2.41-2.81(6H,m), 3.18-3.31(2H,m),3.38-3.62(2H,m),3.93(3H,s),4.41(2H,s),5.97-6.08(1H,br), 6.31(1H,s),7.79-7.88(1H,br),8.12(1H,s)

### Example 7

5-((3R)-3-tert-Butoxycarbonylaminomethylpyrrolidin-1-yl)pentylamine (1 .00 g) as starting compound was reacted and treated in the same manner as in Example 1 using cyclohexanecarbonyl chloride (0.52 ml) and 4-amino-5-chloro-2-methoxybenzoic acid (0.70 g) to give 4-amino-5-chloro-N-((3R)-1-(5-cyclohexanecarbonylaminopentyl)pyrrolidin-3-ylmethyl)-2-methoxybenzamide.
¹H-NMR (CDCl₃,ppm) δ : 1.15-2.15(18H,m), 2.08-2.75(7H,m), 3.15-3.27(2H,m), 3.31-3.50(2H,m), 3.89(3H,s), 4.39(2H,s), 5.48-5.59(1H,br), 6.30(1H,s), 7.77-7.89(1H,br), 8.08(1H,s)

### Example 8

4-((3R)-3-tert-Butoxycarbonylaminomethylpyrrolidin-1-yl)butylamine (1.20 g) as starting compound was reacted and treated in the same manner as in Example 1 using 1-adamantanecarbonyl chloride (0.98 g) and 4-amino-5-chloro-2-methoxybenzoic acid (0.85 g) to give N-((3R)-1-(4-(1-adamantanecarbonylamino)butyl)pyrrolidin-3-ylmethyl)-4-amino-5-chloro-2-methoxybenzamide.
¹H-NMR (CDCl₃,ppm) δ : 1.42-2.10(21H,m), 2.30-2.81(7H,m), 3.17-3.30(2H,m), 3.36-3.48(2H,m), 3.89(3H,s), 4.38(2H,s), 5.75-5.84(1H,br), 6.30(1H,s), 7.76-7.87(1H,br), 8.09(1H,s)

### Example 9

2-(3-tert-Butoxycarbonylaminomethylpyrrolidin-1-yl)ethylamine (1 g) as starting compound was reacted and treated in the same manner as in Example 1 using benzoyl chloride (0.47 ml) and 4-amino-5-chloro-2-methoxybenzoic acid (0.83 g) to give 4-amino-N-(1-(2-benzoylaminoethyl)pyrrolidin-3-ylmethyl)-5-chloro-2-methoxybenzamide.
¹H-NMR (CDCl₃,ppm) δ : 1.48-1.68(1H,m),1.94-2.10(1H,m), 2.38-2.86(7H,m), 3.32-3.70(4H,m), 3.84(3H,s), 4.41(2H,s), 6.22(1H,s), 6.92-7.06(1H,br), 7.32-7.52(3H,m), 7.73-7.91(3H,m), 8.08(1H,s)

### Example 10

3-(3-tert-Butoxycarbonylaminomethylpyrrolidin-1-yl)propylamine (1.1 g) as starting compound was reacted and treated in the same manner as in Example 1 using benzoyl chloride (0.50 ml) and 4-amino-5-chloro-2-methoxybenzoic acid (0.15 g) to give 4-amino-N-(1 -(3-benzoylaminopropyl)pyrrolidin-3-ylmethyl)-5-chloro-2-methoxybenzamide.
¹H-NMR (CDCl₃,ppm) δ : 1.61-1.74(1H,m), 1.86-1.92(2H,m), 2.06-2.16(1H,m), 2.57-2.70(2H,m), 2.83-3.10(5H,m), 3.37-3.48(2H,m), 3.50-3.64(2H,m), 3.86(3H,s), 4.44(2H,s), 6.28(1H,s), 7.35-7.50(3H,m), 7.77-7.85(3H,m), 8.06(1H,s), 8.12-8.20(1H,br)

### Example 11

3-((3R)-3-tert-Butoxycarbonylaminomethylpyrrolidin-1-yl)propylamine (1 .50 g) was dissolved in ethylene chloride (30 ml) and triethylamine (1.2 ml) was added. Then, a soution of benzoyl chloride (0.68 ml) in methylene chloride was dropwise added under ice-cooling. The mixture was stirred at room temperature for 1 hr, and the reaction mixture was concentrated under reduced pressure. The residue was dissolved in 4N hydrochloric acid-dioxane solution (10 ml) and the mixture was stood at room temperature for 30 min. The reaction mixture was concentrated under reduced pressure. Dimethylformamide (10 ml) was added to the residue and the mixture was neutralized with triethylamine (1.7 ml). 4-Amino-5-chloro-2-methoxybenzoic acid (0.80 g) and benzotriazol-1-yloxytris(dimethylamino)phosphonium hexafluorophosphate (1.93 g) were added, and the mixture was stirred at room temperature for 8 hr. The reaction mixture was concentrated under reduced pressure. Aqueous potassium carbonate solution was added to the residue and the mixture was extracted with ethyl acetate. The extract was dried over magnesium sulfate and concentrated under reduced pressure. The obtained residue was purified by silica gel chromatography to give 0.12 g of 4-amino-N-((3R)-1-(3-benzoylaminopropyl)pyrrolidin-3-ylmethyl)-5-chloro-2-methoxybenzamide.
¹H-NMR (CDCl₃,ppm) δ : 1.45-1.62(1H,m), 1.72-1.84(2H,m), 1.94-2.08(1H,m), 2.30-2.85(7H,m), 3.34-3.48(2H,m), 3.50-3.63(2H,m), 3.86(3H,s), 4.45(2H,s), 6.29(1H,s), 7.35-7.50(3H,m), 7.65-7.83(3H,m), 8.08(1H,s), 8.17-8.28(1H,br)

### Example 12

3-((3S)-3-tert-Butoxycarbonylaminomethylpyrrolidin-1-yl)propylamine (1.50 g) as starting compound was reacted and treated in the same manner as in Example 1 using benzoyl chloride (0.68 ml) and 4-amino-5-chloro-2-methoxybenzoic acid (1.17 g) to give 4-amino-N-((3S)-1-(3-benzoylaminopropyl)pyrrolidin-3-ylmethyl)-5-chloro-2-methoxybenzamide.
¹H-NMR (CDCl₃,ppm) δ : 1.45-1.62(1H,m), 1.72-1.84(2H,m), 1.94-2.08(1H,m), 2.30-2.85(7H,m), 3.34-3.48(2H,m), 3.50-3.63(2H,m), 3.86(3H,s), 4.45(2H,s), 6.29(1H,s), 7.35-7.50(3H,m), 7.65-7.83(3H,m), 8.08(1H,s), 8.17-8.28(1H,br)

### Example 13

3-(3-tert-Butoxycarbonylaminomethylpyrrolidin-1-yl)propylamine (1.00 g) as starting compound was reacted and treated in the same manner as in Example 1 using 4-chlorobenzoyl chloride (0.54 ml) and 4-amino-5-chloro-2-methoxybenzoic acid (0.86 g) to give 4-amino-5-chloro-N-(1-(3-(4-chlorobenzoylamino)propyl)pyrrolidin-3-ylmethyl)-2-methoxybenzamide.
¹H-NMR (CDCl₃,ppm) δ : 1.51-2.17(4H,m), 2.36-2.95(7H,m), 3.30-3.65(4H,m), 3.90(3H,s), 4.40(2H,s), 6.30(1H,s), 7.31-7.44(2H,m), 7.60-7.65(1H,br), 7.70-7.80(2H,m), 8.08(1H,s), 8.35-8.45(1H,br)

### Example 14

3-(3-tert-Butoxycarbonylaminomethylpyrrolidin-1-yl)propylamine (1 .00 g) was dissolved in methylene chloride (20 ml) and triethylamine (0.81 ml) was added. Then, a solution (10 ml) of 3-chlorobenzoyl chloride (0.75 g) in methylene chlride was dropwise added under ice-cooling. The mixture was stirred at room temperature for 3 hr, and the reaction mixture was concentrated under reduced pressure. The residue was dissolved in 4N hydrochloric acid-dioxane solution (20 ml) and the mixture was stood at room temperature for 30 min. The reaction mixture was concentrated under reduced pressure. Dimethylformamide (20 ml) was added to the residue and the mixture was neutralized with triethylamine. 4-Amino-5-chloro-2-methoxybenzoic acid (0.86 g) and benzotriazol-1-yloxytris(dimethylamino)phosphonium hexafluorophosphate (1.89 g) were added, and the mixture was stirred at room temperature for 22 hr. The reaction mixture was concentrated under reduced pressure. Aqueous potassium carbonate solution was added to the residue and the mixture was extracted with chloroform. The extract was dried over magnesium sulfate and concentrated under reduced pressure. The obtained residue was purified by silica gel chromatography to give 0.28 g of 4-amino-5-chloro-N-(1-(3-(3-chlorobenzoylamino)propyl)pyrrolidin-3-ylmethyl)-2-methoxybenzamide.
¹H-NMR (CDCl₃,ppm) δ : 1.55-1.69(1H,m), 1.70-1.85(2H,m), 1.95-2.12(1H,m), 2.25-2.35(1H,m), 2.43-2.95(6H,m), 3.30-3.55(4H,m), 3.88(3H,s), 4.39(2H,s), 6.28(1H,s), 7.32-7.48(3H,m), 7.55-7.60(1H,m), 7.61-7.75(1H,br), 8.06(1H,s)

### Example 15

3-(3-tert-Butoxycarbonylaminomethylpyrrolidin-1-yl)propylamine (1.00 g) was dissolved in methylene chloride (20 ml) and triethylamine (0.81 ml) was added. Then, a solution (10 ml) of 2-chlorobenzoyl chloride (0.75 g) in methylene chlride was dropwise added under ice-cooling. The mixture was stirred at room temperature for 6 hr, and the reaction mixture was concentrated under reduced pressure. The residue was dissolved in 4N hydrochloric acid-dioxane solution (20 ml) and the mixture was stood at room temperature for 2 hr. The reaction mixture was concentrated under reduced pressure. Dimethylformamide (20 ml) was added to the residue and the mixture was neutralized with triethylamine. 4-Amino-5-chloro-2-methoxybenzoic acid (0.86 g) and benzotriazol-1-yloxytris(dimethylamino)phosphonium hexafluorophosphate (1.89 g) were added, and the mixture was stirred at room temperature for 17 hr. The reaction mixture was concentrated under reduced pressure. Aqueous potassium carbonate solution was added to the residue and the mixture was extracted with chloroform. The extract was dried over magnesium sulfate and concentrated under reduced pressure. The obtained residue was purified by silica gel chromatography to give 0.25 g of 4-amino-5-chloro-N-(1-(3-(2-chlorobenzoylamino)propyl)pyrrolidin-3-ylmethyl)-2-methoxybenzamide.
¹H-NMR (CDCl₃,ppm) δ : 1.35-1.50(1H,m), 1.73-1.98(3H,m), 2.30-2.72(7H,m), 3.18-3.25(2H,m), 3.48-3.60(2H,m), 3.89(3H,s), 4.39(2H,s), 6.29(1H,s), 7.22-7.40(2H,m), 7.69-7.85(3H,m), 8.09(1H,s), 8.66-8.79(1H,br)

### Example 16

3-(3-tert-Butoxycarbonylaminomethylpyrrolidin-1-yl)propylamine (1.10 g) was dissolved in methylene chloride (10 ml) and triethylamine (0.89 ml) was added. Then, a solution of 4-nitrobenzoyl chloride (0.80 g) in methylene chlride was dropwise added under ice-cooling. The mixture was stirred at room temperature for 5 hr, and the reaction mixture was concentrated under reduced pressure. The residue was dissolved in 4N hydrochloric acid-dioxane solution (10 ml) and the mixture was stood at room temperature for 30 min. The reaction mixture was concentrated under reduced pressure. Dimethylformamide (10 ml) was added to the residue and the mixture was neutralized with triethylamine (0.92 ml). 4-Amino-5-chloro-2-methoxybenzoic acid (0.44 g) and benzotriazol-1-yloxytris(dimethylamino)phosphonium hexafluorophosphate (1.06 g) were added, and the mixture was stirred at room temperature for 8 hr. The reaction mixture was concentrated under reduced pressure. Aqueous potassium carbonate solution was added to the residue and the mixture was extracted with ethyl acetate. The extract was dried over magnesium sulfate and concentrated under reduced pressure. The obtained residue was purified by silica gel chromatography to give 0. 29 g of 4-amino-5-chloro-2-methoxy-N-(1 -(3-(4-nitrobenzoylamino)propyl)pyrrolidin-3-ylmethyl)benzamide.
¹H-NMR (CDCl₃+CD₃OD,ppm) δ : 1.60-1.79(1H,m), 1.83-1.99(2H,m), 2.05-2.20(1H,m), 2.53-3.08(5H,m), 3.17-3.57(6H,m), 3.91(3H,s), 4.37(2H,s), 6.35(1H,s), 7.94-8.03(3H,m), 8.25-8.32(2H,m)

### Example 17

3-(3-tert-Butoxycarbonylaminomethylpyrrolidin-1-yl)propylamine (1.08 g) as starting compound was reacted and treated in the same manner as in Example 1 using 4-methylbenzoyl chloride (0.56 ml) and 4-amino-5-chloro-2-methoxybenzoic acid (0.48 g) to give 4-amino-5-chloro-2-methoxy-N-(1-(3-(4-methylbenzoylamino)propyl)pyrrolidin-3-ylmethyl)benzamide.
¹H-NMR (CDCl₃+CD₃OD,ppm) δ : 1.82-2.07(3H,m), 2.20-2.32(1H,m), 2.39(3H,s), 2.78-3.90(11H,m), 3.92(3H,s), 6.37(1H,s), 7.20-7.28(2H,m), 7.65-7.72(2H,m), 7.96(1H,s)

### Example 18

3-(3-tert-Butoxycarbonylaminomethylpyrrolidin-1-yl)propylamine (1.03 g) as starting compound was reacted and treated in the same manner as in Example 1 using 4-methoxylbenzoyl chloride (0.68 ml) and 4-amino-5-chloro-2-methoxybenzoic acid (0.41 g) to give 4-amino-5-chloro-2-methoxy-N-(1-(3-(4-methoxybenzoylamino)propyl)pyrrolidin-3-ylmethyl)benzamide.
¹H-NMR (CDCl₃+CD₃OD,ppm) δ : 1.82-2.07(3H,m), 2.20-2.32(1H,m), 2.39(3H,s), 2.78-3.90(11H,m), 3.92(3H,s), 6.37(1H,s), 7.20-7.28(2H,m), 7.65-7.72(2H,m), 7.96(1H,s)

### Example 19

3-(3-tert-Butoxycarbonylaminomethylpyrrolidin-1-yl)propylamine (1.05 g) as starting compound was reacted and treated in the same manner as in Example 1 using 2-thiophenecarbonyl chloride (0.48 ml) and 4-amino-5-chloro-2-methoxybenzoic acid (0.82 g) to give 4-amino-5-chloro-2-methoxy-N-(1-(3-(2-thiophenecarbonylamino)propyl)pyrrolidin-3-ylmethyl)benzamide.
¹H-NMR (CDCl₃,ppm) δ : 1.56-1.72(1H,m), 1.75-1.91 (2H,m), 2.00-2.15(1H,m), 2.47-3.05(7H,m), 3.40-3.61(4H,m), 3.86(3H,s), 4.45(2H,brs), 6.30(1H,s), 7.02-7.08(1H,m), 7.40-7.48(1H,m), 7.53-7.64(1H,m), 7.78-7.85(1H,br), 8.08(1H,s),8.10-8.18(1H,br)

### Example 20

4-(3-tert-Butoxycarbonylaminomethylpyrrolidin-1-yl)butylamine (1 g) as starting compound was reacted and treated in the same manner as in Example 1 using benzoyl chloride (0.43 ml) and 4-amino-5-chloro-2-methoxybenzoic acid (0.74 g) to give 4-amino-N-(1-(4-benzoylaminobutyl)pyrrolidin-3-ylmethyl)-5-chloro-2-methoxybenzamide.
¹H-NMR (CDCl₃,ppm) δ : 1.45-1.75(5H,m),1.90-2.13(1H,m),2.37-2.76(7H,m), 3.33-3.52(4H,m),3.85(3H,s),4.38(2H,brs),6.27(1H,s), 7.12(1H,br), 7.31-7.50(3H,m),7.71-7.86(3H,m),8.08(1H,s)

### Example 21

4-Amino-N-(1-(4-aminobutyl)piperidin-4-ylmethyl)-5-chloro-2-methoxybenzamide (0.8 g) was dissolved in dimethylformamide (10 ml), and benzoic acid (0.25 g) and 1-ethyl-3-(3-dimethylaminopropyl)carbodiimide hydrochloride (0. 44 g) web added. The mixture was stirred at room temperature for 12hr. The reaction mixture was concentrated under reduced pressure. Aqueous potassium carbonate solution was added to the residue and the mixture was extracted with ethyl acetate. The extract was dried over magnesium sulfate and concentrated under reduced pressure. The obtained residue was purified by silica gel chromatography to give 0. 2 g of 4-amino-N-(1-(4-benzoylaminobutyl)piperidin-4-ylmethyl)-5-chloro-2-methoxybenzamide.
Melting point 165∼168°C

### Example 22

4-((3R)-3-tert-Butoxycarbonylaminomethylpyrrolidin-1-yl)butylamine (1.00 g) was dissolved in methylene chloride (20 ml) and potassium carbonate (0.56 g) was added. Then, a solution of 1 - naphthoyl chloride (0.6 g) in methylene chloride was dropwise added under ice-cooling. The mixture was stirred at room temperature for 1 hr, and the reaction mixture was concentrated under reduced pressure. The residue was dissolved in 4N hydrochloric acid-dioxane solution (10 ml) and the mixture was stood at room temperature for 30 min. The reaction mixture was concentrated under reduced pressure. Dimethylformamide (10 ml) was added to the residue and the mixture was neutralized with triethylamine. 4-Amino-5-chloro-2-methoxybenzoic acid (0.39 g) and 1-hydroxybenzotriazole (0.27 g) were added, and the mixture was stirred at 0°C for 15 min. Then, 1-ethyl-3-(3-dimethylaminopropyl)carbodiimide hydrochloride (0.38 g) was added and the mixture was stirred at room temperature for 8 hr. The reaction mixture was concentrated under reduced pressure. Aqueous potassium carbonate solution was added to the residue and the mixture was extracted with ethyl acetate. The extract was dried over magnesium sulfate and concentrated under reduced pressure. The obtained residue was purified by silica gel chromatography to give 0.50 g of 4-amino-5-chloro-2-methoxy-N-((3R)-1-(4-(1-naphthoylamino)butyl)pyrrolidin-3-ylmethyl)benzamide.
¹H-NMR (CDCl₃,ppm) δ : 1.25-1.39(1H,m), 1.57-1.78(4H,m), 2.11-2.34(2H,m), 2.42-2.69(6H,m), 3.13-3.33(2H,m), 3.39-3.52(2H,m), 3.79(3H,s), 4.54(2H,s), 6.26(1H,s), 7.33-7.89(8H,m), 8.01(1H,s), 8.20-8.27(1H,br)

### Example 23

4-((3R)-3-tert-Butoxycarbonylaminomethylpyrrolidin-1-yl)butylamine (1.05 g) was dissolved in methylene chloride (30 ml) and triethylamine (0.81 ml) was added. Then, 2-naphthoyl chloride (0.74 g) was added under ice-cooling. The mixture was stirred at room temperature for 5 hr, and the reaction mixture was concentrated under reduced pressure. The residue was dissolved in 4N hydrochloric acid-dioxane solution (10 ml) and the mixture was stood at room temperature for 30 min. The reaction mixture was concentrated under reduced pressure. Dimethylformamide (30 ml) was added to the residue and the mixture was neutralized with triethylamine (0.66 ml). 4-Amino-5-chloro-2-methoxybenzoic acid (0.32 g) and 1-hydroxybenzotriazole (0.23 g) were added, and the mixture was stirred at 0°C for 15 min. Then, 1-ethyl-3-(3-dimethylaminopropyl)carbodiimide hydrochloride (0.33 g) was added and the mixture was stirred at room temperature for 8 hr. The reaction mixture was concentrated under reduced pressure. Aqueous potassium carbonate solution was added to the residue and the mixture was extracted with ethyl acetate. The extract was dried over magnesium sulfate and concentrated under reduced pressure. The obtained residue was purified by silica gel chromatography to give 0.25 g of 4-amino-5-chloro-2-methoxy-N-((3R)-1-(4-(2-naphthoylamino)butyl)pyrrolidin-3-ylmethyl)benzamide.
¹H-NMR (CDCl₃,ppm) δ : 1.50-1.85(5H,m), 1.90-2.28(2H,m), 2.35-2.85(6H,m), 3.33-3.62(4H,m), 3.82(3H,s), 4.34(2H,s), 6.23(1H,s), 7.25-7.36(1H,br), 7.40-7.60(2H,m), 7.71-7.93(5H,m), 8.06(1H,s), 8.25-8.34(1H,m)

### Example 24

4-Amino-N-(1-(4-aminobutyl)piperidin-4-ylmethyl)-5-chloro-2-methoxybenzamide (0.80 g) was dissolved in dichloromethane (30 ml), and potassium carbonate (0.60 g) was added. Then, a solution of 1-naphthoyl chloride (0.33 ml) in methylene chloride was dropwise added under ice-cooling. The mixture was stirred at room temperature for 1 hr, and the insoluble matter was filtered off. The filtrate was concentrated under reduced pressure. The obtained residue was purified by silica gel chromatography to give 0.45 g of 4-amino-5-chloro-2-methoxy-N-(1-(4-(1-naphthoylamino)butyl)piperidin-4-ylmethyl)benzamide.
Melting point 138∼141°C (0.5 fumarate)

### Example 25

4-Amino-N(1-(4-aminobutyl)piperidin-4-ylmethyl)-5-chloro-2-methoxybenzamide (1.10 g) was dissolved in dichloromethane (30 ml), and potassium carbonate (0.82 g) was added. Then, a solution of 2-naphthoyl chloride (0.33 ml) in methylene chloride was dropwise added under ice-cooling. The mixture was stirred at room temperature for 1 hr, and the insoluble matter was filtered off. The filtrate was concentrated under reduced pressure. The obtained residue was purified by silica gel chromatography to give 0.25 g of 4-amino-5-chloro-2-methoxy-N-(1-(4-(2-naphthoylamino)butyl)piperidin-4-ylmethyl) benzamide.
Melting point 179∼181°C

### Example 26

4-((3R)-3-tert-Butoxycarbonylaminomethylpyrrolidin-1-yl)butylamine (1.00 g) as starting compound was reacted and treated in the same manner as in Example 1 using phenylacetyl chloride (0.50 ml) and 4-mino-5-chloro-2-methoxybenzoic acid (0.54 g) to give 4-amino-5-chloro-2-methoxy-N-((3R)-1-(4-phenylacetylminobutyl)pyrrolidin-3-ylmethyl)benzamide.
¹H-NMR (CDCl₃,ppm) δ : 1.33-1.82(5H,m), 1.88-2.05(1H,m), 2.25-2.70(7H,m), 3.14-3.69(2H,m), 3.51(2H,s), 3.81(3H,s), 4.81(2H,s), 6.35(1H,s), 6.55-6.66(1H,br), 7.14-7.35(5H,m), 7.80-7.95(1H,br), 8.02(1H,s)

### Example 27

5-((3R)-3-tert-Butoxycarbonylaminomethylpyrrolidin-1-yl)pentylamine (1.15 g) was dissolved in methylene chloride (10 ml) and triethylamine (0.84 ml) was added. Then, a solution of benzoyl chloride (0.47 ml) in methylene chloride was dropwise added under ice-cooling. The mixture was stirred at room temperature for 1 hr, and the reaction mixture was concentrated under reduced pressure. The residue was dissolved in 4N hydrochloric acid-dioxane solution (10 ml) and the mixture was stood at room temperature for 30 min. The reaction mixture was concentrated under reduced pressure. Dimethylformamide (30 ml) was added to the residue and the mixture was neutralized with triethylamine (1.1 ml). 4-Amino-5-chloro-2-methoxybenzoic acid (0.55 g) and 1-hydroxybenzotriazole (0.40 g) were added, and the mixture was stirred at 0°C for 15 min. Then, 1-ethyl-3-(3-dimethylaminopropyl)carbodiimide hydrochloride (0.57 g) was added and the mixture was stirred at room temperature for 8 hr. The reaction mixture was concentrated under reduced pressure. Aqueous potassium carbonate solution was added to the residue and the mixture was extracted with ethyl acetate. The extract was dried over magnesium sulfate and concentrated under reduced pressure. The obtained residue was purified by silica gel chromatography to give 0.48 g of 4-amino-N-((3R)-1-(5-benzoylaminopentyl)pyrrolidin-3-yl-methyl)-5-chloro-2-methoxybenzamide.
¹H-NMR (CDCl₃,ppm) δ : 1.35-1.69(7H,m), 1.97-2.13(1H,m), 2.47-2.88(7H,m), 3.35-3.50(4H,m), 3.88(3H,s), 4.47(2H,s), 6.31(1H,s), 6.45-6.55(1H,br), 7.35-7.52(3H,m), 7.75-7.89(3H,m), 8.06(1H,s)

### Example 28

5-((3R)-3-tert-Butoxycarbonylaminomethylpyrrolidin-1-yl)pentylamine (1.51 g) was dissolved in methylene chloride (15 ml) and triethylamine (1.11 ml) was added. Then, a solution of 4-chlorobenzoyl chloride (0.67 ml) in ethylene chloride was dropwise added under ice-cooling. The mixture was stirred at room temperature for 5 hr, and the reaction mixture was concentrated under reduced pressure. The residue was dissolved in 4N hydrochloric acid-dioxane solution (10 ml) and the mixture was stood at room temperature for 30 min. The reaction mixture was concentrated under reduced pressure. Dimethylformamide (30 ml) was added to the residue and the mixture was neutralized with triethylamine (2.22 ml). 4-Amino-5-chloro-2-methoxybenzoic acid (1.07 g) and 1-hydroxybenzotriazole (0.79 g) were added, and the mixture was stirred at 0°C for 15 min. Then, 1-ethyl-3-(3-dimethylaminopropyl)carbodiimide hydrochloride (1.12 g) was added and the mixture was stirred at room temperature for 8 hr. The reaction mixture was concentrated under reduced pressure. Aqueous potassium carbonate solution was added to the residue and the mixture was extracted with ethyl acetate. The extract was dried over magnesium sulfate and concentrated under reduced pressure. The obtained residue was purified by silica gel chromatography to give 1.39 g of 4-amino-5-chloro-N-((3R)-1-(5-(4-chlorobenzoylamino)pentyl)pyrrolidin-3-ylmethyl)-2-methoxybenzamide.
¹H-NMR (CDCl₃,ppm) δ : 1.35-1.69(7H,m), 1.97-2.12(1H,m), 2.45-2.83(7H,m), 3.33-3.52(4H,m), 3.88(3H,s), 4.44(2H,s), 6.30(1H,s), 6.55-6.70(1H,br), 7.35-7.43(2H,m), 7.73-7.79(2H,m), 7.80-7.89(1H,br), 8.05(1H,s)

### Example 29

5-(4-tert-Butoxycarbonylaminomethylpiperidin-1-yl)pentylamine (1.2 g) was dissolved in methylene chloride (20 ml) and potassium carbonate (1.66 g) was added. Then, a solution of benzoyl chloride (0.47 ml) in methylene chloride was dropwise added under ice-cooling. The mixture was stirred at room temperature for 1 hr, and the reaction mixture was concentrated under reduced pressure. The residue was dissolved in 4N hydrochloric acid-dioxane solution (30 ml) and the mixture was stood at room temperature for 30 min. The reaction mixture was concentrated under reduced pressure. Dimethylformamide (30 ml) was added to the residue and the mixture was neutralized with triethylamine (1.1 ml). 4-Amino-5-chloro-2-methoxybenzoic acid (0.81 g) and 1-hydroxybonzotriazole (0.60 g) were added, and the mixture was stirred at 0°C for 20 min. Then, 1-ethyl-3-(3-dimethylaminopropyl)-carbodiimide hydrochloride (0.85 g) was added and the mixture was stirred at room temperature for 8 hr. The reaction mixture was concentrated under reduced pressure. Aqueous potassium carbonate solution was added to the residue and the mixture was extracted with ethyl acetate. The extract was dried over magnesium sulfate and concentrated under reduced pressure. The obtained residue was purified by silica gel chromatography to give 0.68 g of 4-amino-N-(1-(5-benzoylaminopentyl)piperidin-4-ylmethyl)-5-chloro-2-methoxybenzamide.
Melting point 138∼140°C

### Example 30

5-(4-tert-Butoxycarbonylaminomethylpiperidin-1-yl)pentylamine (1.50 g) was dissolved in methylene chloride (30 ml) and triethylamine (1.0 ml) was added. Then, a solution (20 ml) of 3-chlorobenzoyl chloride (0.75 ml) in methylene chloride was dropwise added under ice-cooling. The mixture was stirred at room temperature for 30 hr, and the reaction mixture was concentrated under reduced pressure. The residue was dissolved in 4N hydrochloric acid-isopropyl alcohol solution (80 ml) and the mixture was stood at room temperature for 24 hr. The reaction mixture was concentrated under reduced pressure. Dimethylformamide (40 ml) was added to the residue and the mixture was neutralized with triethylamine (2.30 ml). 4-Amino-5-chloro-2-methoxybenzoic acid (1.01 g) and 1-hydroxybenzotriazole (0.75 g) were added, and the mixture was stirred at 0°C for 15 min. Then, 1-ethyl-3-(3-dimethylaminopropyl)carbodiimide hydrochloride (1.06 g) was added and the mixture was stirred at room temperature for 20 hr. The reaction mixture was concentrated under reduced pressure. Aqueous potassium carbonate solution was added to the residue and the mixture was extracted with chloroform. The extract was dried over magnesium sulfate and concentrated under reduced pressure. The obtained residue was purified by silica gel chromatography to give 0.65 g of 4-amino-5-chloro-N-(1-(5-(3-chlorobenzoylamino)pentyl)piperidin-4-ylmethyl)-2-methoxybenzamide.
Melting point 139∼142°C

### Example 31

5-(4-tert-Butoxycarbonylaminomethylpiperidin-1-yl)pentylamine (1.50 g) was dissolved in methylene chloride (30 ml) and triethylamine (1.04 ml) was added. Then, a solution (20 ml) of 4-methylbenzoyl chloride (0.73 ml) in methylene chloride was dropwise added under ice-cooling. The mixture was stirred at room temperature for 27 hr, and the reaction mixture was concentrated under reduced pressure. The residue was dissolved in 4N hydrochloric acid-isopropyl alcohol solution (80 ml) and the mixture was stood at room temperature for 24 hr. The reaction mixture was concentrated under reduced pressure. Dimethylformamide (40 ml) was added to the residue and the mixture was neutralized with triethylamine (2.30 ml). 4-Amino-5-chloro-2-methoxybenzoic acid (1.01 g) and 1-hydroxybenzotriazole (0.75 g) were added, and the mixture was stirred at 0°C for 15 min. Then, 1-ethyl-3-(3-dimethylaminopropyl)carbodiimide hydrochloride (1.06 g) was added and the mixture was stirred at room temperature for 24 hr. The reaction mixture was concentrated under reduced pressure. Aqueous potassium carbonate solution was added to the residue and the mixture was extracted with chloroform. The extract was dried over magnesium sulfate and concentrated under reduced pressure. The obtained residue was purified by silica gel chromatography to give 0. 55 g of 4-amino-5-chloro-N-(1-(5-(4-methylbenzoylamino)pentyl)piperidin-4-ylmethyl)-2-methoxybenzamide.
Melting point 142∼146°C

### Example 32

5-((3R)-3-tert-Butoxycarbonylaminomethylpyrrolidin-1-yl)pentylamine (1.00 g) as starting compound was reacted and treated in the same manner as in Example 1 using 4-benzenesulfonyl chloride (0.49 ml) and 4-amino-5-chloro-2-methoxybenzoic acid (0.70 g) to give 4-amino-N-((3R)-1-(5-benzenesulfonylaminopentyl)pyrrolidin-3-ylmethyl)-5-chloro-2-methoxybenzamide.
¹H-NMR (CDCl₃,ppm) δ : 1.22-1.63(7H,m), 1.95-2.10(1H,m), 2.30-2.70(7H,m), 2.88-2.98(2H,m), 3.31-3.54(2H,m), 3.89(3H,s), 4.38(2H,s), 5.08-5.20(1H,br), 6.30(1H,s), 7.45-7.60(3H,m), 7.79-7.89(3H,m), 8.09(1H,s)

### Example 33

4-((3R)-3-tert-Butoxycarbonylaminomethylpyrrolidin-1-yl)butylamine (1.00 g) as starting compound was reacted and treated in the same manner as in Example 1 using 1-morpholinecarbonyl chloride (0.48 ml) and 4-amino-5-chloro-2-methoxybenzoic acid (0.74 g) to give 4-amino-5-chloro-N-((3R)-1-(4-(1-morpholine)carbonylaminobutyl)pyrrolidin-3-ylmethyl)-2-methoxybenzamide.
¹H-NMR (CDCl₃,ppm) δ : 1.45-1.79(5H,m), 1.90-2.06(1H,m), 2.28-2.72(7H,m), 3.20-3.48(8H,m), 3.60-3.75(4H,m), 3.89(3H,s), 4.37(2H,s), 4.88-4.97(1H,br), 6.29(1H,s), 7.73-7.85(1H,br), 8.09(1H,s)

### Example 34

2-(3-tert-Butoxycarbonylaminomethylpyrrolidin-1-yl)ethylamine (1 g) was dissolved in methylene chloride (10 ml) and a solution of methyl isocyanate (0.24 ml) in methylene chloride was dropwise added under ice-cooling. The mixture was stirred at room temperature for 1 hr, and the reaction mixture was concentrated under reduced pressure to give 1-(2-(3-methylureido)ethyl)-3-tert-butoxycarbonylaminomethylpyrrolidine.

This was dissolved in 4N hydrochloric acid-diode solution and the mixture was stood at room temperature for 30 min. The reaction mixture was concentrated under reduced pressure. Dimethylformamide was added to the residue and the mixture was neutralized with triethylamine. 4-Amino-5-chloro-2-methoxybenzoic acid (0.83 g) and 1-hydroxybenzotriazole (0.61 g) were added thereto, and the mixture was stirred at 0°C for 15 min. Then, 1-ethyl-3-(3-dimethylaminopropyl)carbodiimide hydrochloride (0.86 g) was added and the mixture was stirred at room temperature for 8 hr. The reaction mixture was concentrated under reduced pressure. Aqueous potassium carbonate solution was added to the residue and the mixture was extracted with ethyl acetate. The extract was dried over magnesium sulfate and concentrated under reduced pressure. The obtained residue was purified by silica gel chromatography to give 0.51 g of 4-amino-5-chloro-2-methoxy-N-(1-(2-(3-methylureido)ethyl)pyrrolidin-3-yl-methyl)benzamide.
¹H-NMR (CDCl₃,ppm) δ : 1.45-1.62(1H,m),1.92-2.08(1H,m), 2.27(3H,s), 2.36-2.92(7H,m), 3.12-3.45(4H,m), 3.91(3H,s), 4.51(2H,s), 5.20-5.32(1H,br), 5.42-5.57(1H,br), 6.33(1H,s), 7.79-7.91(1H,br), 8.02(1H,s)

### Example 35

3-(3-tert-Butoxycarbonylaminomethylpyrrolidin-1-yl)propylamine (1.12 g) as starting compound was reacted and treated in the same manner as in Example 34 using methyl isocyanate (0.38 ml) and 4-amino-5-chloro-2-methoxybenzoic acid (0.96 g) to give 4-amino-5-chloro-2-methoxy-N-(1-(3-(3-methylureido)propyl)pyrrolidin-3-ylmethyl)benzamide.
¹H-NMR (CDCl₃+CD₃OD,ppm) δ : 1.80-2.00(3H,m),2.17-2.38(1H,m), 2.70(3H,s), 2.91-3.80(11H,m), 3.93(3H,s), 6.38(1H,s), 7.98(1H,s)

### Example 36

4-(3-tert-Butoxycarbonylaminomethylpyrrolidin-1-yl)butylamine (1.00 g) as starting compound was reacted and treated in the same manner as in Example 34 using methyl isocyanate (0.24 ml) and 4-amino-5-chloro-2-methoxybenzoic acid (0.74 g) to give 4-amino-5-chloro-2-methoxy-N-(1-(4-(3-methylureido)butyl)pyrrolidin-3-ylmethyl)benzamide.
¹H-NMR (CDCl₃,ppm) δ : 1.45-1.72(5H,m),1.98-2.15(1H,m), 2.51-2.88(7H,m), 2.85(3H,d,J=4.62Hz), 3.12-3.25(2H,m), 3.36-3.62(2H,m), 3.92(3H,s), 4.45(2H,s), 4.80-4.93(1H,br), 5.05-5.17(1H,br), 6.31(1H,s), 7.82-7.94(1H,br), 8.08(1H,s)

### Example 37

5-(3-tert-Butoxycarbonylaminomethylpyrrolidin-1-yl)pentylamine (0.84 g) as starting compound was reacted and treated in the same manner as in Example 34 using methyl isocyanate (0.26 ml) and 4-amino-5-chloro-2-methoxybenzoic acid (0.58 g) to give 4-amino-5-chloro-2-methoxy-N-(1-(5-(3-methylureido)pentyl)pyrrolidin-3-ylmethyl)benzamide.
¹H-NMR (CDCl₃+CD₃OD,ppm) δ : 1.32-1.81 (7H,m),2.08-2.25(1H,m), 2.35(3H,s), 2.63-3.50(11H,m), 3.93(3H,s), 6.39(1H,s), 7.97(1H,s)

### Example 38

5-((3R)-3-tert-Butoxycarbonylaminomethylpyrrolidin-1-yl)-pentylamine (2.0 g) was dissolved in methylene chloride (20 ml) and a solution of ethyl isocyanate (0.55 ml) in methylene chloride was dropwise added under ice-cooling. The mixture was stirred at room temperature for 0.25 hr, and the reaction mixture was concentrated under reduced pressure to give (3R)-3-tert-butoxycarbonylaminomethyl-1-(5-(3-ethylureido)pentyl)pyrrolidine.

This compound was dissolved in 4N hydrochloric acid-dioxane solution (10 ml) and the mixture was stood at room temperature for 2 days. The reaction mixture was concentrated under reduced pressure. Dimethylformamide (10 ml) was added to the residue and the mixture was neutralized with triethylamine (1.0 ml). 4-Amino-5-chloro-2-methoxybenzoic acid (0.57 g) and 1-hydroxybenzotriazole (0.4 g) were added, and the mixture was stirred at 0°C for 15 min. Then, dicyclohexylcarbodiimide (0.56 g) was added and the mixture was stirred at room temperature for 14 hr. The reaction mixture was concentrated under reduced pressure. Aqueous potassium carbonate solution was added to the residue and the mixture was extracted with ethyl acetate. The extract was dried over magnesium sulfate and concentrated under reduced pressure. The obtained residue was purified by silica gel chromatography to give 0.2 g of 4-amino-5-chloro-N-((3R)-1-(5-(3-ethylureido)pentyl)pyrrolidin-3-ylmethyl)-2-methoxybenzamide.
¹H-NMR (CDCl₃,ppm) δ : 1.05-1.82(12H,m), 1.90-2.13(1H,m), 2.20-2.80(7H,m), 3.10-3.65(4H,m), 3.85(3H,s), 4.84(2H,s), 6.37(1H,s), 7.90-7.95(1H,br), 8.05(1H,s)

### Example 39

5-((3R)-3-tert-Butoxycarbonylaminomethylpyrrolidin-1-yl)pentylamine (2.0 g) was dissolved in methylene chloride (20 ml) and a solution of isopropyl isocyanate (0.69 ml) in methylene chloride was dropwise added under ice-cooling. The mixture was stirred at room temperature for 0.25 hr, and the reaction mixture was concentrated under reduced pressure to give (3R)-3-tert-butoxycarbonylaminomethyl-1-(5-(3-isopropylureido)pentyl)pyrrolidine.

This compound was dissolved in 4N hydrochloric acid-dioxane solution (10 ml) and the mixture was stood at room temperature for 14 hr. The reaction mixture was concentrated under reduced pressure. Dimethylformamide (10 ml) was added to the residue and the mixture was neutralized with triethylamine (1.0 ml). 4-Amino-5-chloro-2-methoxybenzoic acid (0.54 g) and 1-hydroxybenzotriazole (0.38 g) were added thereto, and the mixture was stirred at 0°C for 15 min. Then, dicyclohexylcarbodiimide (0.54 g) was added and the mixture was stirred at room temperature for 12 hr. The reaction mixture was concentrated under reduced pressure. Aqueous potassium carbonate solution was added to the residue and the mixture was extracted with ethyl acetate. The extract was dried over magnesium sulfate and concentrated under reduced pressure. The obtained residue was purified by silica gel chromatography to give 0.50 g of 4-amino-5-chloro-N-((3R)-1-(5-(3-isopropylureido)pentyl)pyrrolidin-3-ylmethyl)-2-methoxybenzamide.
¹H-NMR (CDCl₃,ppm) δ : 1.12(6H,d,J=4.06Hz), 1.15-1.69(6H,m), 1.90-2.10(1H,m), 2.21-2.79(7H,m), 3.05-3.60(4H,m), 3.88(3H,s), 4.56(2H,s), 4.70-4.80(1H,m), 4.90-5.01(1H,br), 6.33(1H,s), 7.80-7.95(1H,br), 8.04(1H,s)

### Example 40

2-(3-tert-Butoxycarbonylaminomethylpyrrolidin-1-yl)ethylamine (1 g) as starting compound was reacted and treated in the same manner as in Example 34 using n-propyl isocyanate (0.38 ml) and 4-amino-5-chloro-2-methoxybenzoic acid (0.83 g) to give 4-amino-5-chloro-2-methoxy-N-(1-(2-(3-n-propylureido)ethyl)pyrrolidin-3-ylmethyl)benzamide.
¹H-NMR (CDCl₃,ppm) δ : 0.91(3H,t,J=7.5Hz), 1.38-1.62(5H,m), 1.88-2.08(1H,m), 2.28-2.88(7H,m), 3.07-3.41(4H,m), 3.90(3H,s), 4.55(2H,s), 5.25-5.40(1H,br), 5.42-5.53(1H,br), 6.33(1H,s), 7.80-7.91(1H,br), 8.03(1H,s)

### Example 41

3-(3-tert-Butoxycarbonylaminomethylpyrrolidin-1-yl)propylamine (1.1 g) as starting compound was reacted and treated in the same manner as in Example 34 using n-propyl isocyanate (0.40 ml) and 4-amino-5-chloro-2-methoxybenzoic acid (0.87 g) to give 4-amino-5-chloro-2-methoxy-N-(1-(3-(3-n-propylureido)propyl)pyrrolidin-3-ylmethyl)benzamide.
¹H-NMR (CDCl₃,ppm) δ :0.91(3H,t,J=7.26Hz), 1.50-1.77(5H,m), 1.92-2.08(1H,m), 2.38-2.86(7H,m), 3.11-3.44(6H,m), 3.91(3H,s), 4.46(2H,s), 5.15-5.80(1H,br), 5.36-5.48(1H,br), 6.32(1H,s), 7.77-7.86(1H,br), 8.06(1H,s)

### Example 42

4-(3-tert-Butoxycarbonylaminomethylpyrrolidin-1-yl)butylamine (1.10 g) as starting compound was reacted and treated in the same manner as in Example 34 using n-propyl isocyanate (0.42 ml) and 4-amino-5-chloro-2-methoxybenzoic acid (0.82 g) to give 4-amino-5-chloro-2-methoxy-N-(1-(4-(3-n-propylureido)butyl)pyrrolidin-3-ylmethyl)benzamide.
¹H-NMR (CDCl₃,ppm) δ :0.90(3H,t,J=7.26Hz), 1.43-1.70(7H,m), 1.97-2.13(1H,m), 2.46-2.81(7H,m), 3.09-3.25(4H,m), 3.31-3.59(2H,m), 3.90(3H,s), 4.43(2H,s), 4.78-6.85(1H,br), 4.90-5.05(1H,br), 6.31(1H,s), 7.83-7.92(1H,br), 8.08(1H,s)

### Example 43

4-((3R)-3-tert-Butoxycarbonylaminomethylpyrrolidin-1-yl)butylamine (2.00 g) as starting compound was reacted and treated in the same manner as in Example 34 using n-propyl isocyanate (0.76 ml) and 4-amino-5-chloro-2-methoxybenzoic acid (1.49 g) to give 4-amino-5-chloro-2-methoxy-N-((3R)-1-(4-(3-n-propylureido)butyl)pyrrolidin-3-ylmethyl)benzamide.
¹H-NMR (CDCl₃,ppm) δ : 0. 87(3H,t,J=7.26Hz), 1.40-1.60(7H,m), 1.92-2.08(1H,m), 2.34-2.71(7H,m), 3.05-3.25(4H,m), 3.25-3.55(2H,m), 3.88(3H,s), 4.53(2H,s), 4.95-5.03(1H,br), 5.03-5.28(1H,br), 6.32(1H,s), 7.83-7.92(1H,br), 8.04(1H,s)

### Example 44

4-((3S)-3-tert-Butoxycarbonylaminomethylpyrrolidin-1-yl)butylamine (1.50g) as starting compound was reacted and treated in the same manner as in Example 34 using n-propyl isocyanate (0.57 ml) and 4-amino-5-chloro-2-methoxybenzoic acid (1.11 g) to give 4-amino-5-chloro-2-methoxy-N-((3S)-1-(4-(3-n-propylureido)butyl)pyrrolidin-3-ylmethyl)benzamide.
¹H-NMR (CDCl₃,ppm) δ : 0.87(3H,t,J=7.26Hz), 1.40-1.60(7H,m), 1.92-2.08(1H,m), 2.34-2.71(7H,m), 3.05-3.25(4H,m), 3.25-3.55(2H,m), 3.88(3H,s), 4.53(2H,s), 4.95-5.03(1H,br), 5.03-5.28(1H,br), 6.32(1H,s), 7.83-7.92(1H,br), 8.04(1H,s)

### Example 45

4-Amino-N-(1-(4-aminobutyl)piperidin-4-ylmethyl)-5-chloro-2-methoxybenzamide (1.0 g) was dissolved in dimethylformamide (10 ml) and n-propyl isocyanate (0.26 ml) was dropwise added under ice-cooling. The mixture was stirred at room temperature for 1 hr, and the reaction mixture was concentrated under reduced pressure. The obtained residue was purified by silica gel chromatography to give 0.2 g of 4-amino-5-chloro-2-methoxy-N-(1-(4-(3-n-propylureido)butyl)piperidin-4-ylmethyl)benzamide.
Melting Point 114∼115°C

### Example 46

5-((3R)-3-tert-Butoxycarbonylaminomethylpyrrolidin-1-yl)pentylamine (1.50 g) was dissolved in methylene chloride (15 ml) and a solution of n-propyl isocyanate (0.49 ml) in methylene chloride was dropwise added under ice-cooling. The mixture was stirred at room temperature for 1 hr. The reaction mixture was concentrated under reduced pressure. This residue was dissolved in 4N hydrochloric acid-dioxane solution (10 ml) and the mixture was stood at room temperature for 30 min. The reaction mixture was concentrated under reduced pressure. Dimethylformamide (30 ml) was added to the residue and the mixture was neutralized with triethylamine (2.2 ml). 4-Amino-5-chloro-2-methoxybenzoic acid (1.06 g) and 1-hydroxybenzotriazole (0.78 g) were added thereto, and the mixture was stirred at 0°C for 15 min. Then, 1-ethyl-3-(3-dimethylaminopropyl)carbodiimide hydrochloride (1.11 g) was added and the mixture was stirred at room temperature for 8 hr. The reaction mixture was concentrated under reduced pressure. Aqueous potassium carbonate solution was added to the residue and the mixture was extracted with ethyl acetate. The extract was dried over magnesium sulfate and concentrated under reduced pressure. The obtained residue was purified by silica gel chromatography to give 0.27 g of 4-amino-5-chloro-2-methoxy-N-((3R)-1-(5-(3-n-propylureido)pentyl)pyrrolidin-3-ylmethyl)benzamide.
¹H-NMR (CDCl₃,ppm) δ : 0.90(3H,t,J=7.26Hz), 1.28-1.67(9H,m), 1.98-2.14(1H,m), 2.25-2.81(7H,m), 3.04-3.25(4H,m), 3.25-3.58(2H,m), 3.90(3H,s), 4.47(2H,s), 4.80-4.93(2H,m), 6.32(1H,s), 7.83-7.91(1H,br), 8.05(1H,s)

### Example 47

5-((3S)-3-tert-Butoxycarbonylaminomethylpyrrolidin-1-yl)pentylamine (1.50 g) as starting compound was reacted and treated in the same manner as in Example 34 using n-propyl isocyanate (0.49 ml) and 4-amino-5-chloro-2-methoxybenzoic acid (0.61 g) to give 4-amino-5-chloro-2-methoxy-N-((3S)-1-(5-(3-n-propylureido)pentyl)pyrrolidin-3-ylmethyl)benzamide.
¹H-NMR (CDCl₃,ppm) δ : 0.90(3H,t,J=7.26Hz), 1.28-1.67(9H,m), 1.98-2.14(1H,m), 2.25-2.81(7H,m), 3.04-3.25(4H,m), 3.25-3.58(2H,m), 3.90(3H,s), 4.47(2H,s), 4.80-4.93(2H,m), 6.32(1H,s), 7.83-7.91(1H,br), 8.05(1H,s)

### Example 48

5-(4-tert-Butoxycarbonylaminomethy]piperidin-1-yl)pentylamine (1.1 g) was dissolved in methylene chloride (20 ml) and a solution of n-propyl isocyanate (0.34 ml) in methylene chloride was dropwise added under ice-cooling. The mixture was stirred at room temperature for 1 hr, and the reaction mixture was concentrated under reduced pressure to give 4-tert-butoxycarbonylaminomethyl-1-(5-(3-n-propylureido)pentyl)piperidine.

This compound was dissolved in 4N hydrochloric acid-dioxane solution (30 ml) and the mixture was stood at room temperature for 60 min. The reaction mixture was concentrated under reduced pressure. Dimethylformamide (30 ml) was added to the residue and the mixture was neutralized with triethylamine (1.22 ml). 4-Amino-5-chloro-2-methoxybenzoic acid (0.89 g) and 1-hydroxybenzotriazole (0.66 g) were added thereto, and the mixture was stirred at 0°C for 15 min. Then, dicyclohexylcarbodiimide (1.00 g) was added and the mixture was stirred at room temperature for 8 hr. The precipitated crystals were filtered off and the filtrate was concentrated under reduced pressure. Aqueous potassium carbonate solution was added to the residue and the mixture was extracted with ethyl acetate. The extract was dried over magnesium sulfate and concentrated under reduced pressure. The obtained residue was purified by silica gel chromatography to give 0.39 g of 4-amino-5-chloro-2-methoxy-N-(1-(5-(3-n-propylureido)pentyl)piperidin-4-ylmethyl)benzamide.
Melting point 144∼147°C

### Example 49

6-((3R)-3-tert-Butoxycarbonylaminomethylpyrrolidin-1-yl)hexylamine (1.50 g) as starting compound was reacted and treated in the same manner as in Example 34 using n-propyl isocyanate (0.50 ml) and 4-amino-5-chloro-2-methoxybenzoic acid (1.01 g) to give 4-amino-5-chloro-2-methoxy-N-((3R)-1-(6-(3-n-propylureido)hexyl)pyrrolidin-3-ylmethyl)benzamide.
¹H-NMR (CDCl₃+CD₃OD,ppm) δ : 0.90(3H,t,J=7.26Hz), 1.25-1.75(11H,m), 1.95-2.12(1H,m), 2.25-2.42(1H,m), 2.93-3.75(12H,m), 3.95(3H,s), 6.42(1H,s), 7.92(1H,s)

### Example 50

6-((3S)-3-tert-Butoxycarbonylaminomethylpyrrolidin-1-yl)hexylamine (1.00 g) as starting compound was reacted and treated in the same manner as in Example 34 using n-propyl isocyanate (0.33 ml) and 4-amino-5-chloro-2-methoxybenzoic acid (0.67 g) to give 4-amino-5-chloro-2-methoxy-N-((3S)-1-(6-(3-n-propylureido)hexyl)pyrrolidin-3-ylmethyl)benzamide.
¹H-NMR (CDCl₃+CD₃OD,ppm) δ : 0.90(3H,t,J=7.26Hz), 1.25-1.75(11H,m), 1.95-2.12(1H,m), 2.25-2.42(1H,m), 2.93-3.75(12H,m), 3.95(3H,s), 6.42(1H,s), 7.92(1H,s)

### Example 51

5-((3R)-3-tert-Butoxycarbonylaminomethylpyrrolidin-1-yl)pentylamine (2.00 g) as starting compound was reacted and treated in the same manner as in Example 34 using n-butyl isocyanate (0.79 ml) and 4-amino-5-chloro-2-methoxybenzoic acid (0.54 g) to give 4-amino-N-((3R)-1-(5-(3-n-butylureido)pentyl)pyrrolidin-3-ylmethyl)-5-chloro-2-methoxybenzamide.
¹H-NMR (CDCl₃,ppm) δ : 0.85-0.95(3H,t,J=7.26Hz), 1.25-1.63(9H,m), 1.94-2.09(1H,m), 2.35-2.71(7H,m), 3.07-3.21(4H,m), 3.25-3.58(2H,m), 3.89(3H,s), 4.46(2H,s), 4.70-4.83(2H,m), 6.32(1H,s), 7.83-7.90(1H,br), 8.06(1H,s)

### Example 52

2-(3-tert-Butoxycarbonylaminomethylpyrrolidin-1-yl)ethylamine (1 g) as starting compound was reacted and treated in the same manner as in Example 34 using phenyl isocyanate (0.40 ml) and 4-amino-5-chloro-2-methoxybenzoic acid (0.83 g) to give 4-amino-5-chloro-2-methoxy-N-(1-(2-(3-phenylureido)ethyl)pyrrolidin-3-ylmethyl)benzamide.
¹H-NMR (CDCl₃,ppm) δ : 1.55-1.72(1H,m),2.01-2.18(1H,m), 2.43-3.22(7H,m), 3.28-3.61(4H,m), 3.90(3H,s), 4.39(2H,s), 6.29(1H,s), 6.31-6.43(1H,br), 6.92-7.47(5H,m), 7.62-7.72(1H,br), 7.85-7.93(1H,br), 8.06(1H,s)

### Example 53

3-(3-tert-Butoxycarbonylaminomethylpyrrolidin-1-yl)propylamine (0.74 g) as starting compound was reacted and treated in the same manner as in Example 34 using phenyl isocyanate (0.33 ml) and 4-amino-5-chloro-2-methoxybenzoic acid (0.58 g) to give 4-amino-5-chloro-2-methoxy-N-(1-(3-(3-phenylureido)propyl)pyrrolidin-3-ylmethyl)benzamide.
¹H-NMR (CDCl₃+CD₃OD,ppm) δ : 1.82-2.08(3H,m), 2.19-2.38(1H,m), 2.97-3.74(11H,m), 3.91(3H,s), 6.35(1H,s), 6.91-7.01(1H,m), 7.18-7.45(4H,m), 7.97(1H,s)

### Example 54

4-(3-tert-Butoxycarbonylaminomethylpyrrolidin-1-yl)butylamine (0.92 g) as starting compound was reacted and treated in the same manner as in Example 34 using phenyl isocyanate (0.37 ml) and 4-amino-5-chloro-2-methoxybenzoic acid (0.68 g) to give 4-amino-5-chloro-2-methoxy-N-(1-(4-(3-phenylureido)butyl)pyrrolidin-3-ylmethyl)benzamide.
¹H-NMR (CDCl₃,ppm) δ : 1.43-1.69(5H,m), 1.95-2.17(1H,m), 2.35-2.85(7H,m), 3.15-3.41(4H,m), 3.90(3H,s), 4.43(2H,s), 5.78-5.92(1H,br), 6.30(1H,s), 6.93-7.03(1H,br), 7.20-7.44(5H,m), 7.85-7.94(1H,br), 8.10(1H,s)

### Example 55

5-(3-tert-Butoxycarbonylaminomethylpyrrolidin-1-yl)pentylamine (0.87 g) as starting compound was reacted and treated in the same manner as in Example 34 using phenyl isocyanate (0.33 ml) and 4-amino-5-chloro-2-methoxybenzoic acid (0.63 g) to give 4-amino-5-chloro-2-methoxy-N-(1-(5-(3-phenylureido)pentyl)pyrrolidin-3-ylmethyl)benzamide.
¹H-NMR (CDCl₃+CD₃OD,ppm) δ : 1.35-1.96(7H,m), 2.12-2.38(1H,m), 2.72-3.30(11H,m), 3.93(3H,s), 6.38(1H,s), 6.95-7.02(1H,m), 7.20-7.29(2H,m), 7.29-7.38(2H,m), 7.95(1H,s)

### Example 56

5-((3R)-3-tert-Methoxycarbonylaminomethylpyrrolidin-1-yl)pentylamine (1.50 g) was dissolved in methylene chloride (15 ml) and a solution of phenyl isocyanate (0.58 ml) in methylene chloride was dropwise added under ice-cooling. The mixture was stirred at room temperature for 1 hr, and the reaction mixture was concentrated under reduced pressure. This residue was dissolved in 4N hydrochloric acid-dioxane solution (10 ml), and the mixture was stood at room temperature for 30 min. The reaction mixture was concentrated under reduced pressure. Dimethylformamide (30 ml) was added to the residue and the mixture was neutralized with triethylamine (2.2 ml). 4-Amino-5-chloro-2-methoxybenzoic acid (1.06 g) and 1-hydroxybenzotriazole (0.78 g) were added thereto, and the mixture was stirred at 0°C for 15 min. Then, 1-ethyl-3-(3-dimethylaminopropyl)carbodiimide hydrochloride (1.11 g) was added and the mixture was stirred at room temperature for 8 hr. The reaction mixture was concentrated under reduced pressure. Aqueous potassium carbonate solution was added to the residue and the mixture was extracted with ethyl acetate. The extract was dried over magnesium sulfate and concentrated under reduced pressure. The obtained residue was purified by silica gel chromatography to give 0.50 g of 4-amino-5-chloro-2-methoxy-N-((3R)-1-(5-(3-phenylureido)pentyl)pyrrolidin-3-ylmethyl)benzamide.
¹H-NMR (CDCl₃,ppm) δ : 1.35-1.63(7H,m), 1.90-2.04(1H,m), 2.05-2.80(7H,m), 3.12-3.38(3H,m), 3.55-3.69(1H,m), 3.88(3H,s), 4.47(2H,s), 5.58-5.68(1H,br), 6.31(1H,s), 6.91-7.00(1H,br), 7.18-7.40(5H,m), 7.86-7.94(1H,br), 8.05(1H,s)

### Example 57

5-((3S)-3-tert-Butoxycarbonylaminomethylpyrrolidin-1-yl)pentylamine (1.52 g) as starting compound was reacted and treated in the same manner as in Example 34 using phenyl isocyanate (0.58 ml) and 4-amino-5-chloro-2-methoxybenzoic acid (0.75 g) to give 4-amino-5-chloro-2-methoxy-N-((3S)-1-(5-(3-phenylureido)pentyl)pyrrolidin-3-ylmethyl)benzamide.
¹H-NMR (CDCl₃,ppm) δ : 1.35-1.63(7H,m), 1.90-2.04(1H,m), 2.05-2.80(7H,m), 3.12-3.38(3H,m), 3.55-3.69(1H,m), 3.88(3H,s), 4.47(2H,s), 5.58-5.68(1H,br), 6.31(1H,s), 6.91-7.00(1H,br), 7.18-7.40(5H,m), 7.86-7.94(1H,br), 8.05(1H,s)

### Example 58

5-(4-tert-Butoxycarbonylaminomethylpiperidin-1-yl)pentylamine (2.00 g) was dissolved in methylene chloride (80 ml) and phenyl isocyanate (0.80 ml) was dropwise added under ice-cooling. The mixture was stirred at room temperature for 15 hr, and the reaction mixture was concentrated under reduced pressure to give 4-tert-butoxycarbonylaminomethyl-1-(5-(3-phenylureido)pentyl)piperidine.

This compound was dissolved in 4N hydrochloric acid-isopropyl alcohol solution (60 ml) and the mixture was stood at room temperature for 18 hr. The reaction mixture was concentrated under reduced pressure. Dimethylformamide (60 ml) was added to the residue and the mixture was neutralized with triethylamine (2.50 ml). 4-Amino-5-chloro-2-methoxybenzoic acid (1.11 g) and 1-hydroxybenzotriazole (0.82 g) were added thereto, and the mixture was stirred at 0°C for 15 min. Then, 1-ethyl-3-(3-dimethylaminopropyl)carbodiimide hydrochloride (1.16 g) was added and the mixture was stirred at room temperature for 20 hr. The reaction mixture was concentrated under reduced pressure. Aqueous potassium carbonate solution was added to the residue and the mixture was extracted with chloroform. The extract was dried over magnesium sulfate and concentrated under reduced pressure. The obtained residue was purified by silica gel chromatography to give 1.25 g of 4-amino-5-chloro-2-methoxy-N-(1-(5-(3-phenylureido)pentyl)piperidin-4-ylmethyl)benzamide.
Melting point 214∼217°C

### Example 59

5-(4-tert-Butoxycarbonylaminomethylpiperidin-1-yl)pentylamine (2.00 g) was dissolved in methylene chloride (80 ml) and a solution of 4-chlorophenyl isocyanate (1.13 g) in methylene chloride was dropwise added under ice-cooling. The mixture was stirred at room temperature for 15 hr, and the reaction mixture was concentrated under reduced pressure to give 4-tert-butoxycarbonylaminomethyl-1-(5-(3-(4-chlorophenyl)ureido)pentyl)piperidine.

This compound was dissolved in 4N hydrochloric acid-dioxane solution (60 ml) and the mixture was stood at room temperature for 14 hr. The reaction mixture was concentrated under reduced pressure. Dimethylformamide (40 ml) was added to the residue and the mixture was neutralized with triethylamine (2.5 ml). 4-Amino-5-chloro-2-methoxybenzoic acid (1.11 g) and 1-hydroxybenzotriazole (0.82 g) were added thereto, and the mixture was stirred at 0°C for 15 min. Then, 1-ethyl-3-(3-dimethylaminopropyl)carbodiimide hydrochloride (1.16 g) was added and the mixture was stirred at room temperature for 17 hr. The reaction mixture was concentrated under reduced pressure. Aqueous potassium carbonate solution was added to the residue and the mixture was extracted with ethyl acetate. The extract was dried over magnesium sulfate and concentrated under reduced pressure. The obtained residue was purified by silica gel chromatography to give 0.76 g of 4-amino-5-chloro-N-(1 -(5-(3-(4-chlorophenyl)ureido)pentyl)-piperidin-4-ylmethyl)-2-methoxybenzamide.
Melting point 204∼206°C

### Example 60

6-((3R)-3-tert-Butoxycarbonylaminomethylpyrrolidin-1-yl)hexylamine (1.50 g) as starting compound was reacted and treated in the same manner as in Example 34 using phenyl isocyanate (0.57 ml) and 4-amino-5-chloro-2-methoxybenzoic acid (1.01 g) to give 4-amino-5-chloro-2-methoxy-N-((3R)-1-(6-(3-phenylureido)hexyl)pyrrolidin-3- ylmethyl)benzamide.
¹H-NMR (CDCl₃+CD₃OD,ppm) δ : 1.35-1.75(9H,m), 1.90-2.04(1H,m), 2.20-2.36(1H,m), 2.91-3.85(10H,m), 3.93(3H,s), 6.44(1H,s), 6.90-6.99(1H,m), 7.15-7.46(4H,m), 7.91(1H,s), 8.20-8.30(1H,br)

### Example 61

6-((3S)-3-tert-Butoxycarbonylaminomethylpyrrolidin-1-yl)hexylamine (1.00 g) as starting compound was reacted and treated in the same manner as in Example 34 using phenyl isocyanate (0.38 ml) and 4-amino-5-chloro-2-methoxybenzoic acid (0.67 g) to give 4-amino-5-chloro-2-methoxy-N-((3S)-1-(6-(3-phenylureido)hexyl)pyrrolidin-3-ylmethyl)benzamide.
¹H-NMR (CDCl₃+CD₃OD,ppm) δ : 1.35-1.75(9H,m), 1.90-2.04(1H,m), 2.20-2.36(1H,m), 2.91-3.85(10H,m), 3.93(3H,s), 6.44(1H,s), 6.90-6.99(1H,m), 7.15-7.46(4H,m), 7.91(1H,s), 8.20-8.30(1H,br)

### Example 62

2-(3-tert-Butoxycarbonylaminomethylpyrrolidin-1-yl)ethylamine (1 g) as starting compound was reacted and treated in the same manner as in Example 34 using methyl isothiocyanate (0.25 ml) and 4-amino-5-chloro-2-methoxybenzoic acid (0.83 g) to give 4-amino-5-chloro-2-methoxy-N-(1 -(2-(3-methylthioureido)ethyl)pyrrolidin-3-ylmethyl)benzamide.
¹H-NMR (CDCl₃,ppm) δ : 1.53-1.71 (1H,m),1.98-2.16(1H,m), 2.42-3.28(7H,m), 3.06(3H,d,J=5.3Hz), 3.50-3.87(4H,m), 3.92(3H,s), 4.41(2H,s), 6.30(1H,s), 6.98-7.14(1H,br), 7.22-7.30(1H,br), 7.80-7.90(1H,br), 8.00(1H,s)

### Example 63

3-(3-tert-Butoxycarbonylaminomethylpyrrolidin-1-yl)propylamine (1.06 g) as starting compound was reacted and treated in the sane manner as in Example 34 using methyl isothiocyanate (0.32 ml) and 4-amino-5-chloro-2-methoxybenzoic acid (0.91 g) to give 4-amino-5-chloro-2-methoxy-N-(1-(3-(3-methylthioureido)propyl)pyrrolidin-3-ylmethyl)benzamide.
¹H-NMR (CDCl₃,ppm) δ : 1.05-2.87(4H,m),2.93-3.01(3H,br),3.31-3.71(11H,m), 3.91(3H,s),4.41(2H,s),6.30(1H,s),7.75-7.94(1H,br),8.07(1H,s)

### Example 64

4-(3-tert-Butoxycarbonylaminomethylpyrrolidin-1-yl)butylamine (1.01 g) as starting compound was reacted and treated in the sane manner as in Example 34 using methyl isothiocyanate (0.28 ml) and 4-amino-5-chloro-2-methoxybenzoic acid (0.82 g) to give 4-amino-5-chloro-2-methoxy-N-(1-(4-(3-methylthioureido)butyl)pyrrolidin-3-ylmethyl)benzamide.
¹H-NMR (CDCl₃,ppm) δ : 1.62-1.80(5H,m), 2.08-2.21(1H,m), 2.63-3.05(7H,m), 3.05(3H,d,J=4.62Hz), 3.38-3.63(4H,m), 3.92(3H,s), 4.44(2H,s), 6.31(1H,s), 6.69-6.90(1H,br), 6.90-7.05(1H,br), 7.85-7.94(1H,br), 8.07(1H,s)

### Example 65

2-(3-tert-Butoxycarbonylaminomethylpyrrolidin-1-yl)ethylamine (1 g) as starting compound was reacted and treated in the same manner as in Example 34 using phenyl isothiocyanate (0.49 ml) and 4-amino-5-chloro-2-methoxybenzoic acid (0.83 g) to give 4-amino-5-chloro-2-methoxy-N-(1-(2-(3-phenylthioureido)ethyl)pyrrolidin-3-ylmethyl)benzamide.
¹H-NMR (DMSO-D₆,ppm) δ : 1.41-1.57(1H,m),1.82-1.98(1H,m), 2.32-2.80(7H,m), 3.14-3.66(4H,m), 3.82(3H,s), 5.91(2H,s), 6.45(1H,s), 7.05-7.43(5H,m), 7.55-7.62(1H,br), 7.82-7.90(1H,br), 7.94-8.02(1H,br), 8.31(1H,s)

### Example 66

4-(3-tert-Butoxycarbonylaminomethylpyrrolidin-1-yl)butylamine (1.00 g) as starting compound was reacted and treated in the same manner as in Example 34 using phenyl isothiocyanate (0.44 ml) and 4-amino-5-chloro-2-methoxybenzoic acid (0.74 g) to give 4-amino-5-chloro-2-methoxy-N-(1-(4-(3-phenylthioureido)butyl)pyrrolidin-3-ylmethyl)benzamide.
¹H-NMR (CDCl₃,ppm) δ : 1.43-1.69(5H,m),1.95-2.17(1H,m), 2.35-2.85(7H,m), 3.15-3.41(4H,m), 3.90(3H,s), 4.43(2H,s), 5.78-5.92(1H,br), 6.30(1H,s), 6.93-7.03(1H,br), 7.20-7.44(5H,m), 7.85-7.94(1H,br), 8.10(1H,s)

### Example 67

2-(4-(3-tert-Butoxycarbonylaminomethylpyrrolidin-1-yl)butyl)-2,3-dihydro-1H-isoindole-1,3-dione (1.1 g) was dissolved in trifluoroacetic acid (10 ml) and the mixture was stood at room temperature for 30 min. The reaction mixture was concentrated under reduced pressure. Dimethylformamide (10 ml) was added to the residue and the mixture was neutralized with triethylamine (0.8 ml). 4-Amino-5-chloro-2-methoxybenzoic acid (0.51 g) and 1-hydroxybenzotriazole (0. 40 g) were added thereto, and the mixture was stirred at 0°C for 15 min. Then, 1-ethyl-3-(3-dimethylaminopropyl)carbodiimide hydrochloride (0.57 g) was added and the mixture was stirred at room temperature for 8 hr. The reaction mixture was concentrated under reduced pressure. Aqueous potassium carbonate solution was added to the residue and the mixture was extracted with ethyl acetate. The extract was dried over magnesium sulfate and concentrated under reduced pressure. The obtained residue was purified by silica gel chromatography to give 0.62 g of 4-amino-5-chloro-N-(1-(4-(2,3-dihydro-1,3-dioxo-1H-isoindol-2-yl)butyl)pyrrolidin-3-ylmethyl)-2-methoxybenzamide.
¹H-NMR (CDCl₃,ppm) δ :1.50-1.61(3H,m),1.65-1.76(2H,m),1.96-2.08(1H,m), 2.34-2.42(2H,m),2.45-2.66(3H,m),2.72-2.78(2H,m),3.40(2H,m), 3.65-3.72(2H,m),3.86(3H,s),4.50(2H,s),6.30(1H,s),7.65-7.74(2H,m), 7.78-7.86(2H,m),8.08(1H,s)

### Example 68

2-(2-(3-tert-Butoxycarbonylaminomethylpyrrolidin-1-yl)ethyl)-2,3-dihydro-1H-isoindole-1,3-dione (1.5 g) as starting compound was reacted and treated in the same manner as in Example 67 using 4-amino-5-chloro-2-methoxybenzoic acid (0.81 g) to give 4-amino-5-chloro-N-(1-(2-(-2,3-dihydro-1,3-dioxo-1H-isoindol-2-yl)ethyl)pyrrolidin-3-ylmethyl)-2-methoxybenzamide.
¹H-NMR (CDCl₃,ppm) δ : 1.42-1.62(1H,m),1.88-2.06(1H,m), 2.43-2.97(7H,m), 3.17-3.50(4H,m), 3.88(3H,s), 4.33(2H,s), 6.21(1H,s), 7.62-7.72(2H,m), 7.75-7.85(2H,m), 7.90-8.02(1H,br), 8.07(1H,s)

### Example 69

2-(3-(3-tert-Butoxycarbonylaminomethylpyrrolidin-1-yl)propyl)-2,3-dihydro-1H-isoindole-1,3-dione (1 g) as starting compound was reacted and treated in the same manner as in Example 67 using 4-amino-5-chloro-2-methoxybenzoic acid (0.50 g) to give 4-amino-5-chloro-N-(1-(3-(2,3-dihydro-1,3-dioxo-1H-isoindol-2-yl)propyl)pyrrolidin-3-ylmethyl)-2-methoxybenzamide.
¹H-NMR (CDCl₃,ppm) δ : 1.62-1.74(1H,m), 1.98-2.17(3H,m), 2.61-3.14(7H,m), 3.34-3.48(4H,m), 3.73(2H,s), 3.93(3H,s), 6.37(1H,s), 7.25-7.36(1H,br), 7.72-7.79(2H,m), 7.82-7.87(2H,m), 7.95(1H,s)

### Example 70

2-(5-(3-tert-Butoxycarbonylaminomethylpyrrolidin-1-yl)pentyl)-2,3-dihydro-1H-isoindole-1,3-dione (0.50 g) was dissolved in 4N hydrochloric acid-dioxane solution (10 ml) and the mixture was stood at room temperature for 30 min. The reaction mixture was concentrated under reduced pressure. Dimethylformamide (10 ml) was added to the residue and the mixture was neutralized with triethylamine (0.50 ml). 4-Amino-5-chloro-2-methoxybenzoic acid (0.24 g) and benzotriazol-1-yloxytris(dimethylamino)phosphonium hexafluorophosphate (0.58 g) were added thereto, and the mixture was stirred at room temperature for 8 hr. The reaction mixture was concentrated under reduced pressure. Aqueous potassium carbonate solution was added to the residue and the mixture was extracted with ethyl acetate. The extract was dried over magnesium sulfate and concentrated under reduced pressure. The obtained residue was purified by silica gel chromatography to give 0.16 g of 4-amino-5-chloro-N-(1-(5-(2,3-dihydro-1,3-dioxo-1H-isoindol-2-yl)pentyl)pyrrolidin-3-ylmethyl)-2-methoxybenzamide.
¹H-NMR (CDCl₃,ppm) δ : 1.65-1.85(7H,m), 2.12-2.28(1H,m), 2.65-3.23(7H,m), 3.44-3.79(4H,m), 3.91(3H,s), 4.43(2H,s), 6.31(1H,s), 7.65-7.90(4H,m), 7.99(1H,s), 7.98-8.13(1H,br)

### Example 71

4-(3-tert-Butoxycabonylaminomethylpyrrolidin-1-yl)-N-phenylbutylamide (0.99 g) was dissolved in 4N hydrochloric acid-dioxane solution (10 ml) and the mixture was stood at room temperature for 30 min. The reaction mixture was concentrated under reduced pressure. Dimethylformamide (10 ml) was added to the residue and the mixture was neutralized with triethylamine (1.2 ml). 4-Amino-5-chloro-2-methoxybenzoic acid (0.55 g) and benzotriazol-1-yloxytris(dimethylamino)phosphonium hexafluorophosphate (1.33 g) were added thereto, and the mixture was stirred at room temperature for 8 hr. The reaction mixture was concentrated under reduced pressure. Aqueous potassium carbonate solution was added to the residue and the mixture was extracted with ethyl acetate. The extract was dried over magnesium sulfate and concentrated under reduced pressure. The obtained residue was purified by silica gel chromatography to give 0.35 g of 4-amino-5-chloro-2-methoxy-N-(1-(3-phenylcarbamoylpropyl)pyrrolidin-3-ylmethyl)benzamide.
¹H-NMR (CDCl₃+CD₃OD,ppm) δ : 1.80-2.10(3H,m), 2.15-2.32(1H,m), 2.50-2.59(2H,t,J=6.6Hz), 3.75-3.90(1H,m), 3.05-3.52(8H,m), 3.92(3H,s), 6.38(1H,s), 7.02-7.53(5H,m), 7.95(1H,br)

### Example 72

4-(3-tert-Butoxycarbonylaminomethylpyrrolidin-1-yl)-N-(4-methylphenyl)butylamide (2.04 g) as starting compound was reacted and treated in the same manner as in Example 67 using 4-amino-5-chloro-2-methoxybenzoic acid (1.10 g) to give 4-amino-5-chloro-2-methoxy-N-(1-(3-(4-methylphenyl)carbamoylpropyl)pyrrolidin-3-ylmethyl)benzamide.
¹H-NMR (CDCl₃,ppm) δ : 1.50-1.68(1H,m), 1.80-2.11(3H,m), 2.29(3H,s), 2.33-2.79(9H,m), 3.30-3.41(1H,m), 3.48-3.58(1H,m), 3.80(3H,s), 4.39(2H,s), 6.28(1H,s), 7.05-7.11(2H,m), 7.35-7.42(2H,m), 7.75-7.84(1H,br), 8.07(1H,s), 8.74-8.81(1H,br)

### Example 73

4-(3-tert-Butoxycarbonylaminomethylpyrrolidin-1-yl)-N-(3-chlorophenyl)butylamide (2.10 g) as starting compound was reacted and treated in the same manner as in Example 67 using 4-amino-5-chloro-2-methoxybenzoic acid (1.07 g) to give 4-amino-5-chloro-N-(1-(3-(3-chlorophenyl)carbamoylpropyl)pyrrolidin-3-ylmethyl)-2-methoxybenzamide.
¹H-NMR (CDCl₃,ppm) δ : 1.49-1.65(1H,m), 1.80-2.11(3H,m), 2.35-2.78(9H,m), 3.25-3.35(1H,m), 3.52-3.55(1H,m), 3.87(3H,s), 4.42(2H,s), 6.28(1H,s), 6.98-7.40(3H,m), 7.66-7.70(1H,m), 7.75-7.85(1H,br), 8.04(1H,s), 9.28-9.36(1H,br)

### Example 74

4-Amino-N-(2-(3-tert-butoxycarbonylaminomethylpyrrolidin-1-yl)ethyl)-5-chloro-2-methoxybenzamide (0.97 g) as starting compound was reacted and treated in the same manner as in Example 67 using 4-amino-5-chloro-2-methoxybenzoic acid (0.50 g) to give 4-amino-N-(1-(2-(4-amino-5-chloro-2-methoxybenzoylamino)ethyl)pyrrolidin-3-ylmethyl)-5-chloro-2-methoxybenzamide.
¹H-NMR (DMSO-d₆,ppm) δ : 1.38-1.55(1H,m), 1.78-1.95(1H,m), 3.82(6H,s), 5.89(1H,s), 5.90(1H,s), 6.55(4H,s), 7.66(1H,s), 7.71(1H,s), 7.90-8.01(1H,br), 8.05-8.16(1H,br)

### Example 75

4-Amino-N-(3-(3-tert-butoxycarbonylaminomethylpyrrolidin-1-yl)-propyl)-5-chloro-2-methoxybenzamide (1.70 g) as starting compound was reacted and treated in the same manner as in Example 67 using 4-amino-5-chloro-2-methoxybenzoic acid (0.76 g) to give 4-amino-N-(1-(3-(4-amino-5-chloro-2-methoxybenzoylamino)propyl)pyrrolidin-3-ylmethyl)-5-chloro-2-methoxybenzamide.
¹H-NMR (CDCl₃,ppm) δ : 1.65-2.20(3H,m), 2.42-3.58(12H,m), 3.90(6H,s), 4.39(4H,s), 6.30(2H,s), 7.80-7.98(2H,br), 8.08(2H,br)

### Example 76

4-Amino-N-(4-(3-tert-butoxycarbonylaminomethylpyrrolidin-1-yl)butyl)-5-chloro-2-methoxybenzamide (1.35 g) as starting compound was reacted and treated in the same manner as in Example 67 using 4-amino-5-chloro-2-methoxybenzoic acid (0.60 g) to give 4-amino-N-(1-(4-(4-amino-5-chloro-2-methoxybenzoylamino)butyl)pyrrolidin-3-ylmethyl)-5-chloro-2-methoxybenzamide.
¹H-NMR (CDCl₃,ppm) δ : 1.50-1.70(4H,m), 1.90-2.15(2H,m), 2.43-2.81(7H,m), 3.33-3.50(4H,m), 3.85(3H,s), 3.86(3H,s), 4.38(4H,s), 6.25(2H,s), 6.26(2H,s), 7.64-7.72(1H,br), 7.83-7.91(1H,br), 8.07(1H,s), 8.08(1H,s)

### Example 77

4-Amino-N-(4-((3R)-3-tert-butoxycarbonylaminomethylpyrrolidin-1-yl)butyl)-5-chloro-2-methoxybenzamide (2.28 g) was dissolved in 4N hydrochloric acid-dioxane solution (10 ml) and the mixture was stood at room temperature for 30 min. The reaction mixture was concentrated under reduced pressure. Dimethylformamide (30 ml) was added to the residue and the mixture was neutralized with triethylamine (2.23 ml). 4-Amino-5-chloro-2-methoxybenzoic acid (1.07 g) and 1-hydroxybenzotriazole (0.79 g) were added thereto, and the mixture was stirred at 0°C for 15 min. Then, 1-ethyl-3-(3-dimethylaminopropyl)carbodiimide hydrochloride (1.12 g) was added, and the mixture was stirred at room temperature for 8 hr. The reaction mixture was concentrated under reduced pressure. Aqueous potassium carbonate solution was added to the residue and the mixture was extracted with ethyl acetate. The extract was dried over magnesium sulfate and concentrated under reduced pressure. The obtained residue was purified by silica gel chromatography to give 0.60 g of 4-amino-N-((3R)-1-(4-(4-amino-5-chloro-2-methoxybenzoylamino)butyl)pyrrolidin-3-ylmethyl)-5-chloro-2-methoxybenzamide.
¹H-NMR (CDCl₃+CD₃OD,ppm) δ : 1.45-1.70(5H,m), 1.95-2.14(1H,m), 2.21-2.84(11H,m), 3.39(3H,s), 3.41(3H,s), 6.34(1H,s), 6.36(1H,s), 7.97(1H,s), 7.98(1H,s)

### Example 78

4-Amino-N-(5-((3R)-3-tert-butoxycarbonylaminomethylpyrrolidin-1-yl)pentyl)-5-chloro-2-methoxybenzamide (1.45 g) was dissolved in 4N hydrochloric acid-dioxane solution (10 ml) and the mixture was stood at room temperature for 30 min. The reaction mixture was concentrated under reduced pressure. Dimethylformamide (30 ml) was added to the residue and the mixture was neutralized with triethylamine (1.37 ml). 4-Amino-5-chloro-2-methoxybenzoic acid (0.66 g) and 1-hydroxybenzotriazole (0.49 g) were added thereto, and the mixture was stirred at 0°C for 15 min. Then, 1-ethyl-3-(3-dimethylaminopropyl)carbodiimide hydrochloride (0.69 g) was added, and the mixture was stirred at room temperature for 8 hr. The reaction mixture was concentrated under reduced pressure. Aqueous potassium carbonate solution was added to the residue and the mixture was extracted with ethyl acetate. The extract was dried over magnesium sulfate and concentrated under reduced pressure. The obtained residue was purified by silica gel chromatography to give 0.30 g of 4-amino-N-((3R)-1-(5-(4-amino-5-chloro-2-methoxybenzoylamino)pentyl)pyrrolidin-3-ylmethyl)-5-chloro-2-methoxybenzamide.
¹H-NMR (CDCl₃,ppm) δ : 1.33-1.90(7H,m), 1.93-2.08(1H,m), 2.33-2.76(7H,m), 3.32-3.46(4H,m), 3.87(3H,s), 3.88(3H,s), 4.36(2H,s), 4.38(2H,s), 6.28(2H,s), 7.60-7.72(1H,br), 7.82-7.88(1H,br), 8.07(1H,s), 8.10(1H,s)

### Example 79

N-(3-(3-tert-Butoxycarbonylaminomethylpyrrolidin-1-yl)propyl)-4-pyridinecarboxamide (0.90 g) as starting compound was reacted and treated in the same manner as in Example 67 using 4-amino-5-chloro-2-methoxybenzoic acid (0.50 g) to give 4-amino-5-chloro-2-methoxy-N-(1-(3-(4-pyridinecarbonylamino)propyl)pyrrolidin-3-ylmethyl)benzamide.
¹H-NMR (CDCl₃,ppm) δ : 1.50-2.10(4H,m), 2.35-2.90(7H,m), 3.30-3.62(4H,m), 3.90(3H,s), 4.41(2H,s), 6.30(1H,s), 7.60-7.66(2H,m), 7.71-7.83(1H,br), 8.08(1H,s), 8.68-8.78(3H,m)

### Example 80

N-(3-(3-tert-Butoxycarbonylaminomethylpyrrolidin-1-yl)propyl)-6-chloro-4-methyl-3,4-dihydro-2H-1,4-benzoxazine-8-carboxamide (1.5 g) as starting compound was reacted and treated in the same manner as in Example 67 using 4-amino-5-chloro-2-methoxybenzoic acid (0.65 g) to give N-(3-(3-(4-amino-5-chloro-2-methoxybenzoylaminomethyl)pyrrolidin-1-yl)propyl)-6-chloro-4-methyl-3,4-dihydro-2H-1,4-benzoxazine-8-carboxamide.
¹H-NMR (DMSO-D₆,ppm) δ : 1.60-1.68(1H,m), 2.08-2.30(3H,m), 2.39-2.58(7H,m), 2.86(3H,m), 3.24-3.46(6H,m), 3.70(3H,s), 4.27-4.33(2H,m), 5.90(2H,s), 6.41(1H,s), 6.74(1H,d,J=2.64Hz), 6.85(1H,d,J=2.64Hz), 7.53(1H,s), 8.10-8.19(2H,br)

### Example 81

N-(4-(3-tert-Butoxycarbonylaminomethylpyrrolidin-1-yl)butyl)-6-chloro-4-methyl-3,4-dihydro-2H-1,4-benzoxazine-8-carboxamide (0.7 g) as starting compound was reacted and treated in the same manner as in Example 67 using 4-amino-5-chloro-2-methoxybenzoic acid (0.37 g) to give N-(4-(3-(4-amino-5-chloro-2-methoxybenzoylaminomethyl)pyrrolidin-1-yl)butyl)-6-chloro-4-methyl-3,4-dihydro-2H-1,4-benzoxazine-8-carboxamide.
¹H-NMR (CDCl₃,ppm) δ :1.50-1.75(5H,m),1.98-2.13(1H,m),2.31-2.92(7H,m), 2.89(3H,s),3.29-3.37(2H,m),3.35-3.52(4H,m),3.88(3H,s),4.28-4.43(4H,m), 6.25(1H,s),6.66(1H,d,J=2.64Hz),7.41(1H,d,J=2.64Hz),7.73-7.80(1H,br), 7.75-7.88(1H,br),8.07(1H,s)

### Example 82

N-(2-(3-tert-Butoxycarbonylaminomethylpyrrolidin-1-yl)ethyl)-1-methyl-1H-indole-3-carboxamide (2.16 g) as starting compound was reacted and treated in the same manner as in Example 67 using 4-amino-5-chloro-2-methoxybenzoic acid (1.1 g) to give N-(2-(3-(4-amino-5-chloro-2-methoxybenzoylaminomethyl)pyrrolidin-1-yl)ethyl)-1-methyl-1H-indole-3-carboxamide.
¹H-NMR (CDCl₃,ppm) δ : 1.57-1.78(1H,m),2.02-2.18(1H,m), 2.50-2.98(7H,m), 3.39-3.73(4H,m), 3.75(3H,s), 3.81(3H,s), 4.31(2H,s), 6.11(1H,s), 7.18-7.36(4H,m), 7.66-7.75(1H,br), 7.88-7.97(1H,br), 7.99-8.10(1H,m), 8.08(1H,s)

### Example 83

N-(3-(3-tert-Butoxycarbonylaminomethylpyrrolidin-1-yl)propyl)-1-methyl-1H-indole-3-carboxamide (0.44 g) as starting compound was reacted and treated in the same manner as in Example 67 using 4-amino-5-chloro-2-methoxybenzoic acid (0.26 g) to give N-(3-(3-(4-amino-5-chloro-2-methoxybenzoylaminomethyl)pyrrolidin-1-yl)propyl)-1-methyl-1H-indole-3-carboxamide.
¹H-NMR (CDCl₃,ppm) δ : 1.60-1.75(1H,m), 1.91-2.18(3H,m), 2.60-3.14(7H.m), 3.36-3.49(2H,m), 3.53-3.80(2H,m), 3.84(3H,s), 3.86(3H,s), 4.38(2H,s), 6.24(1H,s), 7.18-7.45(4H,m), 7.70-7.88(2H,m), 8.05(1H,s), 8.08-8.14(1H,m)

### Example 84

N-(4-(3-tert-Butoxycarbonylaminomethylpyrrolidin-1-yl)butyl)-1-methyl-1H-indole-3-carboxamide (0.67 g) as starting compound was reacted and treated in the same manner as in Example 67 using 4-amino-5-chloro-2-methoxybenzoic acid (0.42 g) to give N-(4-(3-(4-amino-5-chloro-2-methoxybenzoylaminomethyl)pyrrolidin-1-yl)butyl)-1-methyl-1H-indole-3-carboxamide.
¹H-NMR (CDCl₃,ppm) δ : 1.50-1.78(5H,m),1.92-2.08(1H,m),2.36-2.80(7H,m), 3.35-3.60(4H,m),3.80(3H,s),3.85(3H,s),4.31(2H,s),6.24(1H,s), 7.20-7.37(4H,m),7.65(1H,s),7.80-7.85(1H,br),7.90-7.97(1H,br),8.07(1H,s)

### Example 85

N-(4-((3R)-3-tert-Butoxycarbonylaminomethylpyrrolidin-1-yl)butyl)-1-methyl-1H-indole-3-carboxamide (1.94 g) was dissolved in 4N hydrochloric acid-dioxane solution (40 ml) and the mixture was stood at room temperature for 60 min. The reaction mixture was concentrated under reduced pressure. Dimethylformamide (30 ml) was added to the residue and the mixture was neutralized with triethylamine (1.25 ml). 4-Amino-5-chloro-2-methoxybenzoic acid (0.91 g) and 1-hydroxybenzotriazole (0.67 g) were added thereto, and the mixture was stirred at 0°C for 15 min. Then, 1-ethyl-3-(3-dimethylaminopropyl)carbodiimide hydrochloride (0.95 g) was added, and the mixture was stirred at room temperature for 8 hr. The reaction mixture was concentrated under reduced pressure. Aqueous potassium carbonate solution was added to the residue and the mixture was extracted with ethyl acetate. The extract was dried over magnesium sulfate and concentrated under reduced pressure. The obtained residue was purified by silica gel chromatography to give 0.75 g of N-(4-((3R)-3-(4-amino-5-chloro-2-methoxybenzoylaminomethyl)pyrrolidin-1-yl)butyl)-1-methyl-1H-indole-3-carboxamide.
¹H-NMR(CDCl₃,ppm) δ :1.50-1.78(5H,m), 1.89-2.13(1H,m), 2.40-2.81(7H,m), 3.35-3.56(4H,m), 3.80(3H,s), 3.84(3H,s), 4.35(2H,s), 6.25(1H,s), 6.33-6.45(1H,br), 7.20-7.38(3H,m), 7.65(1H,s), 7.78-7.88(1H,br), 7.96-8.00(1H,m), 8.05(1H,s)

### Example 86

N-(4-((3S)-3-tert-Butoxycarbonylaminomethylpyrrolidin-1-yl)-butyl)-1-methyl-1H-indole-3-carboxamide (1.94 g) as starting compound was reacted and treated in the same manner as in Example 67 using 4-amino-5-chloro-2-methoxybenzoic acid (0.67 g) to give N-(4-((3S)-3-(4-amino-5-chloro-2-methoxybenzoylaminomethyl)pyrrolidin-1-yl)butyl)-1-methyl-1H-indole-3-carboxamide.
¹H-NMR (CDCl₃,ppm) δ :1.50-1.78(5H,m), 1.89-2.13(1H,m), 2.40-2.81(7H,m), 3.35-3.56(4H,m), 3.80(3H,s), 3.84(3H,s), 4.35(2H,s), 6.25(1H,s), 6.33-6.45(1H,br), 7.20-7.38(3H,m), 7.65(1H,s), 7.78-7.88(1H,br), 7.96-8.00(1H,m), 8.05(1H,s)

### Example 87

N-(5-(3-tert-Butoxycarbonylaminomethylpyrrolidin-1-yl)pentyl)-1-methyl-1H-indole-3-carboxamide (0.89 g) as starting compound was reacted and treated in the same manner as in Example 67 using 4-amino-5-chloro-2-methoxybenzoic acid (0.42 g) to give N-(5-(3-(4-amino-5-chloro-2-methoxybenzoylaminomethyl)pyrrolidin-1-yl)pentyl)-1-methyl-1H-indole-3-carboxamide.
¹H-NMR (CDCl₃+CD₃OD,ppm) δ : 1.20-1.78(7H,m), 2.05-2.20(1H,m), 2.58-3.53(11H,m), 3.83(3H,s), 3.90(3H,s), 6.35(1H,s), 7.20-7.41(3H,m), 7.72(1H,s), 7.95-8.08(2H,m)

### Example 88

N-(5-((3R)-3-tert-Butoxycarbonylaminomethylpyrrolidin-1-yl)-pentyl)-1-methyl-1H-indole-3-carboxamide (1.53 g) was dissolved in 4N hydrochloric acid-dioxane solution (10 ml) and the mixture was stood at room temperature for 30 min. The reaction mixture was concentrated under reduced pressure. Dimethylformamide (30 ml) was added to the residue and the mixture was neutralized with triethylamine (1.45 ml). 4-Amino-5-chloro-2-methoxybenzoic acid (0.70 g) and 1-hydroxybenzotriazole (0.51 g) were added thereto, and the mixture was stirred at 0°C for 15 min. Then, 1-ethyl-3-(3-dimethylaminopropyl)carbodiimide hydrochloride (0.73 g) was added, and the mixture was stirred at room temperature for 8 hr. The reaction mixture was concentrated under reduced pressure. Aqueous potassium carbonate solution was added to the residue and the mixture was extracted with ethyl acetate. The extract was dried over magnesium sulfate and concentrated under reduced pressure. The obtained residue was purified by silica gel chromatography to give 0.89 g of N-(5-((3R)-3-(4-amino-5-chloro-2-methoxybenzoylaminomethyl)pyrrolidin-1-yl)pentyl)-1-methyl-1H-indole-3-carboxamide.
¹H-NMR(CDCl₃,ppm) δ : 1.38-1.71 (7H,m), 1.95-2.10(1H,m), 2.40-2.79(7H,m), 3.37-3.53(4H,m), 3.81(3H,s), 3.87(3H,s), 4.39(2H,s), 5.98-6.10(1H,br), 6.28(1H,s), 7.18-7.36(3H,m), 7.68(1H,s), 7.80-7.88(1H,br), 7.94-7.97(1H,m), 8.07(1H,s)

### Example 89

N-(5-((3S)-3-tert-Butoxycarbonylaminomethylpyrrolidin-1-yl)pentyl)-1-methyl-1H-indole-3-carboxamide (1.48 g) as starting compound was reacted and treated in the same manner as in Example 67 using 4-amino-5-chloro-2-methoxybenzoic acid (0.68 g) to give N-(5-((3S)-3-(4-amino-5-chloro-2-methoxybenzoylaminomethyl)pyrrolidin-1-yl)pentyl)-1-methyl-1H-indole-3-carboxamide.
¹H-NMR(CDCl₃,ppm) δ : 1.38-1.71 (7H,m), 1.95-2.10(1H,m), 2.40-2.79(7H,m), 3.37-3.53(4H,m), 3.81(3H,s), 3.87(3H,s), 4.39(2H,s), 5.98-6.10(1H,br), 6.28(1H,s), 7.18-7.36(3H,m), 7.68(1H,s), 7.80-7.88(1H,br), 7.94-7.97(1H,m), 8.07(1H,s)

### Example 90

N-(5-(4-tert-Butoxycarbonylaminomethylpiperidin-1-yl)pentyl)-1-methyl-1H-indole-3-carboxamide (1.12 g) was dissolved in 4N hydrochloric acid-diode solution (30 ml) and the mixture was stood at room temperature for 1 hr. The reaction mixture was concentrated under reduced pressure. Dimethylformamide (30 ml) was added to the residue and the mixture was neutralized with triethylamine (0.68 ml). 4-Amino-5-chloro-2-methoxybenzoic acid (0.49 g) and 1-hydroxybenzotriazole (0.36 g) were added thereto, and the mixture was stirred at 0°C for 20 min. Then, 1-ethyl-3-(3-dimethylaminopropyl)carbodiimide hydrochloride (0.52 g) was added, and the mixture was stirred at room temperature for 12 hr. The reaction mixture was concentrated under reduced pressure. Aqueous potassium carbonate solution was added to the residue and the mixture was extracted with ethyl acetate. The extract was dried over magnesium sulfate and concentrated under reduced pressure. The obtained residue was purified by silica gel chromatography to give 0.60 g of N-(5-(4-(4-amino-5-chloro-2-methoxybenzoylaminomethyl)piperidin-1-yl)pentyl)-1-methyl-1H-indole-3-carboxamide.
¹H-NMR(CDCl₃,ppm) δ : 1.25-1.79(11H,m), 1.85-2.04(2H,m), 2.28-2.41(2H,m), 2.89-3.03(2H,m), 3.25-3.36(2H,m), 3.42-3.55(2H,m), 3.80(3H,s), 3.87(3H,s), 4.42(2H,s), 5.97-6.15(1H,br), 6.29(1H,s), 7.20-7.38(3H,m), 7.66(1H,s), 7.65-7.82(1H,br), 7.90-7.96(1H,m), 8.10(1H,s)

### Example 91

N-(6-((3R)-3-tert-Butoxycarbonylaminomethylpyrrolidin-1-yl)hexyl)-1-methyl-1H-indole-3-carboxamide (0.62 g) as starting compound was reacted and treated in the same manner as in Example 67 using 4-amino-5-chloro-2-methoxybenzoic acid (0.28 g) to give N-(6-((3R)-3-(4-amino-5-chloro-2-methoxybenzoylaminomethyl)pyrrolidin-1-yl)hexyl)-1-methyl-1H-indole-3-carboxamide.
¹H-NMR(DMSO-d₆,ppm) δ :1.20-1.71(9H,m), 1.80-1.94(1H,m), 2.05-2.20(1H,m), 2.49(6H,s), 5.90-5.95(1H,br), 6.48(1H,s), 7.07-7.25(3H,m), 7.42-7.52(1H,m), 7.80-7.91(1H,br), 8.08-8.18(1H,m), 8.31(1H,s)

### Example 92

N-(6-((3S)-3-tert-Butoxycarbonylaminomethylpyrrolidin-1-yl)hexyl)-1-methyl-1H-indole-3-carboxamide (1.00 g) as starting compound was reacted and treated in the same manner as in Example 67 using 4-amino-5-chloro-2-methoxybenzoic acid (0.50 g) to give N-(6-((3S)-3-(4-amino-5-chloro-2-methoxybenzoylaminomethyl)pyrrolidin-1-yl)hexyl)-1-methyl-1H-indole-3-carboxamide.
¹H-NMR(DMSO-d₆,ppm) δ :1.20-1.71(9H,m), 1.80-1.94(1H,m), 2.05-2.20(1H,m), 2.49(6H,s), 5.90-5.95(1H,br), 6.48(1H,s), 7.07-7.25(3H,m), 7.42-7.52(1H,m), 7.80-7.91(1H,br), 8.08-8.18(1H,m), 8.31(1H,s)

### Example 93

N-(4-((3R)-3-tert-Butoxycarbonylaminomethylpyrrolidin-1-yl)butyl)-1-methyl-1H-indole-2-carboxamide (0.95 g) as starting compound was reacted and treated in the same manner as in Example 67 using 4-amino-5-chloro-2-methoxybenzoic acid (0.45 g) to give N-(4-((3R)-3-(4-amino-5-chloro-2-methoxybenzoylaminomethyl)pyrrolidin-1-yl)butyl)-1-methyl-1H-indole-2-carboxamide.
¹H-NMR (CDCl₃,ppm) δ : 1.44-1.80(5H,m), 1.89-2.08(1H,m), 2.31-2.78(7H,m), 3.31-3.52(4H,m), 3.82(3H,s), 4.05(3H,s), 4.35(2H,s), 6.23(1H,s), 6.81(1H,s), 7.05-7.38(4H,m), 7.55-7.64(1H,m), 7.72-7.83(1H,br), 8.07(1H,s)

### Example 94

N-(4-((3R)-3-tert-Butoxycarbonylaminomethylpyrrolidin-1-yl)-butyl)-1-isopropyl-1H-indole-3-carboxamide (1.34 g) was dissolved in 4N hydrochloric acid-dioxane solution (30 ml) and the mixture was stood at room temperature for 60 min. The reaction mixture was concentrated under reduced pressure. Dimethylformamide (30 ml) was added to the residue and the mixture was neutralized with triethylamine (0.81 ml). 4-Amino-5-chloro-2-methoxybenzoic acid (0.59 g) and 1-hydroxybenzotriazole (0. 44 g) were added thereto, and the mixture was stirred at 0°C for 15 min. Then, 1-ethyl-3-(3-dimethylaminopropyl)carbodiimide hydrochloride (0.62 g) was added, and the mixture was stirred at room temperature for 8 hr. The reaction mixture was concentrated under reduced pressure. Aqueous potassium carbonate solution was added to the residue and the mixture was extracted with ethyl acetate. The extract was dried over magnesium sulfate and concentrated under reduced pressure. The obtained residue was purified by silica gel chromatography to give 0.62 g of N-(4-((3R)-3-(4-amino-5-chloro-2-methoxybenzoylaminomethyl)pyrrolidin-1-yl)butyl)-1-isopropyl-1H-indole-3-carboxamide.
¹H-NMR (CDCl₃,ppm) δ : 1.53(6H,d,J=6.6Hz), 1.55-1.78(5H,m), 1.88-2.05(1H,m), 2.31-2.78(7H,m), 3.33-3.55(4H,m), 3.85(3H,s), 4.36(2H,s), 6.25(1H,s), 6.25-6.35(1H,br), 7.28-7.45(3H,m), 7.75-7.85(2H,m), 7.90-7.95(1H,m), 8.07(1H,s)

### Example 95

N-(4-((3R)-3-tert-Butoxycarbonylaminomethylpyrrolidin-1-yl)butyl)-1-benzyl-1H-indole-3-carboxamide (1.35 g) was dissolved in 4N hydrochloric acid-dioxane solution (30 ml) and the mixture was stood at room temperature for 60 min. The reaction mixture was concentrated under reduced pressure. Dimethylformamide (30 ml) was added to the residue and the mixture was neutralized with triethylamine (0.74 ml). 4-Amino-5-chloro-2-methoxybenzoic acid (0.54 g) and 1-hydroxybenzotriazole (0.40 g) were added thereto, and the mixture was stirred at 0°C for 15 min. Then, 1-ethyl-3-(3-dimethylaminopropyl)carbodiimide hydrochloride (0.56 g) was added, and the mixture was stirred at room temperature for 8 hr. The reaction mixture was concentrated under reduced pressure. Aqueous potassium carbonate solution was added to the residue and the mixture was extracted with ethyl acetate. The extract was dried over magnesium sulfate and concentrated under reduced pressure. The obtained residue was purified by silica gel chromatography to give 1.03 g of N-(4-((3R)-3-(4-amino-5-chloro-2-methoxybenzoylaminomethyl)pyrrolidin-1-yl)butyl)-1-benzyl-1N-indole-3-carboxamide.
¹H-NMR (CDCl₃,ppm) δ : 1.42-1.78(5H,m), 1.85-2.03(1H,m), 2.29-2.78(7H,m), 3.35-3.64(4H,m), 3.83(3H,s), 4.31(2H,s), 5.31(2H,s), 6.22(1H,s), 6.30-6.40(1H,br), 7.08-7.35(6H,m), 7.69(1H,s), 7.75-7.84(1H,br), 7.95-8.03(1H,m), 8.07(1H,s)

### Example 96

N-(4-(3-tert-Butoxycarbonylaminomethylpyrrolidin-1-yl)butyl)-N-methylbenzamide (0.57 g) as starting compound was reacted and treated in the same manner as in Example 67 using 4-amino-5-chloro-2-methoxybenzoic acid (0.30 g) to give 4-amino-N-(1-(4-(N-benzoyl-N-methylamino)butyl)pyrrolidin-3-ylmethyl)-5-chloro-2-methoxybenzamide.
¹H-NMR (CDCl₃,ppm) δ : 1.25-1.43(1H,m), 1.45-1.79(4H,m), 1.90-2.11(1H,m), 2.22-3.12(10H,m), 3.23-3.55(4H,m), 3.86(3H,s), 4.47(2H,s), 6.30(1H,s), 7.30-7.45(5H,m), 7.75-7.89(1H,br), 8.07(1H,s)

### Example 97

N-(4-(3-tert-Butoxycarbonylaminomethylpyrrolidin-1-yl)butyl)-1-methyl-1H-indole-3-carboxamide (1.00 g) as starting compound was reacted and treated in the same manner as in Example 67 using 4-amino-5-chloro-2-cyclopropylmethyloxybenzoic acid (0.56 g) to give N-(4-(3-(4-amino-5-chloro-2-cyclopropylmethyloxybenzoylaminomethyl)-pyrrolidin-1-yl)butyl)-1-methyl-1H-indole-3-carboxamide.
¹H-NMR (CDCl₃,ppm) δ : 0.45-0.53(2H,m), 0.62-0.75(2H,m), 1.20-1.35(1H,m), 1.50-1.79(5H,m), 1.98-2.13(1H,m), 2.39-3.00(7H,m), 3.38-3.55(4H,m), 3.70-3.85(5H,m), 4.35(2H,s), 6.20(1H,s), 6.35-6.45(1H,br), 7.18-7.35(3H,m), 7.71(1H,s), 7.95-8.05(1H,br), 8.09(1H,s), 8.10-8.15(1H,br)

### Example 98

N-(4-(4-tert-Butoxycarbonylaminomethylpiperidin-1-yl)butyl)-1-methyl-1H-indole-3-carboxamide (1.40 g) was dissolved in 4N hydrochloric acid-dioxane solution (50 ml) and the mixture was stood at room temperature for 2.5 hr. The reaction mixture was concentrated under reduced pressure. Dimethylformamide (30 ml) was added to the residue and the mixture was neutralized with triethylamine (1.45 ml). 4-Amino-5-chloro-2-methorybenzoic acid (0.64 g) and 1-hydroxybenzotriazole (0.49 g) were added thereto, and the mixture was stirred at 0°C for 15 min. Then, 1-ethyl-3-(3-dimethylaminopropyl)carbodiimide hydrochloride (0.67 g) was added, and the mixture was stirred at room temperature for 16 hr. The reaction mixture was concentrated under reduced pressure. Aqueous potassium carbonate solution was added to the residue and the mixture was extracted with ethyl acetate. The extract was dried over magnesium sulfate and concentrated under reduced pressure. The obtained residue was purified by silica gel chromatography to give 0.63 g of N-(4-(4-(4-amino-5-chloro-2-methoxybenzoylaminomethyl)piperidin-1-yl)butyl)-1-methyl-1H-indole-3-carboxamide.
¹H-NMR (CDCl₃,ppm) δ : 1.20-1.38(3H,m), 1.45-1.99(6H,m), 2.30-2.43(2H,m), 2.88-2.99(4H,m), 3.25-3.33(2H,m), 3.45-3.56(2H,m), 3.81(3H,s), 3.88(3H,s), 4.35(2H,s), 6.15-6.22(1H,br), 6.27(1H,s), 7.20-7.35(3H,m), 7.63(1H,s), 7.65-7.75(1H,br), 7.88-7.95(1H,m), 8.10(1H,s)

### Example 99

N-(3-((3R)-3-tert-Butoxycarbonylaminomethylpyrrolidin-1-yl)propyl)-3-phenylpropylamide (1.74 g) as starting compound was reacted and treated in the same manner as in Example 67 using 4-amino-5-chloro-2-methoxybenzoic acid (0.90 g) to give 4-amino-5-chloro-2-methoxy-N-((3R)-1-(3-(3-phenylpropionylamino)propyl)pyrrolidin-3-ylmethyl)benzamide.
¹H-NMR (CDCl₃,ppm) δ : 1.45-1.68(3H,m), 1.90-2.04(1H,m), 2.25-2.72(9H,m), 2.88-2.99(2H,m), 3.25-3.50(4H,m), 3.88(3H,s), 4.43(2H,s), 6.30(1H,s), 6.75-6.86(1H,br), 7.14-7.33(5H,m), 7.72-7.82(1H,br), 8.09(1H,s)

### Example 100

N-(2-((3R)-3-tert-Butoxycarbonylaminomethylpyrrolidin-1-yl)ethyl)-4-phenylbutylamide (1.55 g) was dissolved in 4N hydrochloric acid-dioxane solution (10 ml) and the mixture was stood at room temperature for 30 min. The reaction mixture was concentrated under reduced pressure. Dimethylformamide (30 ml) was added to the residue and the mixture was neutralized with triethylamine (1.7 ml). 4-Amino-5-chloro-2-methoxybenzoic acid (0.80 g) and 1-hydroxybenzotriazole (0.59 g) were added thereto, and the mixture was stirred at 0°C for 15 min. Then, 1-ethyl-3-(3-dimethylaminopropyl)carbodiimide hydrochloride (0.84 g) was added, and the mixture was stirred at room temperature for 8 hr. The reaction mixture was concentrated under reduced pressure. Aqueous potassium carbonate solution was added to the residue and the mixture was extracted with ethyl acetate. The extract was dried over magnesium sulfate and concentrated under reduced pressure. The obtained residue was purified by silica gel chromatography to give 1.02 g of 4-amino-5-chloro-2-methoxy-N-((3R)-1-(2-(4-phenylbutyrylamino)ethyl)pyrrolidin-3-ylmethyl)benzamide.
¹H-NMR (CDCl₃,ppm) δ : 1.40-1.60(1H,m), 1.88-2.04(3H,m), 2.12-2.23(2H,m), 2.33-2.75(9H,m), 3.25-3.55(4H,m), 3.87(3H,s), 4.47(2H,s), 6.25-6.38(2H,br), 7.10-7.32(5H,m), 7.75-7.88(1H,br), 8.08(1H,s)

### Example 101

3-tert-Butoxycarbonylaminomethyl-1-(2-methylsulfonylaminoethyl)pyrrolidine (2 g) as starting compound was reacted and treated in the same manner as in Example 67 using 4-amino-5-chloro-2-methoxybenzoic acid (1.25 g) to give 4-amino-5-chloro-2-methoxy-N-(2-methylsulfonylaminoethyl)pyrrolidin-3-ylmethyl)benzamide.
¹H-NMR (CDCl₃,ppm) δ : 1.48-1.64(1H,m), 1.93-2.08(1H,m), 2.40-2.79(7H,m), 2.95(3H,s), 3.15-3.58(4H,m), 3.92(3H,s), 4.41(2H,s), 5.18-5.34(1H,br), 6.33(1H,s), 7.79-8.88(1H,br), 8.09(1H,s)

### Example 102

3-tert-Butoxycarbonylaminomethyl-1-(3-(1,1,3-trioxo-2,3-dihydro-1,2-benzisothiazol-2-yl)propyl)pyrrolidine (0.28 g) as starting compound was reacted and treated in the same manner as in Example 67 using 4-amino-5-chloro-2-methoxybenzoic acid (0.13 g) to give 4-amino-5-chloro-2-methoxy-N-(1-(3-(1,1,3-trioxo-2,3-dihydro-1,2-benzisothiazol-2-yl)propyl)pyrrolidin-3-ylmethyl)benzamide.
¹H-NMR (CDCl₃,ppm) δ :1.50-1.58(1H,m),1.95-2.12(3H,m),2.39-2.74(7H,m), 3.30-3.47(2H,m),3.81-3.95(2H,m),3.90(3H,s),4.36(1H,s),6.29(1H,s), 7.80-7.94(3H,m),8.04-8.08(1H,m),8.09(1H,s)

### Example 103

3-tert-Butoxycarbonylaminomethyl-1-(3-(2,3-dihydro-2-oxobenzimidazol-1-yl)propyl)pyrrolidine (1.31 g) as starting compound was reacted and treated in the same manner as in Example 67 using 4-amino-5-chloro-2-methoxybenzoic acid (0.7 g) to give 4-amino-5-chloro-N-(1-(3-(2,3-dihydro-2-oxobenzimidazol-1-yl)propyl)pyrrolidin- 3-ylmethyl)-2-methoxybenzamide.
¹H-NMR (CDCl₃,ppm) δ :1.48-1.68(1H,m),1.93-2.11(1H,m), 2.40-2.80(7H,m), 3.43(2H,t,J=6.3Hz), 3.85(3H,s), 3.95(2H,t,J=6.9Hz), 4.40(2H,s), 6.26(1H,s), 6.98-7.22(4H,m), 7.77-7.89(1H,br), 8.07(1H,s), 9.18-9.30(1H,br)

### Example 104

4-Amino-5-chloro-2-methoxy-N-(piperidin-4-ylmethyl)benzamide hydrochloride (8.0 g) was dissolved in dimethylformamide (100 ml) and toluene (100 ml). Potassium carbonate (9.9 g) and 4-bromopropylphthalimide (6.4 g) were added, and the mixture was stirred at 70°C for 5 hr. The reaction mixture was concentrated under reduced pressure and the residue was extracted with ethyl acetate. The extract was dried over magnesium sulfate and concentrated under reduced pressure. The obtained residue was purified by silica gel chromatography to give 4.0 g of 4-amino-5-chloro-N-(1-(3-(2,3-dihydro-1,3-dioxo-1H-isoindol-2-yl)propyl)piperidin-4-ylmethyl)-2-methoxybenzamide.
¹H-NMR (CDCl₃,ppm) δ :1.05-1.22(2H,m), 1.41-1.70(3H,m), 1.75-1.95(4H,m), 2.33-2.45(2H,m), 2.80-2.93(2H,m), 3.18-3.25(2H,m), 3.72-3.80(2H,m), 3.88(3H,s), 4.43(2H,s), 6.30(1H,s), 7.65-7.88(4H,m), 8.08(1H,s)

### Example 105

4-Amino-5-chloro-2-methoxy-N-(piperidin-4-ylmethyl)benzamide hydrochloride (15.0 g) was dissolved in dimethylforaamide (100 ml) and toluene (150 ml). Potassium carbonate (18.6 g) and 4-bromobutylphthalimide (12.7 g) were added, and the mixture was stirred at 70°C for 2 hr. The reaction mixture was concentrated under reduced pressure and the residue was extracted with ethyl acetate. The extract was dried over magnesium sulfate and concentrated under reduced pressure. The obtained residue was purified by silica gel chromatography to give 13.6 g of 4-amino-5-chloro-N-(1-(4-(2,3-dihydro-1,3-dioxo-1H-isoindol-2-yl)butyl)piperidin-4-ylmethyl)-2-methoxybenzamide.
¹H-NMR(CDCl₃,ppm) δ : 1.20-1.39(2H,m), 1.55-1.98(9H,m), 2.28-2.41(2H,m), 2.85-2.97(2H,m), 3.25-3.37(2H,m), 3.66-3.78(2H,m), 3.89(3H,s), 4.42(2H,s), 6.29(1H,s), 7.65-7.86(4H,m), 8.09(1H,s)

### Example 106

4-Amino-5-chloro-2-methoxy-N-(piperidin-4-ylmethyl)benzamide hydrochioride (23.0 g) was dissolved in dimethylformamide (250 ml). Potassium carbonate (28.5 g) and 5-bromopentylphthalimide (20.4 g) were added, and the mixture was stirred at 50°C for 8 hr. The reaction mixture was concentrated under reduced pressure and the residue was extracted with ethyl acetate. The extract was dried over magnesium sulfate and concentrated under reduced pressure. The obtained residue was purified by silica gel chromatography to give 20.0 g of 4-amino-5-chloro-N-(1-(5-(2,3-dihydro-1,3-dioxo-1H-isoindol-2-yl)pentyl)piperidin-4-ylmethyl)-2-methoxybenzamide.
¹H-NMR (CDCl₃,ppm) δ :1.20-1.98(11H,m), 2.00-2.11(2H,m), 2.30-2.40(2H,m), 2.85-2.97(2H,m), 3.25-3.37(2H,m), 3.66-3.78(2H,m), 3.89(3H,s), 4.42(2H,s), 6.29(1H,s), 7.63-7.85(4H,m), 8.09(1H,s)

### Example 107

4-Amino-5-chloro-2-methoxy-N-(piperidin-4-ylmethyl)benzamide hydrochloride (8.5 g) was dissolved in dimethylformamide (100 ml). Potassium carbonate (10.5 g) and 6-bromoherylphthalimide (7.2 g) were added, and the mixture was stirred at 70°C for 16 hr. The reaction mixture was concentrated under reduced pressure and the residue was extracted with ethyl acetate. The extract was dried over magnesium sulfate and concentrated under reduced pressure. The obtained residue was purified by silica gel chromatography to give 4.2 g of 4-amino-5-chloro-N-(1-(6-(2,3-dihydro-1,3-dioxo-1H-isoindol-2-yl)hexyl)piperidin-4-ylmethyl)-2-methoxybenzamide.
¹H-NMR (CDCl₃,ppm) δ :1.30-1.82(13H,m), 1.95-2.08(2H,m), 2.33-2.42(2H,m), 2.95-3.05(2H,m), 3.25-3.38(2H,m), 3.64-3.75(2H,m), 3.89(3H,s), 4.48(2H,s), 6.32(1H,s), 7.68-7.82(4H,m), 8.08(1H,s)

### Example 108

4-Amino-N-(1-(3-aminopropyl)piperidin-4-ylmethyl)-5-chloro-2-methoxybenzamide (0.70 g) was dissolved in dimethylformamide (10 ml). Triethylamine (0.30 ml) was added, and a solution of benzoyl chloride (0.25 ml) in methylene chloride was dropwise added under ice-cooling. The mixture was stirred at room temperature. Insoluble matter was filtered off and the filtrate was concentrated under reduced pressure. The obtained residue was purified by silica gel chromatography to give 0.15 g of 4-amino-N-(1-(3-benzoylaminopropyl)piperidin-4-ylmethyl)-5-chloro-2-methoxybenzamide.
¹H-NMR (CDCl₃,ppm) δ : 1.25-1.50(2H,m), 1.62-1.95(5H,m), 2.05-2.21(2H,m), 2.62-2.75(2H,m), 3.10-3.20(2H,m), 3.25-3.38(2H,m), 3.48-3.63(2H,m), 3.86(3H,s), 4.43(2H,s), 6.29(1H,s), 7.33-7.50(3H,m), 7.65-7.75(1H,br), 7.76-7.90(2H,m), 8.09(1H,s), 8.20-8.35(1H,br)

### Example 109

4-Amino-N-(1-(4-aminobutyl)piperidin-4-ylmethyl)-5-chloro-2-methoxybenzamide (1.00 g) was dissolved in a mixed solvent of dichloromethane (20 ml) and dimethylformamide (15 ml). Triethylamine (0.57 ml) was added, and a solution of benzenesulfonyl chloride (0.35 ml) in methylene chloride was dropwise added under ice-cooling. The mixture was stirred at room temperature. Insoluble matter was filtered off and the filtrate was concentrated under reduced pressure. The obtained residue was purified by silica gel chromatography to give 0.57 g of 4-amino-N-(1-(4-benzenesulfonylaminobutyl)piperidin-4-yl-methyl)-5-chloro-2-methoxybenzamide.
¹H-NMR (CDCl₃,ppm) δ : 1.45-1.83(9H,m), 1.98-2.15(2H,m), 2.31-2.47(2H,m), 2.88-3.10(4H,m), 3.30-3.42(2H,m), 3.89(3H,s), 4.40(2H,s), 6.28(1H,s), 7.40-7.59(3H,m), 7.78-7.90(3H,m), 8.10(1H,s)

### Example 110

4-Amino-N-(1-(5-aminopentyl)piperidin-4-ylmethyl)-5-chloro-2-methoxybenzamide (0.53 g) was dissolved in dimethylformamide (20 ml). 1-Methylindazole-3-carboxylic acid (0.27 g) and 1-hydroxybenzotriazole (0.25 g) were added, and the mixture was stirred at 0°C for 15 min. Then, 1-ethyl-3-(3-dimethylaminopropyl)carbodiimide (0.35 g) was added, and the mixture was stirred at room temperature for 16 hr. The reaction mixture was concentrated under reduced pressure. Aqueous potassium carbonate solution was added to the residue and the mixture was extracted with ethyl acetate. The extract was dried over magnesium sulfate and concentrated under reduced pressure. The obtained residue was purified by silica gel chromatography to give 0.20 g of N-(5-(4-(4-amino-5-chloro-2-methoxybenzoylaminomethyl)piperidin-1-yl)pentyl)-1-methyl-1H-indazole-3-carboxamide.
Melting point 74∼77°C

### Example 111

5-(4-tert-Butoxycarbonylaminomethylpiperidin-1-yl)pentylamine (1.42 g) was dissolved in methylene chloride (15 ml). Triethylamine (1.39 ml) was added, and a solution of 2-chlorobenzoyl chloride (0.84 ml) in methylene chloride was dropwise added under ice-cooling. The mixture was stirred at room temperature for 2 hr. The reaction mixture was concentrated under reduced pressure. This was dissolved in 4N hydrochloric acid-dioxane solution (10 ml) and the mixture was stood at room temperature for 30 min. The reaction mixture was concentrated under reduced pressure. Dimethylformamide (30 ml) was added to the residue and the mixture was neutralized with triethylamine (2.05 ml). 4-Amino-5-chloro-2-methoxybenzoic acid (0.99 g) and 1-hydroxybenzotriazole (0.73 g) were added, and the mixture was stirred at 0°C for 15 min. Then, 1-ethyl-3-(3-dimethylaminopropyl)carbodiimide hydrochloride (1.04 g) was added, and the mixture was stirred at room temperature for 8 hr. The reaction mixture was concentrated under reduced pressure. Aqueous potassium carbonate solution was added to the residue and the mixture was extracted with ethyl acetate. The extract was dried over magnesium sulfate and concentrated under reduced pressure. The obtained residue was purified by silica gel chromatography to give 0.63 g of 4-amino-5-chloro-N-(1-(5-(2-chlorobenzoyl)aminopentyl)piperidin-4-ylmethyl)-2-methoxybenzamide.
¹H-NMR (CDCl₃,ppm) δ : 1.38-1.92(11H,m), 2.15-2.38(2H,m), 2.52-2.70(2H,m), 3.15-3.39(4H,m), 3.42-3.55(2H,m), 3.90(3H,s), 4.45(2H,s), 6.31(1H,s), 6.32-6.48(1H,br), 7.25-7.42(4H,m), 7.58-7.68(1H,m), 7.72-7.83(1H,br), 8.07(1H,s)

### Example 112

5-(4-tert-Butoxycarbonylaminomethylpiperidin-1-yl)pentylamine (1.39 g) was dissolved in methylene chloride (15 ml). Triethylamine (1.36 ml) was added, and a solution of 4-chlorobenzoyl chloride (0.83 ml) in methylene chloride was dropwise added under ice-cooling. The mixture was stirred at room temperature for 2 hr. The reaction mixture was concentrated under reduced pressure. This was dissolved in 4N hydrochloric acid-dioxane solution (10 ml) and the mixture was stood at room temperature for 30 min. The reaction mixture was concentrated under reduced pressure. Dimethylformamide (30 ml) was added to the residue and the mixture was neutralized with triethylamine (1.99 ml). 4-Amino-5-chloro-2-methoxybenzoic acid (0.96 g) and 1-hydroxybenzotriazole (0.71 g) were added, and the mixture was stirred at 0°C for 15 min. Then, 1-ethyl-3-(3-dimethylaminopropyl)carbodiimide hydrochloride (1.00 g) was added, and the mixture was stirred at room temperature for 8 hr. The reaction mixture was concentrated under reduced pressure. Aqueous potassium carbonate solution was added to the residue and the mixture was extracted with ethyl acetate. The extract was dried over magnesium sulfate and concentrated under reduced pressure. The obtained residue was purified by silica gel chromatography to give 0.50 g of 4-amino-5-chloro-N-(1-(5-(4-chlorobenzoyl)aminopentyl)piperidin-4-ylmethyl)-2-methoxybenzamide.
Melting point 196∼198°C

### Example 113

5-(4-tert-Butoxycarbonylaminomethylpiperidin-1-yl)pentylamine (1.50 g) was dissolved in methylene chloride (15 ml). Triethylamine (1.47 ml) was added, and a solution of benzenesulfonyl chloride (0.90 ml) in methylene chloride was dropwise added under ice-cooling. The mixture was stirred at room temperature for 2 hr. The reaction mixture was concentrated under reduced pressure. This was dissolved in 4N hydrochloric acid-dioxane solution (10 ml) and the mixture was stood at room temperature for 30 min. The reaction mixture was concentrated under reduced pressure. Dimethylformamide (30 ml) was added to the residue and the mixture was neutralized with triethylamine (1.35 ml). 4-Amino-5-chloro-2-methoxybenzoic acid (0.65 g) and 1-hydroxybenzotriazole (0.48 g) were added, and the mixture was stirred at 0°C for 15 min. Then, 1-ethyl-3-(3-dimethylaminopropyl)carbodiimide hydrochloride (0.68 g) was added, and the mixture was stirred at room temperature for 8 hr. The reaction mixture was concentrated under reduced pressure. Aqueous potassium carbonate solution was added to the residue and the mixture was extracted with ethyl acetate. The extract was dried over magnesium sulfate and concentrated under reduced pressure. The obtained residue was purified by silica gel chromatography to give 0.80 g of 4-amino-N-(1-(5-benzenesulfonylaminopentyl)piperidin-4-ylmethyl)-5-chloro-2-methoxybenzamide.
¹H-NMR (CDCl₃,ppm) δ : 1.20-1.81 (11H,m), 1.90-2.05(2H,m), 2.28-2.40(2H,m), 2.88-3.05(4H,m), 3.28-3.40(2H,m), 3.90(3H,s), 4.41(2H,s), 4.95-5.11(1H,br), 6.30(1H,s), 7.48-7.62(3H,m), 7.71-7.79(1H,br), 7.85-7.90(2H,m), 8.09(1H,s)

### Example 114

4-Amino-N-(1-(6-aminohexyl)piperidin-4-ylmethyl)-5-chloro-2-methoxybenzamide (1.00 g) was dissolved in dimethylformamide (30 ml). 1-Methylindole-3-carboxylic acid (0.48 g) and 1-hydroxybenzotriazole (0.37 g) were added and the mixture was stirred at 0°C for 15 min. Then, 1-ethyl-3-(3-dimethylaminopropyl)carbodiimide (0.53 g) was added, and the mixture was stirred at room temperature for 19 hr. The reaction mixture was concentrated under reduced pressure. Aqueous potassium carbonate solution was added to the residue and the mixture was extracted with ethyl acetate. The extract was dried over magnesium sulfate and concentrated under reduced pressure. The obtained residue was purified by silica gel chromatography to give 0.25 g of N-(6-(4-(4-amino-5-chloro-2-methoxybenzoylaminomethyl)piperidin-1-yl)hexyl)-1-methyl-1H-indole-3-carboxamide.
¹H-NMR (CDCl₃,ppm) δ : 1.20-1.78(13H,m), 1.85-2.04(2H,m), 2.28-2.41(2H,m), 2.87-3.01(2H,m), 3.28-3.38(2H,m), 3.42-3.58(2H,m), 3.81(3H,s), 3.88(3H,s), 4.42(2H,s), 5.92-6.05(1H,br), 6.27(1H,s), 7.20-7.40(3H,m), 7.65(1H,s), 7.65-7.82(1H,br), 7.87-7.96(1H,m), 8.10(1H,s)

### Example 115

4-Amino-N-(1-(6-aminohexyl)piperidin-4-ylmethyl)-5-chloro-2-methoxybenzamide (1.00 g) was dissolved in dichloromethane (25 ml). Triethylamine (0.42 ml) was added, and a solution of benzoyl chloride (0.32 ml) in methylene chloride was dropwise added under ice-cooling. The mixture was stirred at room temperature for 14 hr, and insoluble litter was filtered off. The filtrate was concentrated under reduced pressure. The obtained residue was purified by silica gel chromatography to give 0.35 g of 4-amino-N-(1-(6-benzoylaminohexyl)piperidin-4-ylmethyl)-5-chloro-2-methoxybenzamide.
Melting point 159∼162°C

### Example 116

4-Amino-N-(1-(6-aminohexyl)piperidin-4-ylmethyl)-5-chloro-2-methoxybenzamide (1.00 g) was dissolved in dimethylformamide (15 ml). A solution of phenyl isocyanate (0.30 ml) in methylene chloride was dropwise added under ice-cooling. The mixture was stirred at room temperature for 12 hr, and the reaction mixture was concentrated under reduced pressure. The obtained residue was purified by silica gel chromatography to give 0.30 g of 4-amino-5-chloro-2-methoxy-N-(1-(6-phenylureido)hexyl)piperidin-4-ylmethyl)benzamide.
Melting point 163∼166°C

### Comparative Example 1

(1) 2-(Aminomethyl)-4-benzylmorpholine obtianed by hydrolysis of 2-(acetylaminomethyl)-4-benzylmorpholine described in Journal of Medicinal Chemistry, vol. 33, p. 1406 (1990) was reacted and treated in the same manner as in Preparation Example 9, whereby the following compound was produced.
   4-benzyl-2-(tert-butoxycarbonylaminomethyl)morpholine
   ¹H-NMR (CDCl₃,ppm) δ : 1.43(9H,s), 1.85-1.95(1H,m), 2.08-2.22(1H,m), 2.60-2.78(2H,m), 3.00-3.10(1H,m), 3.20-3.35(1H,m), 3.48(2H,s), 3.50-3.69(2H,m), 3.80-3.86(1H,m), 4.83-4.92(1H,br), 7.20-7.34(5H,m)
(2) The compound obtained in the above (1) was reacted and treated in the same manner as in Preparation Example 13, whereby the following compound was produced.
   2-(tert-butoxycarbonylaminomethyl)morpholine
   ¹H-NMR (CDCl₃,ppm) δ : 1.43(9H,s), 1.80-1.95(1H,br), 2.51-2.62(1H,m), 2.79-2.90(3H,m), 2.95-3.08(1H,m), 3.20-3.35(1H,m), 3.45-3.65(2H,m), 3.80-3.89(1H,m), 4.90-5.05(1H,br)
(3) The compound obtained in the above (2) was reacted and treated in the same manner as in Preparation Example 17, whereby the following compound was produced.
   2-(4-(2-tert-butoxycarbonylaminomethylmorpholin-4-yl)butyl)-2,3-dihydro-1H-isoindole-1,3-dione
   ¹H-NMR (CDCl₃,ppm) δ : 1.44(9H,s), 1.43-1.88(5H,m), 2.04-2.14(1H,m), 2.30-2.40(2H,m), 2.65-2.77(2H,m), 3.03-3.13(1H,m), 3.20-3.38(1H,m), 3.48-3.87(4H,m), 3.83-3.94(1H,m), 7.65-7.90(4H,m)
(4) 2-(4-(2-tert-Butoxycarbonylaminomethylmorpholin-4-yl)butyl)-2,3-dihydro-1H-isoindole-1,3-dione (2.00 g) as starting compound was reacted and treated in the same manner as in Example 67 using 4-amino-5-chloro-2-methoxybenzoic acid (0.97 g) to give 4-amino-5-chloro-N-(4-(4-(2,3-dihydro-1,3-dioxo-1H-isoindol-2-yl)butyl)morpholin-2-ylmethyl)-2-methoxybenzamide.
   ¹H-NMR (CDCl₃,ppm) δ : 1.45-1.78(5H,m), 1.82-1.99(1H,m), 2.00-2.18(1H,m), 2.28-2.45(2H,m), 2.65-2.83(2H,m), 3.25-3.45(1H,m), 3.58-3.78(5H,m), 3.89(3H,s), 4.38(2H,s), 6.29(1H,s), 7.65-7.89(4H,m), 7.90-8.01(1H,br), 8.09(1H,s)

### Comparative Example 2

(1) The compound obtained in Comparative Example 1 (3) was reacted and treated in the same manner as in Preparation Example 40, whereby the following compound was produced.
   4-(2-tert-butoxycarbonylaminomethylmorpholin-4-yl)butylamine
   ¹H-NMR (CDCl₃+CD₃OD,ppm) δ : 1.44(9H,s), 1.42-1.70(3H,m), 1.80-1.92(1H,m), 2.06-2.18(1H,m), 2.28-2.38(2H,m), 2.65-2.82(2H,m), 3.03-3.13(1H,m), 3.20-3.36(1H,m), 3.48-3.70(5H,m), 3.80-3.90(1H,m)
(2) The compound obtained in the above (1) was reacted and treated in the same manner as in Preparation Example 55 using 1-methyl-1H-indole-3-carboxylic acid, whereby the following compound was produced.
   N-(4-(2-tert-butoxycarbonylaminomethylmorpholin-4-yl)butyl)-1-methyl-1H-indole-3-carboxamide
   ¹H-NMR (CDCl₃,ppm) δ : 1.44(9H,s), 1.50-1.91(5H,m), 2.04-2.16(1H,m), 2.30-2.45(2H,m), 2.65-2.79(2H,m), 2.98-3.10(1H,m), 3.18-3.34(1H,m), 3.45-3.69(4H,m), 3.80-3.90(1H,m), 3.82(3H,s), 4.78-4.90(1H,br), 6.05-6.18(1H,br), 7.20-7.41(3H,m), 7.65(1H,s), 7.88-7.94(1H,m)
(3) N-(4-(2-tert-Butoxycarbonylaminomethylmorpholin-4-yl)butyl)-1-methyl-1H-indole-3-carboxamide (1.96 g) as starting compound was reacted and treated in the same manner as in Example 67 using 4-amino-5-chloro-2-methoxybenzoic acid (0.89 g) to give N-(4-(2-(4-amino-5-chloro-2-methoxybenzoylaminomethyl)morpholin-4-yl)butyl)-1-methyl-1H-indole-3-carboxamide.
   ¹H-NMR (CDCl₃,ppm) δ : 1.50-1.72(4H,m), 1.80-1.95(2H,m), 2.03-2.17(1H,m), 2.31-2.43(2H,m), 2.66-2.82(2H,m), 3.25-3.40(1H,m), 3.44-3.55(2H,m), 3.56-3.72(3H,m), 3.81(3H,s), 3.86(3H,s), 4.41(2H,s), 6.13-6.22(1H,br), 6.28(1H,s), 7.20-7.38(3H,m), 7.65(1H,s), 7.65-7.75(1H,br), 7.88-7.98(1H,m), 8.09(1H,s)

### Comparative Example 3

2-(4-(4-(2-Hydroxyethyl)piperidin-1-yl)butyl)-2,3-dihydro-1H-isoindole-1,3-dione (0.33 g) and 5-chloro-2-methoxy-4-tritylaminobenzoic acid (0.44 g) were dissolved in tetrahydrofuran. 1,3-Carbodiimidazole (0.18 g) and 1,5-diazabicyclo[5.4.0]undecene (0.23 g) were added, and the mixture was stirred overnight at 40°C. The reaction mixture was concentrated under reduced pressure and the obtained residue was extracted with chloroform. The extract was dried over magnesium sulfate and concentrated under reduced pressure. The obtained residue was purified by silica gel chromatography to give 2-(1-(4-(1,3-dioxo-2,3-dihydro-1H-isoindol-2-yl)butyl)piperidin-4-yl)-ethyl 5-chloro-2-methoxy-4-tritylaminobenzoate. This was dissolved in 4N hydrochloric acid-dioxane solution (15 ml) and the mixture was stood at room temperature for 14 hr. The reaction mixture was concentrated under reduced pressure. Aqueous potassium carbonate solution was added to the residue and the mixture was extracted with chloroform. The extract was dried over magnesium sulfate and concentrated under reduced pressure. The obtained residue was purified by silica gel chromatography to give 2-(1-(4-(1,3-dioxo-2,3-dihydro-1H-isoindol-2-yl)butyl)piperidin-4-yl)ethyl 4-amino-5-chloro-2-methoxybenzoate.
¹H-NMR (CDCl₃,ppm) δ : 1.22-1.80(11H,m), 1.85-2.03(2H,m), 2.30-2.35(2H,m), 2.85-2.99(2H,m), 2.65-2.75(2H,m), 3.79(3H,s), 4.20-4.33(2H,m), 4.45(2H,s), 6.29(1H,s), 7.65-7.87(5H,m)

The list of the compounds produced in the above Examples and Comparative Examples is given below. In the Tables, Me means methyl, Et means ethyl, n-Pr and Pr mean n-propyl, i-Pr means isopropyl, n-Bu and Bu mean n-butyl i-Bu means isobutyl, sec-Bu means secondary butyl, t-Bu means tert-butyl, n-C₅H₁₁ means n-pentyl, i-C₅H₁₁ means isopentyl, t-C₅H₁₁ means tert-pentyl, n-C₆H₁₃ means n-hexyl, Ph means phenyl, and Bzl means benzyl.

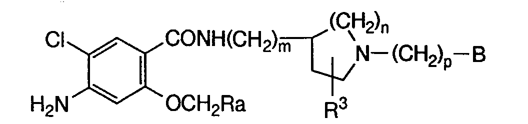

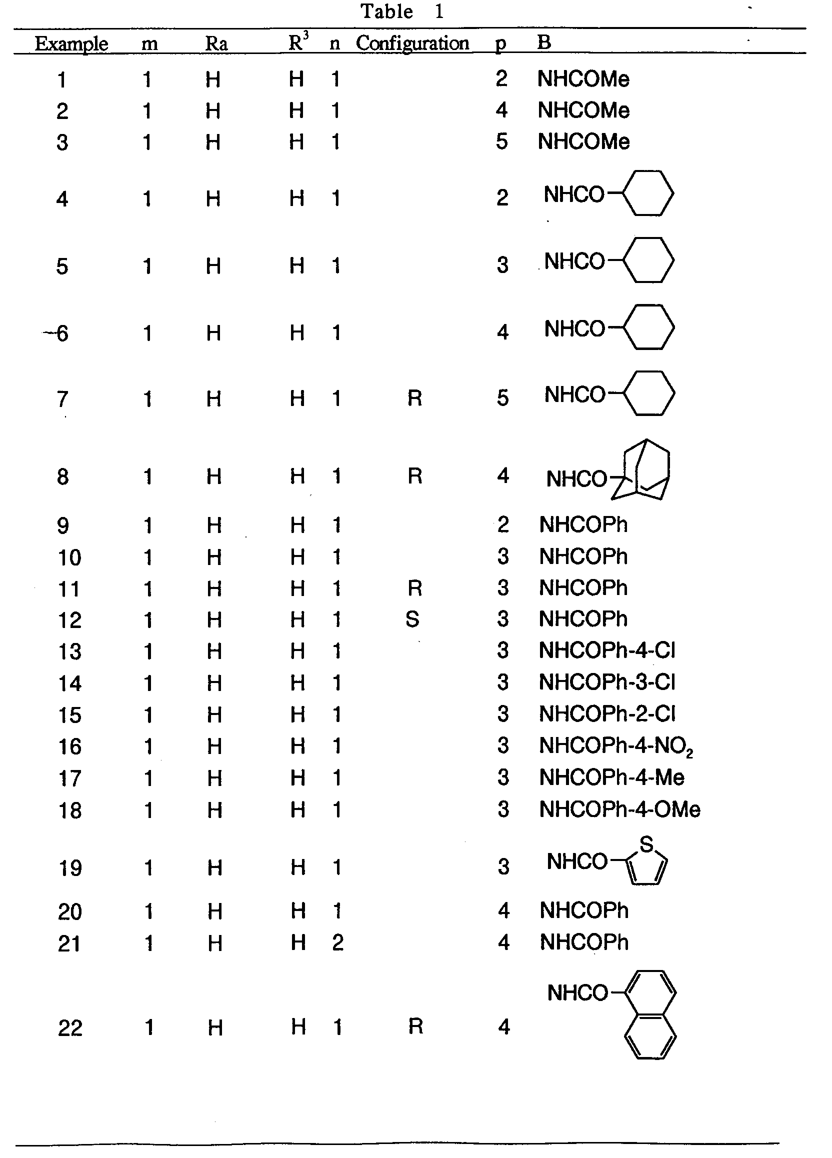

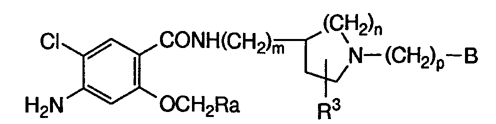

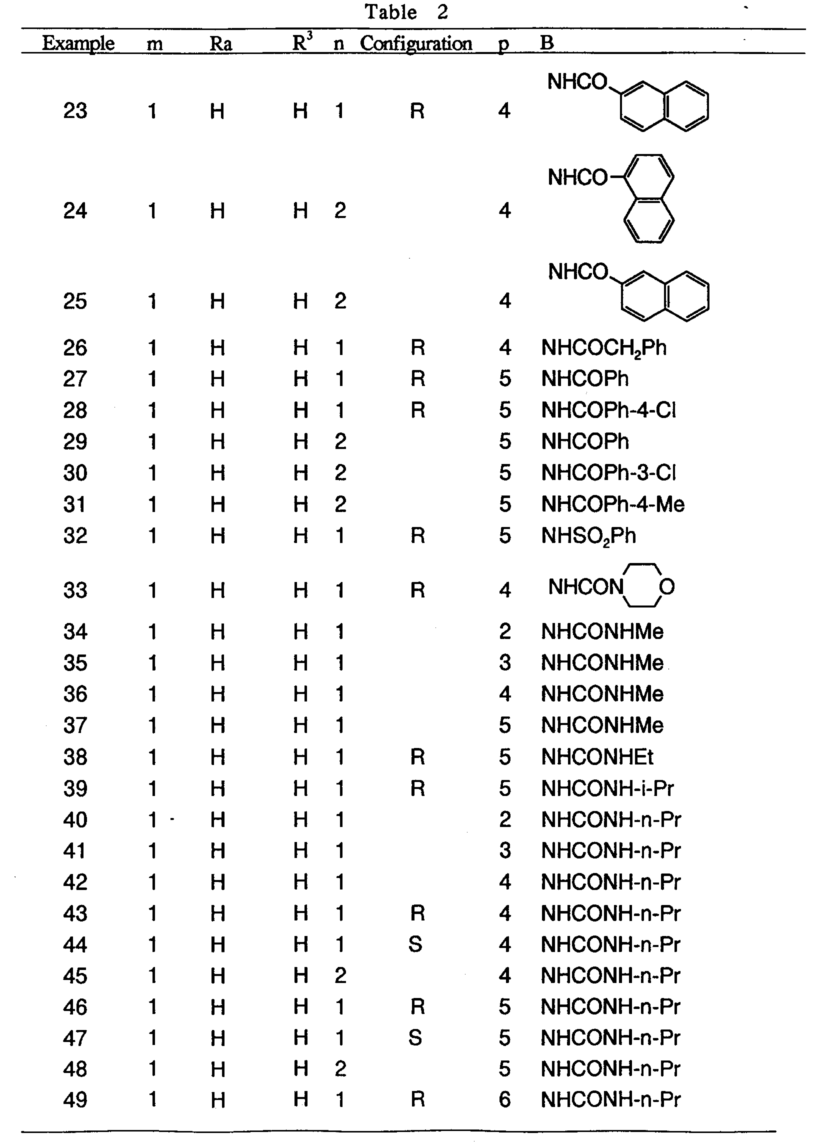

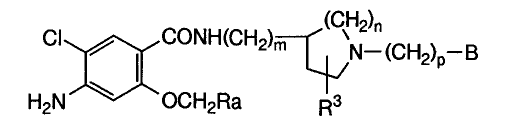

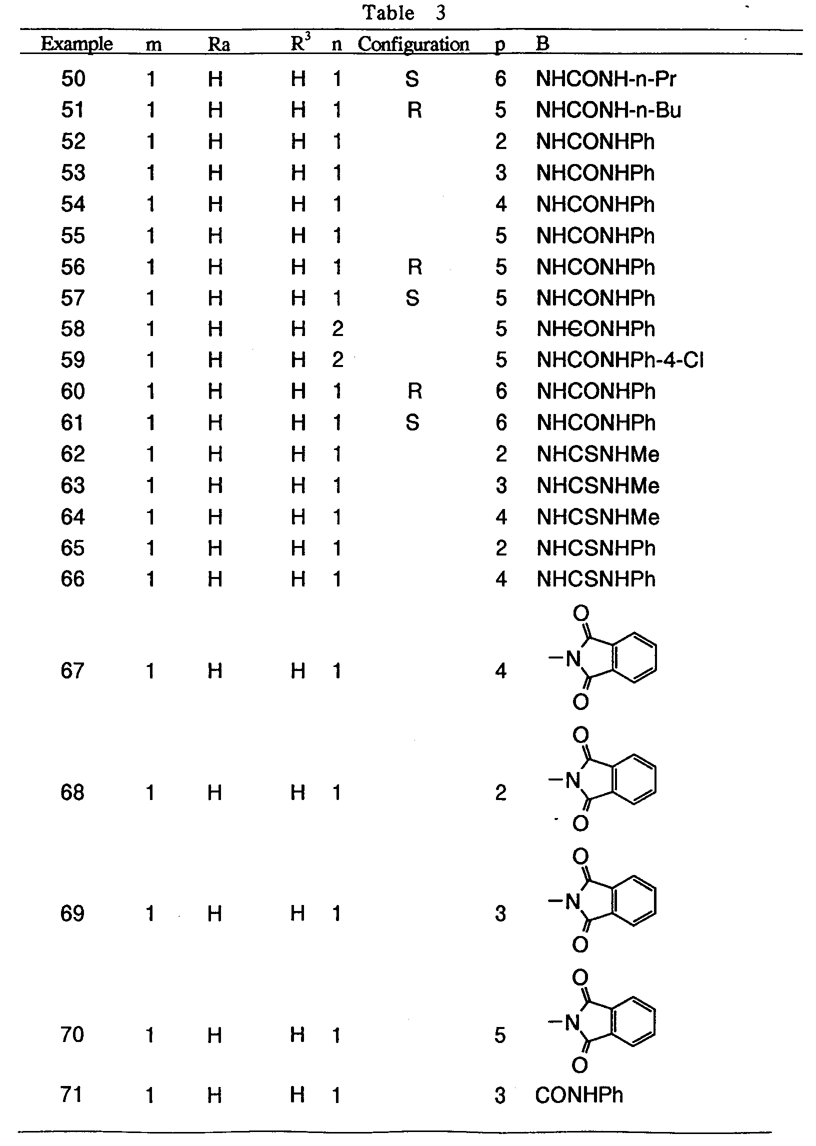

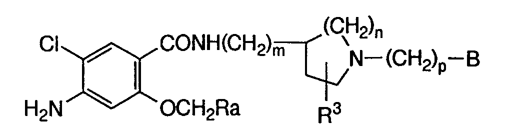

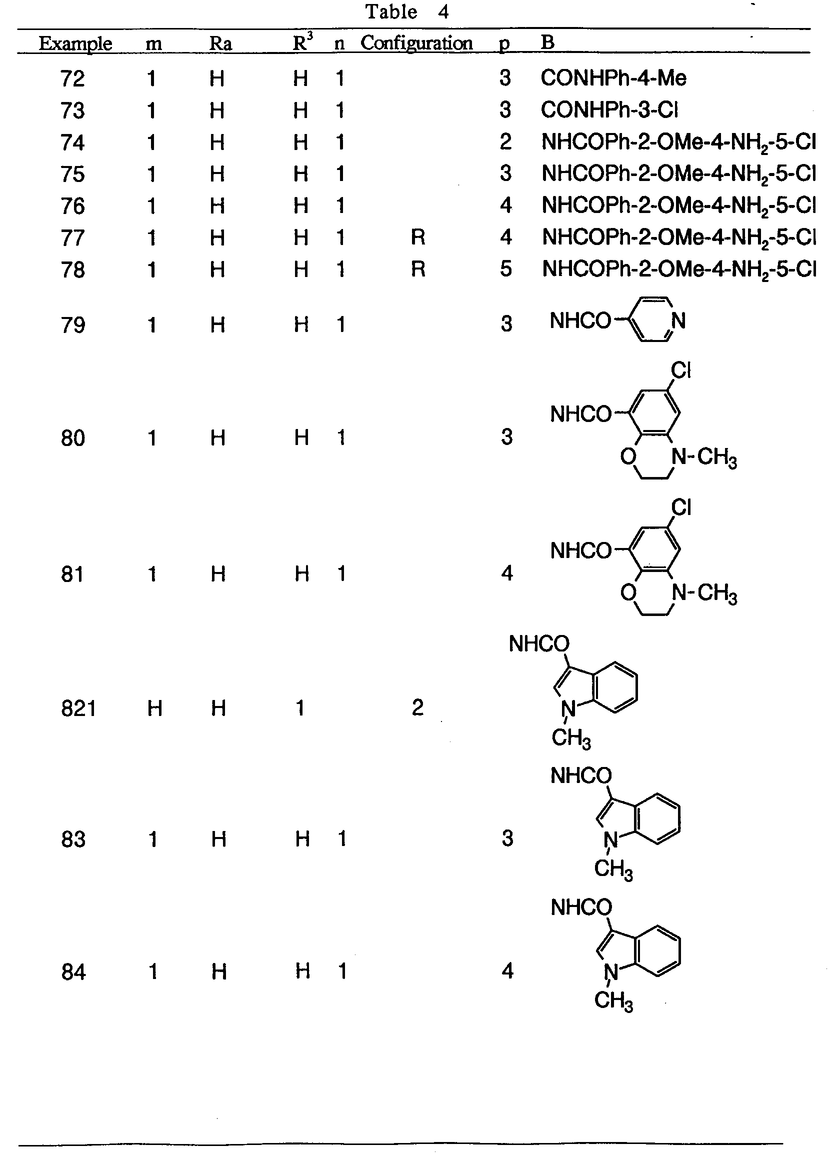

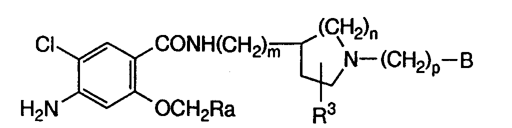

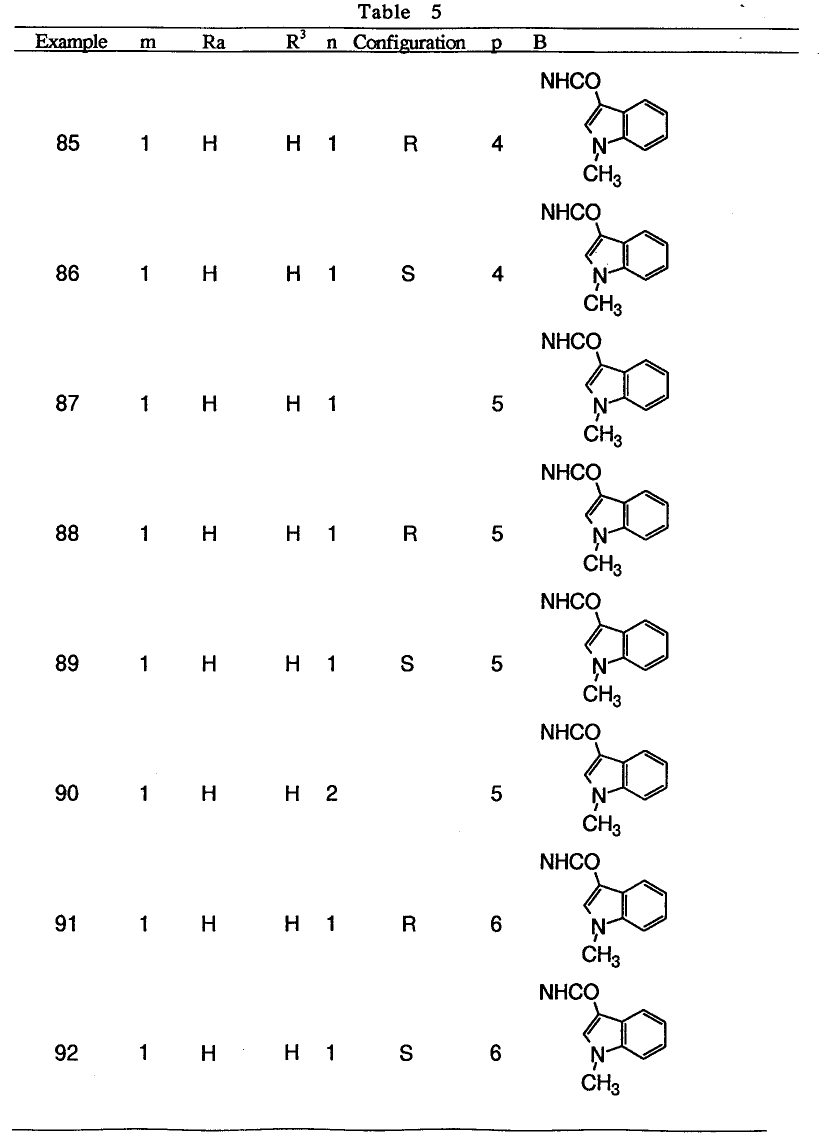

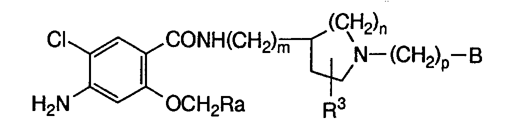

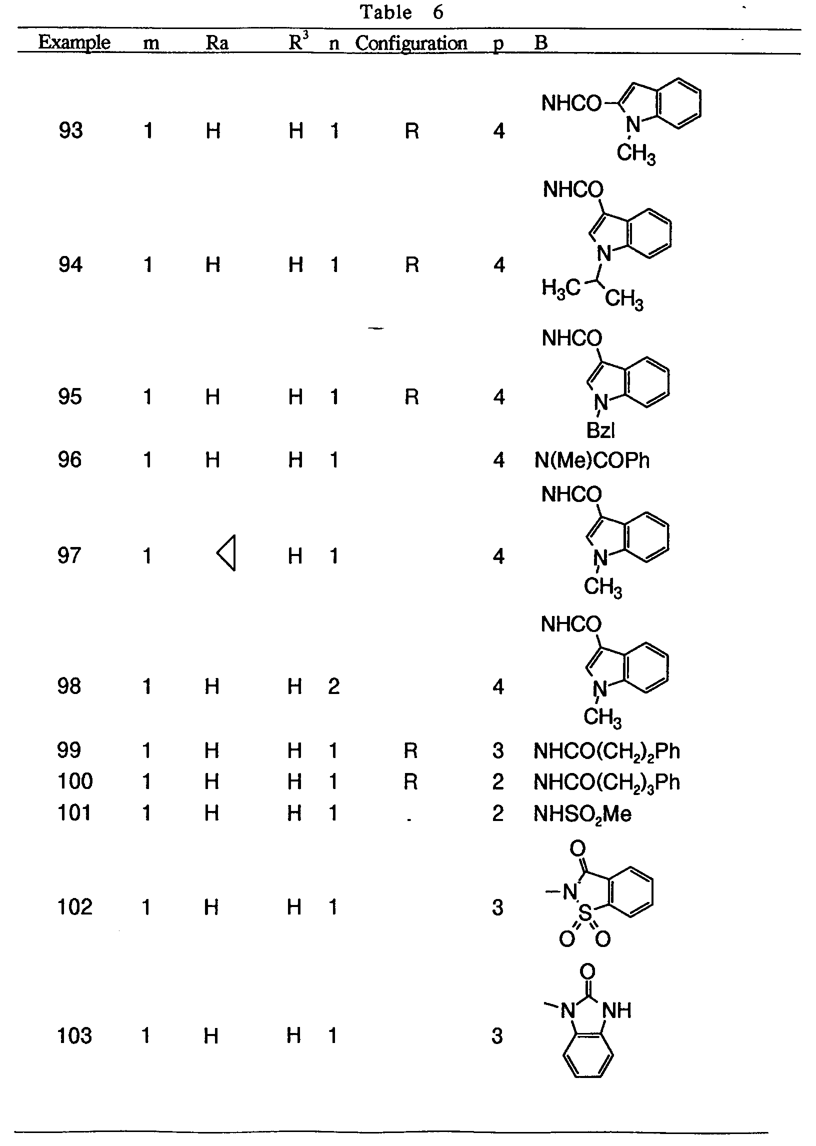

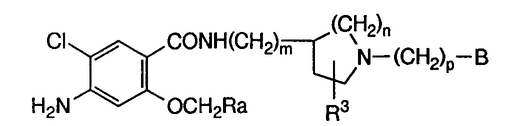

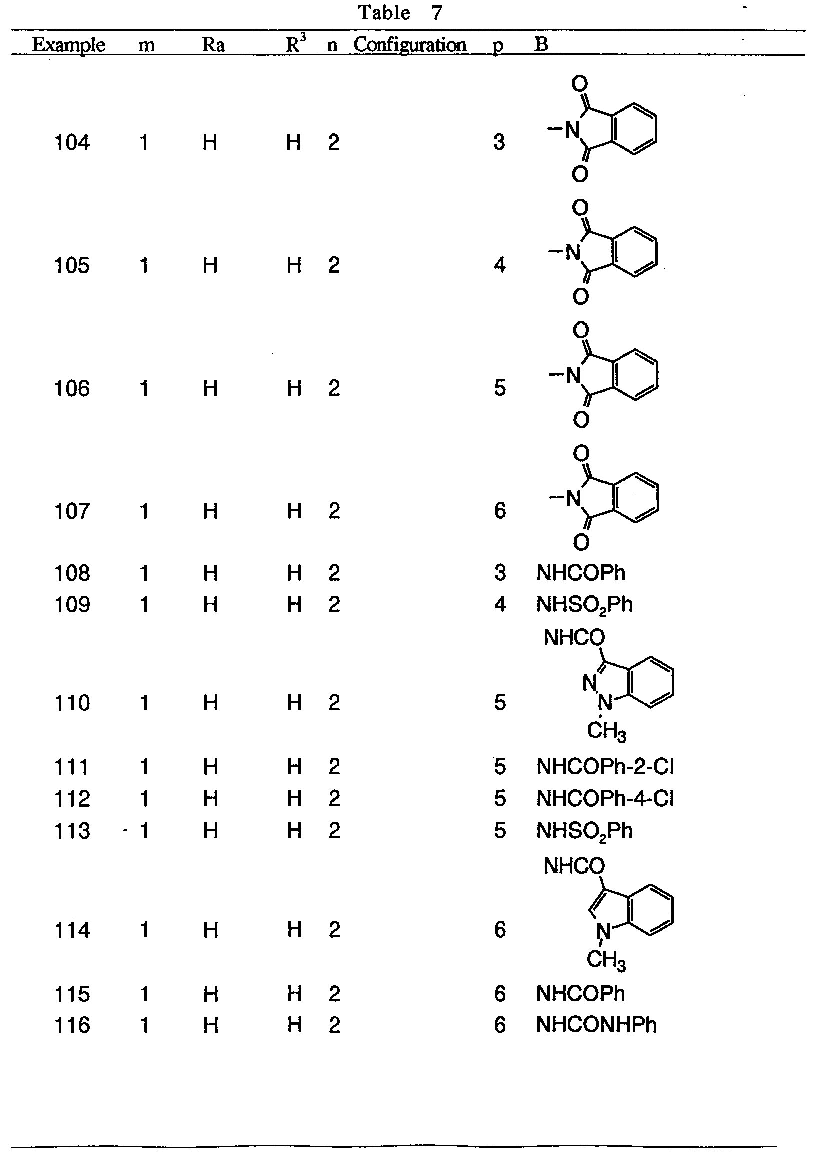

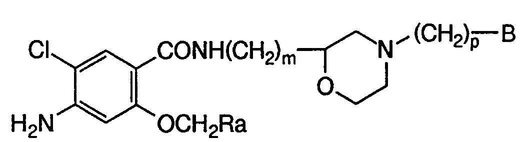

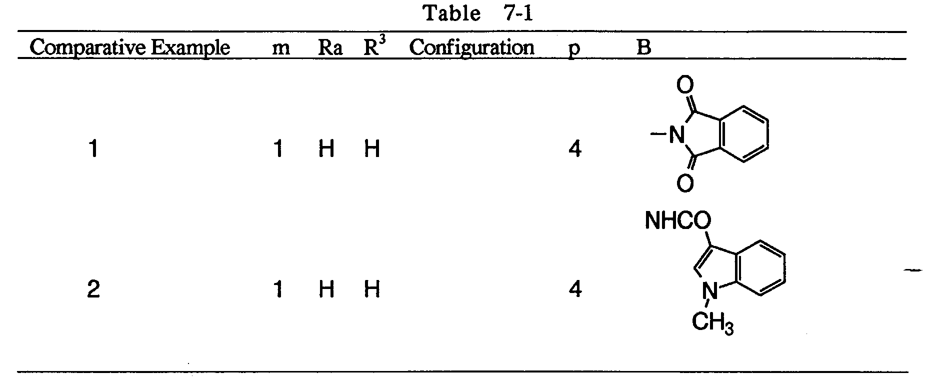

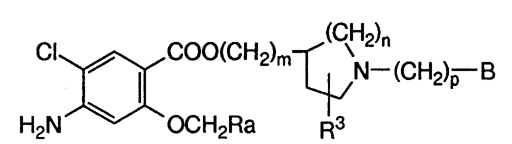

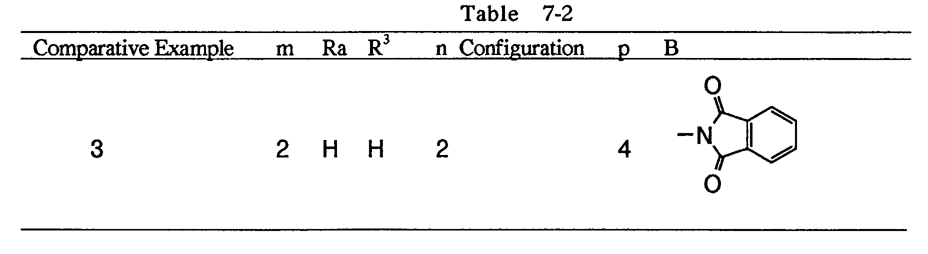

In the same manner as in the above Preparation Examples and Examples, the following preferable compounds can be produced. The present invention is not limited to these compounds exemplified, and it is needless to say that the compounds wherein p is 1, 2, 3 or 6 can be also produced in the same manner.

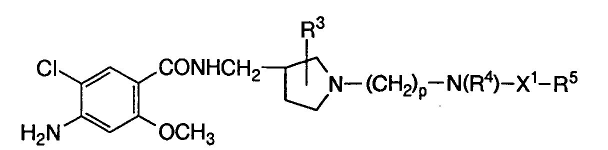

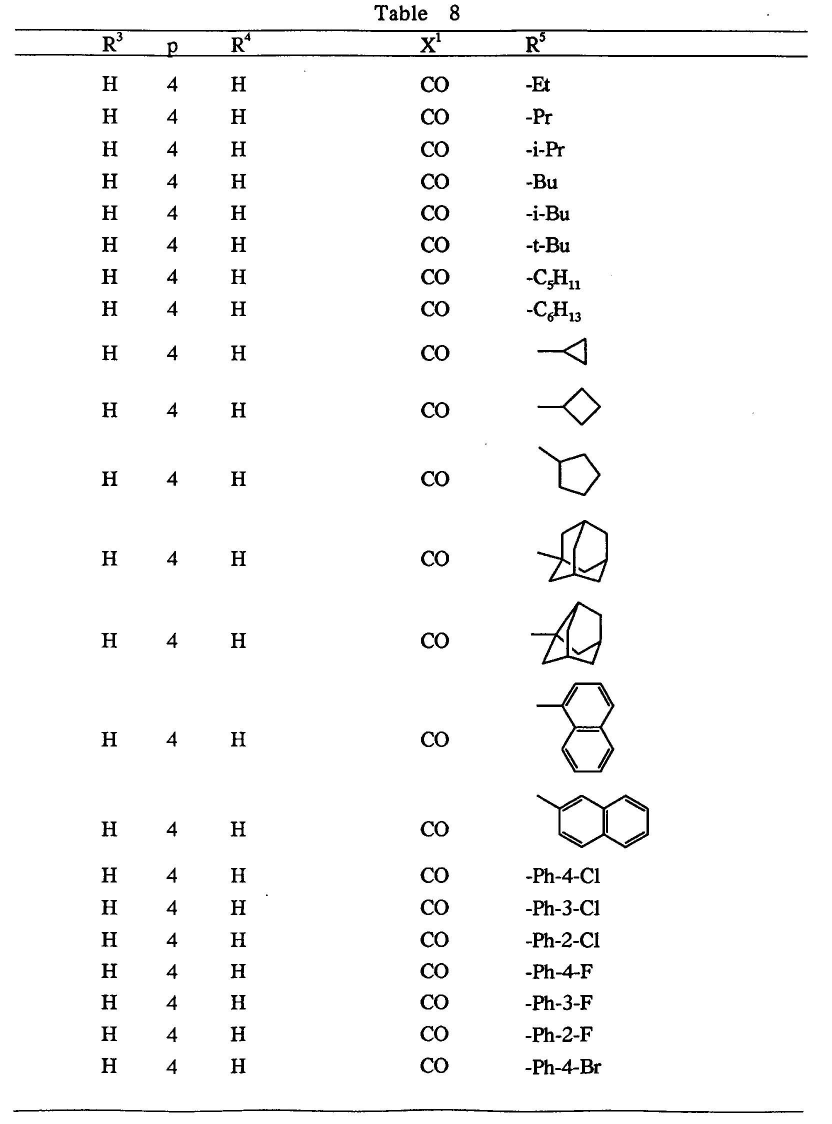

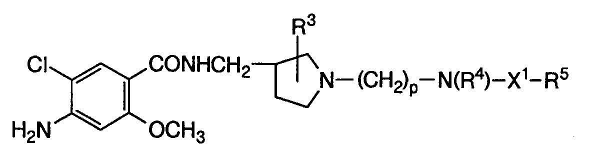

**Table 9**

| R³ | p | R⁴ | X¹ | R⁵ |
|---|---|---|---|---|
| H | 4 | H | CO | -Ph-3-Br |
| H | 4 | H | CO | -Ph-2-Br |
| H | 4 | H | CO | -Ph-4-I |
| H | 4 | H | CO | -Ph-3-I |
| H | 4 | H | CO | -Ph-2-I |
| H | 4 | H | CO | -Ph-2,3-Cl |
| H | 4 | H | CO | -Ph-2,4-Cl |
| H | 4 | H | CO | -Ph-2,5-Cl |
| H | 4 | H | CO | -Ph-2,6-Cl |
| H | 4 | H | CO | -Ph-3,4-Cl |
| H | 4 | H | CO | -Ph-3,5-Cl |
| H | 4 | H | CO | -Ph-2,3,5-Cl |
| H | 4 | H | CO | -Ph-2,4,6-Cl |
| H | 4 | H | CO | -Ph-2,3-F |
| H | 4 | H | CO | -Ph-2,4-F |
| H | 4 | H | CO | -Ph-2,5-F |
| H | 4 | H | CO | -Ph-2,6-F |
| H | 4 | H | CO | -Ph-3,4-F |
| H | 4 | H | CO | -Ph-3,5-F |
| H | 4 | H | CO | -Ph-2,3,4-F |
| H | 4 | H | CO | -Ph-2,3,6-F |
| H | 4 | H | CO | -Ph-2,4,5-F |
| H | 4 | H | CO | -Ph-2,4,6-F |
| H | 4 | H | CO | -Ph-3,4,5-F |
| H | 4 | H | CO | -Ph-3,5-Br |
| H | 4 | H | CO | -Ph-4-Me |
| H | 4 | H | CO | -Ph-3-Me |
| H | 4 | H | CO | -Ph-2-Me |
| H | 4 | H | CO | -Ph-4-Et |
| H | 4 | H | CO | -Ph-4-Pr |
| H | 4 | H | CO | -Ph-4-i-Pr |
| H | 4 | H | CO | -Ph-4-Bu |

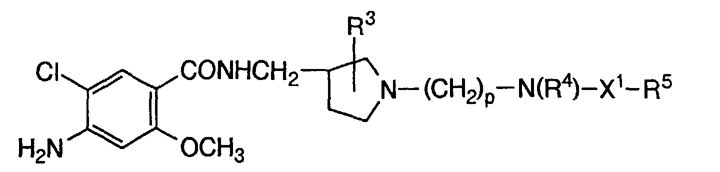

**Table 10**

| R³ | p | R⁴ | X¹ | R⁵ |
|---|---|---|---|---|
| H | 4 | H | CO | -Ph-4-t-Bu |
| H | 4 | H | CO | -Ph-2,3-Me |
| H | 4 | H | CO | -Ph-2,4-Me |
| H | 4 | H | CO | -Ph-2,5-Me |
| H | 4 | H | CO | -Ph-2,6-Me |
| H | 4 | H | CO | -Ph-3,4-Me |
| H | 4 | H | CO | -Ph-3,5-Me |
| H | 4 | H | CO | -Ph-3,4,5-Me |
| H | 4 | H | CO | -Ph-4-OMe |
| H | 4 | H | CO | -Ph-3-OMe |
| H | 4 | H | CO | -Ph-2-OMe |
| H | 4 | H | CO | -Ph-4-OEt |
| H | 4 | H | CO | -Ph-2-OEt |
| H | 4 | H | CO | -Ph-4-OPr |
| H | 4 | H | CO | -Ph-4-O-i-Pr |
| H | 4 | H | CO | -Ph-4-OBu |
| H | 4 | H | CO | -Ph-2,3-OMe |
| H | 4 | H | CO | -Ph-2,4-OMe |
| H | 4 | H | CO | -Ph-2,5-OMe |
| H | 4 | H | CO | -Ph-2,6-OMe |
| H | 4 | H | CO | -Ph-3,4-OMe |
| H | 4 | H | CO | -Ph-3,5-OMe |
| H | 4 | H | CO | -Ph-2,3,4-OMe |
| H | 4 | H | CO | -Ph-2,4,5-OMe |
| H | 4 | H | CO | -Ph-3,4,5-OMe |
| H | 4 | H | CO | -Ph-4-CH₂Ph |
| H | 4 | H | CO | -Ph-3-CH₂Ph |
| H | 4 | H | CO | -Ph-2-CH₂Ph |
| H | 4 | H | CO | -Ph-4-OH |
| H | 4 | H | CO | -Ph-3-OH |
| H | 4 | H | CO | -Ph-2,3-OH |
| H | 4 | H | CO | -Ph-2,4-OH |

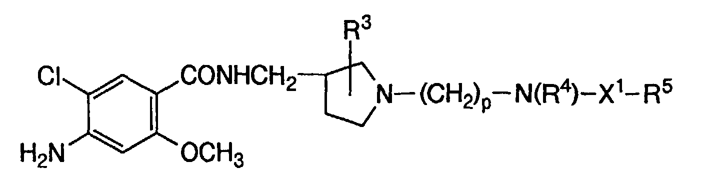

**Table 11**

| R³ | p | R⁴ | X¹ | R⁵ |
|---|---|---|---|---|
| H | 4 | H | CO | -Ph-2,5-OH |
| H | 4 | H | CO | -Ph-2,6-OH |
| H | 4 | H | CO | -Ph-3,4-OH |
| H | 4 | H | CO | -Ph-3,5-OH |
| H | 4 | H | CO | -Ph-2,3,4-OH |
| H | 4 | H | CO | -Ph-2,4,6-OH |
| H | 4 | H | CO | -Ph-4-NO₂ |
| H | 4 | H | CO | -Ph-3-NO₂ |
| H | 4 | H | CO | -Ph-2-NO₂ |
| H | 4 | H | CO | -Ph-2,4-NO₂ |
| H | 4 | H | CO | -Ph-3,5-NO₂ |
| H | 4 | H | CO | -Ph-4-NH₂ |
| H | 4 | H | CO | -Ph-3-NH₂ |
| H | 4 | H | CO | -Ph-3,4-NH₂ |
| H | 4 | H | CO | -Ph-3,5-NH₂ |
| H | 4 | H | CO | -Ph-2-F-5-Me |
| H | 4 | H | CO | -Ph-3-F-2-Me |
| H | 4 | H | CO | -Ph-3-F-4-Me |
| H | 4 | H | CO | -Ph-5-F-2-Me |
| H | 4 | H | CO | -Ph-3-Br-4-Me |
| H | 4 | H | CO | -Ph-4-Cl-2-OMe |
| H | 4 | H | CO | -Ph-5-Cl-2-OMe |
| H | 4 | H | CO | -Ph-3-F-4-OMe |
| H | 4 | H | CO | -Ph-2-Br-5-OMe |
| H | 4 | H | CO | -Ph-2-NH₂-3-OMe |
| H | 4 | H | CO | -Ph-3-NH₂-4-OMe |
| H | 4 | H | CO | -Ph-4-NH₂-3-OMe |
| H | 4 | H | CO | -Ph-3-NH₂-4-OH |
| H | 4 | H | CO | -Ph-4-NH₂-3-OH |
| H | 4 | H | CO | -Ph-3-NH₂-2-OH |
| H | 4 | H | CO | -Ph-4-NH₂-2-OH |
| H | 4 | H | CO | -Ph-5-NH₂-2-OH |

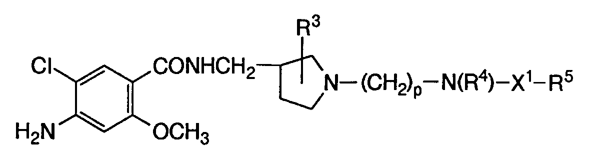

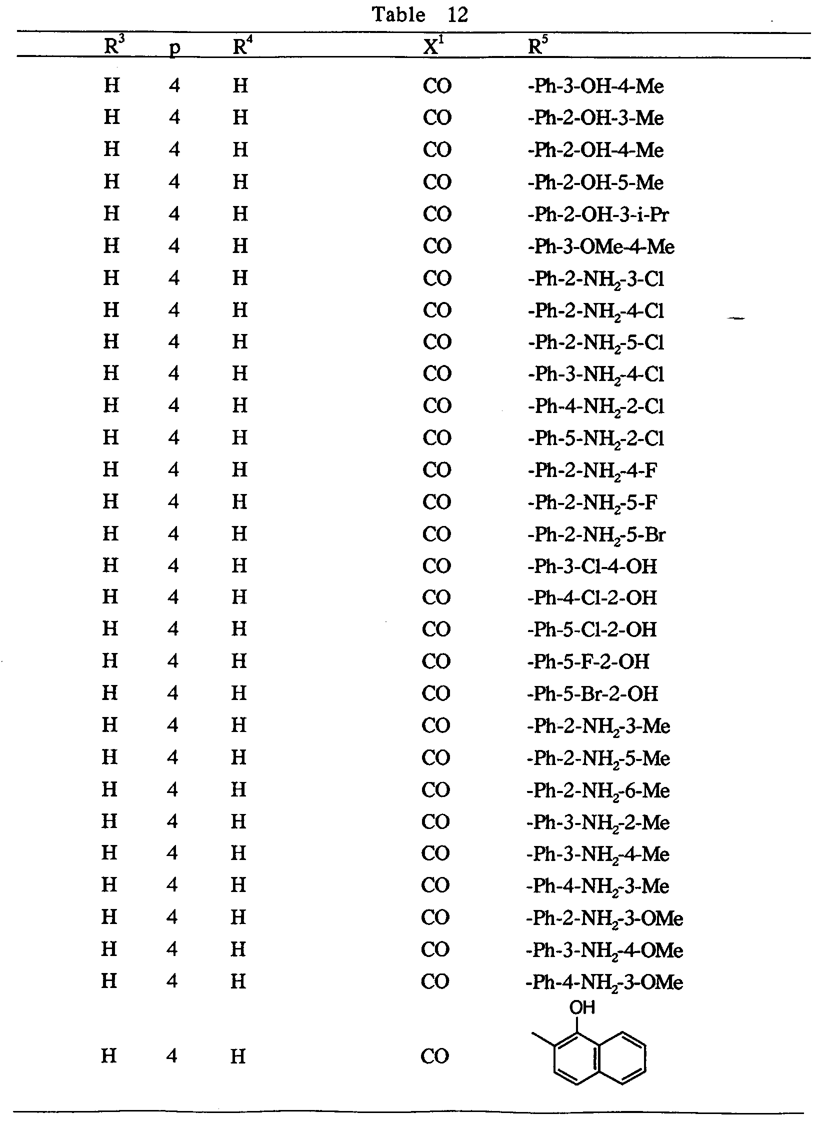

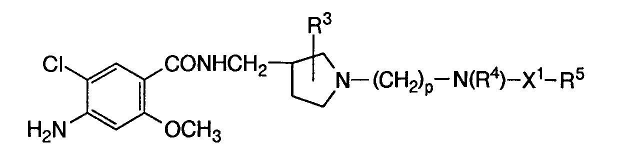

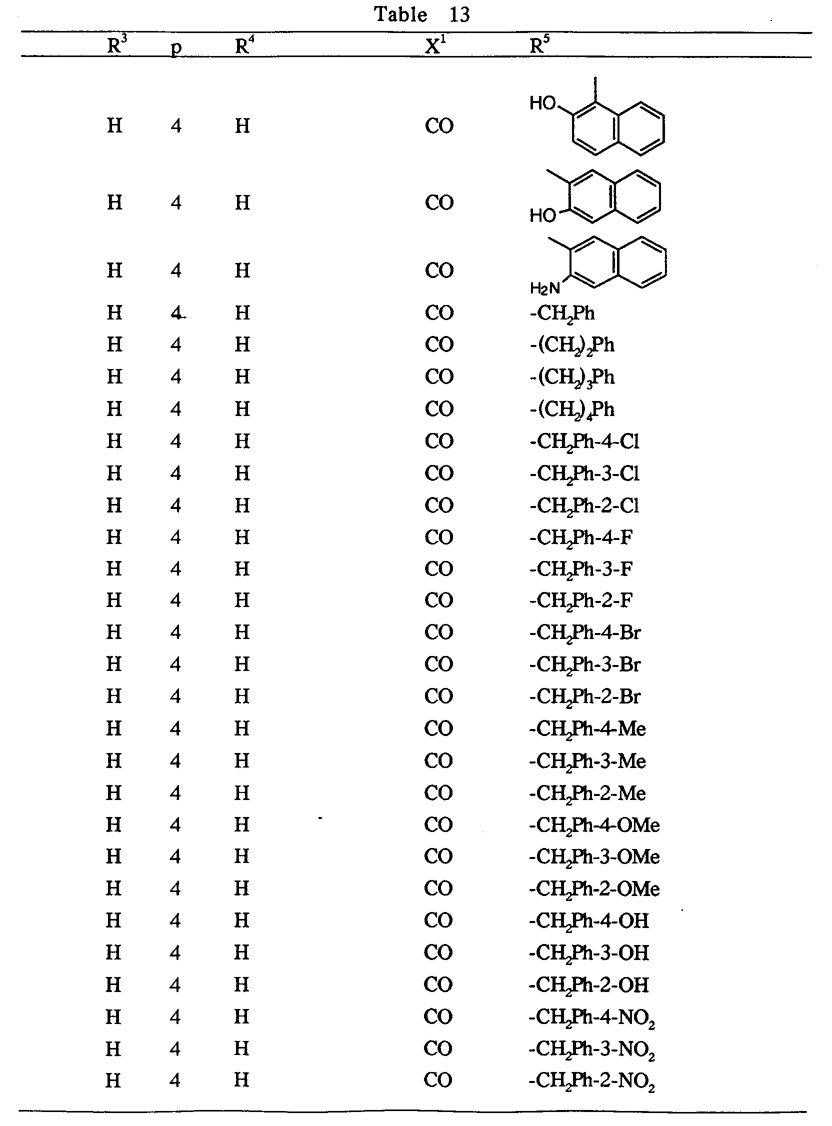

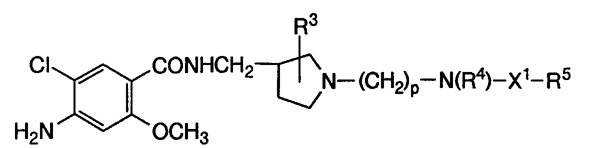

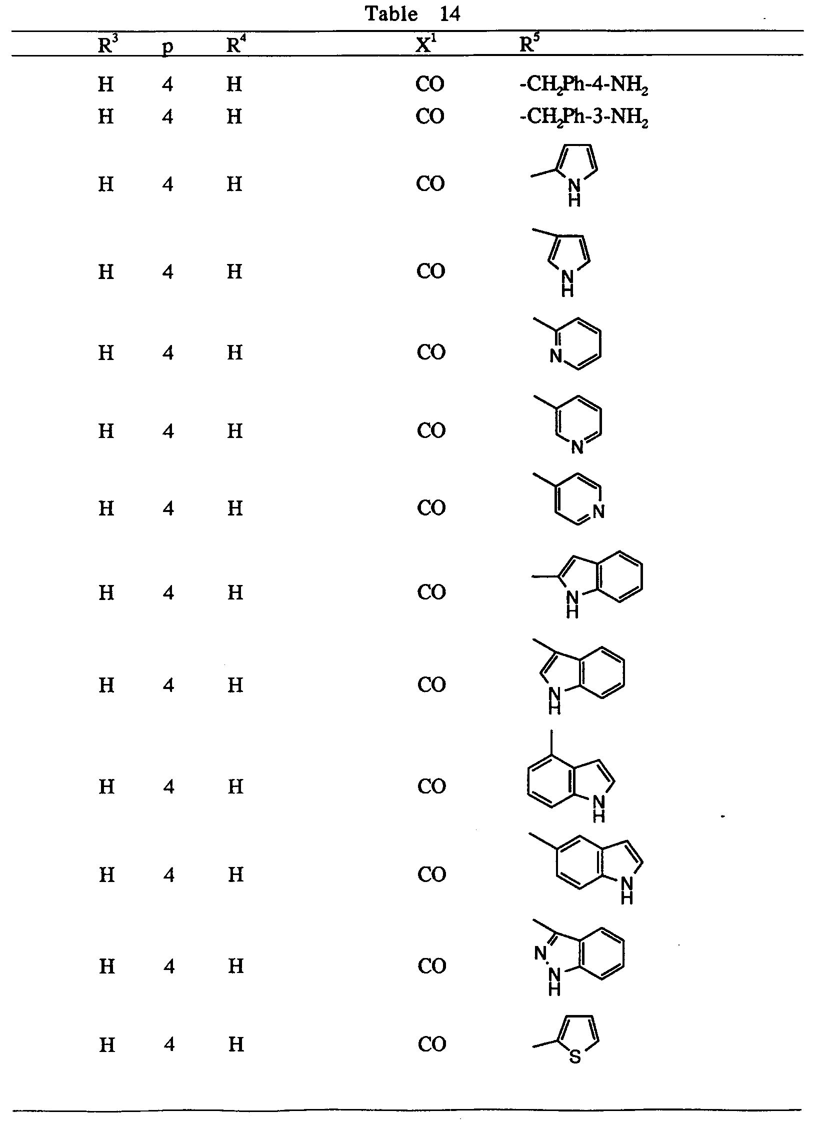

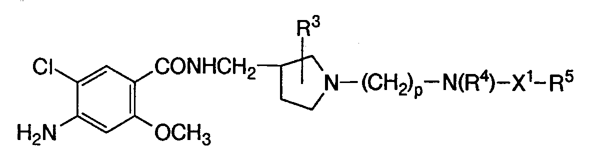

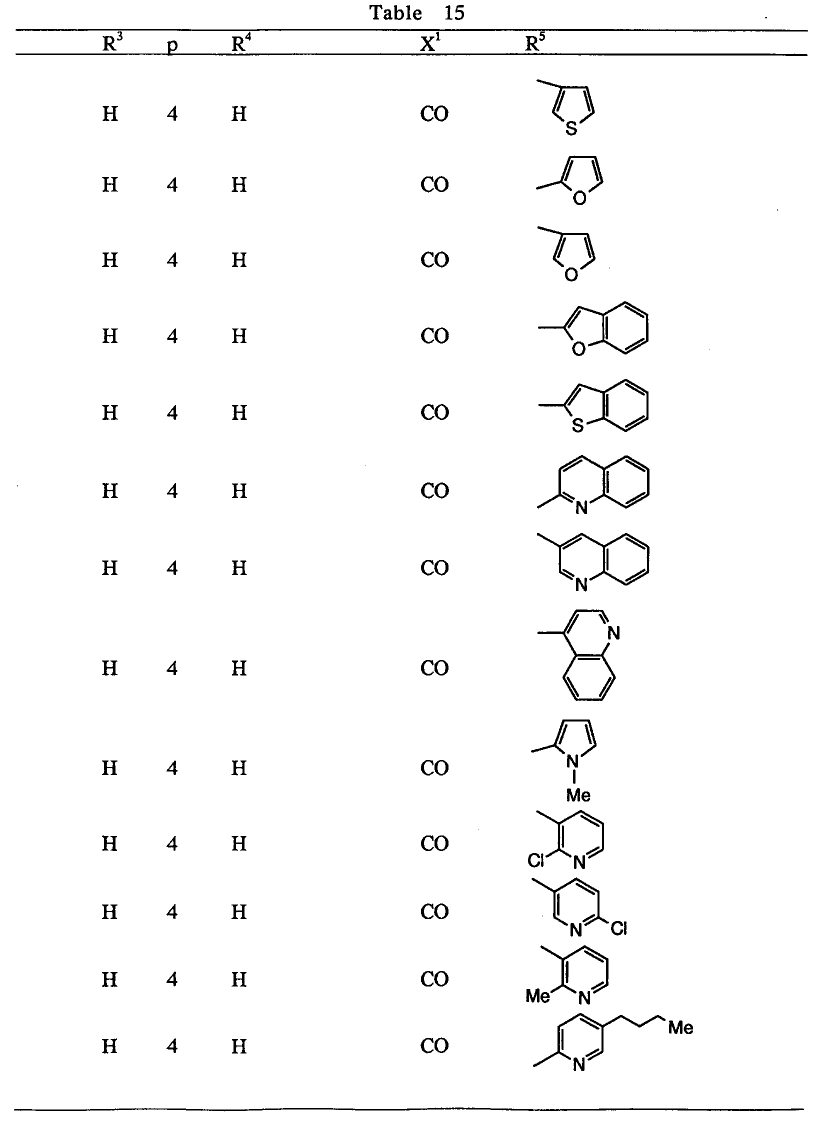

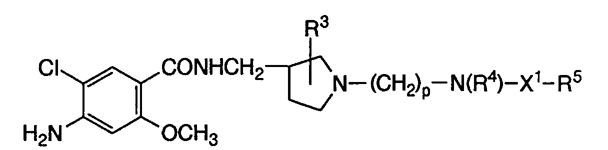

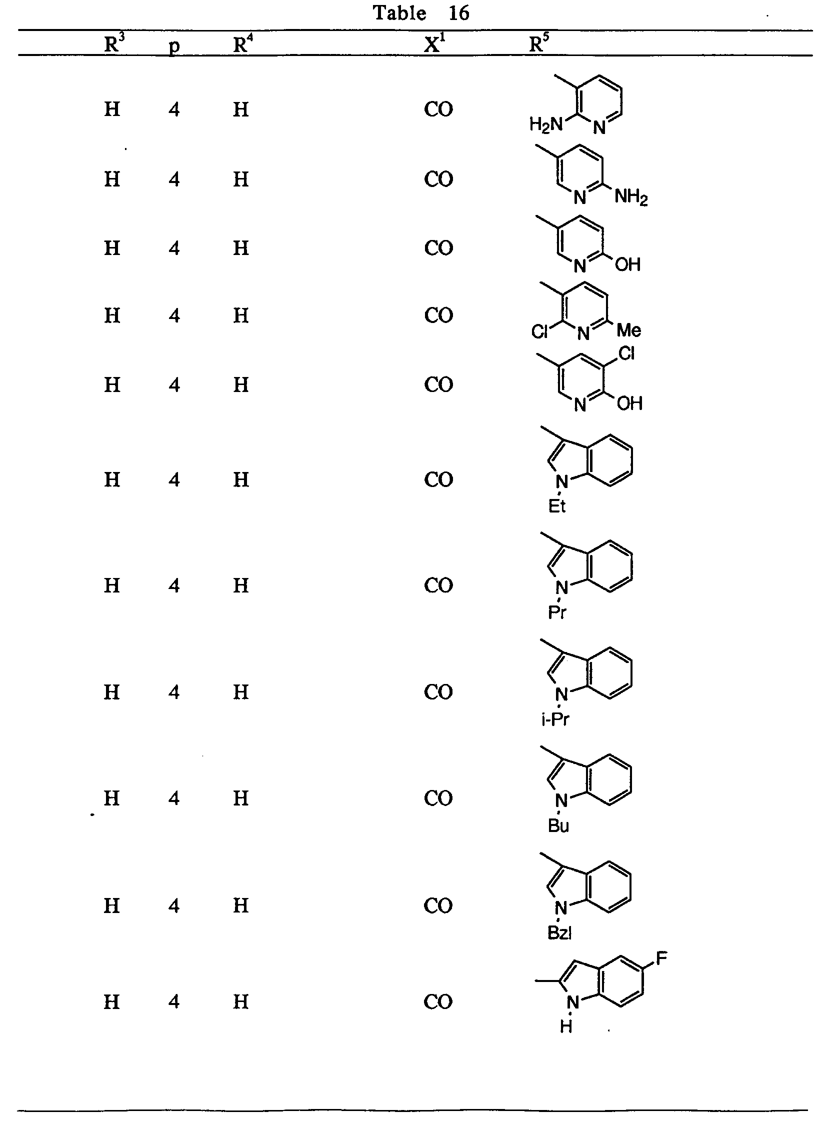

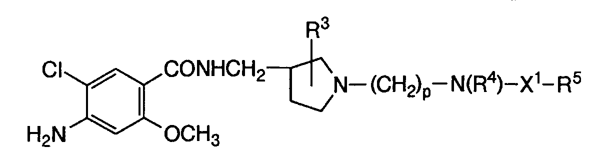

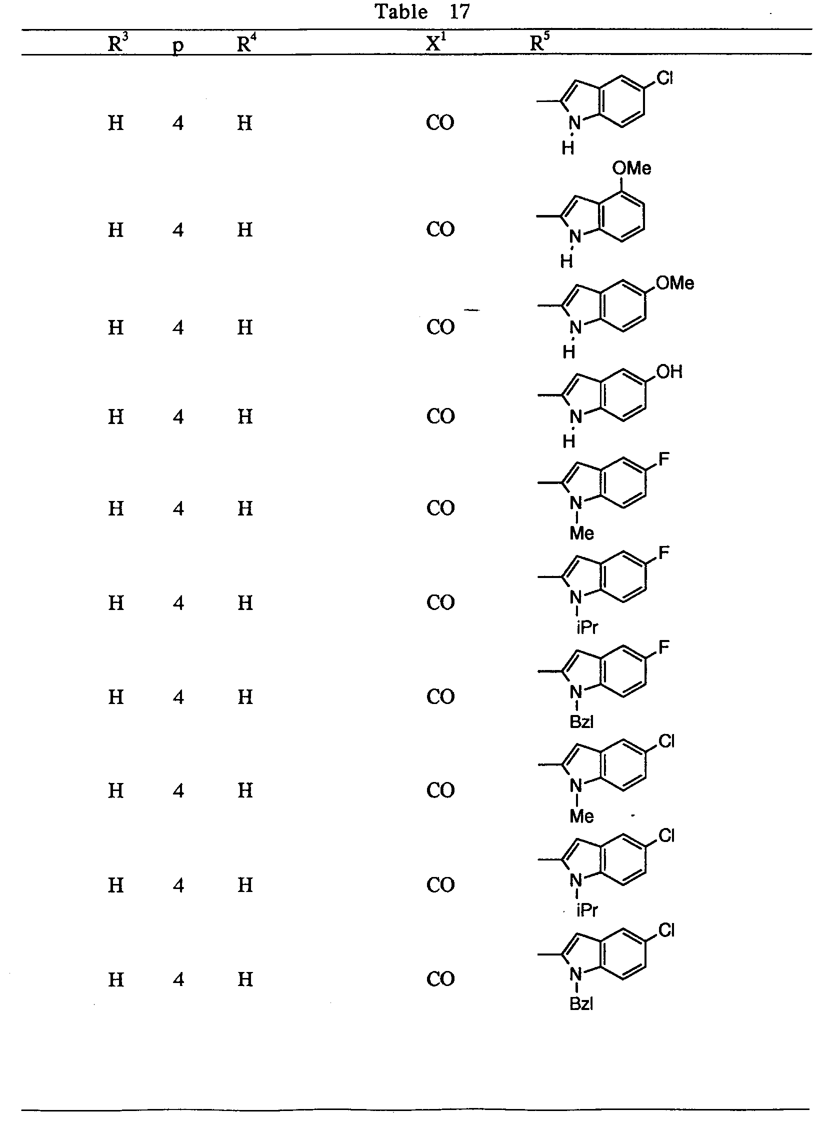

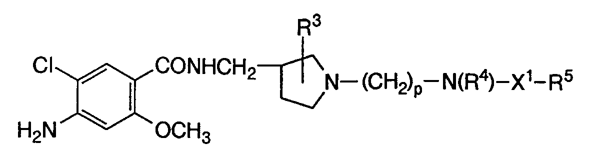

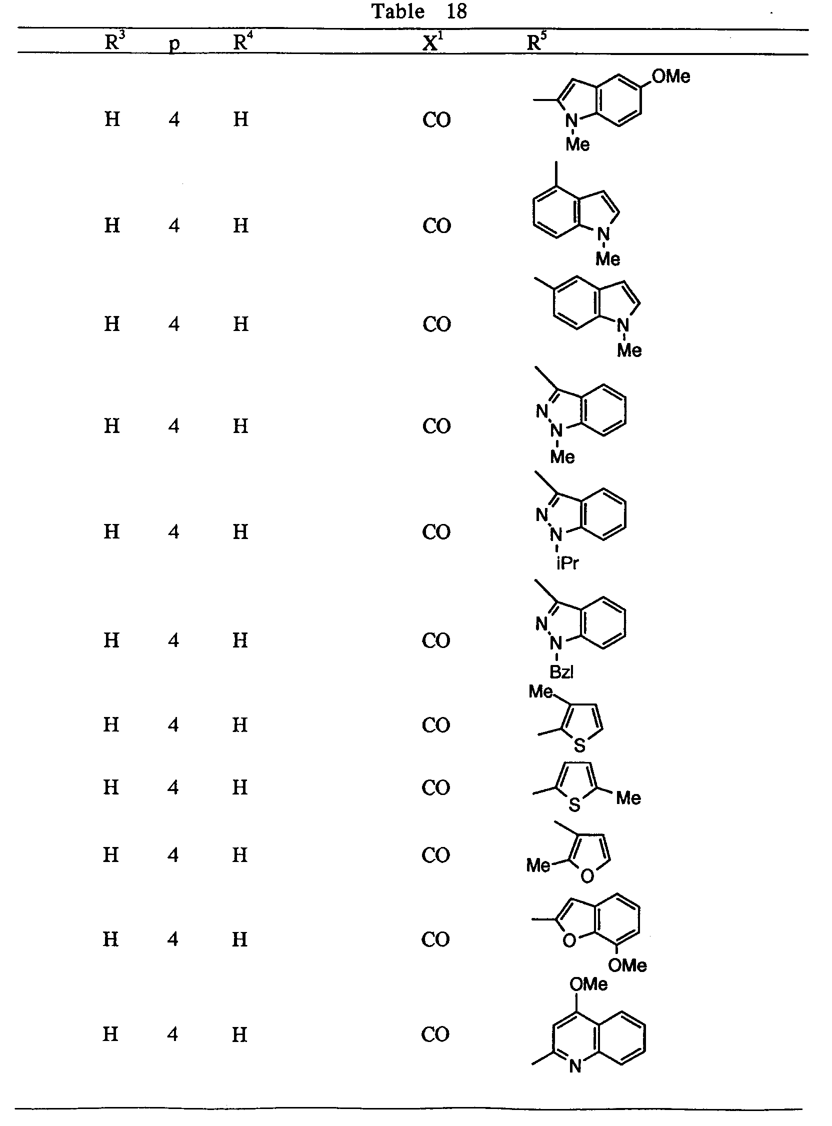

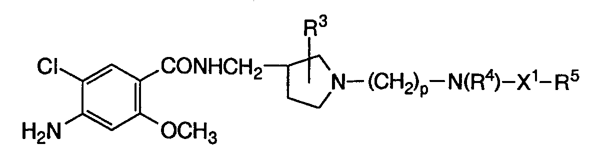

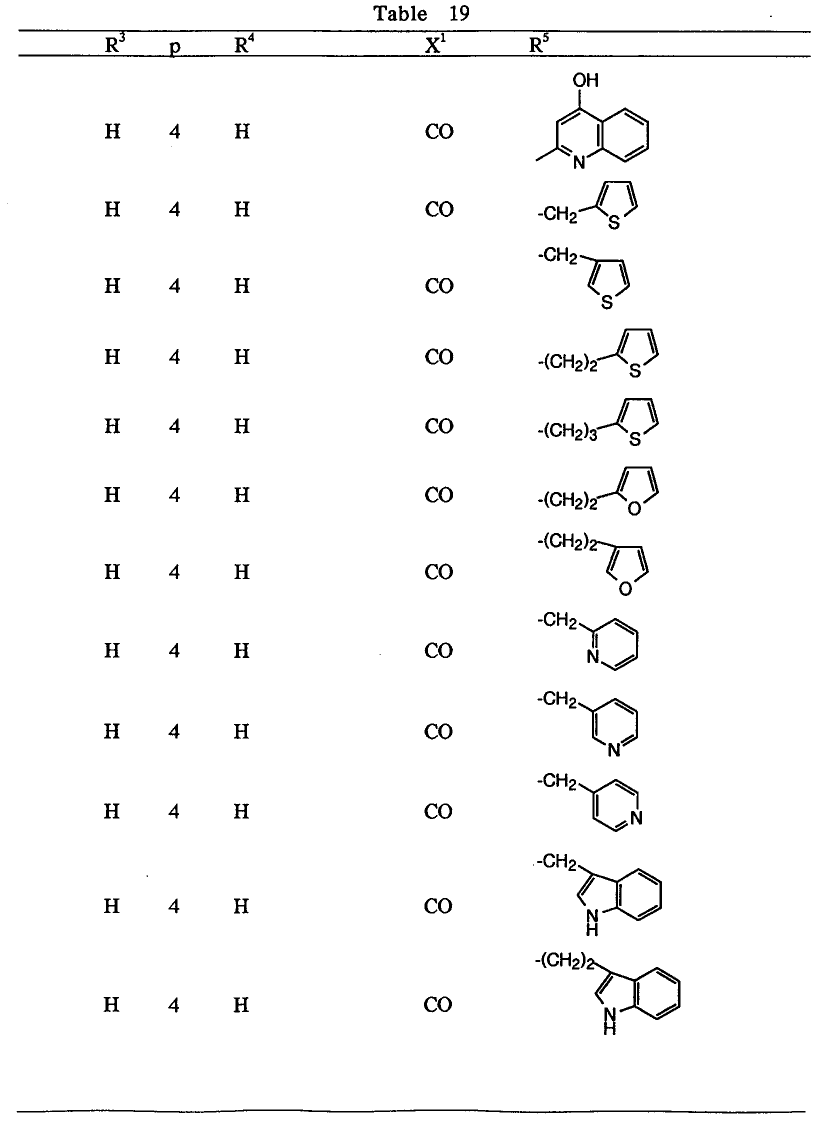

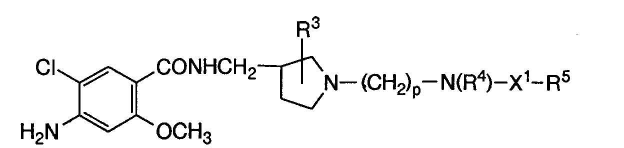

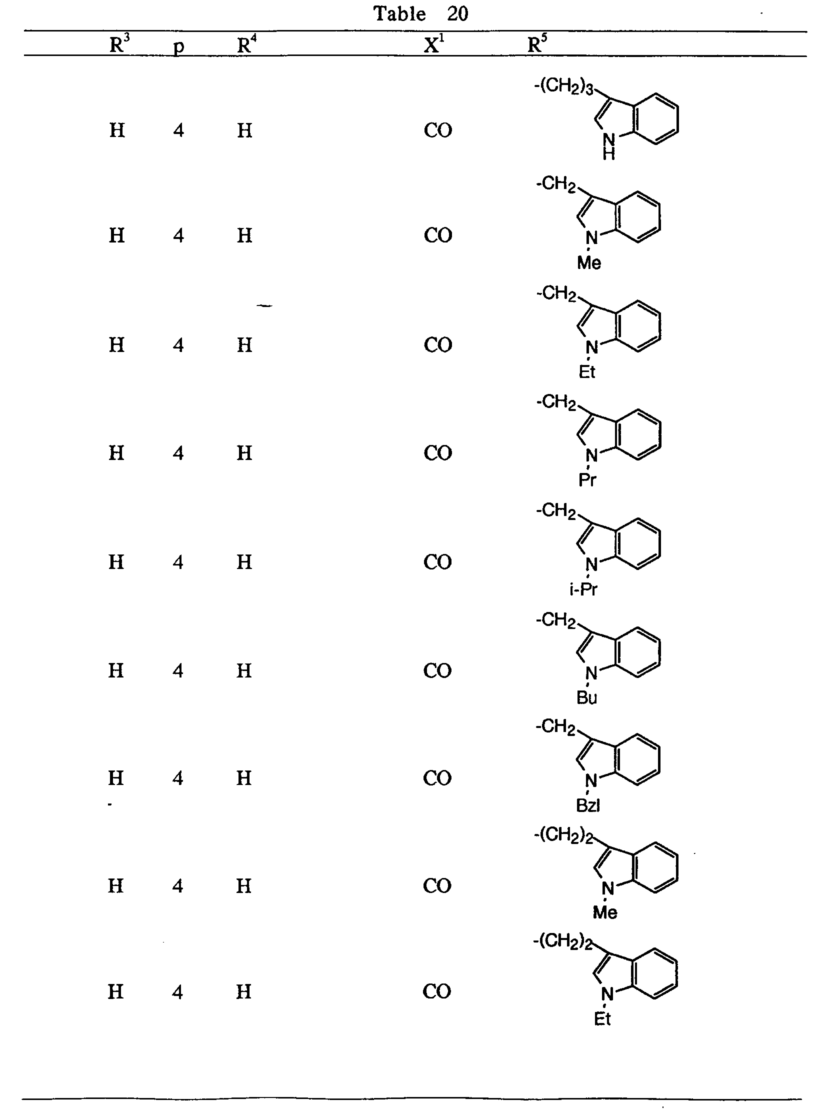

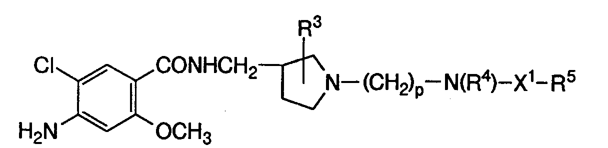

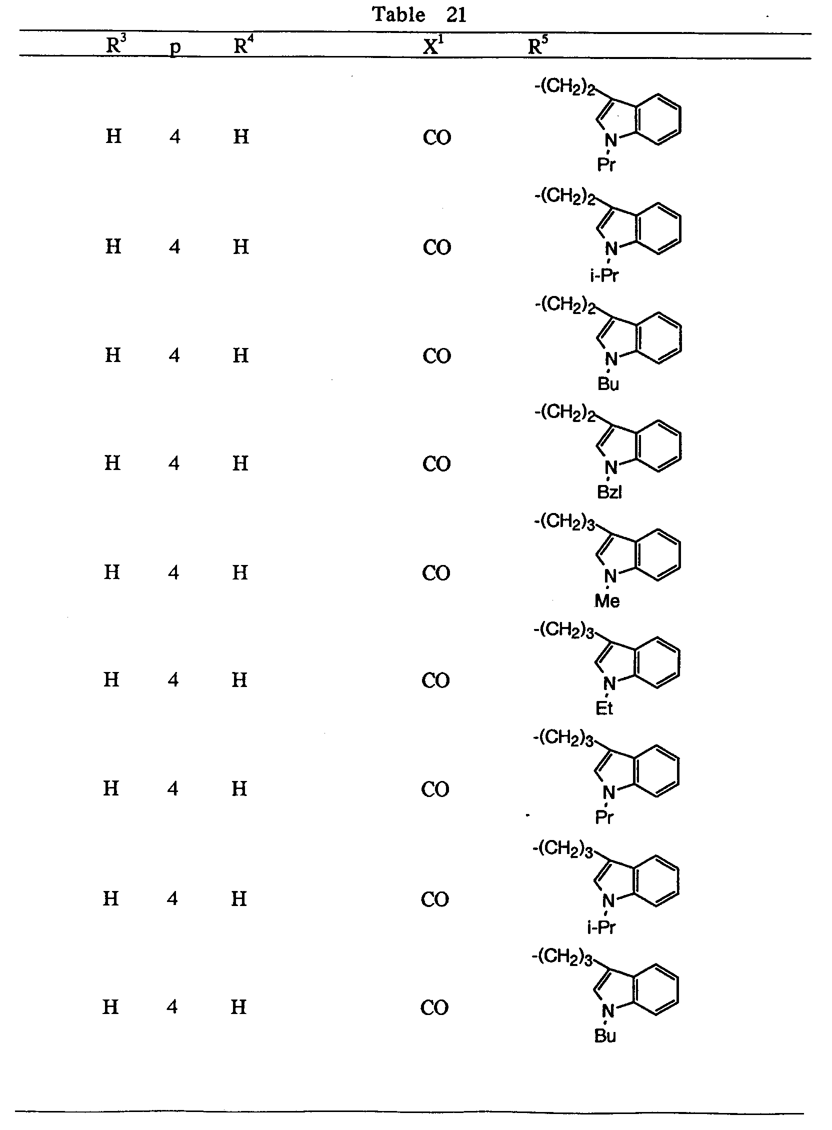

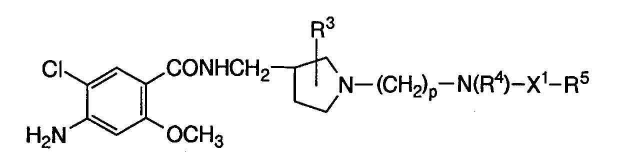

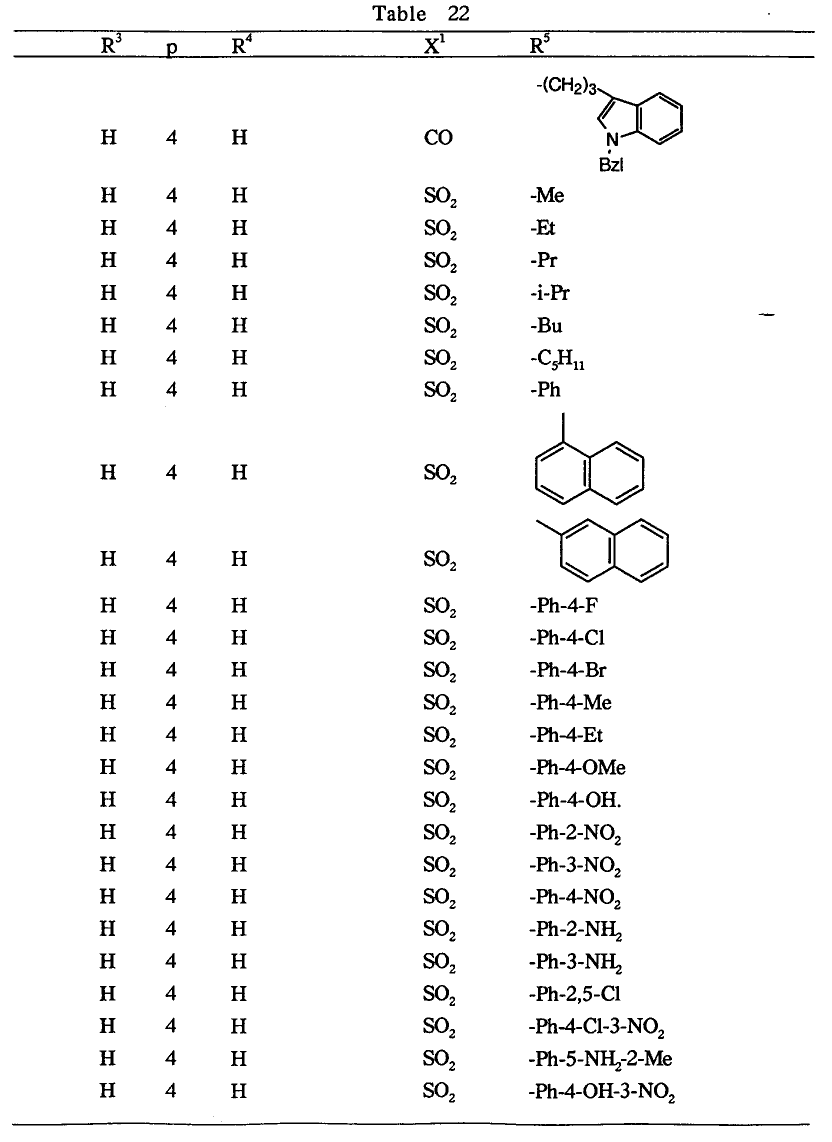

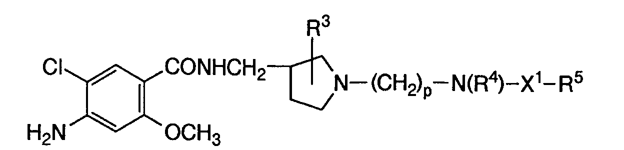

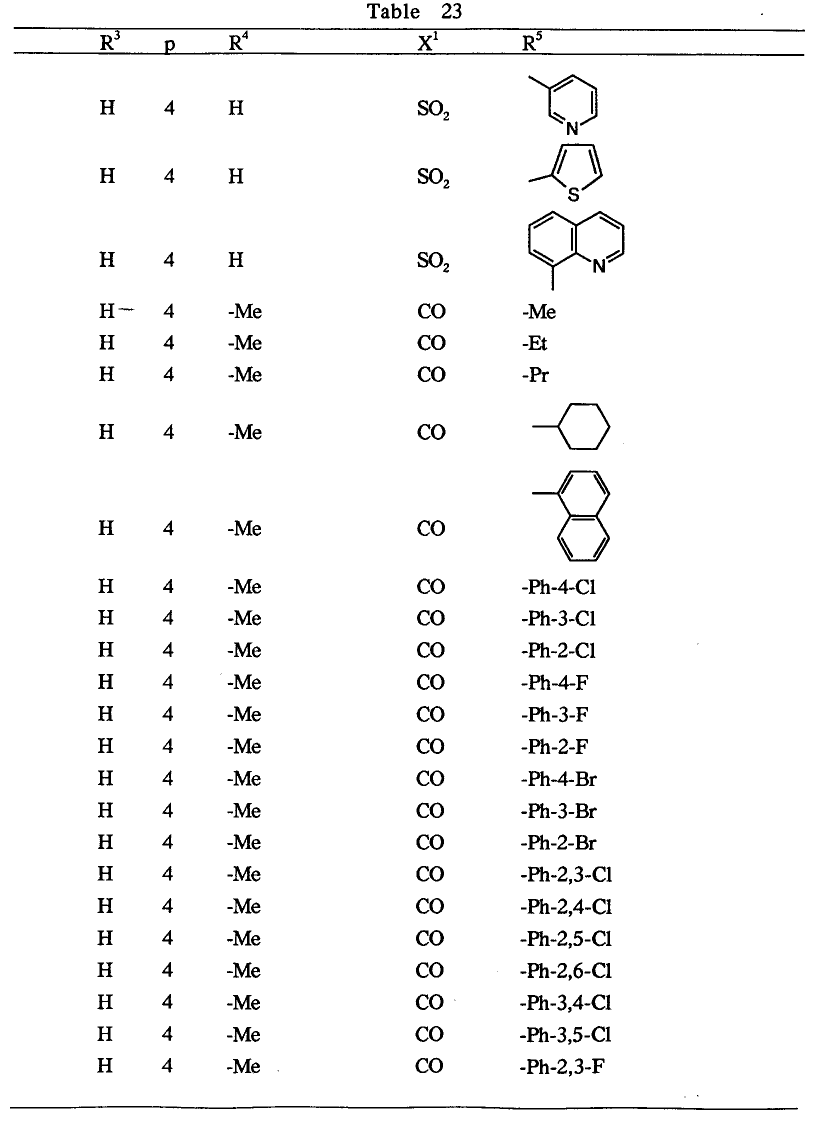

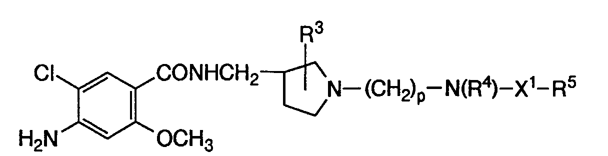

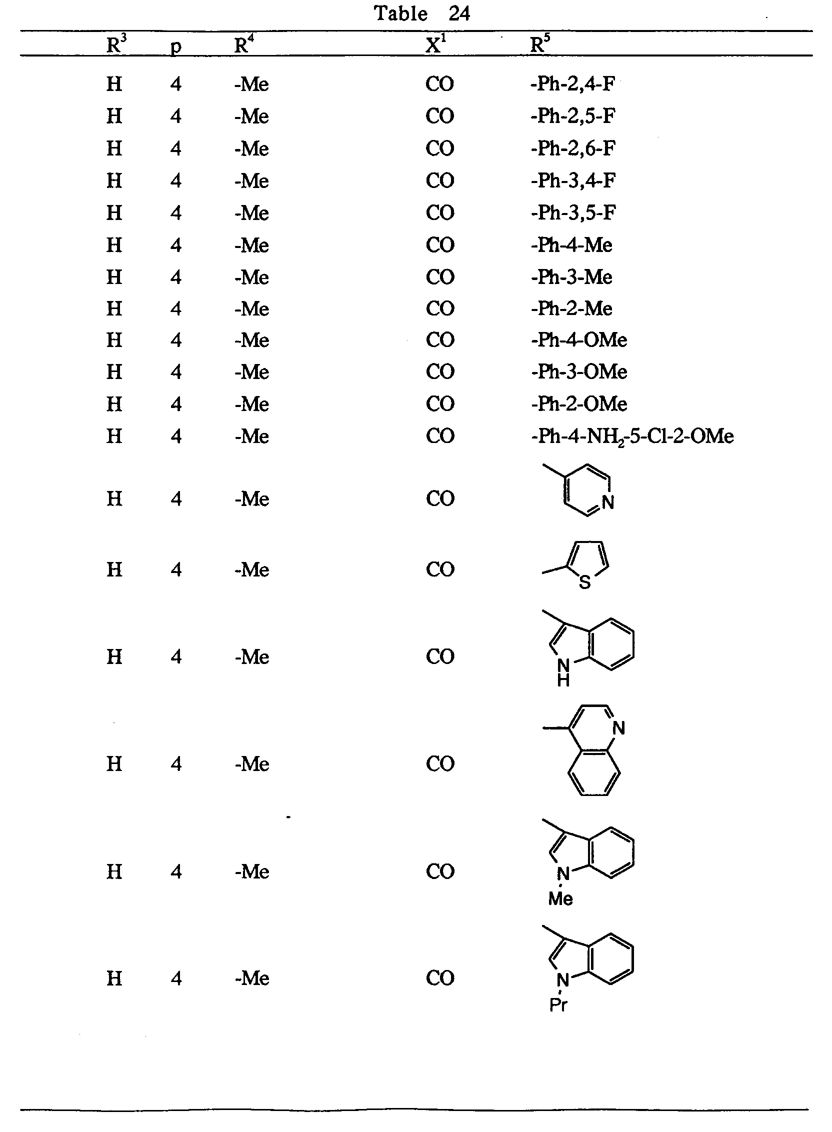

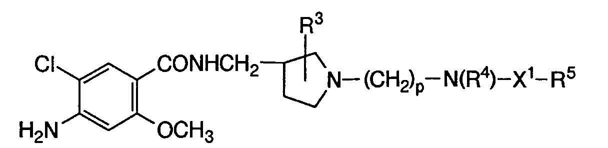

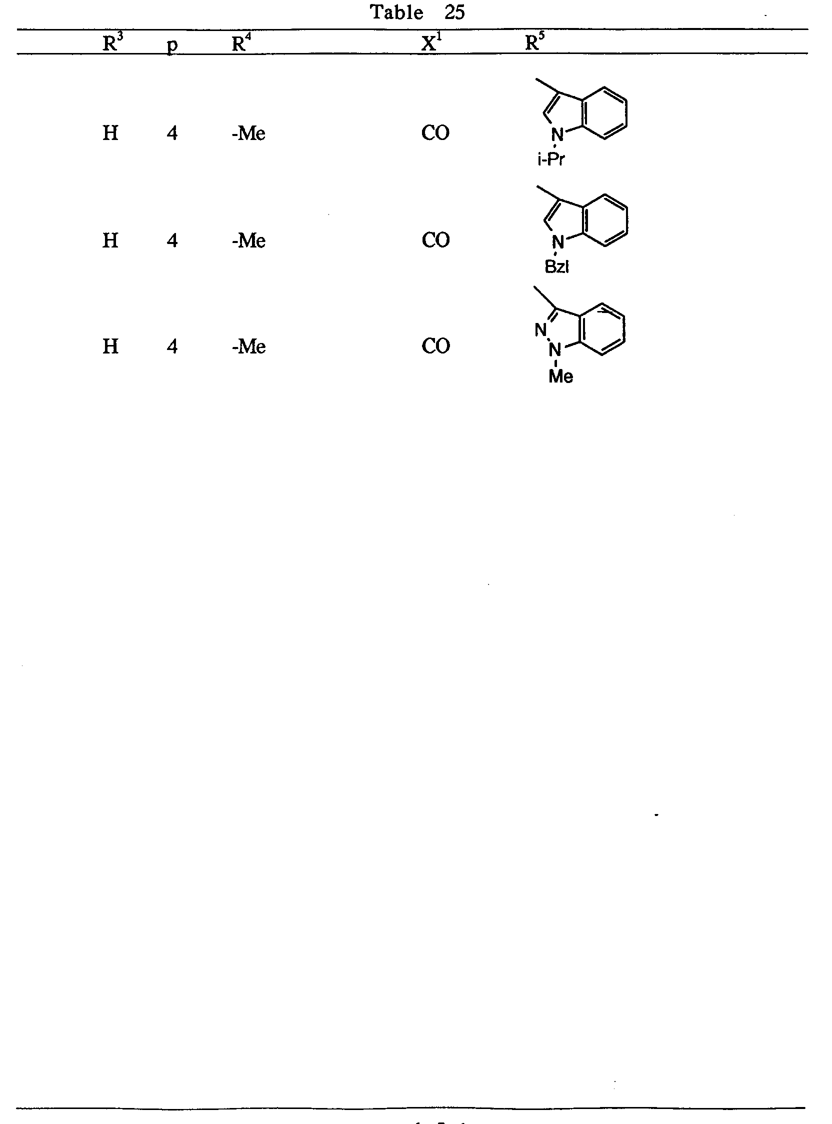

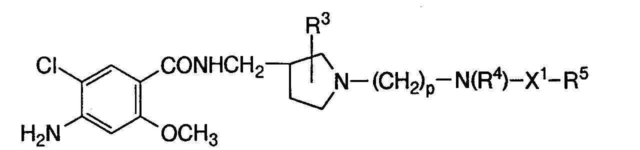

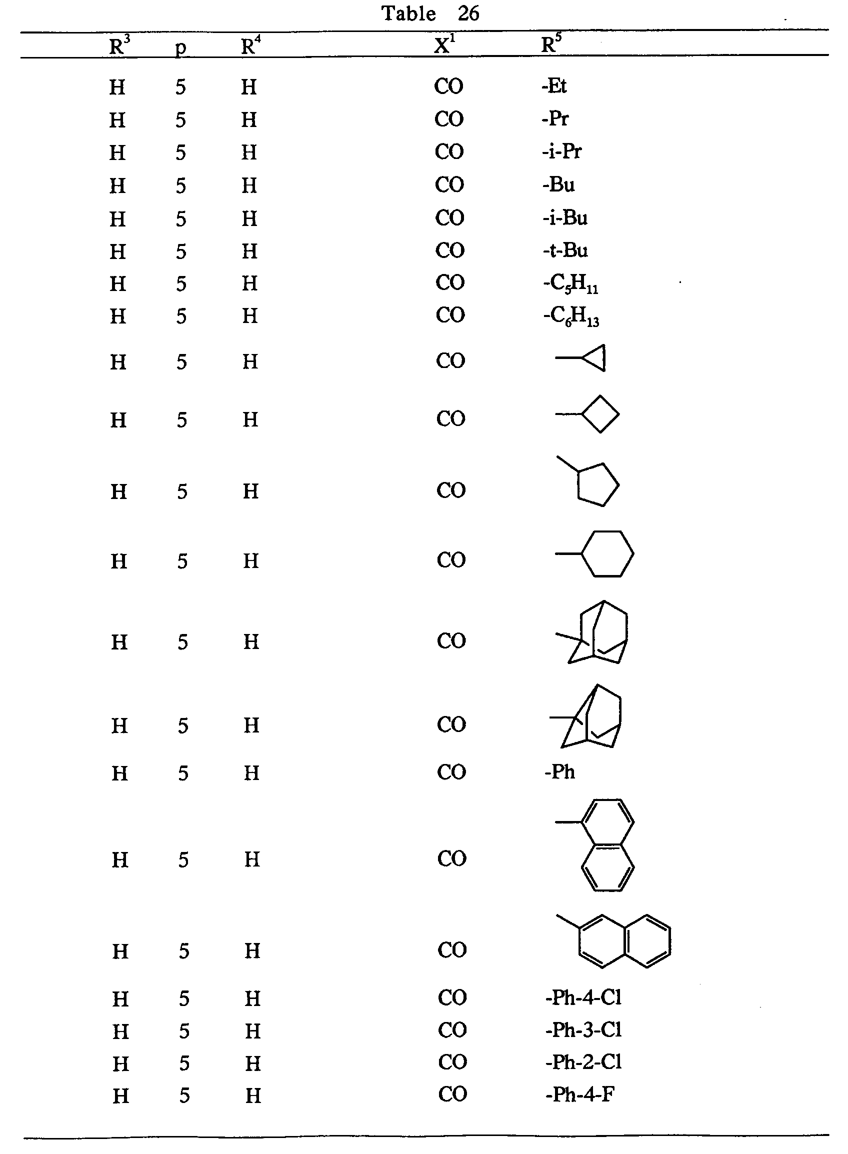

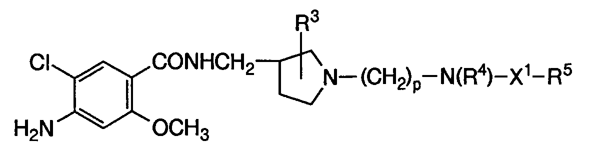

**Table 27**

| R³ | p | R⁴ | X¹ | R⁵ |
|---|---|---|---|---|
| H | 5 | H | CO | -Ph-3-F |
| H | 5 | H | CO | -Ph-2-F |
| H | 5 | H | CO | -Ph-4-Br |
| H | 5 | H | CO | -Ph-3-Br |
| H | 5 | H | CO | -Ph-2-Br |
| H | 5 | H | CO | -Ph-4-I |
| H | 5 | H | CO | -Ph-3-I |
| H | 5 | H | CO | -Ph-2-I |
| H | 5 | H | CO | -Ph-2,3-Cl |
| H | 5 | H | CO | -Ph-2,4-Cl |
| H | 5 | H | CO | -Ph-2,5-Cl |
| H | 5 | H | CO | -Ph-2,6-Cl |
| H | 5 | H | CO | -Ph-3,4-Cl |
| H | 5 | H | CO | -Ph-3,5-Cl |
| H | 5 | H | CO | -Ph-2,3,5-Cl |
| H | 5 | H | CO | -Ph-2,4,6-Cl |
| H | 5 | H | CO | -Ph-2,3-F |
| H | 5 | H | CO | -Ph-2,4-F |
| H | 5 | H | CO | -Ph-2,5-F |
| H | 5 | H | CO | -Ph-2,6-F |
| H | 5 | H | CO | -Ph-3,4-F |
| H | 5 | H | CO | -Ph-3,5-F |
| H | 5 | H | CO | -Ph-2,3,4-F |
| H | 5 | H | CO | -Ph-2,3,6-F |
| H | 5 | H | CO | -Ph-2,4,5-F |
| H | 5 | H | CO | -Ph-2,4,6-F |
| H | 5 | H | CO | -Ph-3,4,5-F |
| H | 5 | H | CO | -Ph-3,5-Br |
| H | 5 | H | CO | -Ph-4-Me |
| H | 5 | H | CO | -Ph-3-Me |
| H | 5 | H | CO | -Ph-2-Me |
| H | 5 | H | CO | -Ph-4-Et |

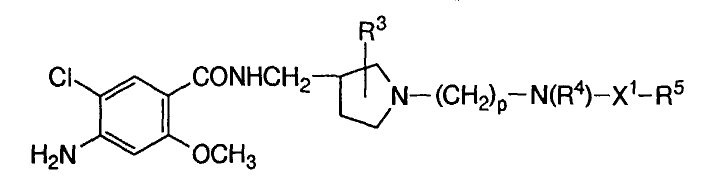

**Table 28**

| R³ | p | R⁴ | X¹ | R⁵ |
|---|---|---|---|---|
| H | 5 | H | CO | -Ph-4-Pr |
| H | 5 | H | CO | -Ph-4-i-Pr |
| H | 5 | H | CO | -Ph-4-Bu |
| H | 5 | H | CO | -Ph-4-t-Bu |
| H | 5 | H | CO | -Ph-2,3-Me |
| H | 5 | H | CO | -Ph-2,4-Me |
| H | 5 | H | CO | -Ph-2,5-Me |
| H | 5 | H | CO | -Ph-2,6-Me |
| H | 5 | H | CO | -Ph-3,4-Me |
| H | 5 | H | CO | -Ph-3,5-Me |
| H | 5 | H | CO | -Ph-3,4,5-Me |
| H | 5 | H | CO | -Ph-4-OMe |
| H | 5 | H | CO | -Ph-3-OMe |
| H | 5 | H | CO | -Ph-2-OMe |
| H | 5 | H | CO | -Ph-4-OEt |
| H | 5 | H | CO | -Ph-2-OEt |
| H | 5 | H | CO | -Ph-4-OPr |
| H | 5 | H | CO | -Ph-4-O-i-Pr |
| H | 5 | H | CO | -Ph-4-OBu |
| H | 5 | H | CO | -Ph-2,3-OMe |
| H | 5 | H | CO | -Ph-2,4-OMe |
| H | 5 | H | CO | -Ph-2,5-OMe |
| H | 5 | H | CO | -Ph-2,6-OMe |
| H | 5 | H | CO | -Ph-3,4-OMe |
| H | 5 | H | CO | -Ph-3,5-OMe |
| H | 5 | H | CO | -Ph-2,3,4-OMe |
| H | 5 | H | CO | -Ph-2,4,5-OMe |
| H | 5 | H | CO | -Ph-3,4,5-OMe |
| H | 5 | H | CO | -Ph-4-CH₂Ph |
| H | 5 | H | CO | -Ph-3-CH₂Ph |
| H | 5 | H | CO | -Ph-2-CH₂Ph |
| H | 5 | H | CO | -Ph-4-OH |

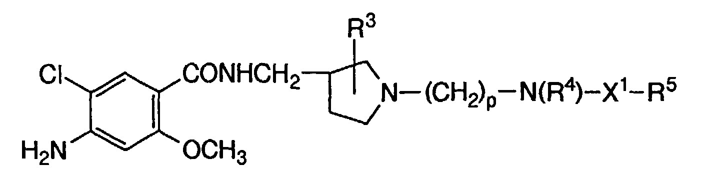

**Table 29**

| R³ | p | R⁴ | X¹ | R⁵ |
|---|---|---|---|---|
| H | 5 | H | CO | -Ph-3-OH |
| H | 5 | H | CO | -Ph-2,3-OH |
| H | 5 | H | CO | -Ph-2,4-OH |
| H | 5 | H | CO | -Ph-2,5-OH |
| H | 5 | H | CO | -Ph-2,6-OH |
| H | 5 | H | CO | -Ph-3,4-OH |
| H | 5 | H | CO | -Ph-3,5-OH |
| H | 5 | H | CO | -Ph-2,3,4-OH |
| H | 5 | H | CO | -Ph-2,4,6-OH |
| H | 5 | H | CO | -Ph-4-NO₂ |
| H | 5 | H | CO | -Ph-3-NO₂ |
| H | 5 | H | CO | -Ph-2-NO₂ |
| H | 5 | H | CO | -Ph-2,4-NO₂ |
| H | 5 | H | CO | -Ph-3,5-NO₂ |
| H | 5 | H | CO | -Ph-4-NH₂ |
| H | 5 | H | CO | -Ph-3-NH₂ |
| H | 5 | H | CO | -Ph-3,4-NH₂ |
| H | 5 | H | CO | -Ph-3,5-NH₂ |
| H | 5 | H | CO | -Ph-2-F-5-Me |
| H | 5 | H | CO | -Ph-3-F-2-Me |
| H | 5 | H | CO | -Ph-3-F-4-Me |
| H | 5 | H | CO | -Ph-5-F-2-Me |
| H | 5 | H | CO | -Ph-3-Br-4-Me |
| H | 5 | H | CO | -Ph-4-Cl-2-OMe |
| H | 5 | H | CO | -Ph-5-Cl-2-OMe |
| H | 5 | H | CO | -Ph-3-F-4-OMe |
| H | 5 | H | CO | -Ph-2-Br-5-OMe |
| H | 5 | H | CO | -Ph-2-NH₂-3-OMe |
| H | 5 | H | CO | -Ph-3-NH₂-4-OMe |
| H | 5 | H | CO | -Ph-4-NH₂-3-OMe |
| H | 5 | H | CO | -Ph-3-NH₂-4-OH |
| H | 5 | H | CO | -Ph-4-NH₂-3-OH |

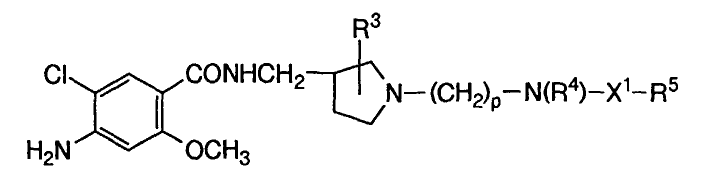

**Table 30**

| R³ | p | R⁴ | X¹ | R⁵ |
|---|---|---|---|---|
| H | 5 | H | CO | -Ph-3-NH₂-2-OH |
| H | 5 | H | CO | -Ph-4-NH₂-2-OH |
| H | 5 | H | CO | -Ph-5-NH₂-2-OH |
| H | 5 | H | CO | -Ph-3-OH-4-Me |
| H | 5 | H | CO | -Ph-2-OH-3-Me |
| H | 5 | H | CO | -Ph-2-OH-4-Me |
| H | 5 | H | CO | -Ph-2-OH-5-Me |
| H | 5 | H | CO | -Ph-2-OH-3-i-Pr |
| H | 5 | H | CO | -Ph-3-OMe-4-Me |
| H | 5 | H | CO | -Ph-2-NH₂-3-Cl |
| H | 5 | H | CO | -Ph-2-NH₂-4-Cl |
| H | 5 | H | CO | -Ph-2-NH₂-5-Cl |
| H | 5 | H | CO | -Ph-3-NH₂-4-Cl |
| H | 5 | H | CO | -Ph-4-NH₂-2-Cl |
| H | 5 | H | CO | -Ph-5-NH₂-2-Cl |
| H | 5 | H | CO | -Ph-2-NH₂-4-F |
| H | 5 | H | CO | -Ph-2-NH₂-5-F |
| H | 5 | H | CO | -Ph-2-NH₂-5-Br |
| H | 5 | H | CO | -Ph-3-Cl-4-OH |
| H | 5 | H | CO | -Ph-4-Cl-2-OH |
| H | 5 | H | CO | -Ph-5-Cl-2-OH |
| H | 5 | H | CO | -Ph-5-F-2-OH |
| H | 5 | H | CO | -Ph-5-Br-2-OH |
| H | 5 | H | CO | -Ph-2-NH₂-3-Me |
| H | 5 | H | CO | -Ph-2-NH₂-5-Me |
| H | 5 | H | CO | -Ph-2-NH₂-6-Me |
| H | 5 | H | CO | -Ph-3-NH₂-2-Me |
| H | 5 | H | CO | -Ph-3-NH₂-4-Me |
| H | 5 | H | CO | -Ph-4-NH₂-3-Me |
| H | 5 | H | CO | -Ph-2-NH₂-3-OMe |
| H | 5 | H | CO | -Ph-3-NH₂-4-OMe |
| H | 5 | H | CO | -Ph-4-NH₂-3-OMe |

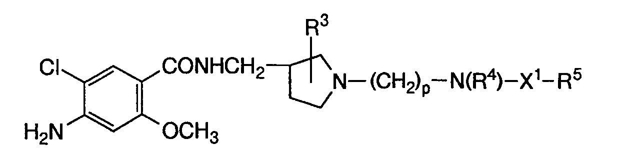

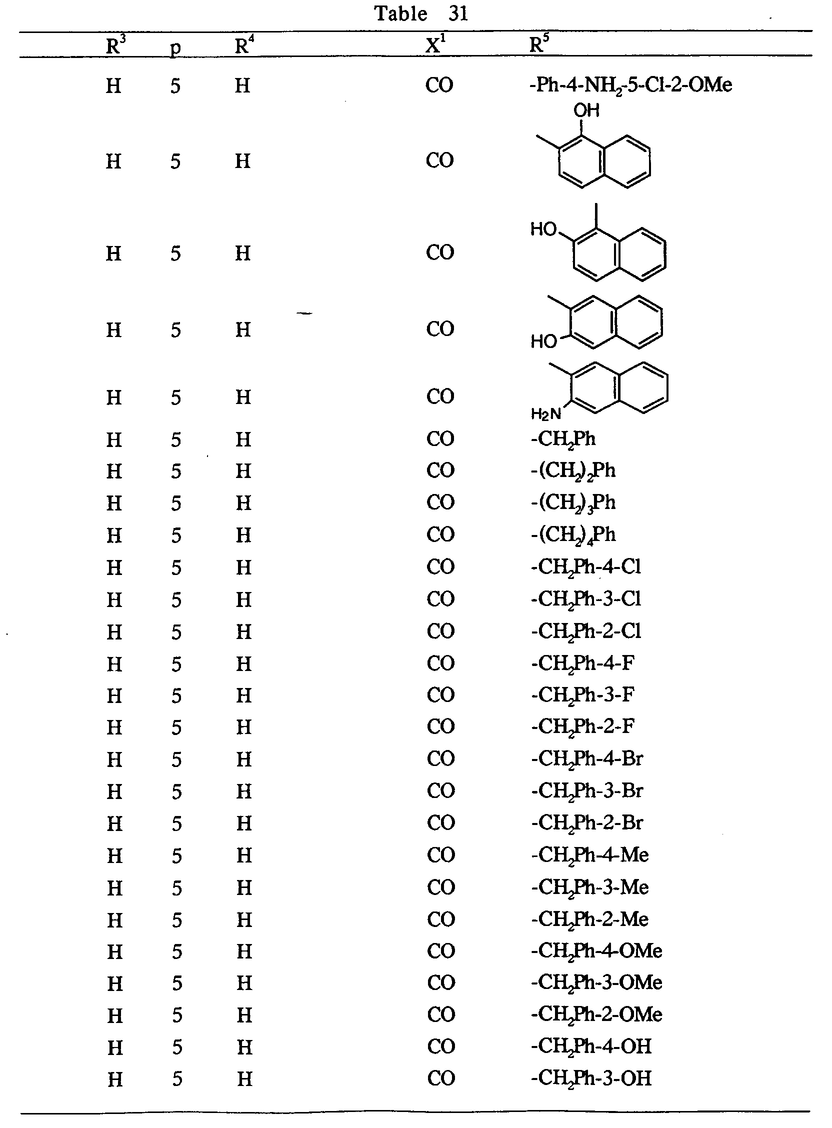

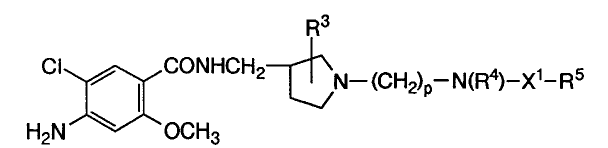

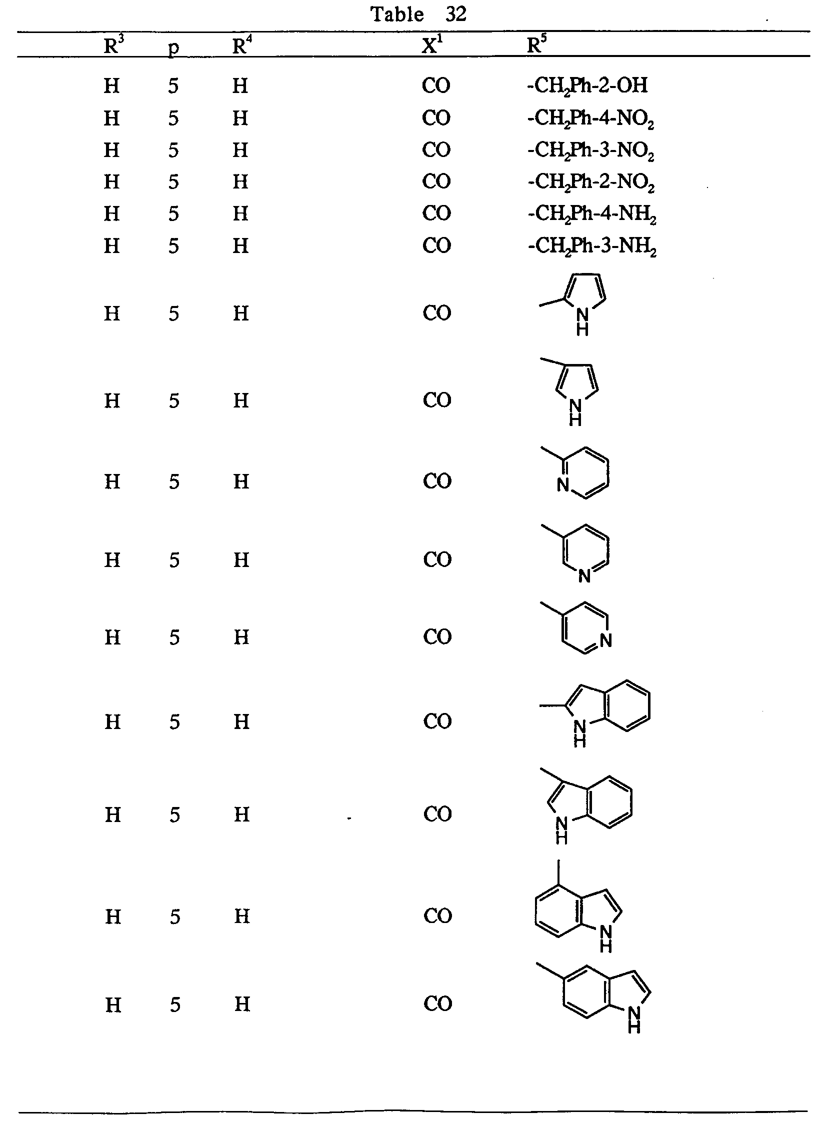

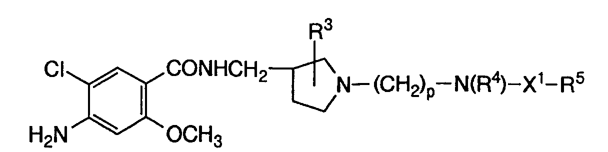

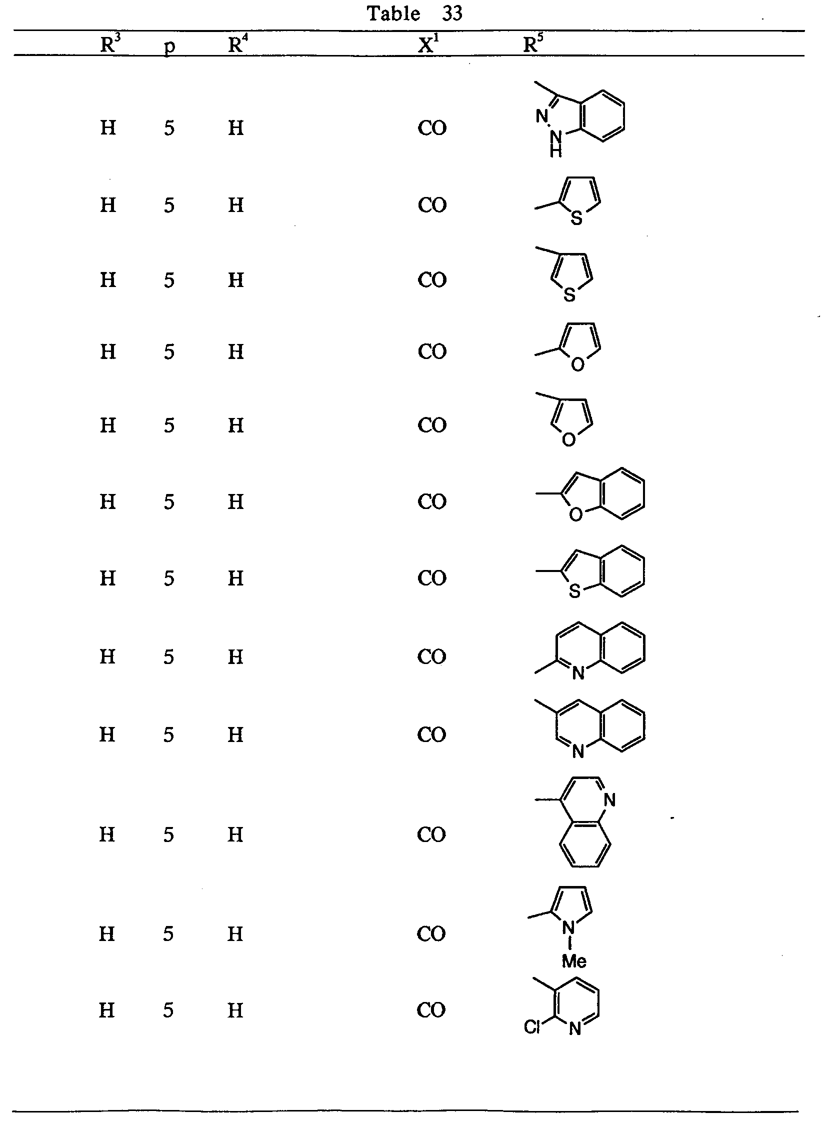

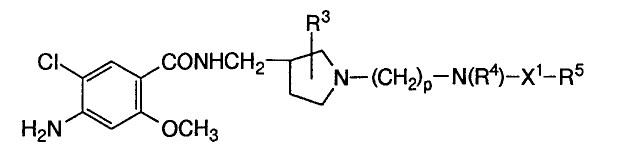

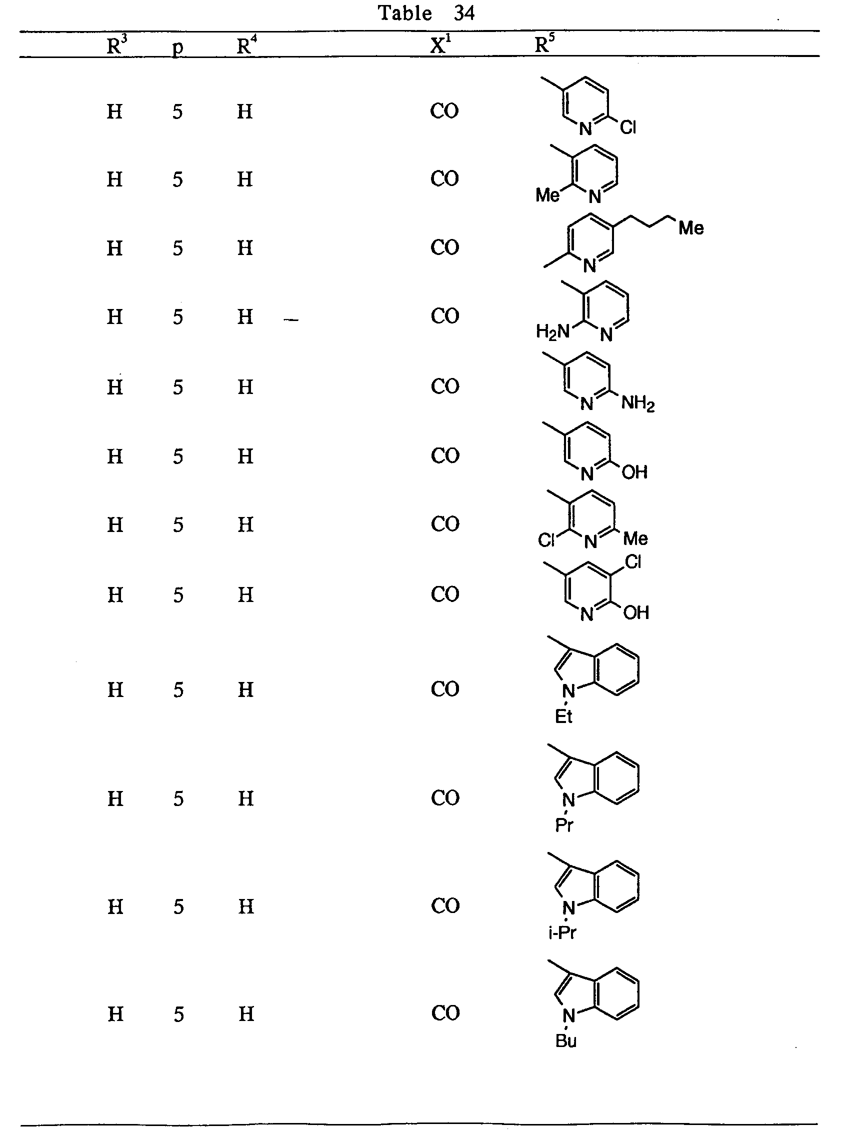

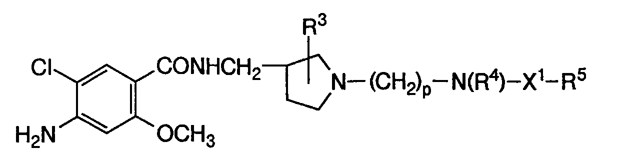

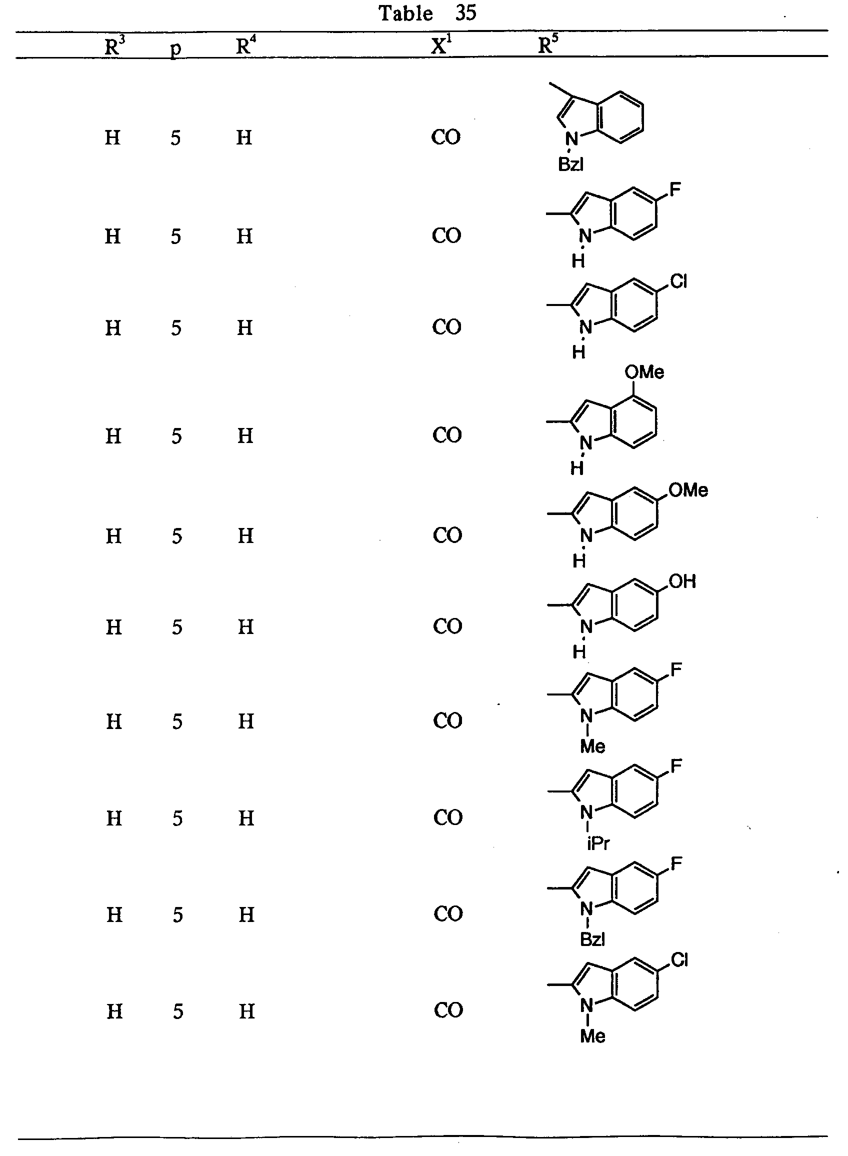

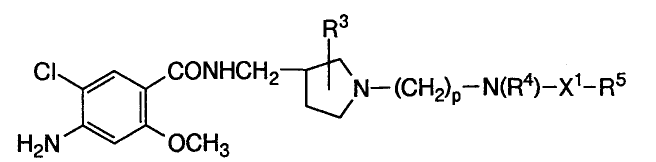

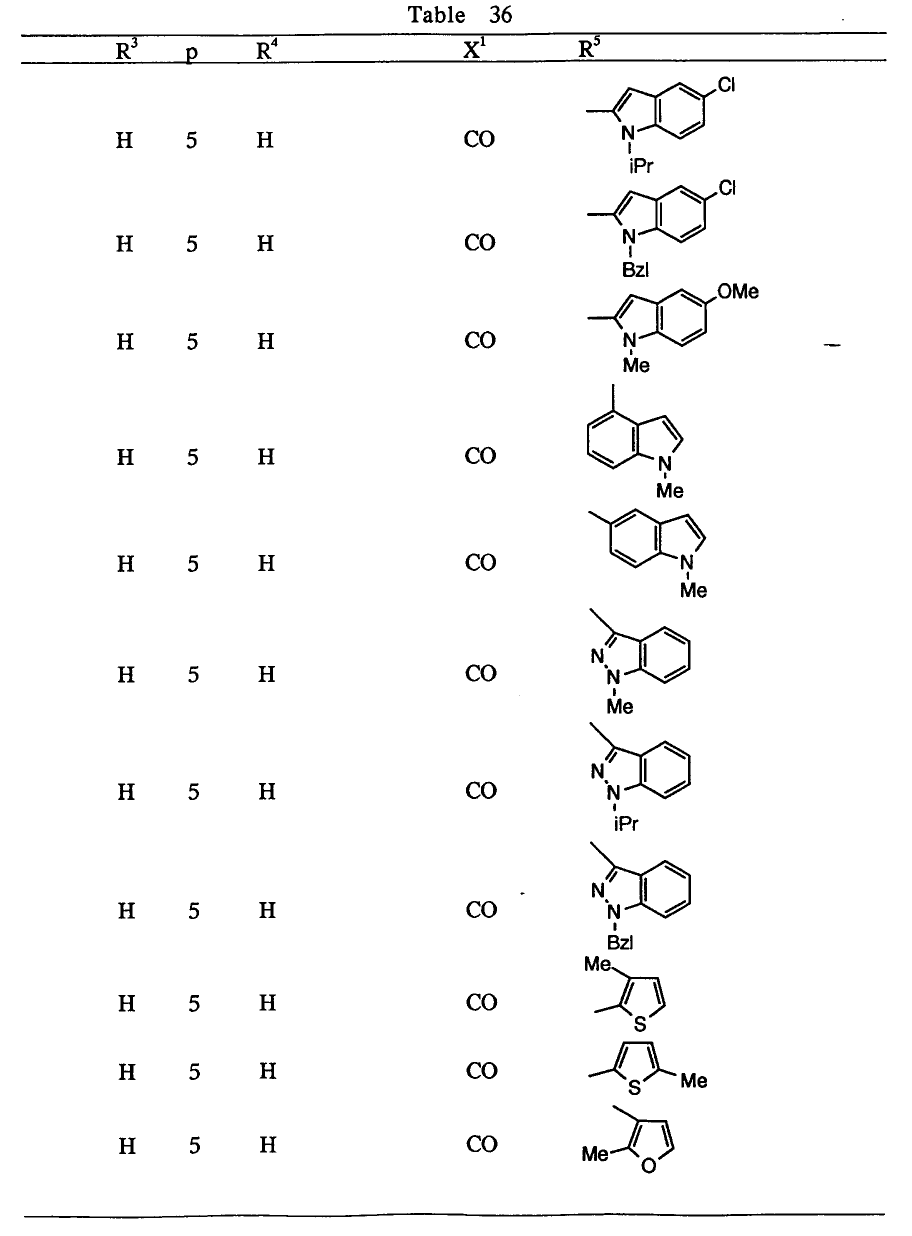

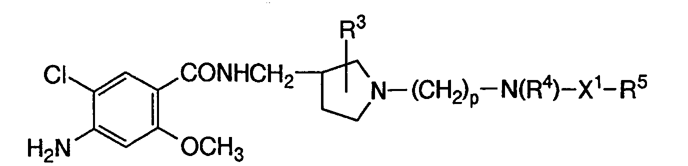

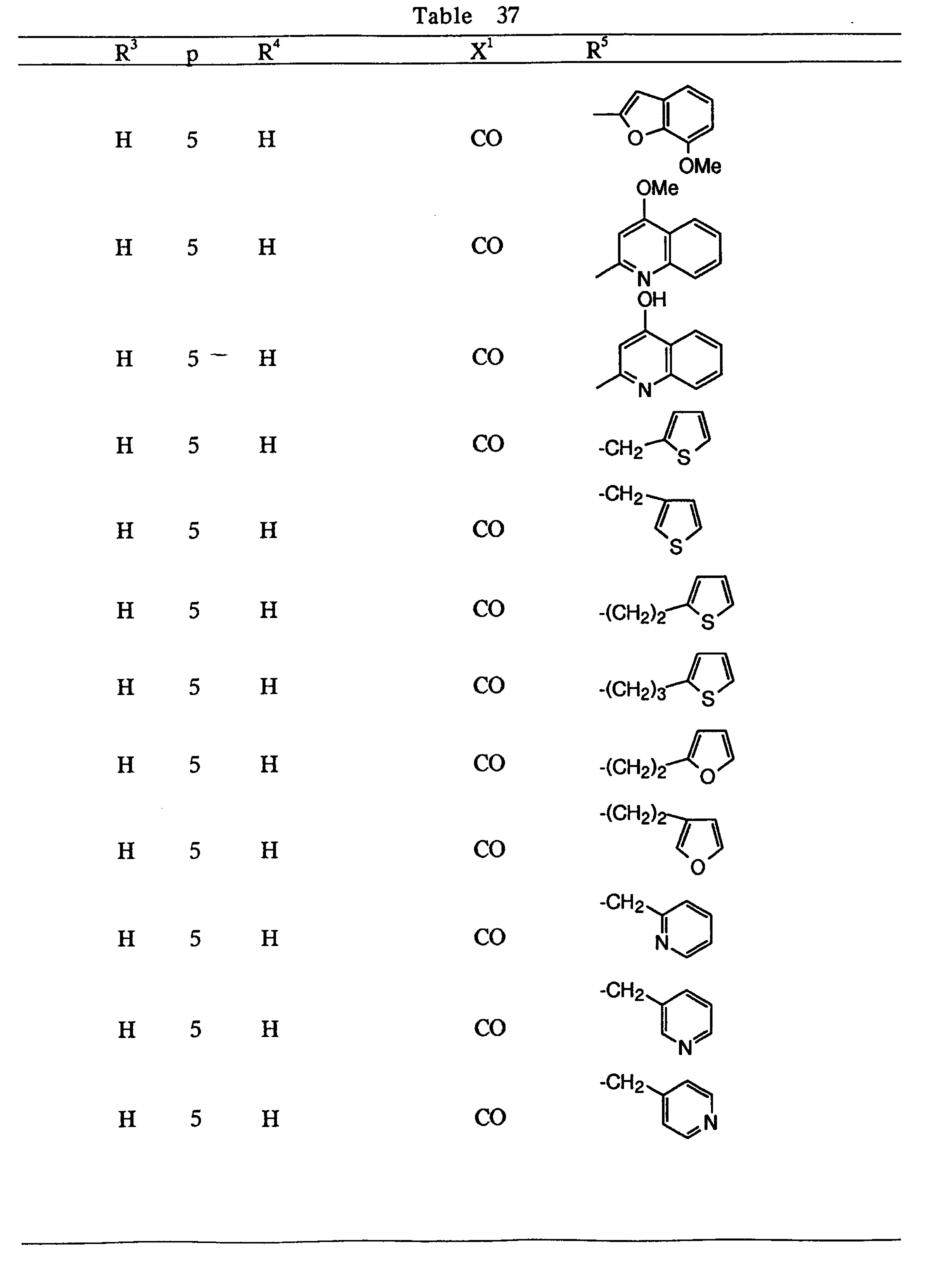

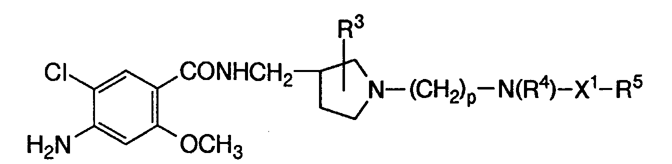

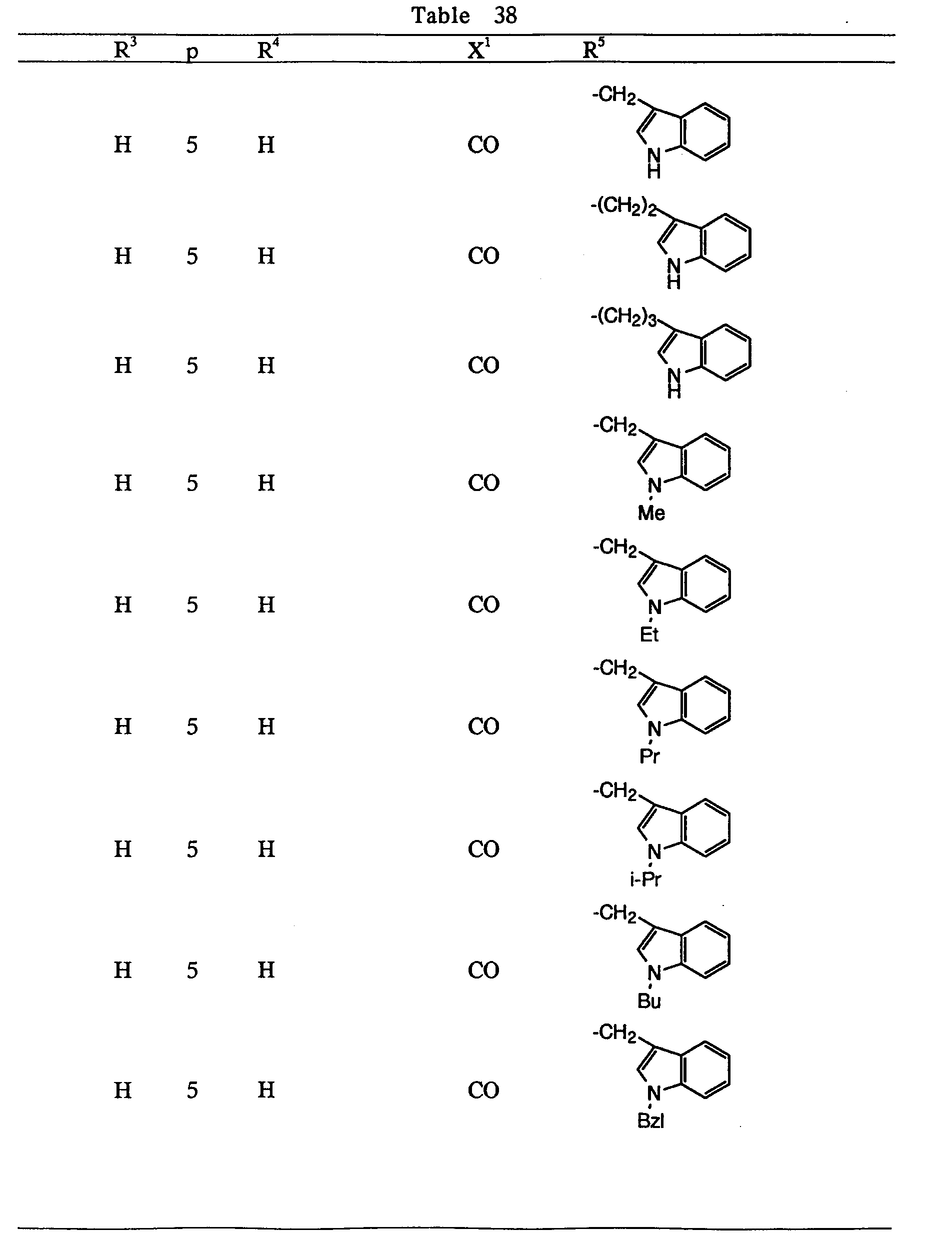

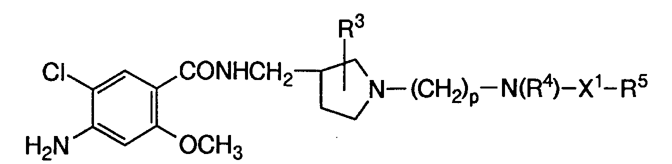

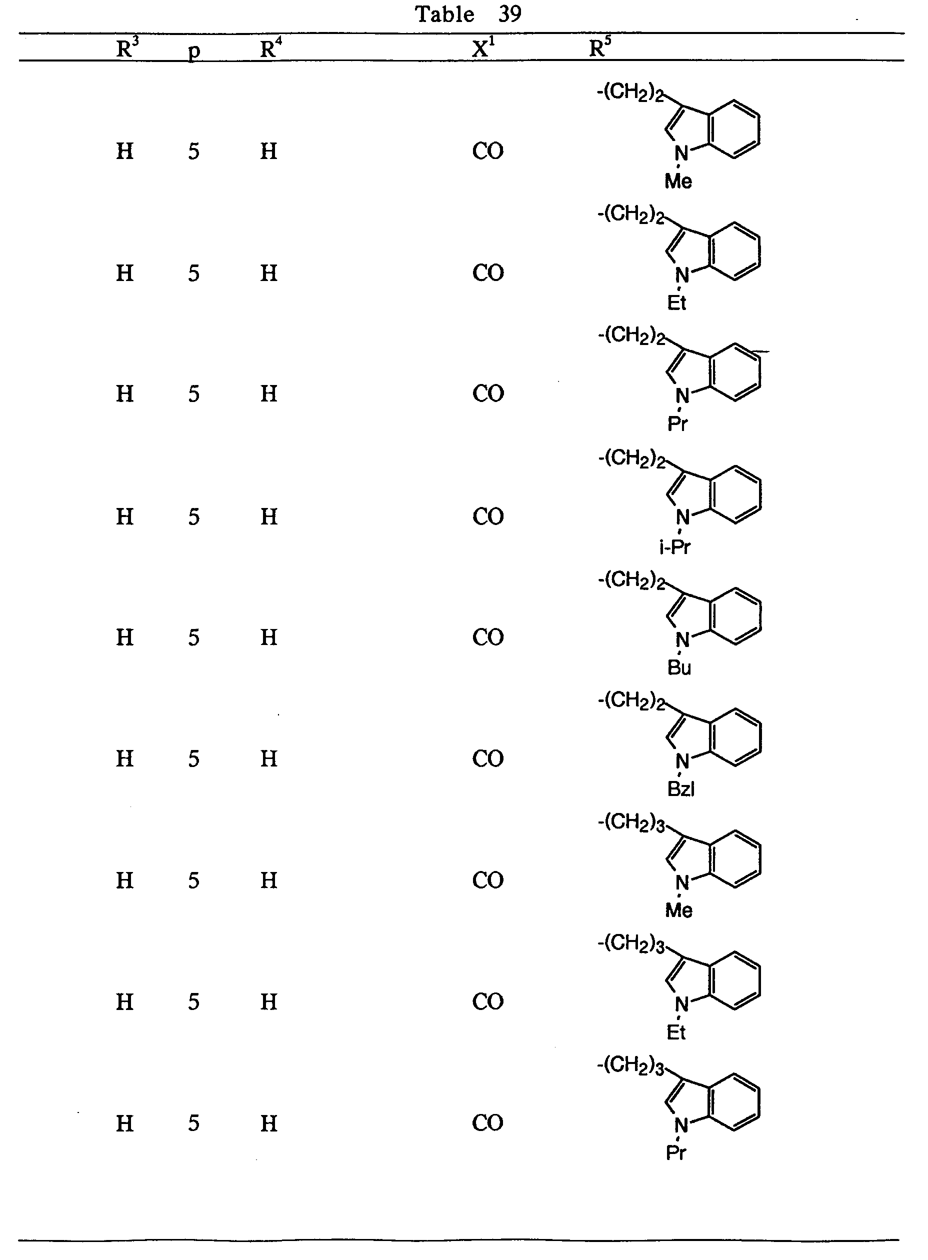

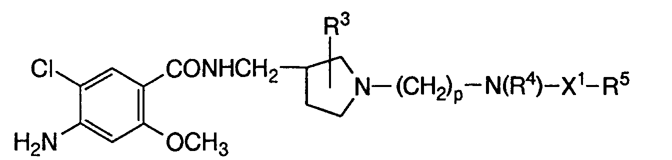

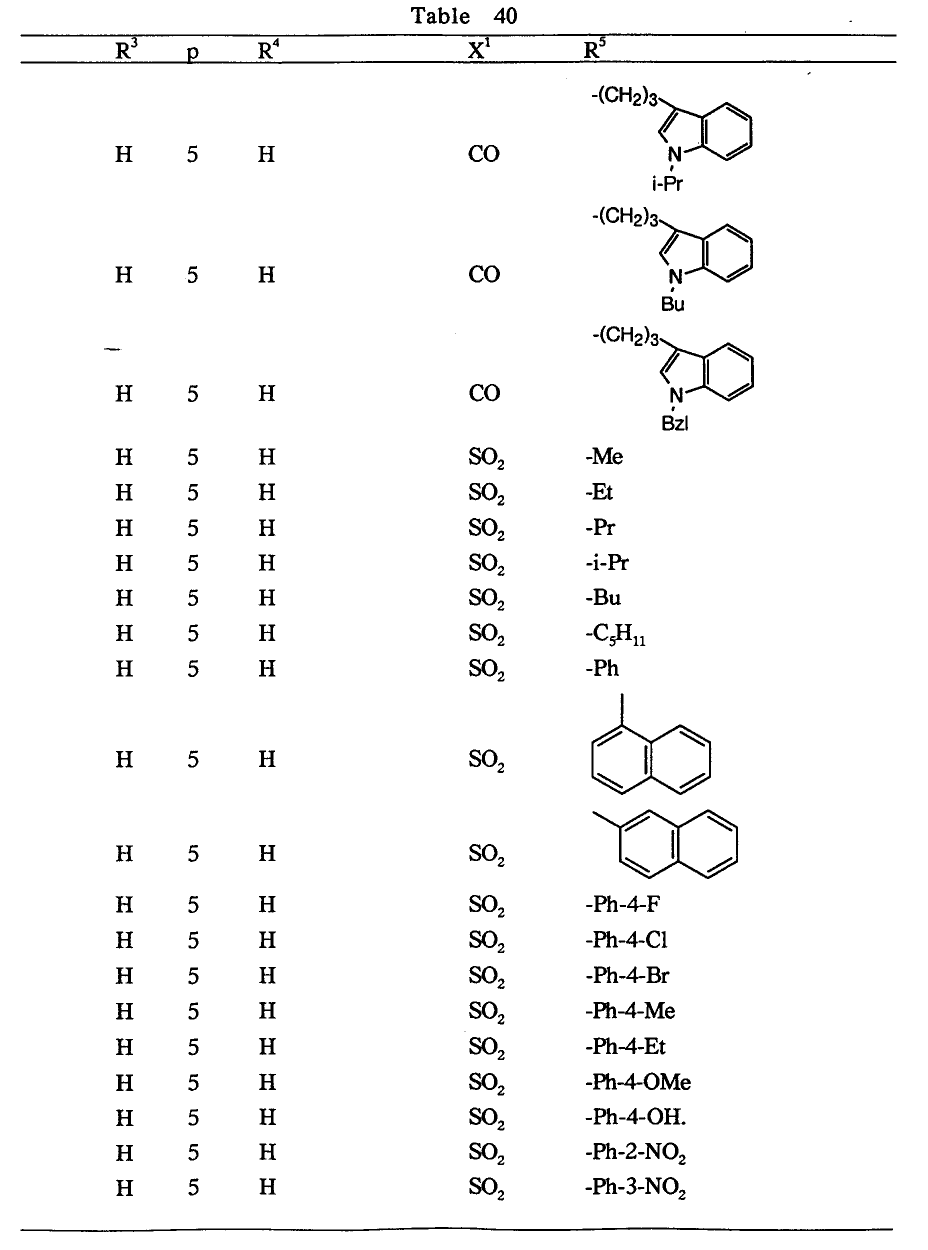

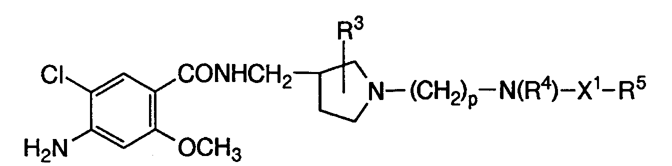

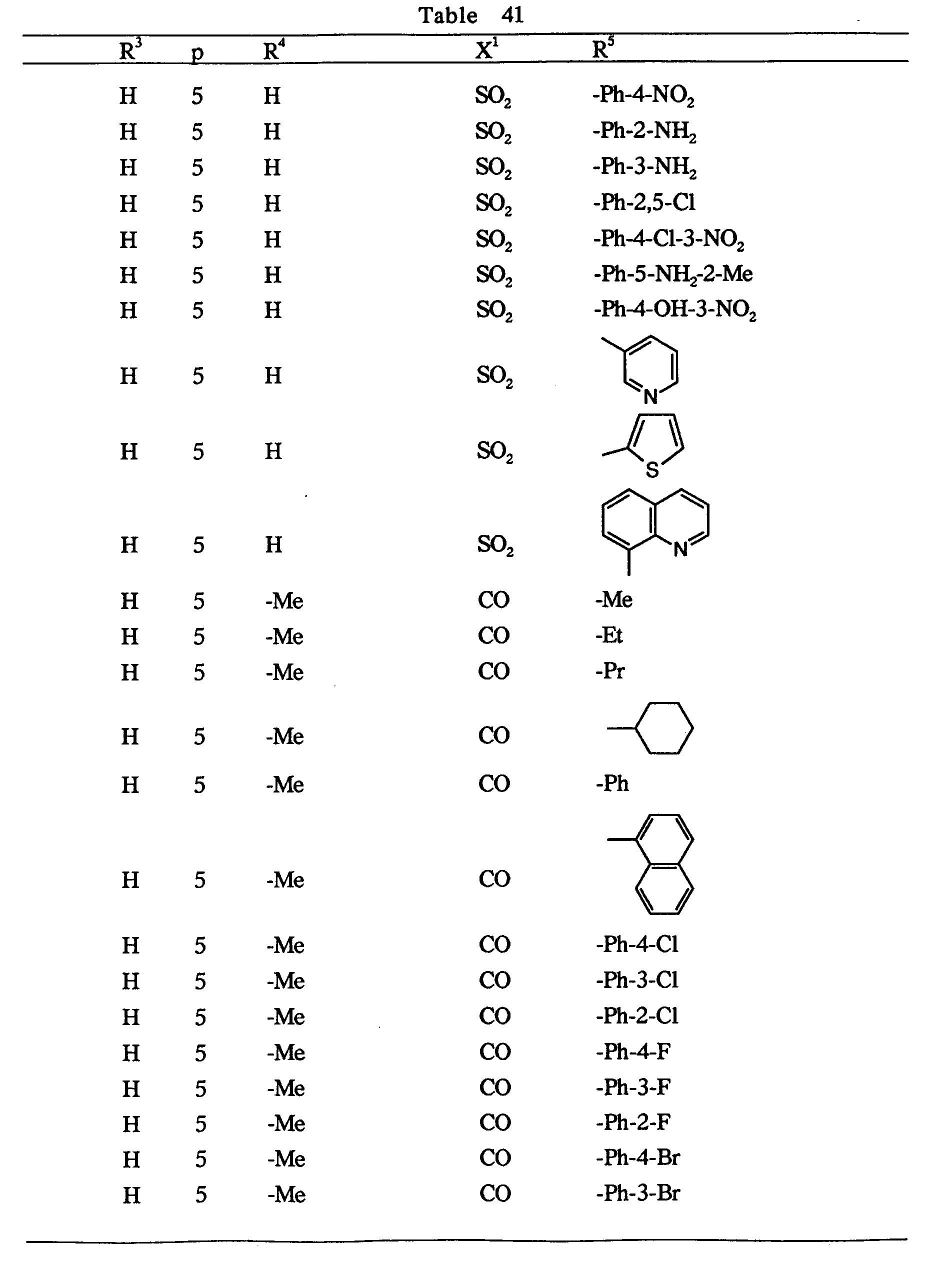

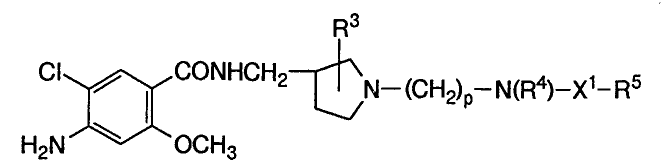

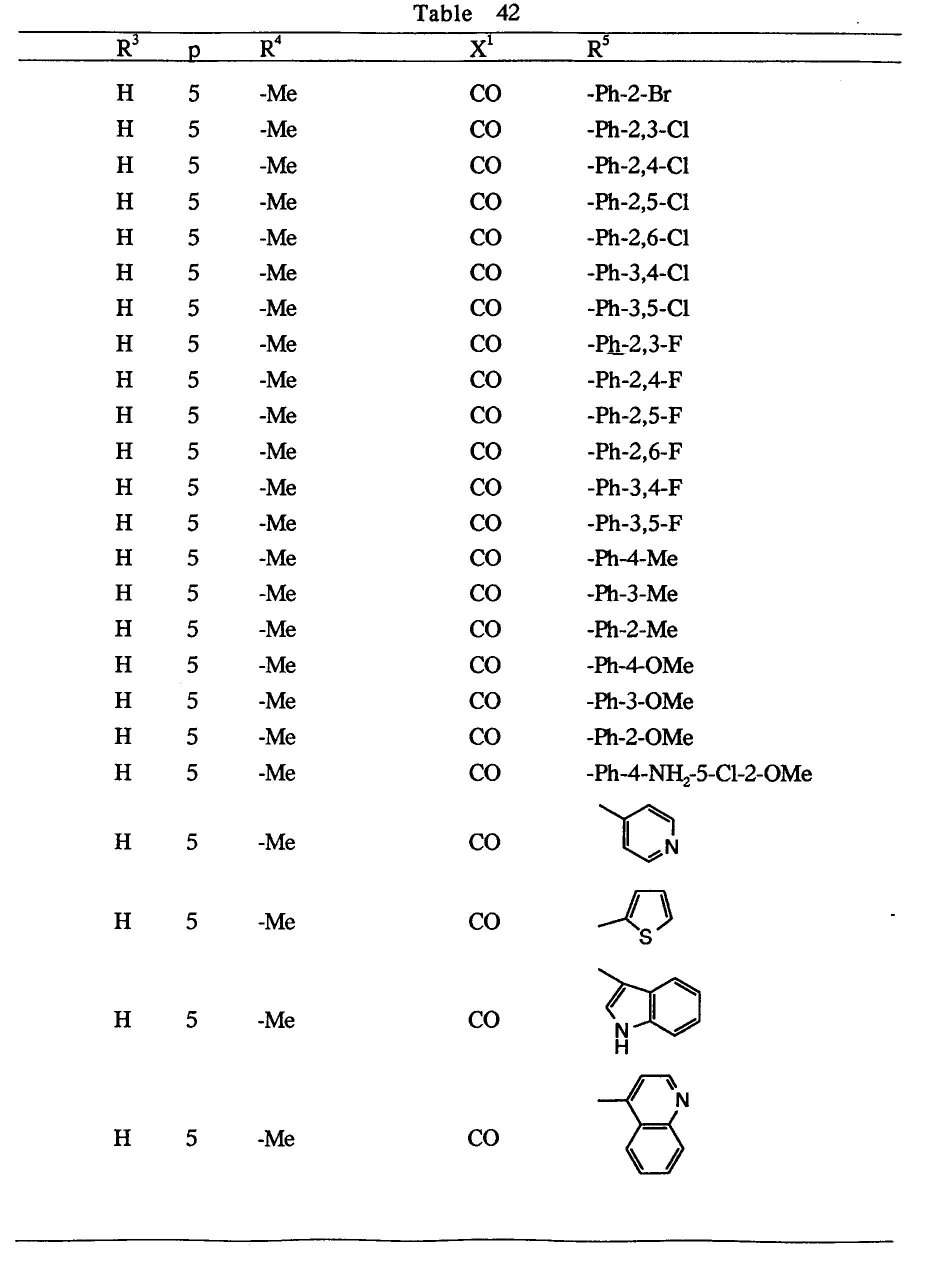

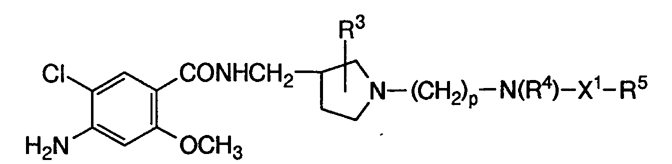

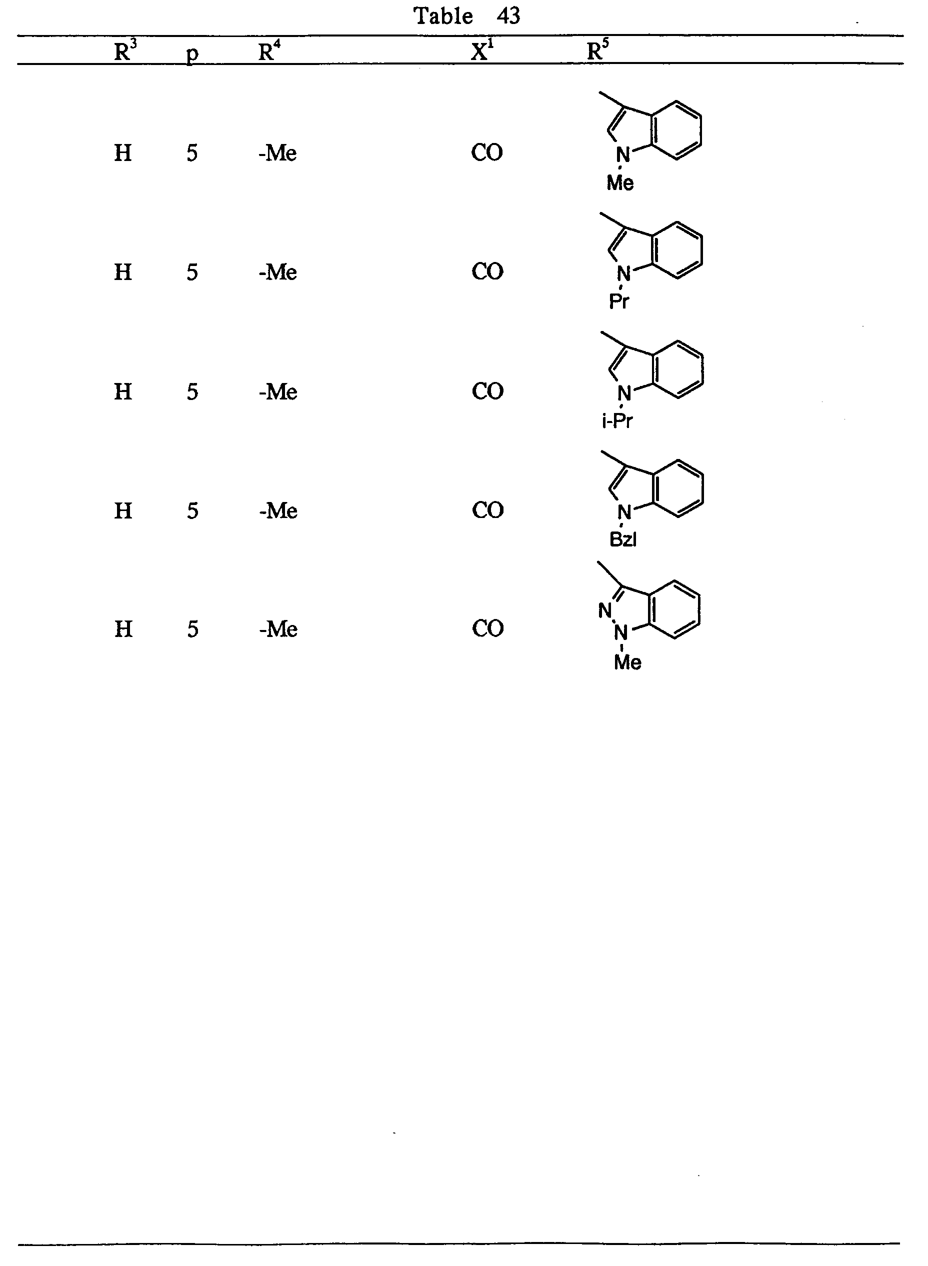

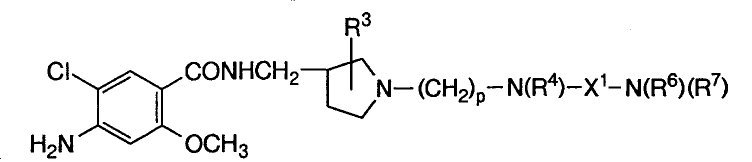

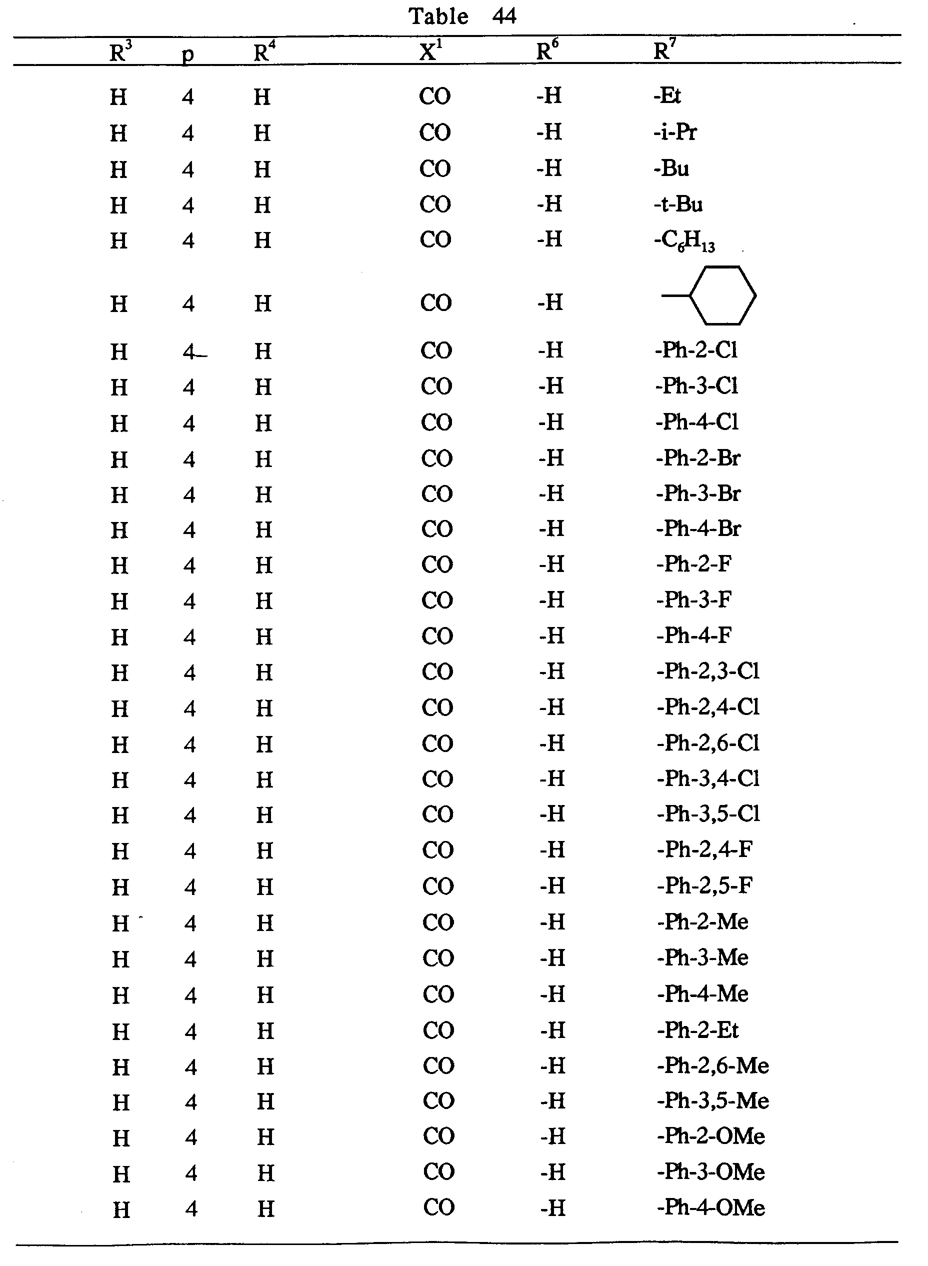

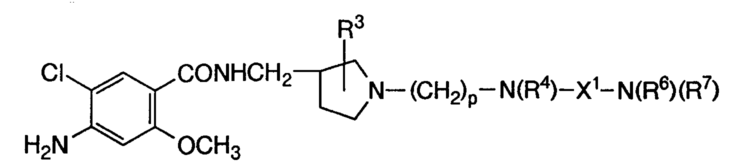

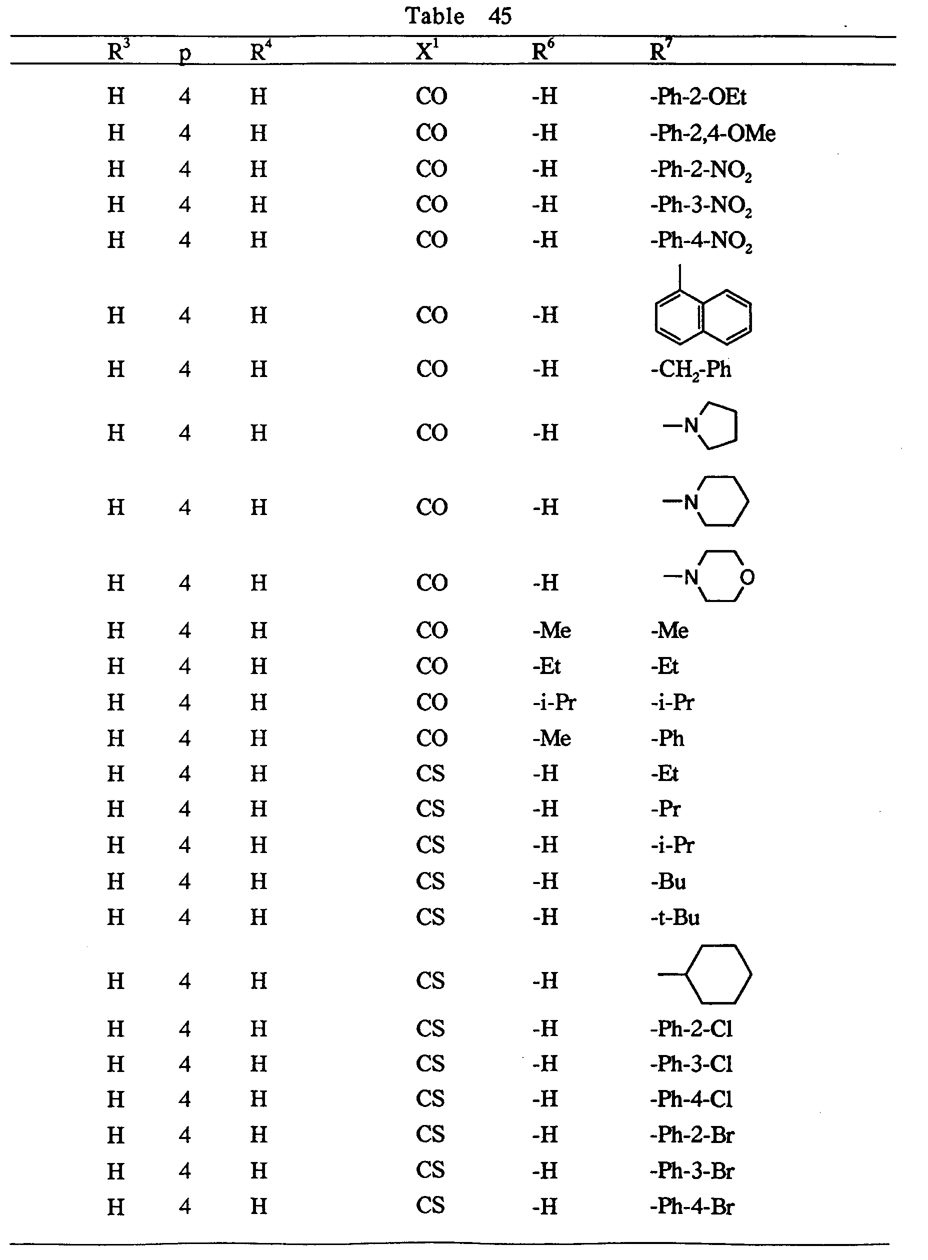

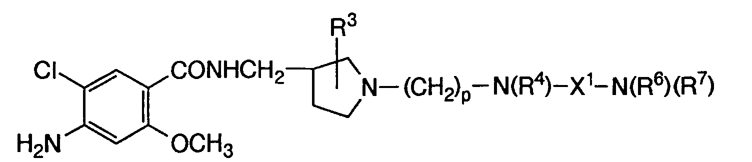

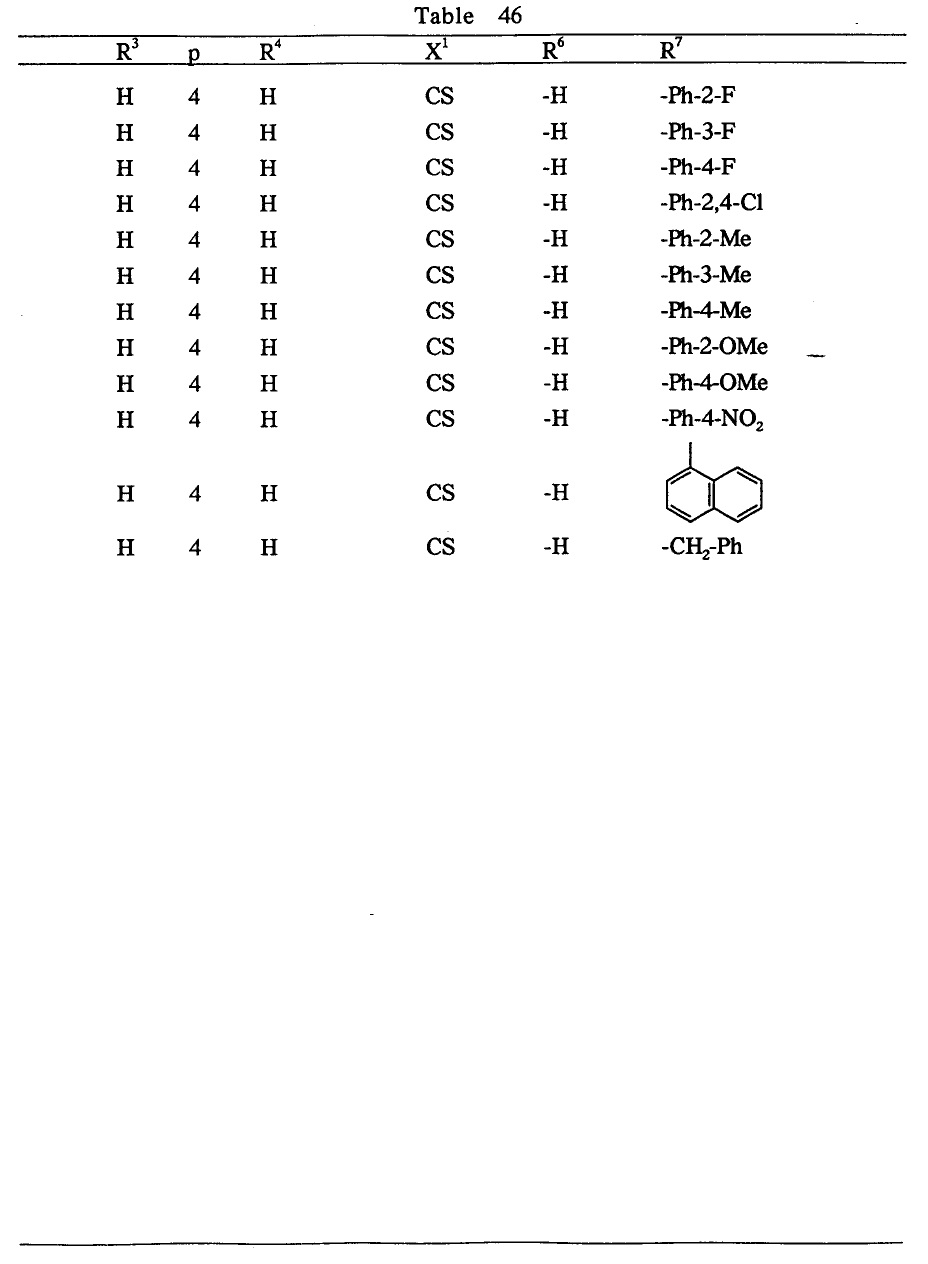

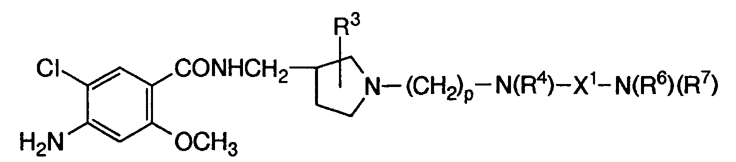

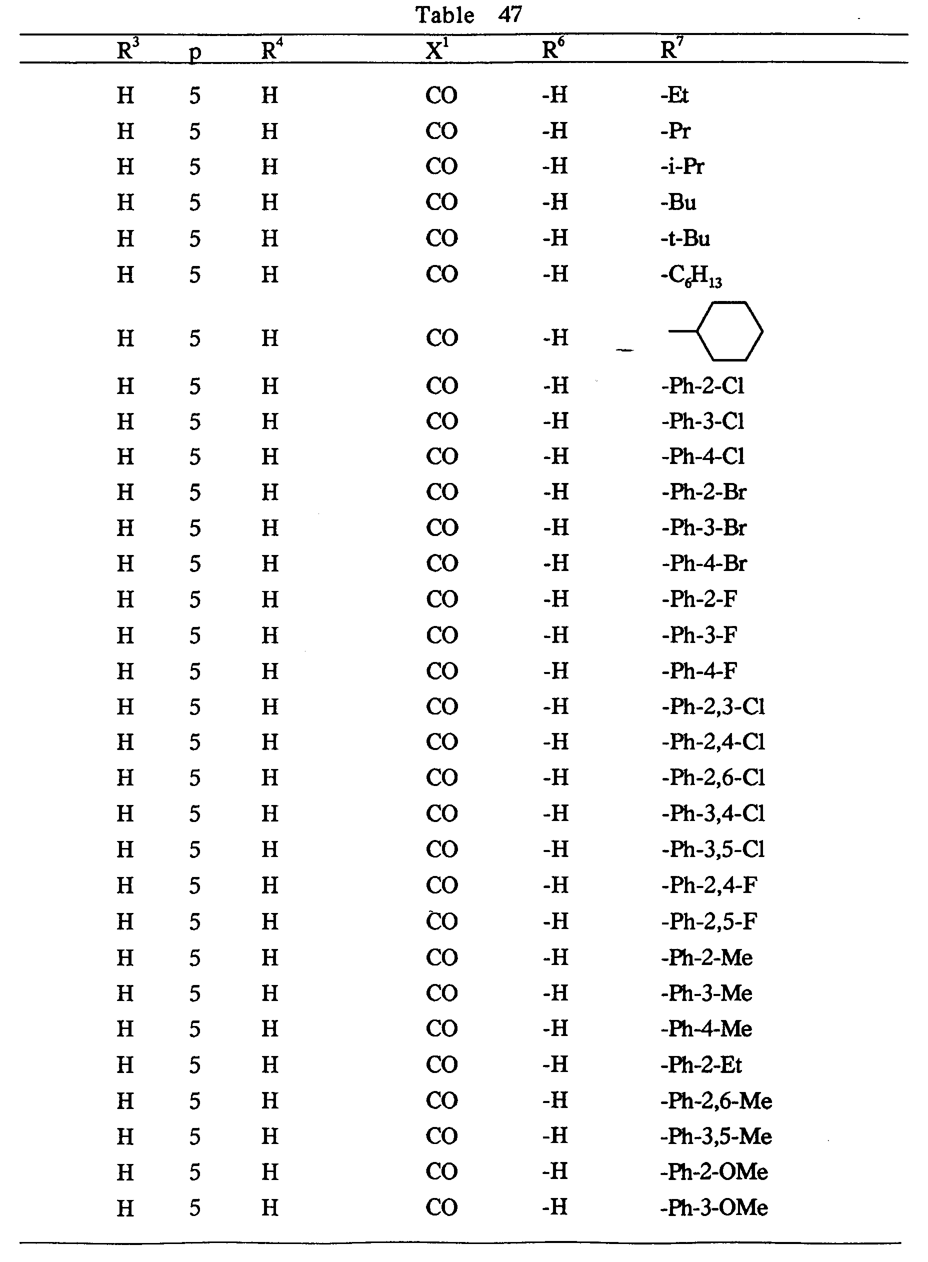

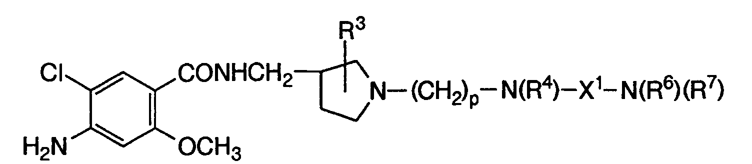

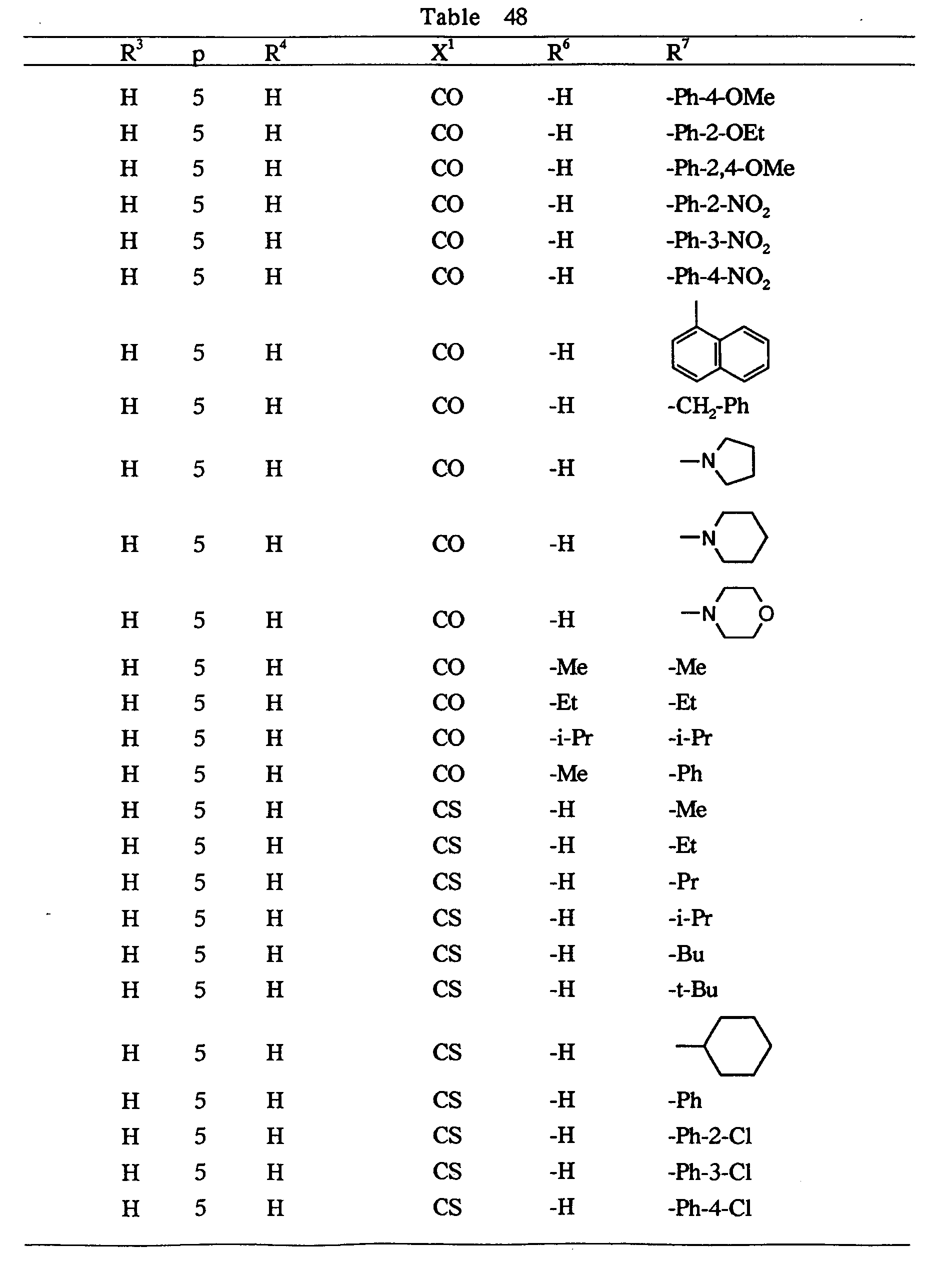

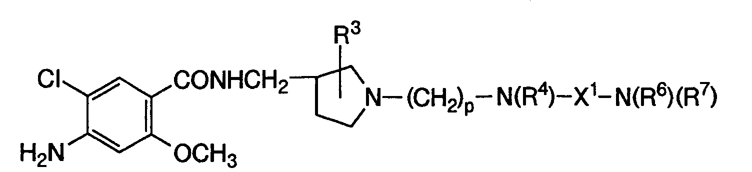

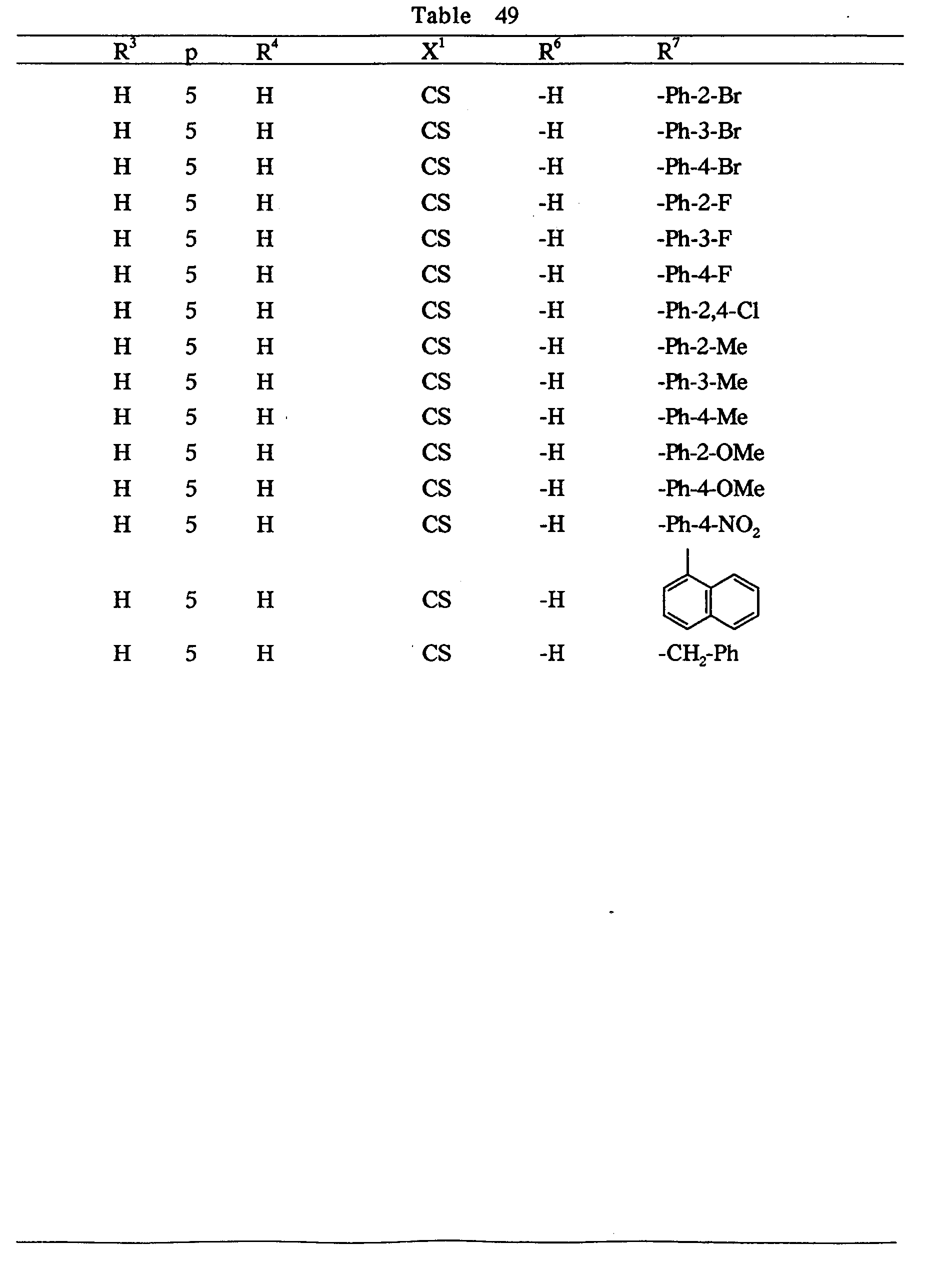

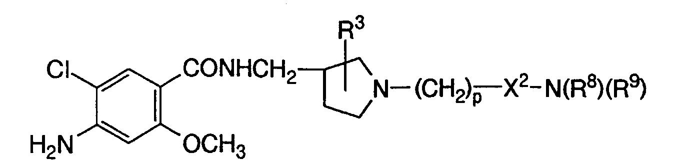

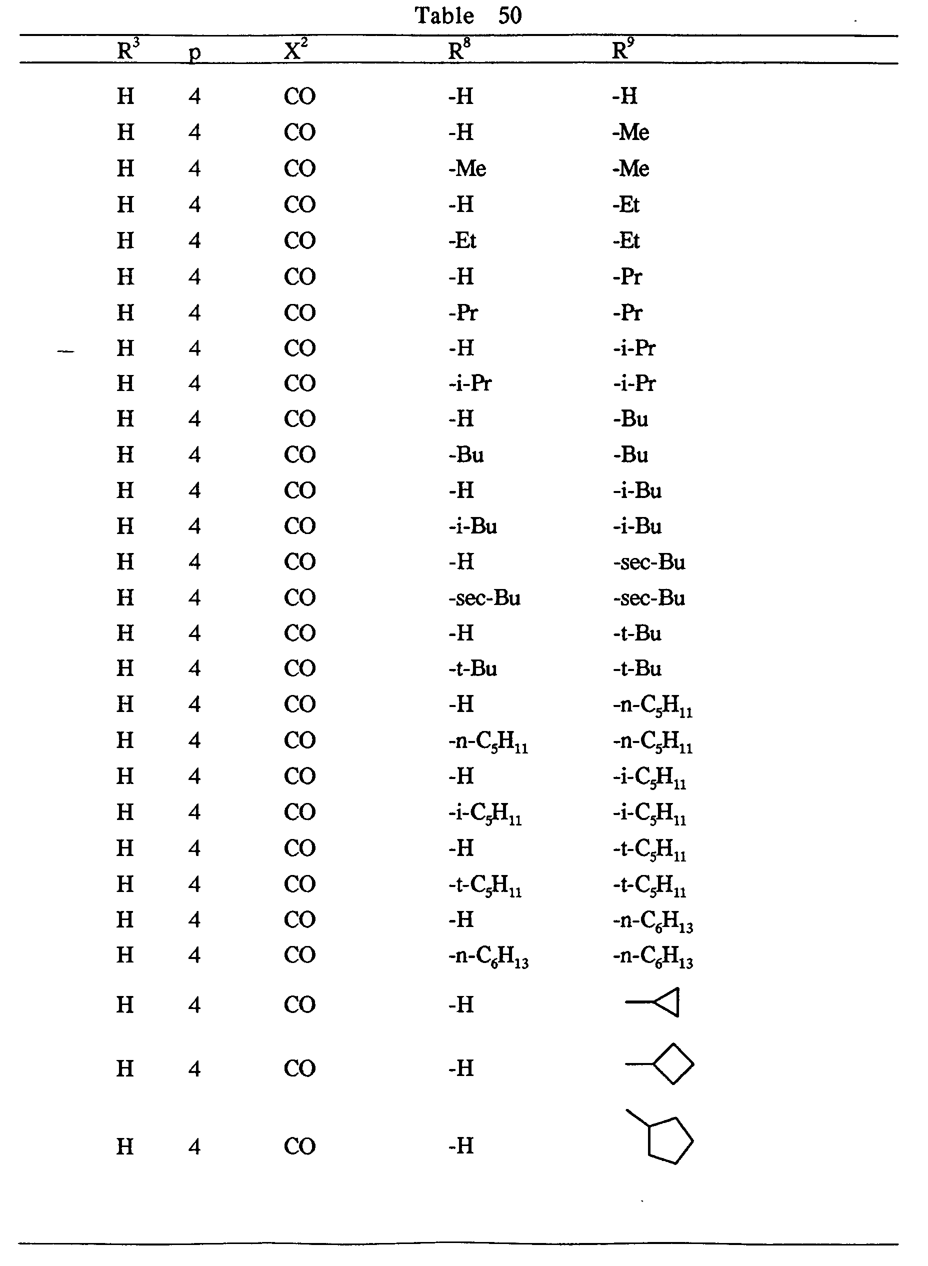

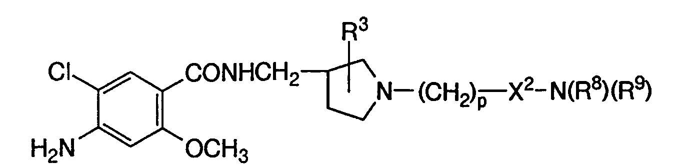

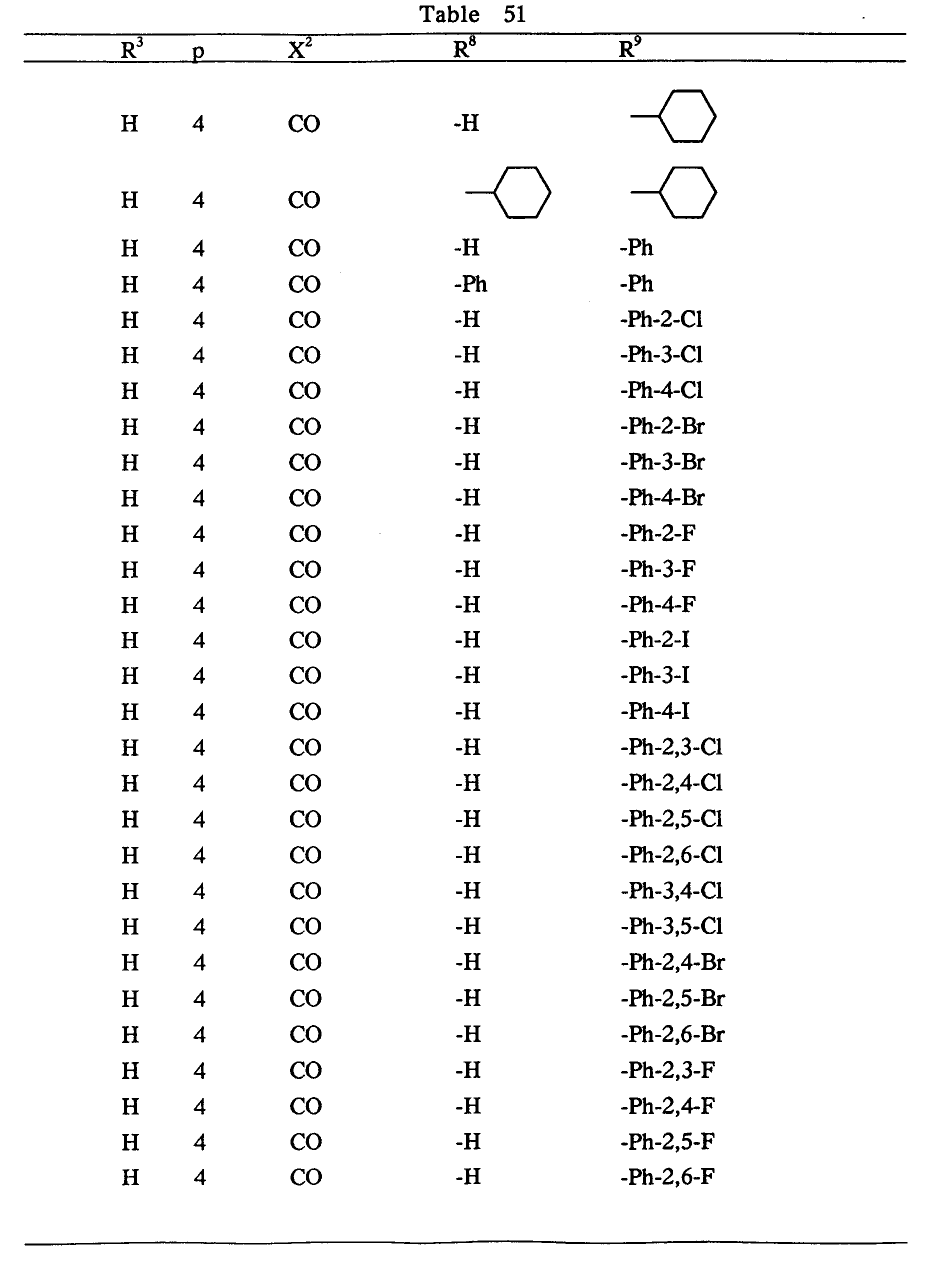

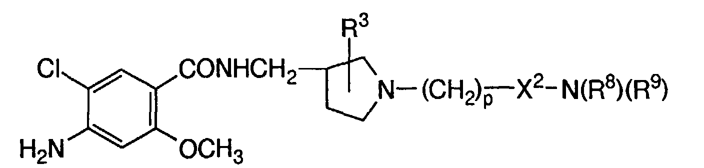

**Table 52**

| R³ | p | X² | R⁸ | R⁹ |
|---|---|---|---|---|
| H | 4 | CO | -H | -Ph-3,4-F |
| H | 4 | CO | -H | -Ph-3,5-F |
| H | 4 | CO | -H | -Ph-2-Cl-4-F |
| H | 4 | CO | -H | -Ph-3-Cl-4-F |
| H | 4 | CO | -H | -Ph-4-Cl-2-F |
| H | 4 | CO | -H | -Ph-2-Br-4-F |
| H | 4 | CO | -H | -Ph-4-Br-2-F |
| H | 4 | CO | -H | -Ph-2-Cl-4-Br |
| H | 4 | CO | -H | -Ph-2-F-4-I |
| H | 4 | CO | -H | -Ph-2-Me |
| H | 4 | CO | -H | -Ph-3-Me |
| H | 4 | CO | -H | -Ph-4-Me |
| H | 4 | CO | -H | -Ph-2-Et |
| H | 4 | CO | -H | -Ph-3-Et |
| H | 4 | CO | -H | -Ph-4-Et |
| H | 4 | CO | -H | -Ph-2-Pr |
| H | 4 | CO | -H | -Ph-4-Pr |
| H | 4 | CO | -H | -Ph-2-i-Pr |
| H | 4 | CO | -H | -Ph-4-i-Pr |
| H | 4 | CO | -H | -Ph-4-Bu |
| H | 4 | CO | -H | -Ph-2-t-Bu |
| H | 4 | CO | -H | -Ph-4-t-Bu |
| H | 4 | CO | -H | -Ph-4-sec-Bu |
| H | 4 | CO | -H | -Ph-2,3-Me |
| H | 4 | CO | -H | -Ph-2,4-Me |
| H | 4 | CO | -H | -Ph-2,5-Me |
| H | 4 | CO | -H | -Ph-2,6-Me |
| H | 4 | CO | -H | -Ph-3,4-Me |
| H | 4 | CO | -H | -Ph-3,5-Me |
| H | 4 | CO | -H | -Ph-2,4,6-Me |
| H | 4 | CO | -H | -Ph-2,6-Et |
| H | 4 | CO | -H | -Ph-2-Me-6-Et |

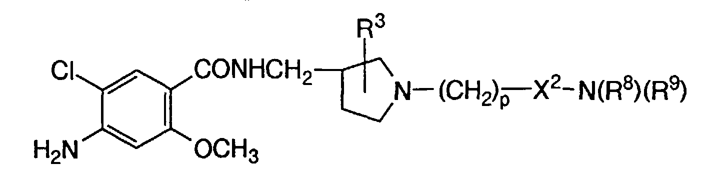

**Table 53**

| R³ | p | X² | R⁸ | R⁹ |
|---|---|---|---|---|
| H | 4 | CO | -H | -Ph-2-Me-6-i-Pr |
| H | 4 | CO | -H | -Ph-2-OMe |
| H | 4 | CO | -H | -Ph-3-OMe |
| H | 4 | CO | -H | -Ph-4-OMe |
| H | 4 | CO | -H | -Ph-2-OEt |
| H | 4 | CO | -H | -Ph-3-OEt |
| H | 4 | CO | -H | -Ph-4-OEt |
| H | 4 | CO | -H | -Ph-2-OPr |
| H | 4 | CO | -H | -Ph-3-OPr |
| H | 4 | CO | -H | -Ph-4-OPr |
| H | 4 | CO | -H | -Ph-2-O-i-Pr |
| H | 4 | CO | -H | -Ph-3-O-i-Pr |
| H | 4 | CO | -H | -Ph-4-O-i-Pr |
| H | 4 | CO | -H | -Ph-4-O-n-Bu |
| H | 4 | CO | -H | -Ph-2-CH₂Ph |
| H | 4 | CO | -H | -Ph-2-OH |
| H | 4 | CO | -H | -Ph-3-OH |
| H | 4 | CO | -H | -Ph-4-OH |
| H | 4 | CO | -H | -Ph-2-NO₂ |
| H | 4 | CO | -H | -Ph-3-NO₂ |
| H | 4 | CO | -H | -Ph-4-NO₂ |
| H | 4 | CO | -H | -Ph-2-NH₂ |
| H | 4 | CO | -H | -Ph-3-NH₂ |
| H | 4 | CO | -H | -Ph-4-NH₂ |
| H | 4 | CO | -H | -Ph-2-OH-6-Me |
| H | 4 | CO | -H | -Ph-2-OH-5-Me |
| H | 4 | CO | -H | -Ph-3-OH-2-Me |
| H | 4 | CO | -H | -Ph-4-OH-2-Me |
| H | 4 | CO | -H | -Ph-2-OH-4-Me |
| H | 4 | CO | -H | -Ph-2-OMe-5-Me |
| H | 4 | CO | -H | -Ph-2-OMe-6-Me |
| H | 4 | CO | -H | -Ph-4-OMe-2-Me |

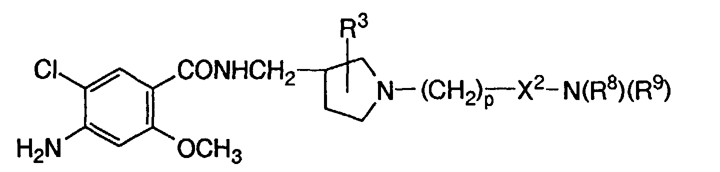

**Table 54**

| R³ | p | X² | R⁸ | R⁹ |
|---|---|---|---|---|
| H | 4 | CO | -H | -Ph-5-OMe-2-Me |
| H | 4 | CO | -H | -Ph-5-t-Bu-2-OH |
| H | 4 | CO | -H | -Ph-3-Cl-4-Me |
| H | 4 | CO | -H | -Ph-2-Cl-4-Me |
| H | 4 | CO | -H | -Ph-2-Cl-5-Me |
| H | 4 | CO | -H | -Ph-2-Cl-6-Me |
| H | 4 | CO | -H | -Ph-3-Cl-2-Me |
| H | 4 | CO | -H | -Ph-4-Cl-2-Me |
| H | 4 | CO | -H | -Ph-5-Cl-2-Me |
| H | 4 | CO | -H | -Ph-2-Br-4-Me |
| H | 4 | CO | -H | -Ph-3-Br-4-Me |
| H | 4 | CO | -H | -Ph-4-Br-2-Me |
| H | 4 | CO | -H | -Ph-4-Br-3-Me |
| H | 4 | CO | -H | -Ph-2-F-4-Me |
| H | 4 | CO | -H | -Ph-2-F-5-Me |
| H | 4 | CO | -H | -Ph-3-F-2-Me |
| H | 4 | CO | -H | -Ph-3-F-4-Me |
| H | 4 | CO | -H | -Ph-4-F-2-Me |
| H | 4 | CO | -H | -Ph-5-F-2-Me |
| H | 4 | CO | -H | -Ph-3-Cl-4-OMe |
| H | 4 | CO | -H | -Ph-5-Cl-2-OMe |
| H | 4 | CO | -H | -Ph-2-Cl-5-OMe |
| H | 4 | CO | -H | -Ph-3-F-2-OMe |
| H | 4 | CO | -H | -Ph-3-F-4-OMe |
| H | 4 | CO | -H | -Ph-5-Cl-2-OH |
| H | 4 | CO | -H | -Ph-2-Cl-4-OH |
| H | 4 | CO | -H | -Ph-2-Cl-4-NO₂ |
| H | 4 | CO | -H | -Ph-2-Cl-5-NO₂ |
| H | 4 | CO | -H | -Ph-4-Cl-2-NO₂ |
| H | 4 | CO | -H | -Ph-4-Cl-3-NO₂ |
| H | 4 | CO | -H | -Ph-5-Cl-2-NO₂ |
| H | 4 | CO | -H | -Ph-2-F-5-NO₂ |

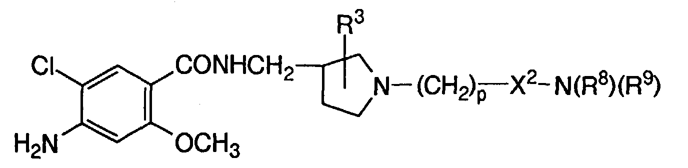

**Table 55**

| R³ | p | X² | R⁸ | R⁹ |
|---|---|---|---|---|
| H | 4 | CO | -H | -Ph-4-F-2-NO₂ |
| H | 4 | CO | -H | -Ph-4-F-3-NO₂ |
| H | 4 | CO | -H | -Ph-4-OH-2,5-Me |
| H | 4 | CO | -H | -Ph-2-OH-3,5-Me |
| H | 4 | CO | -H | -Ph-4-OH-2,5-Me |
| H | 4 | CO | -H | -Ph-2-Cl-4,6-Me |
| H | 4 | CO | -H | -Ph-4-Br-2,6-Me |
| H | 4 | CO | -H | -CH₂Ph |
| H | 4 | CO | -CH₂Ph | -CH₂Ph |
| H | 4 | CO | -H | -CHPh₂ |
| H | 4 | CO | -H | -(CH₂)₂Ph |
| H | 4 | CO | -H | -CH(CH₃)Ph |
| H | 4 | CO | -H | -(CH₂)₃Ph |
| H | 4 | CO | -H | -CH₂CH(CH₃)Ph |
| H | 4 | CO | -H | -(CH₂)₄Ph |
| H | 4 | CO | -H | -CH(CH₃)-(CH₂)₂Ph |
| H | 4 | CO | -H | -CH₂Ph-2-Cl |
| H | 4 | CO | -H | -CH₂Ph-3-Cl |
| H | 4 | CO | -H | -CH₂Ph-4-Cl |
| H | 4 | CO | -H | -CH₂Ph-2-Br |
| H | 4 | CO | -H | -CH₂Ph-3-Br |
| H | 4 | CO | -H | -CH₂Ph-4-Br |
| H | 4 | CO | -H | -CH₂Ph-2-F |
| H | 4 | CO | -H | -CH₂Ph-3-F |
| H | 4 | CO | -H | -CH₂Ph-4-F |
| H | 4 | CO | -H | -CH₂Ph-3-I |
| H | 4 | CO | -H | -CH₂Ph-2-Me |
| H | 4 | CO | -H | -CH₂Ph-3-Me |
| H | 4 | CO | -H | -CH₂Ph-4-Me |
| H | 4 | CO | -H | -CH₂Ph-2-OMe |
| H | 4 | CO | -H | -CH₂Ph-4-OMe |
| H | 4 | CO | -H | -CH₂Ph-2-OEt |

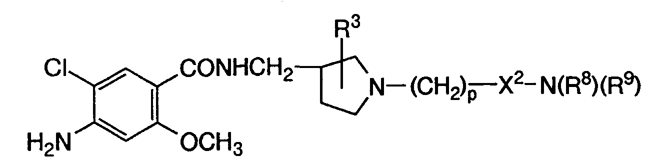

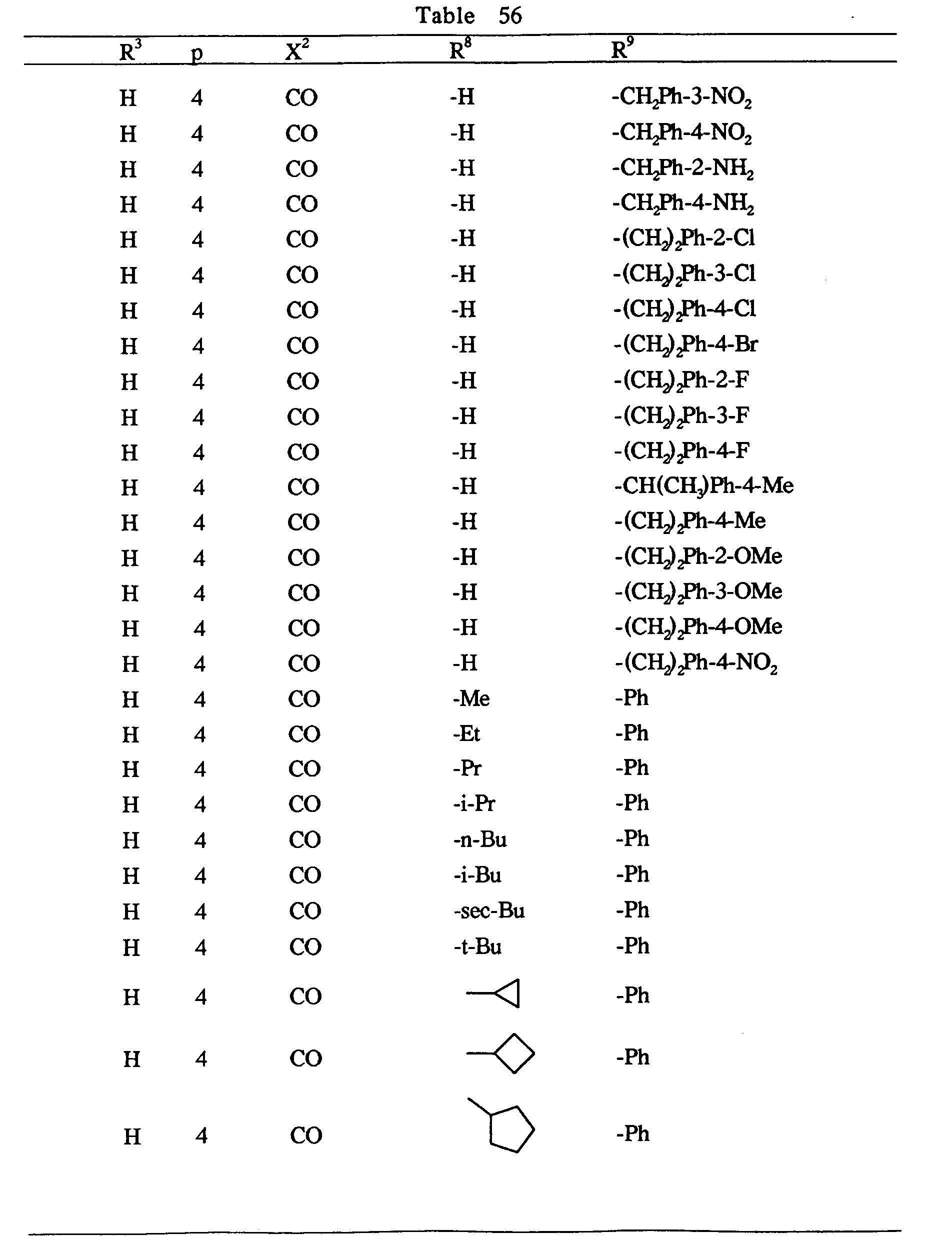

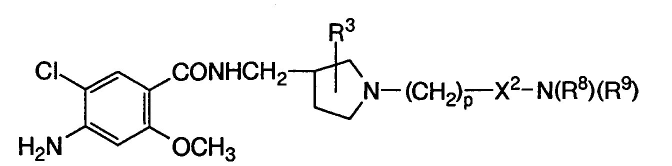

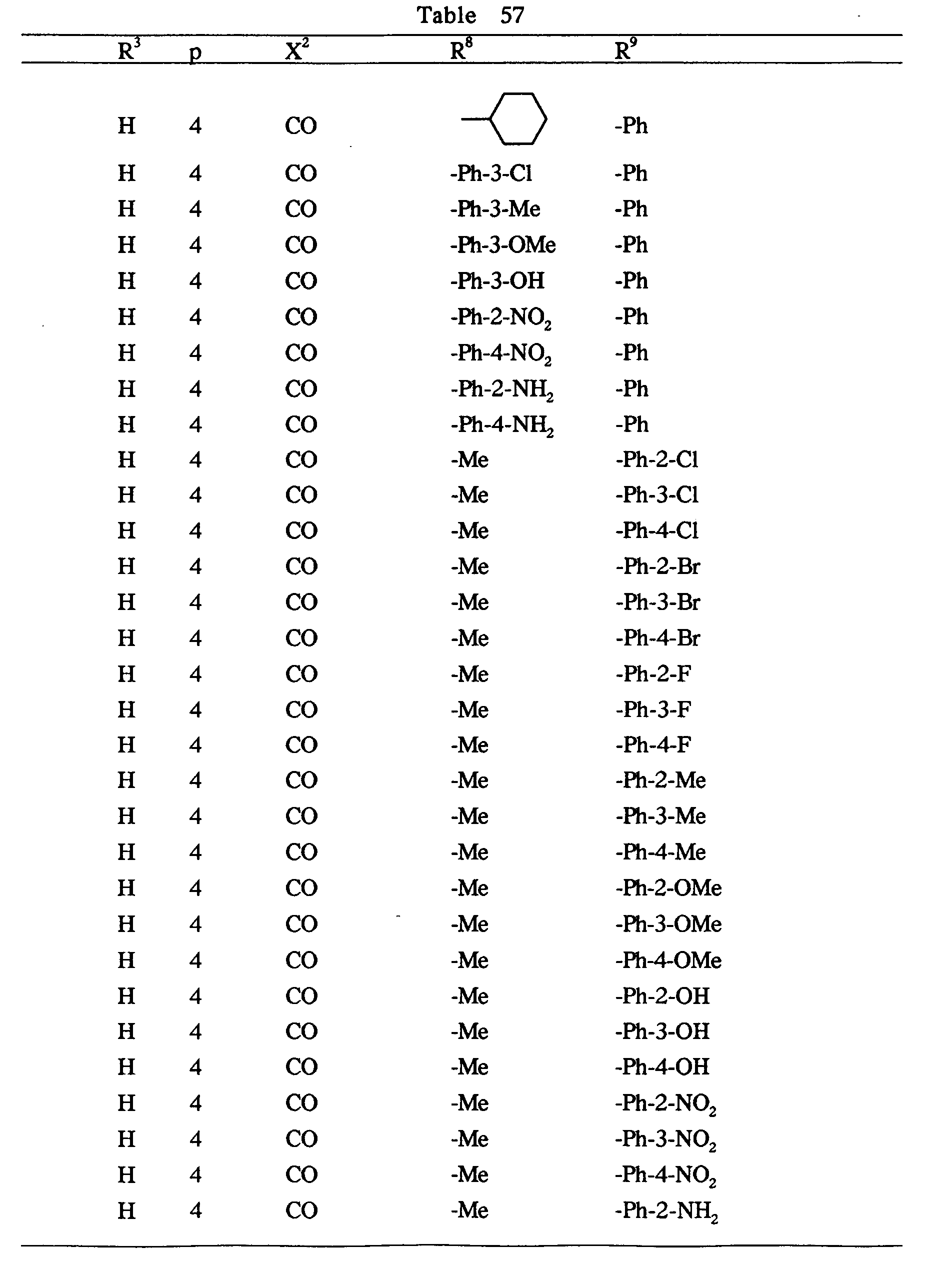

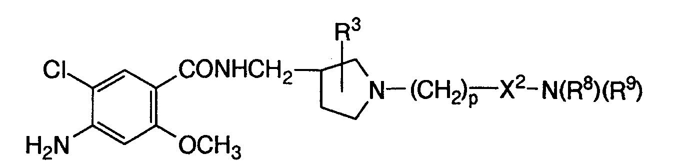

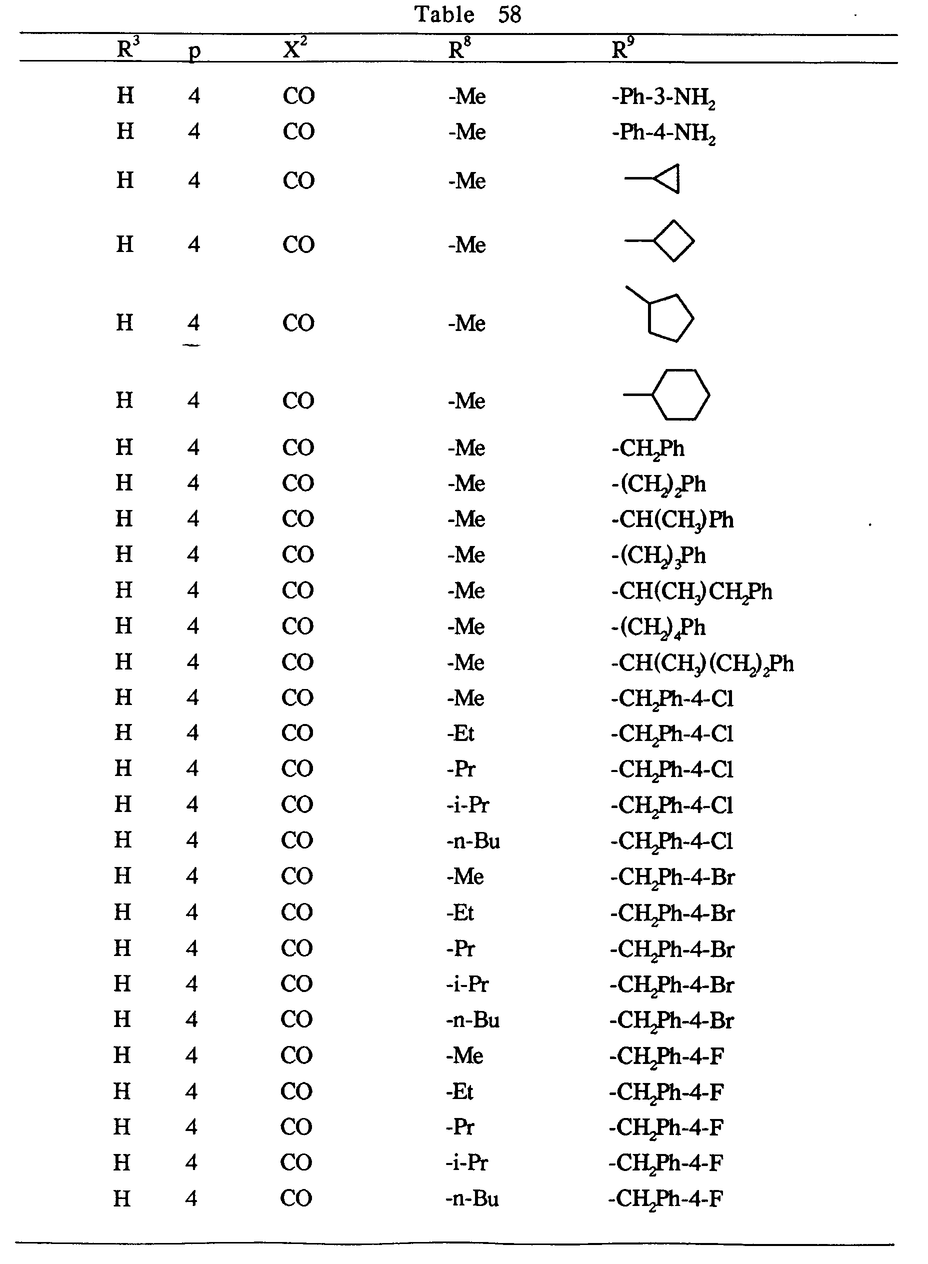

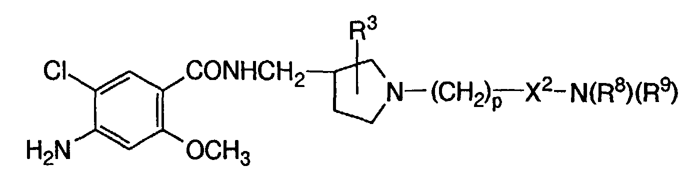

**Table 59**

| R³ | p | X² | R⁸ | R⁹ |
|---|---|---|---|---|
| H | 4 | CO | -Me | -CH₂Ph-4-Me |
| H | 4 | CO | -Et | -CH₂Ph-4-Me |
| H | 4 | CO | -Pr | -CH₂Ph-4-Me |
| H | 4 | CO | -i-Pr | -CH₂Ph-4-Me |
| H | 4 | CO | -n-Bu | -CH₂Ph-4-Me |
| H | 4 | CO | -Me | -CH₂Ph-4-OMe |
| H | 4 | CO | -Et | -CH₂Ph-4-OMe |
| H | 4 | CO | -Pr | -CH₂Ph-4-OMe |
| H | 4 | CO | -i-Pr | -CH₂Ph-4-OMe |
| H | 4 | CO | -n-Bu | -CH₂Ph-4-OMe |
| H | 4 | CO | -Me | -CH₂Ph-4-NO₂ |
| H | 4 | CO | -Et | -CH₂Ph-4-NO₂ |
| H | 4 | CO | -Pr | -CH₂Ph-4-NO₂ |
| H | 4 | CO | -i-Pr | -CH₂Ph-4-NO₂ |
| H | 4 | CO | -n-Bu | -CH₂Ph-4-NO₂ |
| H | 4 | CO | -Me | -CH₂Ph-4-NH₂ |
| H | 4 | CO | -Et | -CH₂Ph-4-NH₂ |
| H | 4 | CO | -Pr | -CH₂Ph-4-NH₂ |
| H | 4 | CO | -i-Pr | -CH₂Ph-4-NH₂ |
| H | 4 | CO | -n-Bu | -CH₂Ph-4-NH₂ |
| H | 4 | CO | -Me | -(CH₂)₂Ph-4-Cl |
| H | 4 | CO | -Et | -(CH₂)₂Ph-4-Cl |
| H | 4 | CO | -Pr | -(CH₂)₂Ph-4-Cl |
| H | 4 | CO | -i-Pr | -(CH₂)₂Ph-4-Cl |
| H | 4 | CO | -Bu | -(CH₂)₂Ph-4-Cl |
| H | 4 | CO | -Me | -(CH₂)₂Ph-4-Br |
| H | 4 | CO | -Et | -(CH₂)₂Ph-4-Br |
| H | 4 | CO | -Pr | -(CH₂)₂Ph-4-Br |
| H | 4 | CO | -i-Pr | -(CH₂)₂Ph-4-Br |
| H | 4 | CO | -Bu | -(CH₂)₂Ph-4-Br |
| H | 4 | CO | -Me | -(CH₂)₂Ph-4-F |
| H | 4 | CO | -Et | -(CH₂)₂Ph-4-F |

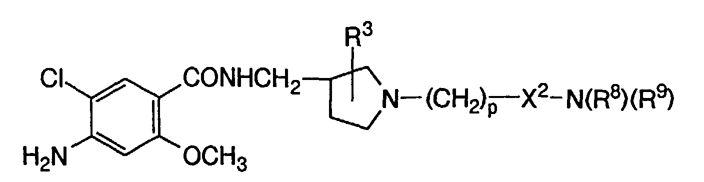

**Table 60**

| R³ | p | X² | R⁸ | R⁹ |
|---|---|---|---|---|
| H | 4 | CO | -Pr | -(CH₂)₂Ph-4-F |
| H | 4 | CO | -i-Pr | -(CH₂)₂Ph-4-F |
| H | 4 | CO | -Bu | -(CH₂)₂Ph-4-F |
| H | 4 | CO | -Me | -(CH₂)₂Ph-4-Me |
| H | 4 | CO | -Et | -(CH₂)₂Ph-4-Me |
| H | 4 | CO | -Pr | -(CH₂)₂Ph-4-Me |
| H | 4 | CO | -i-Pr | -(CH₂)₂Ph-4-Me |
| H | 4 | CO | -Bu | -(CH₂)₂Ph-4-Me |
| H | 4 | CO | -Me | -(CH₂)₂Ph-4-OMe |
| H | 4 | CO | -Et | -(CH₂)₂Ph-4-OMe |
| H | 4 | CO | -Pr | -(CH₂)₂Ph-4-OMe |
| H | 4 | CO | -i-Pr | -(CH₂)₂Ph-4-OMe |
| H | 4 | CO | -Bu | -(CH₂)₂Ph-4-OMe |
| H | 4 | CO | -Me | -(CH₂)₂Ph-4-NO₂ |
| H | 4 | CO | -Et | -(CH₂)₂Ph-4-NO₂ |
| H | 4 | CO | -Pr | -(CH₂)₂Ph-4-NO₂ |
| H | 4 | CO | -i-Pr | -(CH₂)₂Ph-4-NO₂ |
| H | 4 | CO | -Bu | -(CH₂)₂Ph-4-NO₂ |
| H | 4 | CO | -(CH₂)₄- | |
| H | 4 | CO | -(CH₂)₅- | |
| H | 4 | CO | -(CH₂)₂O(CH₂)₂- | |
| H | 4 | CO | -(CH₂)₂S(CH₂)₂- | |
| H | 4 | CO | -(CH₂)₂N(CH₃)(CH₂)₂- | |

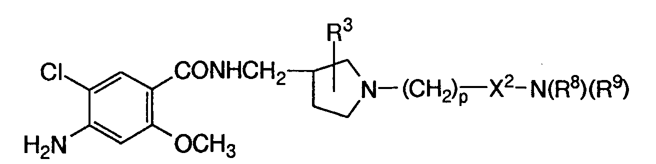

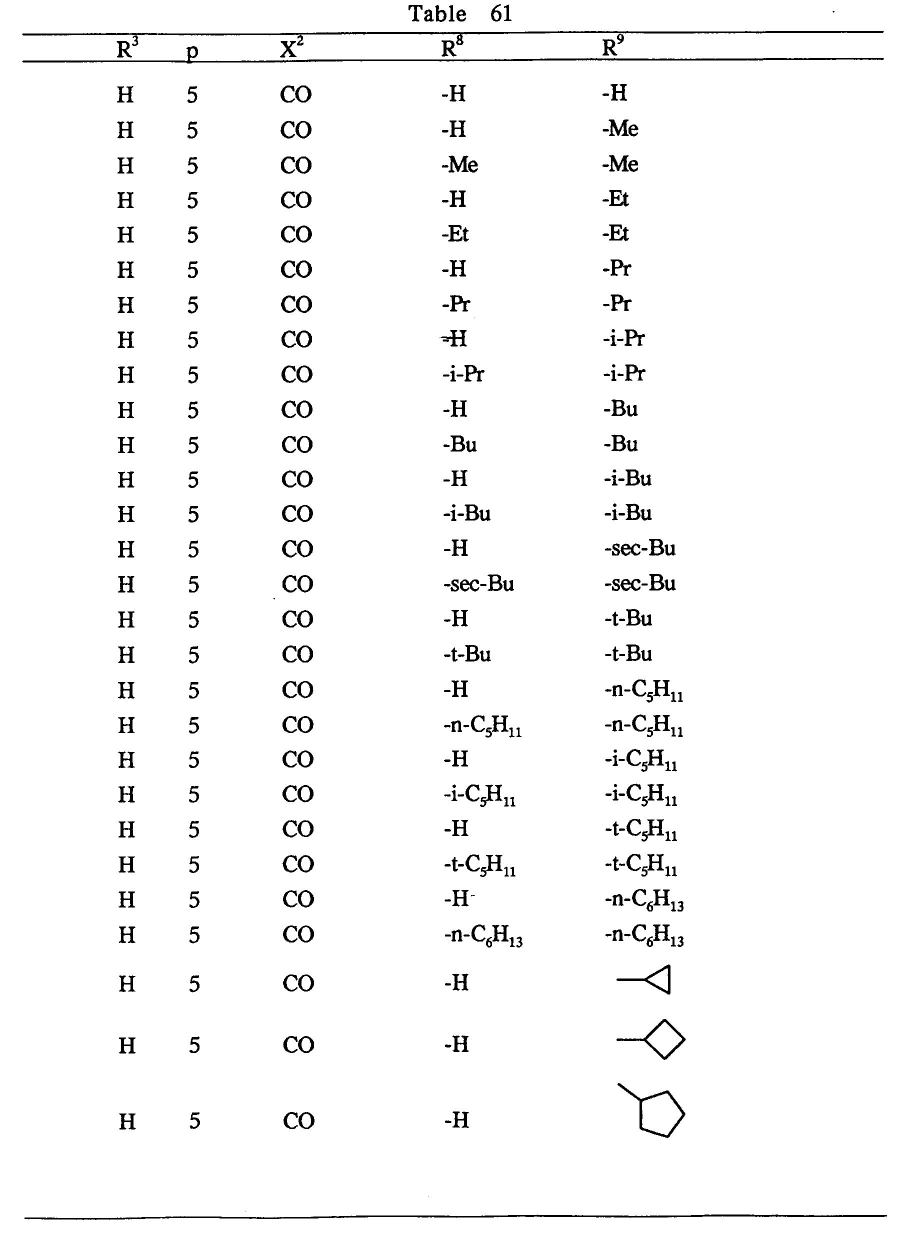

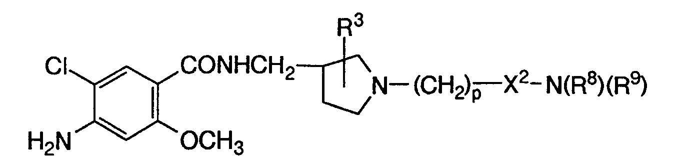

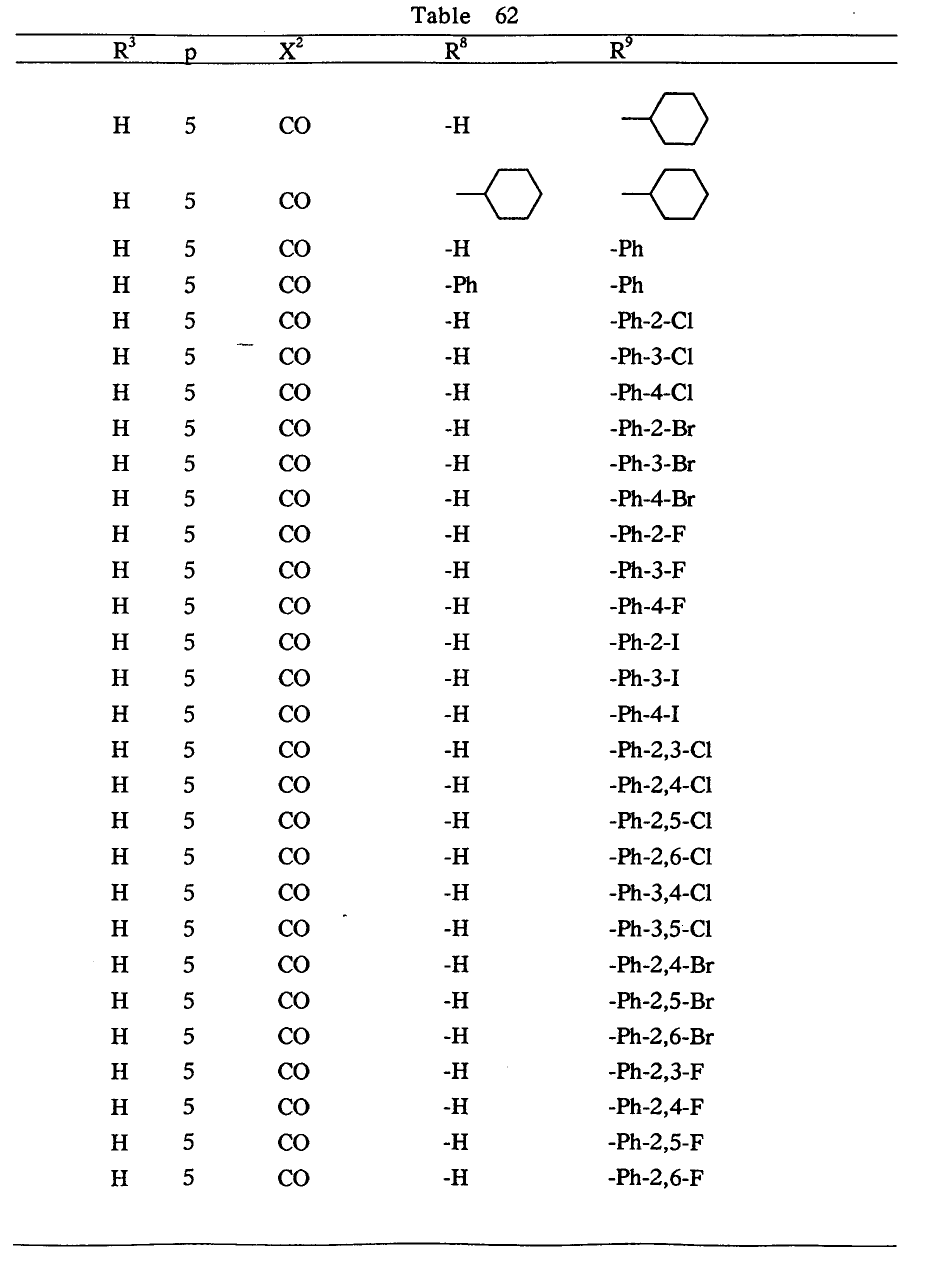

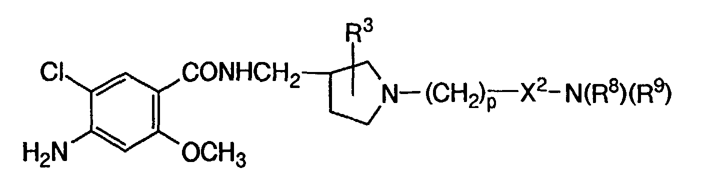

**Table 63**

| R³ | p | X² | R⁸ | R⁹ |
|---|---|---|---|---|
| H | 5 | CO | -H | -Ph-3,4-F |
| H | 5 | CO | -H | -Ph-3,5-F |
| H | 5 | CO | -H | -Ph-2-Cl-4-F |
| H | 5 | CO | -H | -Ph-3-Cl-4-F |
| H | 5 | CO | -H | -Ph-4-Cl-2-F |
| H | 5 | CO | -H | -Ph-2-Br-4-F |
| H | 5 | CO | -H | -Ph-4-Br-2-F |
| H | 5 | CO | -H | -Ph-2-Cl-4-Br |
| H | 5 | CO | -H | -Ph-2-F-4-I |
| H | 5 | CO | -H | -Ph-2-Me |
| H | 5 | CO | -H | -Ph-3-Me |
| H | 5 | CO | -H | -Ph-4-Me |
| H | 5 | CO | -H | -Ph-2-Et |
| H | 5 | CO | -H | -Ph-3-Et |
| H | 5 | CO | -H | -Ph-4-Et |
| H | 5 | CO | -H | -Ph-2-Pr |
| H | 5 | CO | -H | -Ph-4-Pr |
| H | 5 | CO | -H | -Ph-2-i-Pr |
| H | 5 | CO | -H | -Ph-4-i-Pr |
| H | 5 | CO | -H | -Ph-4-Bu |
| H | 5 | CO | -H | -Ph-2-t-Bu |
| H | 5 | CO | -H | -Ph-4-t-Bu |
| H | 5 | CO | -H | -Ph-4-sec-Bu |
| H | 5 | CO | -H | -Ph-2,3-Me |
| H | 5 | CO | -H | -Ph-2,4-Me |
| H | 5 | CO | -H | -Ph-2,5-Me |
| H | 5 | CO | -H | -Ph-2,6-Me |
| H | 5 | CO | -H | -Ph-3,4-Me |
| H | 5 | CO | -H | -Ph-3,5-Me |
| H | 5 | CO | -H | -Ph-2,4,6-Me |
| H | 5 | CO | -H | -Ph-2,6-Et |
| H | 5 | CO | -H | -Ph-2-Me-6-Et |

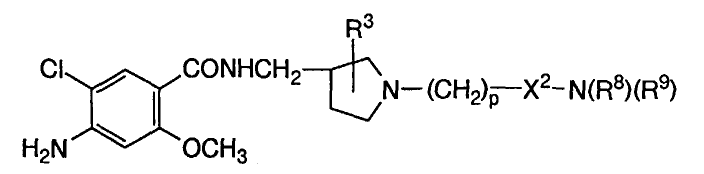

**Table 64**

| R³ | p | X² | R⁸ | R⁹ |
|---|---|---|---|---|
| H | 5 | CO | -H | -Ph-2-Me-6-i-Pr |
| H | 5 | CO | -H | -Ph-2-OMe |
| H | 5 | CO | -H | -Ph-3-OMe |
| H | 5 | CO | -H | -Ph-4-OMe |
| H | 5 | CO | -H | -Ph-2-OEt |
| H | 5 | CO | -H | -Ph-3-OEt |
| H | 5 | CO | -H | -Ph-4-OEt |
| H | 5 | CO | -H | -Ph-2-OPr |
| H | 5 | CO | -H | -Ph-3-OPr |
| H | 5 | CO | -H | -Ph-4-OPr |
| H | 5 | CO | -H | -Ph-2-O-i-Pr |
| H | 5 | CO | -H | -Ph-3-O-i-Pr |
| H | 5 | CO | -H | -Ph-4-O-i-Pr |
| H | 5 | CO | -H | -Ph-4-O-n-Bu |
| H | 5 | CO | -H | -Ph-2-CH₂Ph |
| H | 5 | CO | -H | -Ph-2-OH |
| H | 5 | CO | -H | -Ph-3-OH |
| H | 5 | CO | -H | -Ph-4-OH |
| H | 5 | CO | -H | -Ph-2-NO₂ |
| H | 5 | CO | -H | -Ph-3-NO₂ |
| H | 5 | CO | -H | -Ph-4-NO₂ |
| H | 5 | CO | -H | -Ph-2-NH₂ |
| H | 5 | CO | -H | -Ph-3-NH₂ |
| H | 5 | CO | -H | -Ph-4-NH₂ |
| H | 5 | CO | -H | -Ph-2-OH-6-Me |
| H | 5 | CO | -H | -Ph-2-OH-5-Me |
| H | 5 | CO | -H | -Ph-3-OH-2-Me |
| H | 5 | CO | -H | -Ph-4-OH-2-Me |
| H | 5 | CO | -H | -Ph-2-OH-4-Me |
| H | 5 | CO | -H | -Ph-2-OMe-5-Me |
| H | 5 | CO | -H | -Ph-2-OMe-6-Me |
| H | 5 | CO | -H | -Ph-4-OMe-2-Me |

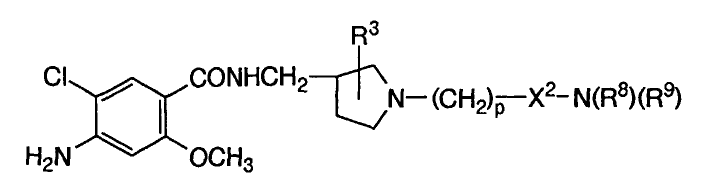

**Table 65**

| R³ | p | X² | R⁸ | R⁹ |
|---|---|---|---|---|
| H | 5 | CO | -H | -Ph-5-OMe-2-Me |
| H | 5 | CO | -H | -Ph-5-t-Bu-2-OH |
| H | 5 | CO | -H | -Ph-3-Cl-4-Me |
| H | 5 | CO | -H | -Ph-2-Cl-4-Me |
| H | 5 | CO | -H | -Ph-2-Cl-5-Me |
| H | 5 | CO | -H | -Ph-2-Cl-6-Me |
| H | 5 | CO | -H | -Ph-3-Cl-2-Me |
| H | 5 | CO | -H | -Ph-4-Cl-2-Me |
| H | 5 | CO | -H | -Ph-5-Cl-2-Me |
| H | 5 | CO | -H | -Ph-2-Br-4-Me |
| H | 5 | CO | -H | -Ph-3-Br-4-Me |
| H | 5 | CO | -H | -Ph-4-Br-2-Me |
| H | 5 | CO | -H | -Ph-4-Br-3-Me |
| H | 5 | CO | -H | -Ph-2-F-4-Me |
| H | 5 | CO | -H | -Ph-2-F-5-Me |
| H | 5 | CO | -H | -Ph-3-F-2-Me |
| H | 5 | CO | -H | -Ph-3-F-4-Me |
| H | 5 | CO | -H | -Ph-4-F-2-Me |
| H | 5 | CO | -H | -Ph-5-F-2-Me |
| H | 5 | CO | -H | -Ph-3-Cl-4-OMe |
| H | 5 | CO | -H | -Ph-5-Cl-2-OMe |
| H | 5 | CO | -H | -Ph-2-Cl-5-OMe |
| H | 5 | CO | -H | -Ph-3-F-2-OMe |
| H | 5 | CO | -H | -Ph-3-F-4-OMe |
| H | 5 | CO | -H | -Ph-5-Cl-2-OH |
| H | 5 | CO | -H | -Ph-2-Cl-4-OH |
| H | 5 | CO | -H | -Ph-2-Cl-4-NO₂ |
| H | 5 | CO | -H | -Ph-2-Cl-5-NO₂ |
| H | 5 | CO | -H | -Ph-4-Cl-2-NO₂ |
| H | 5 | CO | -H | -Ph-4-Cl-3-NO₂ |
| H | 5 | CO | -H | -Ph-5-Cl-2-NO₂ |
| H | 5 | CO | -H | -Ph-2-F-5-NO₂ |

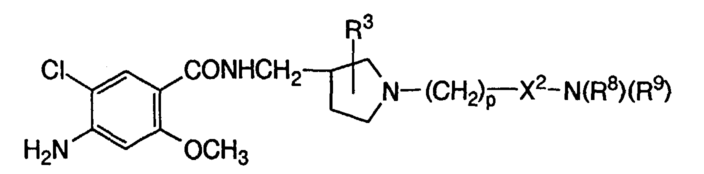

**Table 66**

| R³ | p | X² | R⁸ | R⁹ |
|---|---|---|---|---|
| H | 5 | CO | -H | -Ph-4-F-2-NO₂ |
| H | 5 | CO | -H | -Ph-4-F-3-NO₂ |
| H | 5 | CO | -H | -Ph-4-OH-2,5-Me |
| H | 5 | CO | -H | -Ph-2-OH-3,5-Me |
| H | 5 | CO | -H | -Ph-4-OH-2,5-Me |
| H | 5 | CO | -H | -Ph-2-Cl-4,6-Me |
| H | 5 | CO | -H | -Ph-4-Br-2,6-Me |
| H | 5 | CO | -H | -CH₂Ph |
| H | 5 | CO | -CH₂Ph | -CH₂Ph |
| H | 5 | CO | -H | -CHPh₂ |
| H | 5 | CO | -H | -(CH₂)₂Ph |
| H | 5 | CO | -H | -CH(CH₃)Ph |
| H | 5 | CO | -H | -(CH₂)₃Ph |
| H | 5 | CO | -H | -CH₂CH(CH₃)Ph |
| H | 5 | CO | -H | -(CH₂)₄Ph |
| H | 5 | CO | -H | -CH(CH₃)-(CH₂)₂Ph |
| H | 5 | CO | -H | -CH₂Ph-2-Cl |
| H | 5 | CO | -H | -CH₂Ph-3-Cl |
| H | 5 | CO | -H | -CH₂Ph-4-Cl |
| H | 5 | CO | -H | -CH₂Ph-2-Br |
| H | 5 | CO | -H | -CH₂Ph-3-Br |
| H | 5 | CO | -H | -CH₂Ph-4-Br |
| H | 5 | CO | -H | -CH₂Ph-2-F |
| H | 5 | CO | -H | -CH₂Ph-3-F |
| H | 5 | CO | -H | -CH₂Ph-4-F |
| H | 5 | CO | -H | -CH₂Ph-3-I |
| H | 5 | CO | -H | -CH₂Ph-2-Me |
| H | 5 | CO | -H | -CH₂Ph-3-Me |
| H | 5 | CO | -H | -CH₂Ph-4-Me |
| H | 5 | CO | -H | -CH₂Ph-2-OMe |
| H | 5 | CO | -H | -CH₂Ph-4-OMe |
| H | 5 | CO | -H | -CH₂Ph-2-OEt |

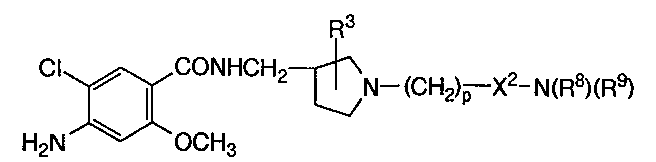

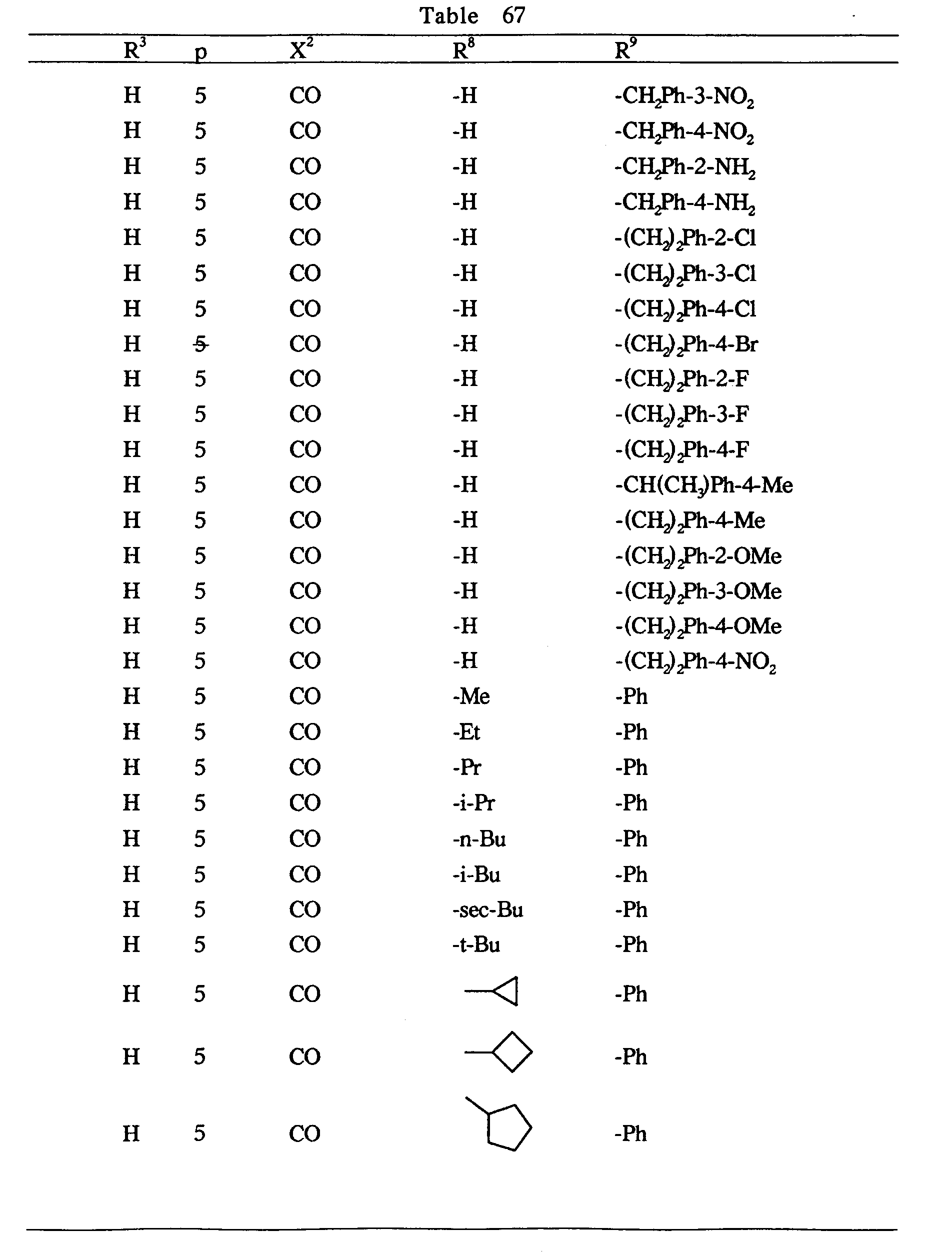

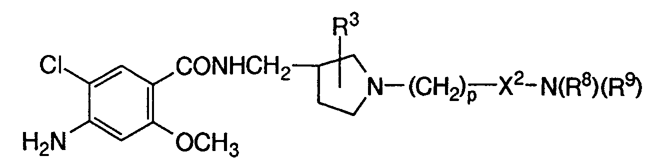

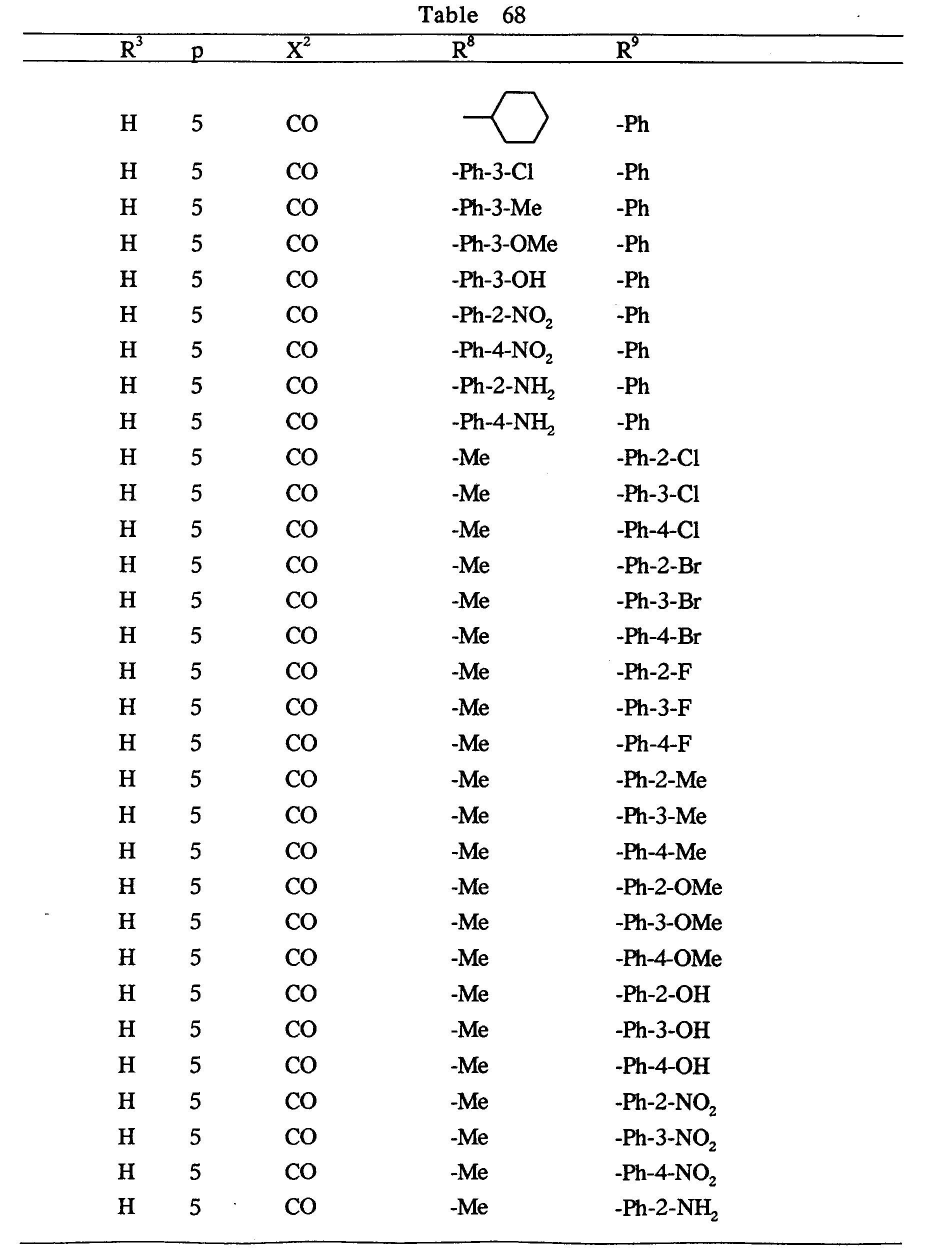

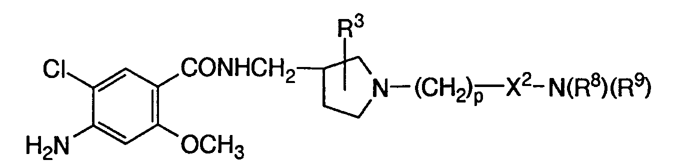

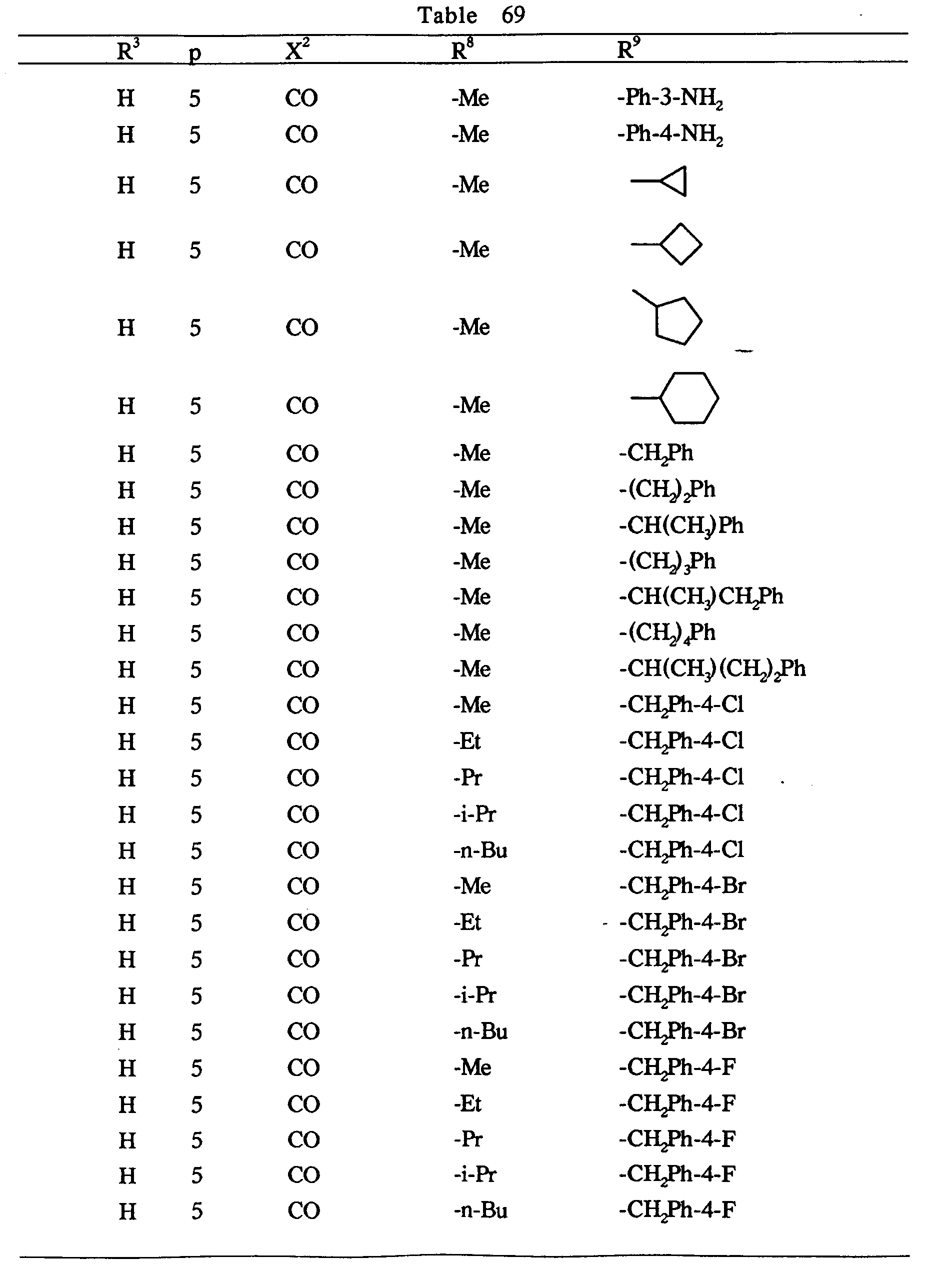

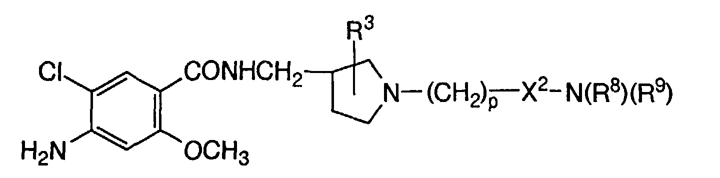

**Table 70**

| R³ | p | X² | R⁸ | R⁹ |
|---|---|---|---|---|
| H | 5 | CO | -Me | -CH₂Ph-4-Me |
| H | 5 | CO | -Et | -CH₂Ph-4-Me |
| H | 5 | CO | -Pr | -CH₂Ph-4-Me |
| H | 5 | CO | -i-Pr | -CH₂Ph-4-Me |
| H | 5 | CO | -n-Bu | -CH₂Ph-4-Me |
| H | 5 | CO | -Me | -CH₂Ph-4-OMe |
| H | 5 | CO | -Et | -CH₂Ph-4-OMe |
| H | 5 | CO | -Pr | -CH₂Ph-4-OMe |
| H | 5 | CO | -i-Pr | -CH₂Ph-4-OMe |
| H | 5 | CO | -n-Bu | -CH₂Ph-4-OMe |
| H | 5 | CO | -Me | -CH₂Ph-4-NO₂ |
| H | 5 | CO | -Et | -CH₂Ph-4-NO₂ |
| H | 5 | CO | -Pr | -CH₂Ph-4-NO₂ |
| H | 5 | CO | -i-Pr | -CH₂Ph-4-NO₂ |
| H | 5 | CO | -n-Bu | -CH₂Ph-4-NO₂ |
| H | 5 | CO | -Me | -CH₂Ph-4-NH₂ |
| H | 5 | CO | -Et | -CH₂Ph-4-NH₂ |
| H | 5 | CO | -Pr | -CH₂Ph-4-NH₂ |
| H | 5 | CO | -i-Pr | -CH₂Ph-4-NH₂ |
| H | 5 | CO | -n-Bu | -CH₂Ph-4-NH₂ |
| H | 5 | CO | -Me | -(CH₂)₂Ph-4-Cl |
| H | 5 | CO | -Et | -(CH₂)₂Ph-4-Cl |
| H | 5 | CO | -Pr | -(CH₂)₂Ph-4-Cl |
| H | 5 | CO | -i-Pr | -(CH₂)₂Ph-4-Cl |
| H | 5 | CO | -Bu | -(CH₂)₂Ph-4-Cl |
| H | 5 | CO | -Me | -(CH₂)₂Ph-4-Br |
| H | 5 | CO | -Et | -(CH₂)₂Ph-4-Br |
| H | 5 | CO | -Pr | -(CH₂)₂Ph-4-Br |
| H | 5 | CO | -i-Pr | -(CH₂)₂Ph-4-Br |
| H | 5 | CO | -Bu | -(CH₂)₂Ph-4-Br |
| H | 5 | CO | -Me | -(CH₂)₂Ph-4-F |
| H | 5 | CO | -Et | -(CH₂)₂Ph-4-F |

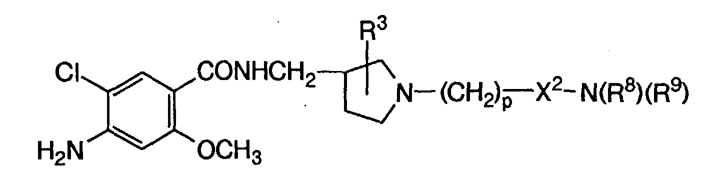

**Table 71**

| R³ | p | X² | R⁸ | R⁹ |
|---|---|---|---|---|
| H | 5 | CO | -Pr | -(CH₂)₂Ph-4-F |
| H | 5 | CO | -i-Pr | -(CH₂)₂Ph-4-F |
| H | 5 | CO | -Bu | -(CH₂)₂Ph-4-F |
| H | 5 | CO | -Me | -(CH₂)₂Ph-4-Me |
| H | 5 | CO | -Et | -(CH₂)₂Ph-4-Me |
| H | 5 | CO | -Pr | -(CH₂)₂Ph-4-Me |
| H | 5 | CO | -i-Pr | -(CH₂)₂-Ph-4-Me |
| H | 5 | CO | -Bu | -(CH₂)₂Ph-4-Me |
| H | 5 | CO | -Me | -(CH₂)₂Ph-4-OMe |
| H | 5 | CO | -Et | -(CH₂)₂Ph-4-OMe |
| H | 5 | CO | -Pr | -(CH₂)₂Ph-4-OMe |
| H | 5 | CO | -i-Pr | -(CH₂)₂Ph-4-OMe |
| H | 5 | CO | -Bu | -(CH₂)₂Ph-4-OMe |
| H | 5 | CO | -Me | -(CH₂)₂Ph-4-NO₂ |
| H | 5 | CO | -Et | -(CH₂)₂Ph-4-NO₂ |
| H | 5 | CO | -Pr | -(CH₂)₂Ph-4-NO₂ |
| H | 5 | CO | -i-Pr | -(CH₂)₂Ph-4-NO₂ |
| H | 5 | CO | -Bu | -(CH₂)₂Ph-4-NO₂ |
| H | 5 | CO | -(CH₂)₄- | |
| H | 5 | CO | -(CH₂)₅- | |
| H | 5 | CO | -(CH₂)₂O(CH₂)₂- | |
| H | 5 | CO | -(CH₂)₂S(CH₂)₂- | |
| H | 5 | CO | -(CH₂)₂N(CH₃)(CH₂)₂- | |

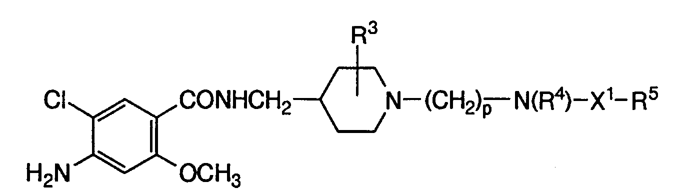

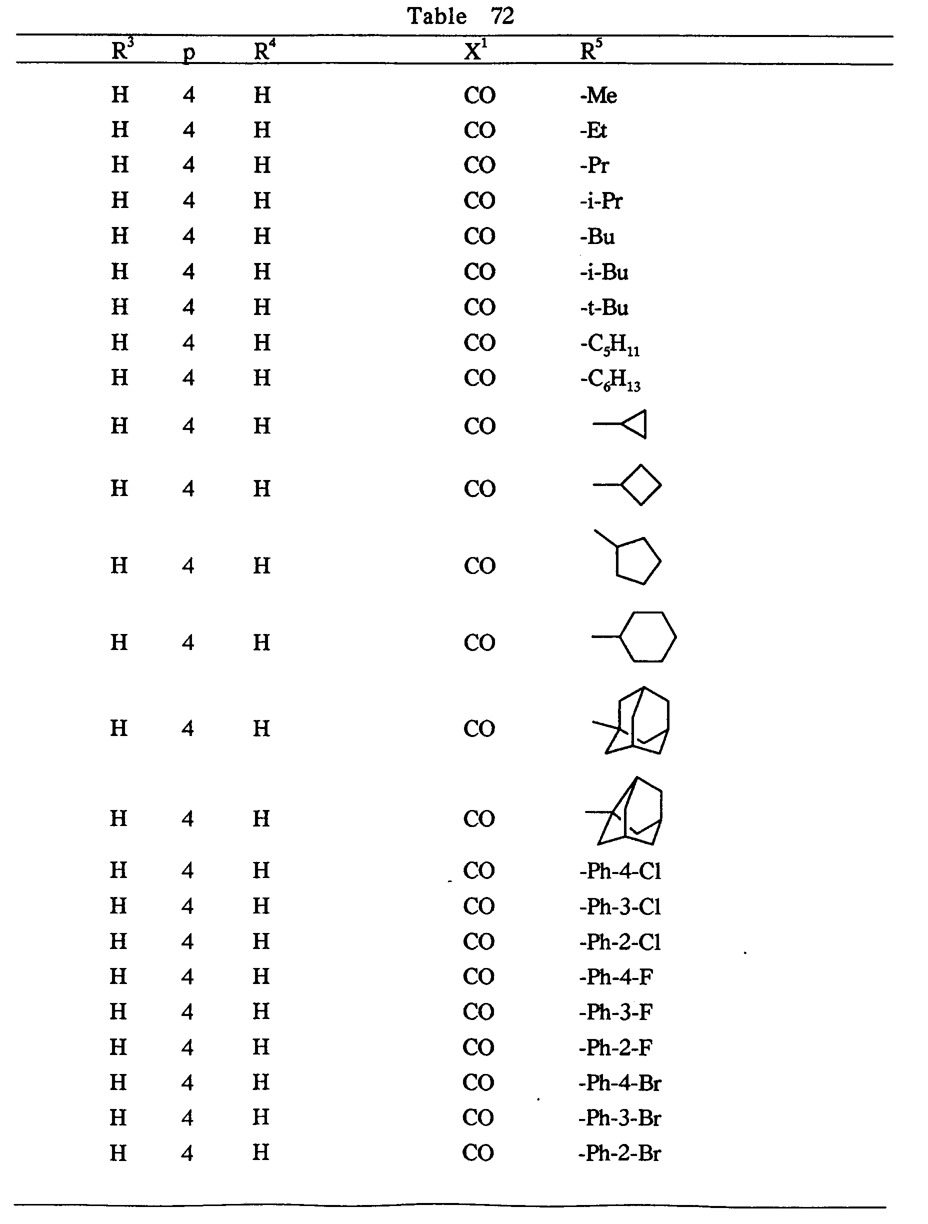

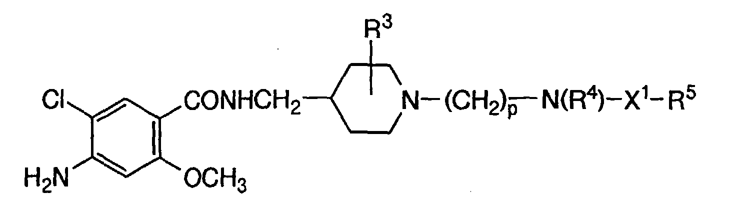

**Table 73**

| R³ | p | R⁴ | X¹ | R⁵ |
|---|---|---|---|---|
| H | 4 | H | CO | -Ph-4-I |
| H | 4 | H | CO | -Ph-3-I |
| H | 4 | H | CO | -Ph-2-I |
| H | 4 | H | CO | -Ph-2,3-Cl |
| H | 4 | H | CO | -Ph-2,4-Cl |
| H | 4 | H | CO | -Ph-2,5-Cl |
| H | 4 | H | CO | -Ph-2,6-Cl |
| H | 4 | H | CO | -Ph-3,4-Cl |
| H | 4 | H | CO | -Ph-3,5-Cl |
| H | 4 | H | CO | -Ph-2,3,5-Cl |
| H | 4 | H | CO | -Ph-2,4,6-Cl |
| H | 4 | H | CO | -Ph-2,3-F |
| H | 4 | H | CO | -Ph-2,4-F |
| H | 4 | H | CO | -Ph-2,5-F |
| H | 4 | H | CO | -Ph-2,6-F |
| H | 4 | H | CO | -Ph-3,4-F |
| H | 4 | H | CO | -Ph-3,5-F |
| H | 4 | H | CO | -Ph-2,3,4-F |
| H | 4 | H | CO | -Ph-2,3,6-F |
| H | 4 | H | CO | -Ph-2,4,5-F |
| H | 4 | H | CO | -Ph-2,4,6-F |
| H | 4 | H | CO | -Ph-3,4,5-F |
| H | 4 | H | CO | -Ph-3,5-Br |
| H | 4 | H | CO | -Ph-4-Me |
| H | 4 | H | CO | -Ph-3-Me |
| H | 4 | H | CO | -Ph-2-Me |
| H | 4 | H | CO | -Ph-4-Et |
| H | 4 | H | CO | -Ph-4-Pr |
| H | 4 | H | CO | -Ph-4-i-Pr |
| H | 4 | H | CO | -Ph-4-Bu |
| H | 4 | H | CO | -Ph-4-t-Bu |

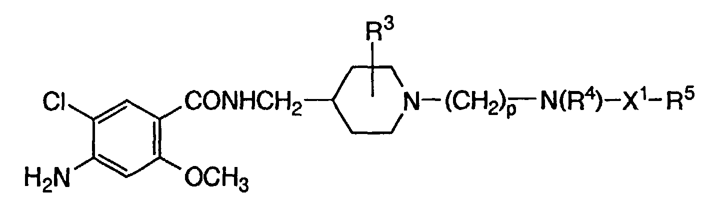

**Table 74**

| R³ | p | R⁴ | X¹ | R⁵ |
|---|---|---|---|---|
| H | 4 | H | CO | -Ph-2,3-Me |
| H | 4 | H | CO | -Ph-2,4-Me |
| H | 4 | H | CO | -Ph-2,5-Me |
| H | 4 | H | CO | -Ph-2,6-Me |
| H | 4 | H | CO | -Ph-3,4-Me |
| H | 4 | H | CO | -Ph-3,5-Me |
| H | 4 | H | CO | -Ph-3,4,5-Me |
| H | 4 | H | CO | -Ph-4-OMe |
| H | 4 | H | CO | -Ph-3-OMe |
| H | 4 | H | CO | -Ph-2-OMe |
| H | 4 | H | CO | -Ph-4-OEt |
| H | 4 | H | CO | -Ph-2-OEt |
| H | 4 | H | CO | -Ph-4-OPr |
| H | 4 | H | CO | -Ph-4-O-i-Pr |
| H | 4 | H | CO | -Ph-4-OBu |
| H | 4 | H | CO | -Ph-2,3-OMe |
| H | 4 | H | CO | -Ph-2,4-OMe |
| H | 4 | H | CO | -Ph-2,5-OMe |
| H | 4 | H | CO | -Ph-2,6-OMe |
| H | 4 | H | CO | -Ph-3,4-OMe |
| H | 4 | H | CO | -Ph-3,5-OMe |
| H | 4 | H | CO | -Ph-2,3,4-OMe |
| H | 4 | H | CO | -Ph-2,4,5-OMe |
| H | 4 | H | CO | -Ph-3,4,5-OMe |
| H | 4 | H | CO | -Ph-4-CH₂Ph |
| H | 4 | H | CO | -Ph-3-CH₂Ph |
| H | 4 | H | CO | -Ph-2-CH₂Ph |
| H | 4 | H | CO | -Ph-4-OH |
| H | 4 | H | CO | -Ph-3-OH |
| H | 4 | H | CO | -Ph-2,3-OH |
| H | 4 | H | CO | -Ph-2,4-OH |

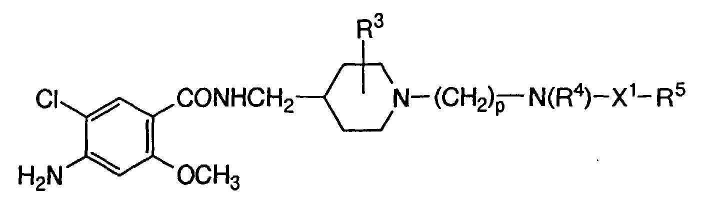

**Table 75**

| R³ | p | R⁴ | X¹ | R⁵ |
|---|---|---|---|---|
| H | 4 | H | CO | -Ph-2,5-OH |
| H | 4 | H | CO | -Ph-2,6-OH |
| H | 4 | H | CO | -Ph-3,4-OH |
| H | 4 | H | CO | -Ph-3,5-OH |
| H | 4 | H | CO | -Ph-2,3,4-OH |
| H | 4 | H | CO | -Ph-2,4,6-OH |
| H | 4 | H | CO | -Ph-4-NO₂ |
| H | 4 | H | CO | -Ph-3-NO₂ |
| H | 4 | H | CO | -Ph-2-NO₂ |
| H | 4 | H | CO | -Ph-2,4-NO₂ |
| H | 4 | H | CO | -Ph-3,5-NO₂ |
| H | 4 | H | CO | -Ph-4-NH₂ |
| H | 4 | H | CO | -Ph-3-NH₂ |
| H | 4 | H | CO | -Ph-3,4-NH₂ |
| H | 4 | H | CO | -Ph-3,5-NH₂ |
| H | 4 | H | CO | -Ph-2-F-5-Me |
| H | 4 | H | CO | -Ph-3-F-2-Me |
| H | 4 | H | CO | -Ph-3-F-4-Me |
| H | 4 | H | CO | -Ph-5-F-2-Me |
| H | 4 | H | CO | -Ph-3-Br-4-Me |
| H | 4 | H | CO | -Ph-4-Cl-2-OMe |
| H | 4 | H | CO | -Ph-5-Cl-2-OMe |
| H | 4 | H | CO | -Ph-3-F-4-OMe |
| H | 4 | H | CO | -Ph-2-Br-5-OMe |
| H | 4 | H | CO | -Ph-2-NH₂-3-OMe |
| H | 4 | H | CO | -Ph-3-NH₂-4-OMe |
| H | 4 | H | CO | -Ph-4-NH₂-3-OMe |
| H | 4 | H | CO | -Ph-3-NH₂-4-OH |
| H | 4 | H | CO | -Ph-4-NH₂-3-OH |
| H | 4 | H | CO | -Ph-3-NH₂-2-OH |
| H | 4 | H | CO | -Ph-4-NH₂-2-OH |

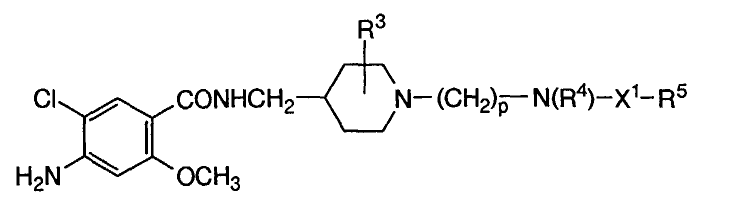

**Table 76**

| R³ | p | R⁴ | X¹ | R⁵ |
|---|---|---|---|---|
| H | 4 | H | CO | -Ph-5-NH₂-2-OH |
| H | 4 | H | CO | -Ph-3-OH-4-Me |
| H | 4 | H | CO | -Ph-2-OH-3-Me |
| H | 4 | H | CO | -Ph-2-OH-4-Me |
| H | 4 | H | CO | -Ph-2-OH-5-Me |
| H | 4 | H | CO | -Ph-2-OH-3-i-Pr |
| H | 4 | H | CO | -Ph-3-OMe-4-Me |
| H | 4 | H | CO | -Ph-2-NH₂-3-Cl |
| H | 4 | H | CO | -Ph-2-NH₂-4-Cl |
| H | 4 | H | CO | -Ph-2-NH₂-5-Cl |
| H | 4 | H | CO | -Ph-3-NH₂-4-Cl |
| H | 4 | H | CO | -Ph-4-NH₂-2-Cl |
| H | 4 | H | CO | -Ph-5-NH₂-2-Cl |
| H | 4 | H | CO | -Ph-2-NH₂-4-F |
| H | 4 | H | CO | -Ph-2-NH₂-5-F |
| H | 4 | H | CO | -Ph-2-NH₂-5-Br |
| H | 4 | H | CO | -Ph-3-Cl-4-OH |
| H | 4 | H | CO | -Ph-4-Cl-2-OH |
| H | 4 | H | CO | -Ph-5-Cl-2-OH |
| H | 4 | H | CO | -Ph-5-F-2-OH |
| H | 4 | H | CO | -Ph-5-Br-2-OH |
| H | 4 | H | CO | -Ph-2-NH₂-3-Me |
| H | 4 | H | CO | -Ph-2-NH₂-5-Me |
| H | 4 | H | CO | -Ph-2-NH₂-6-Me |
| H | 4 | H | CO | -Ph-3-NH₂-2-Me |
| H | 4 | H | CO | -Ph-3-NH₂-4-Me |
| H | 4 | H | CO | -Ph-4-NH₂-3-Me |
| H | 4 | H | CO | -Ph-2-NH₂-3-OMe |
| H | 4 | H | CO | -Ph-3-NH₂-4-OMe |
| H | 4 | H | CO | -Ph-4-NH₂-3-OMe |
| H | 4 | H | CO | -Ph-4-NH₂-5-Cl-2-OMe |

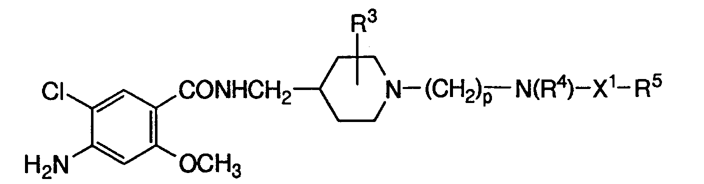

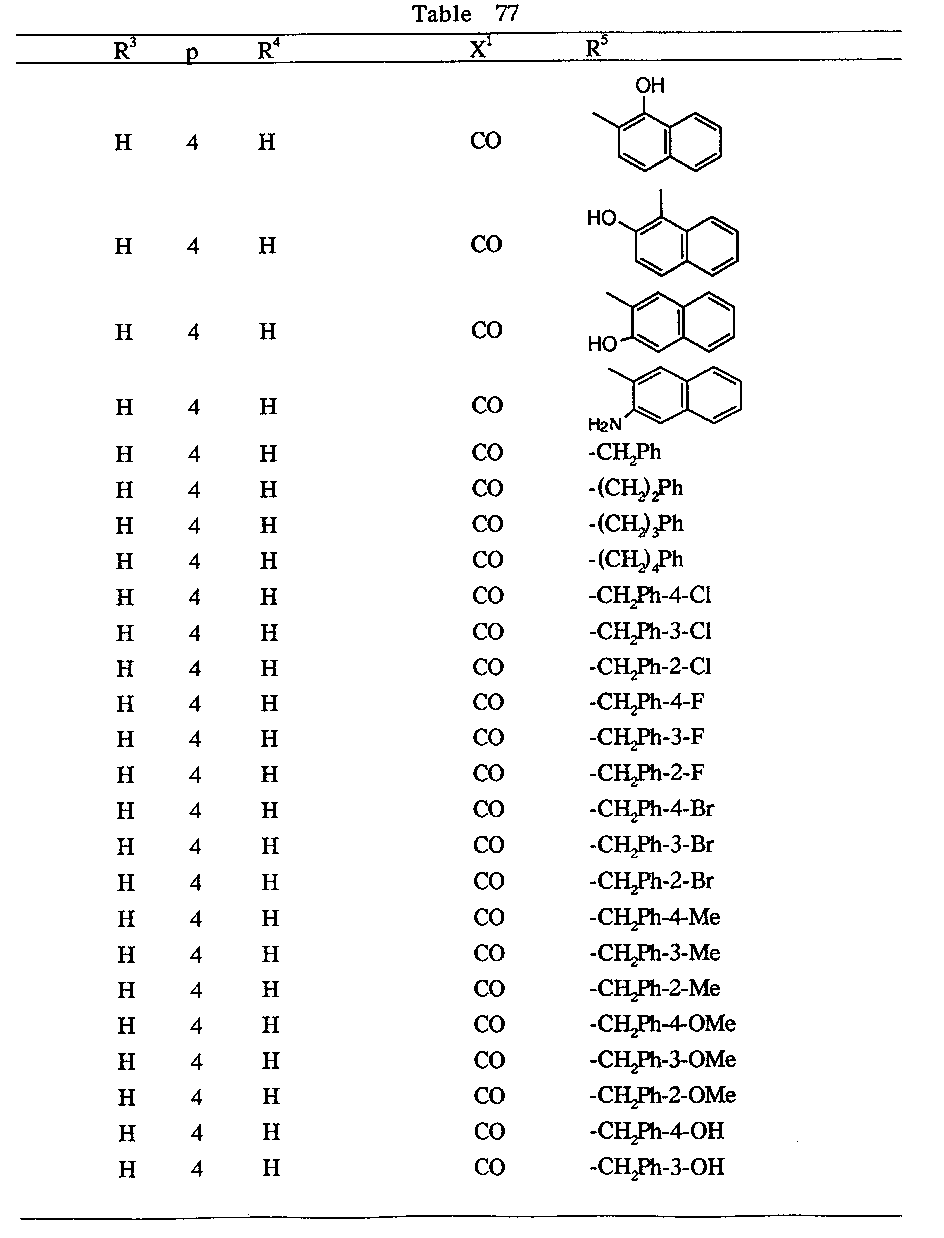

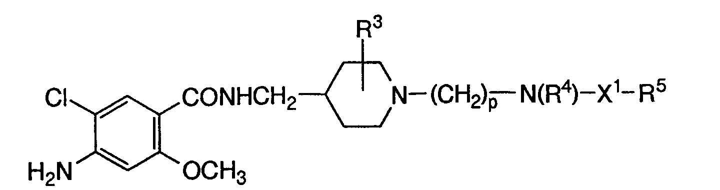

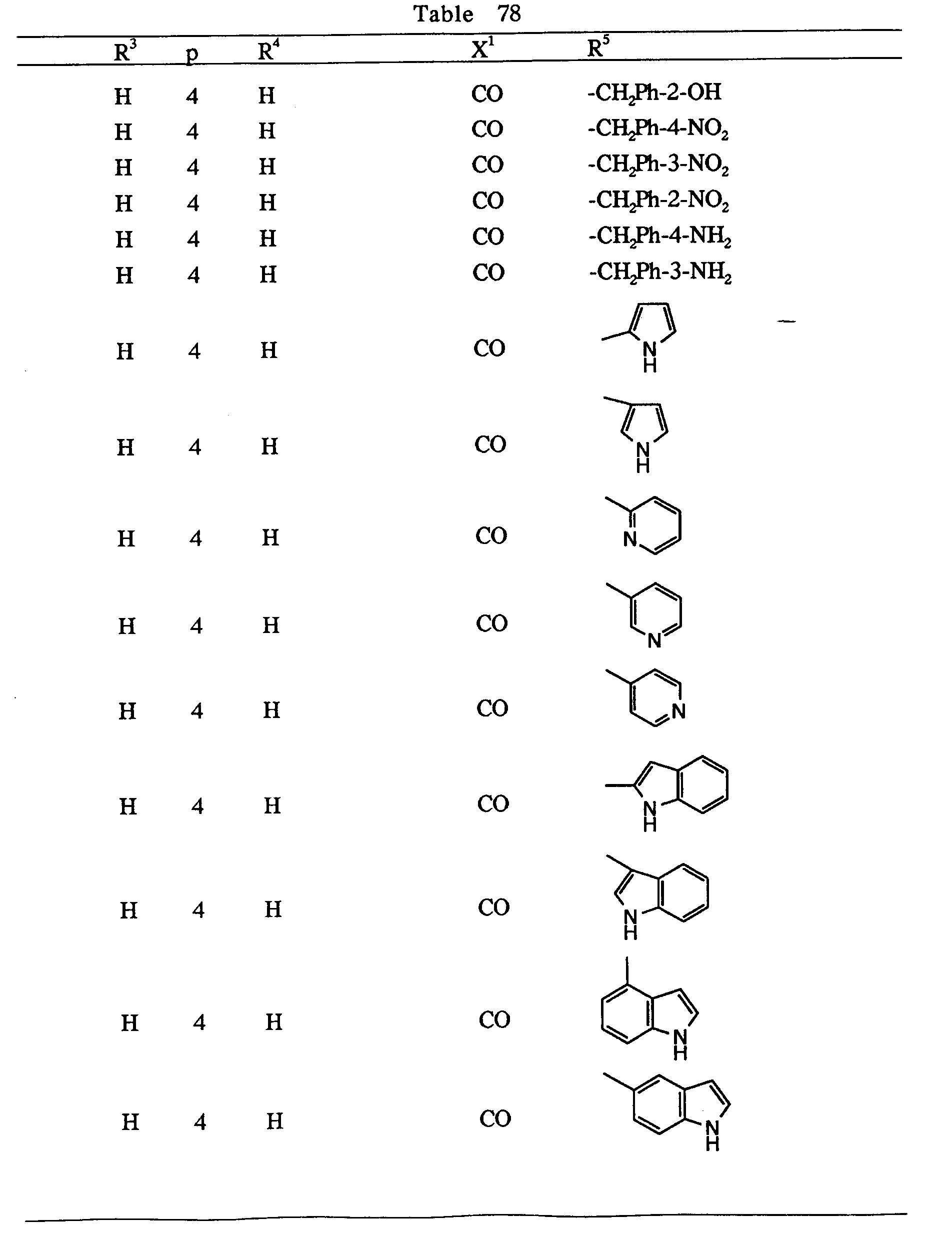

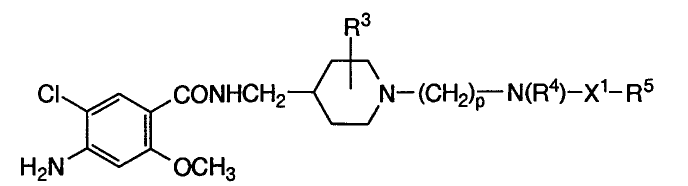

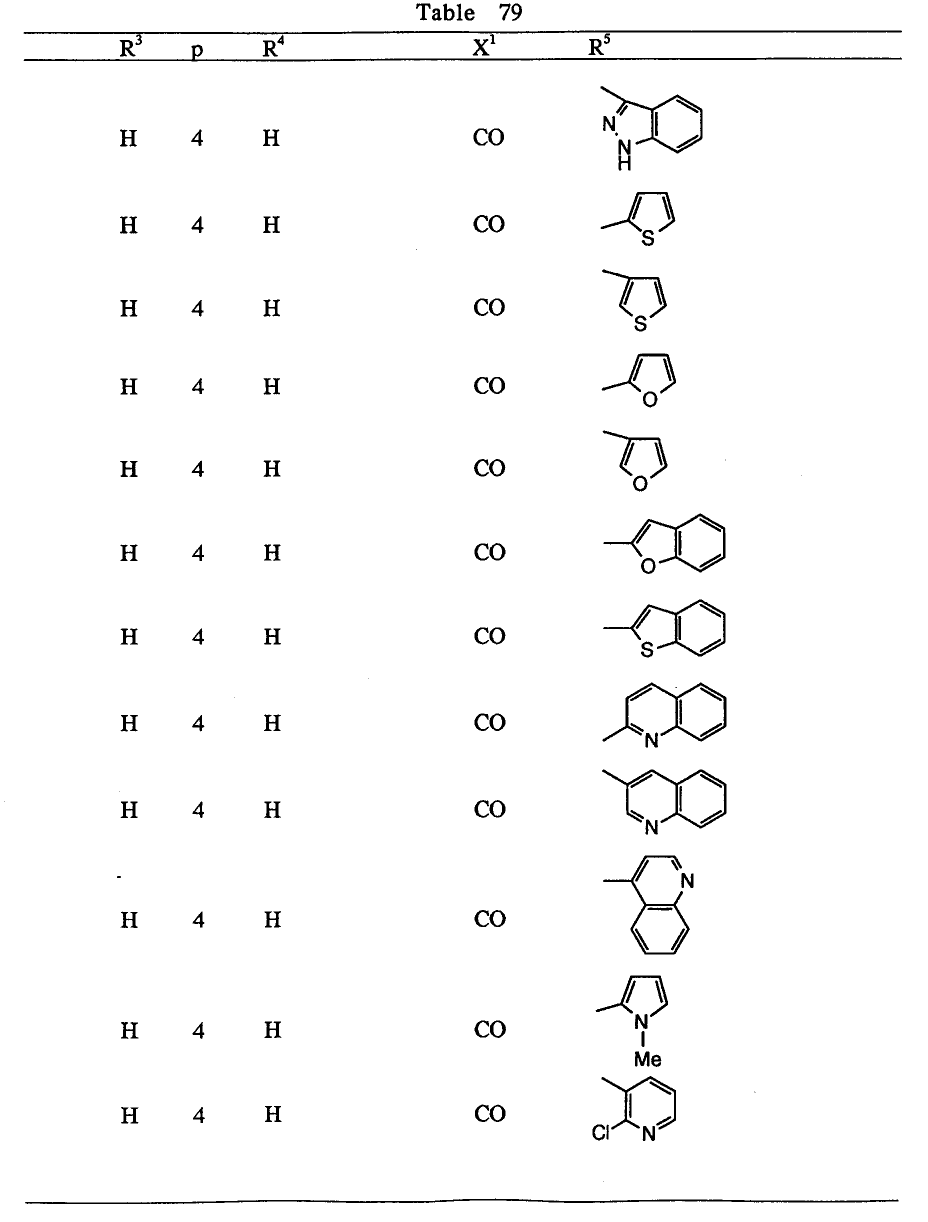

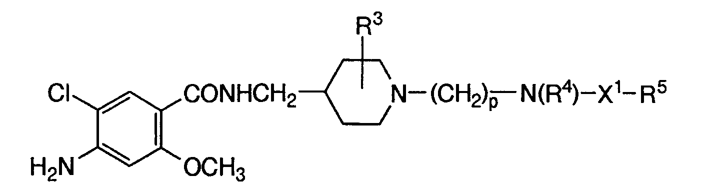

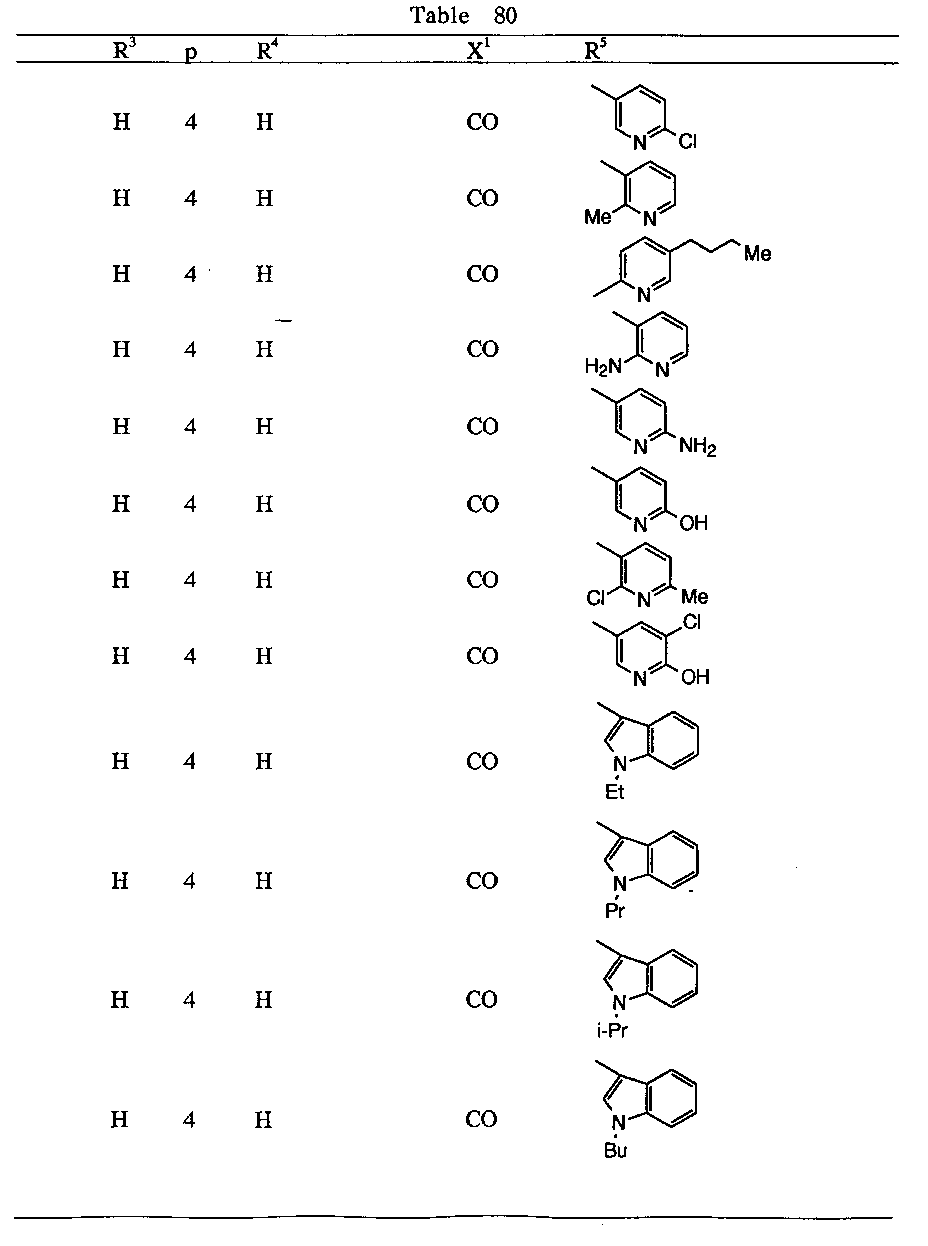

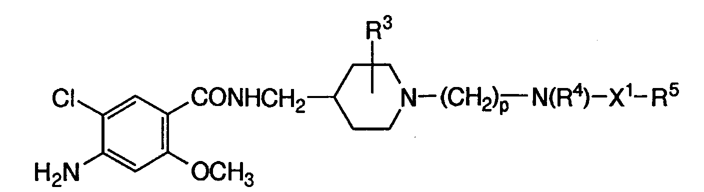

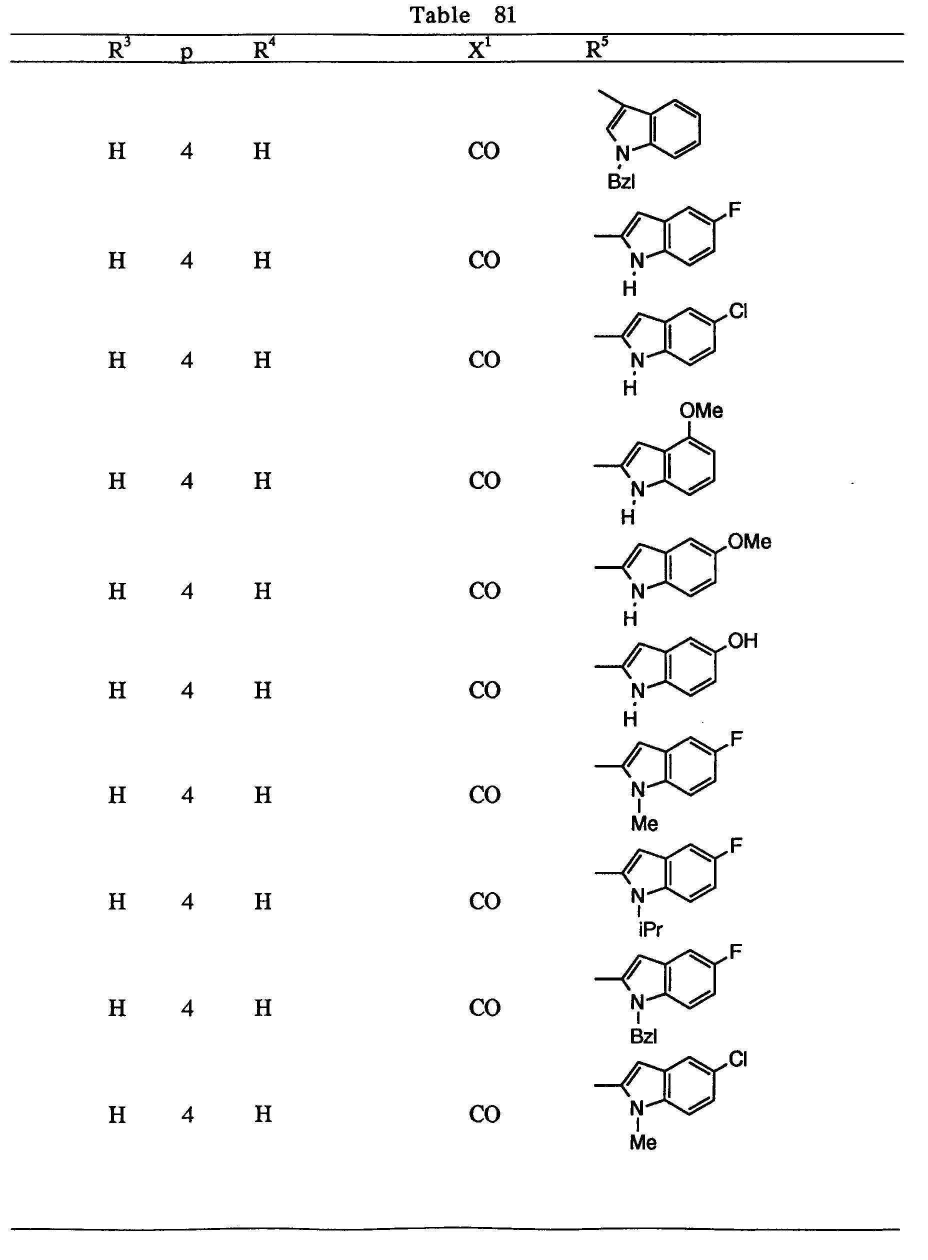

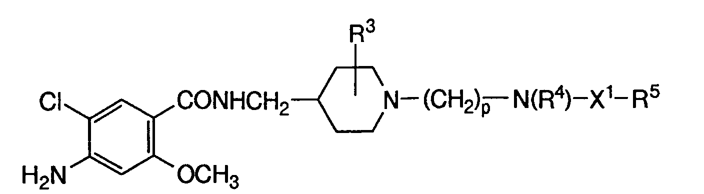

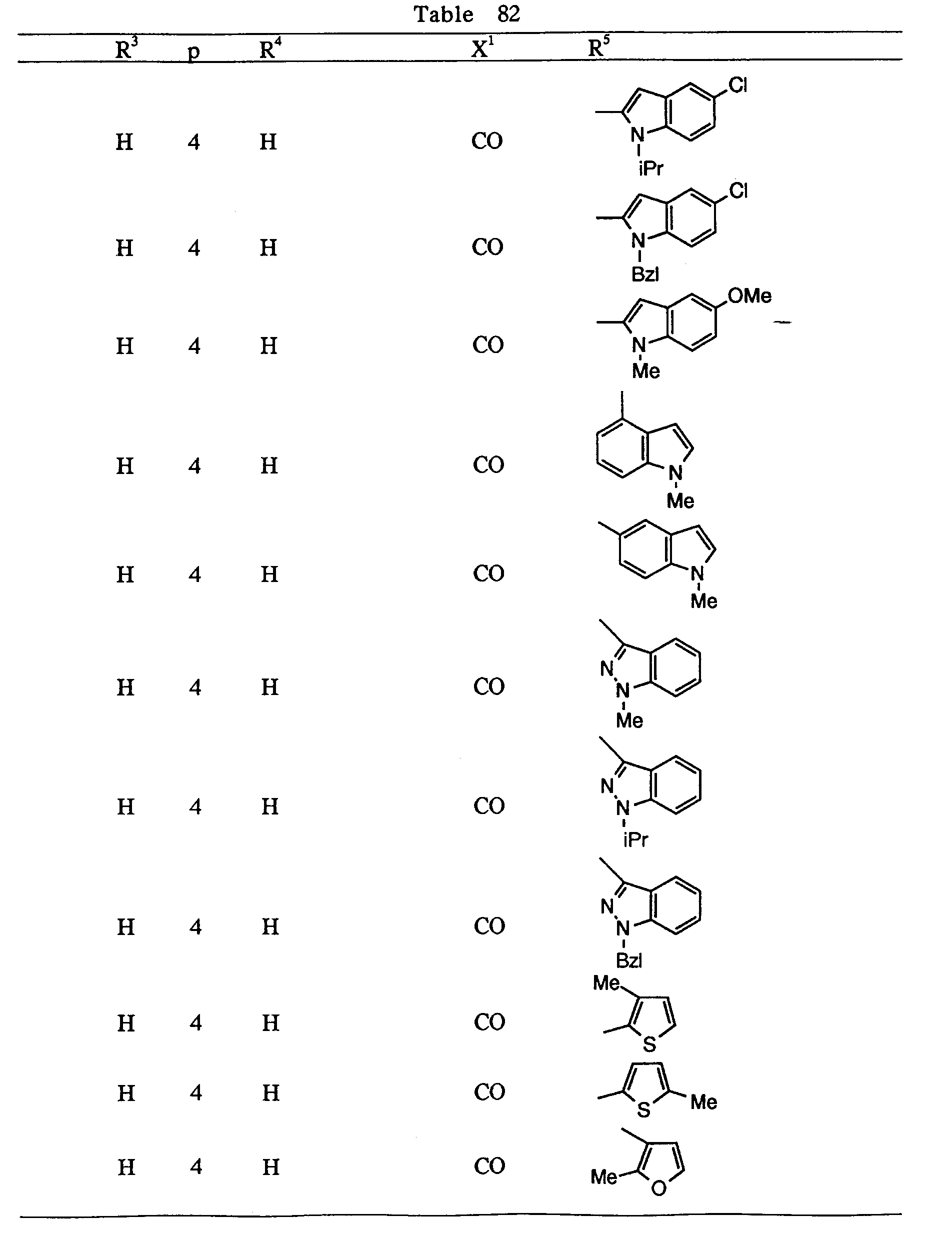

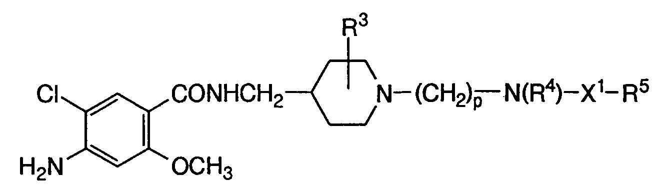

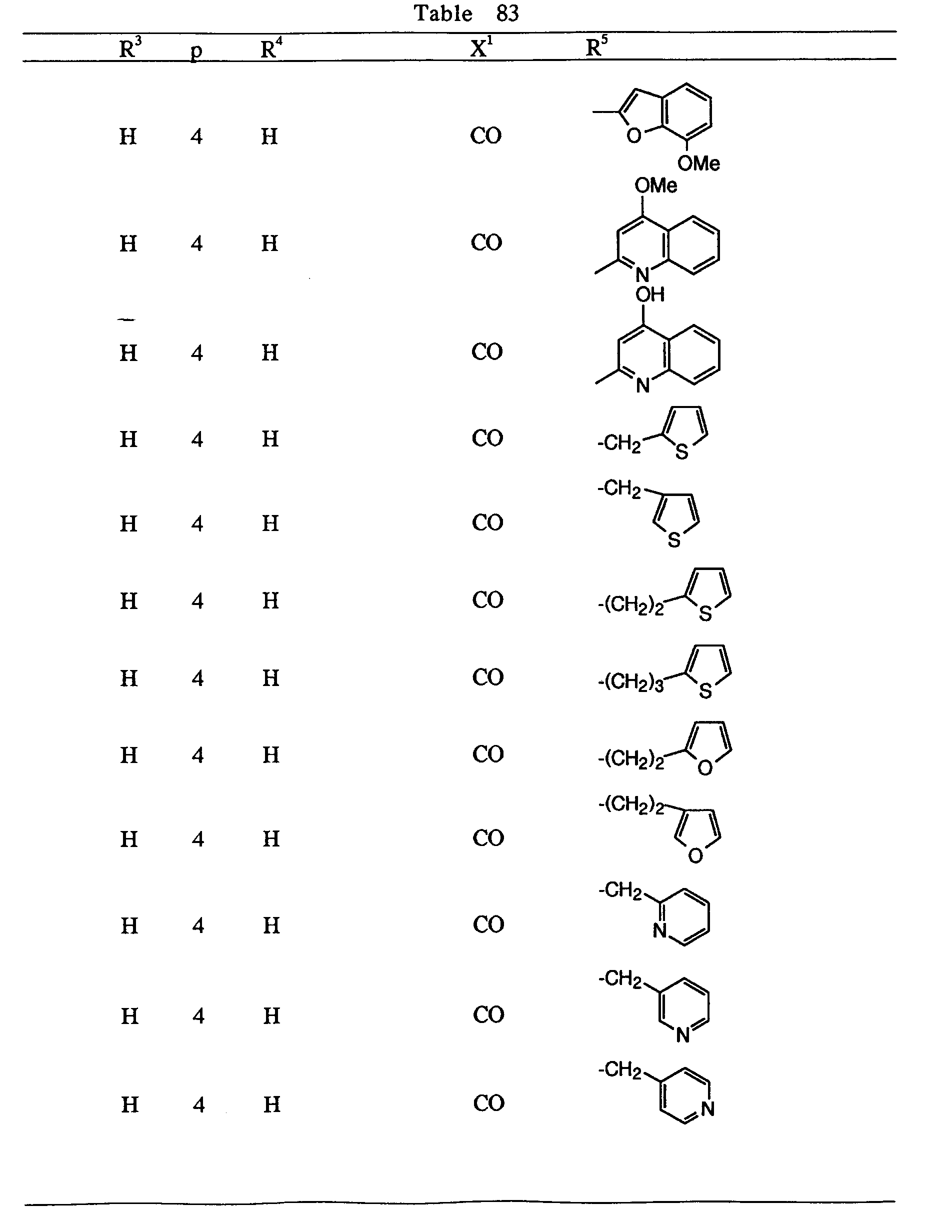

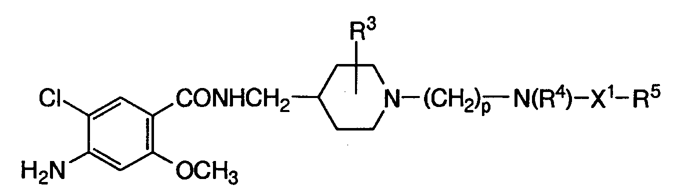

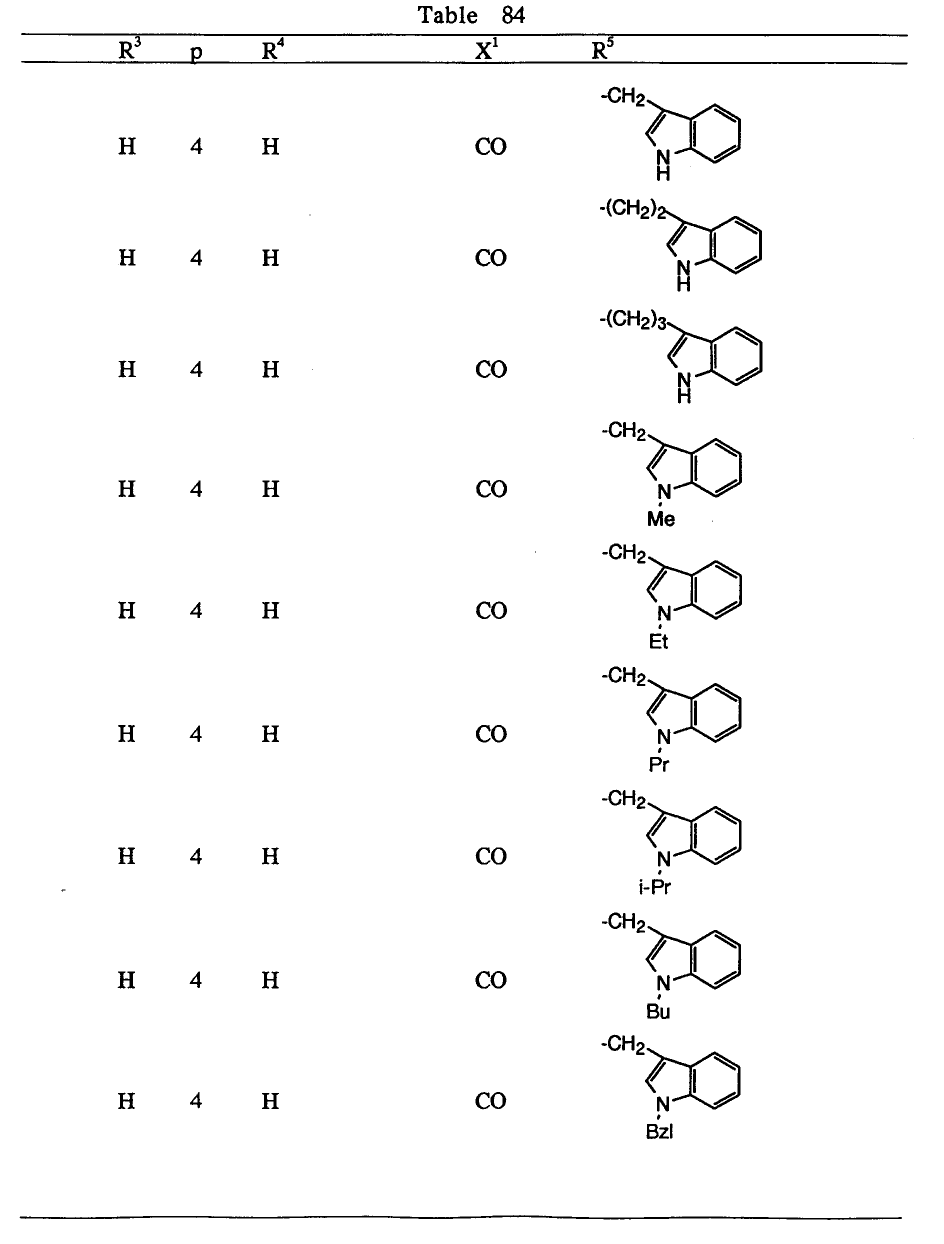

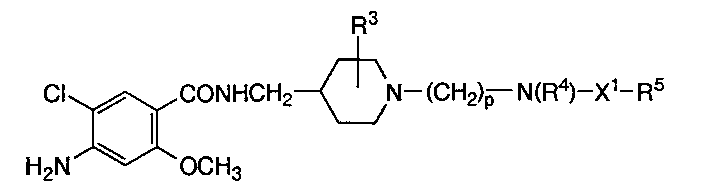

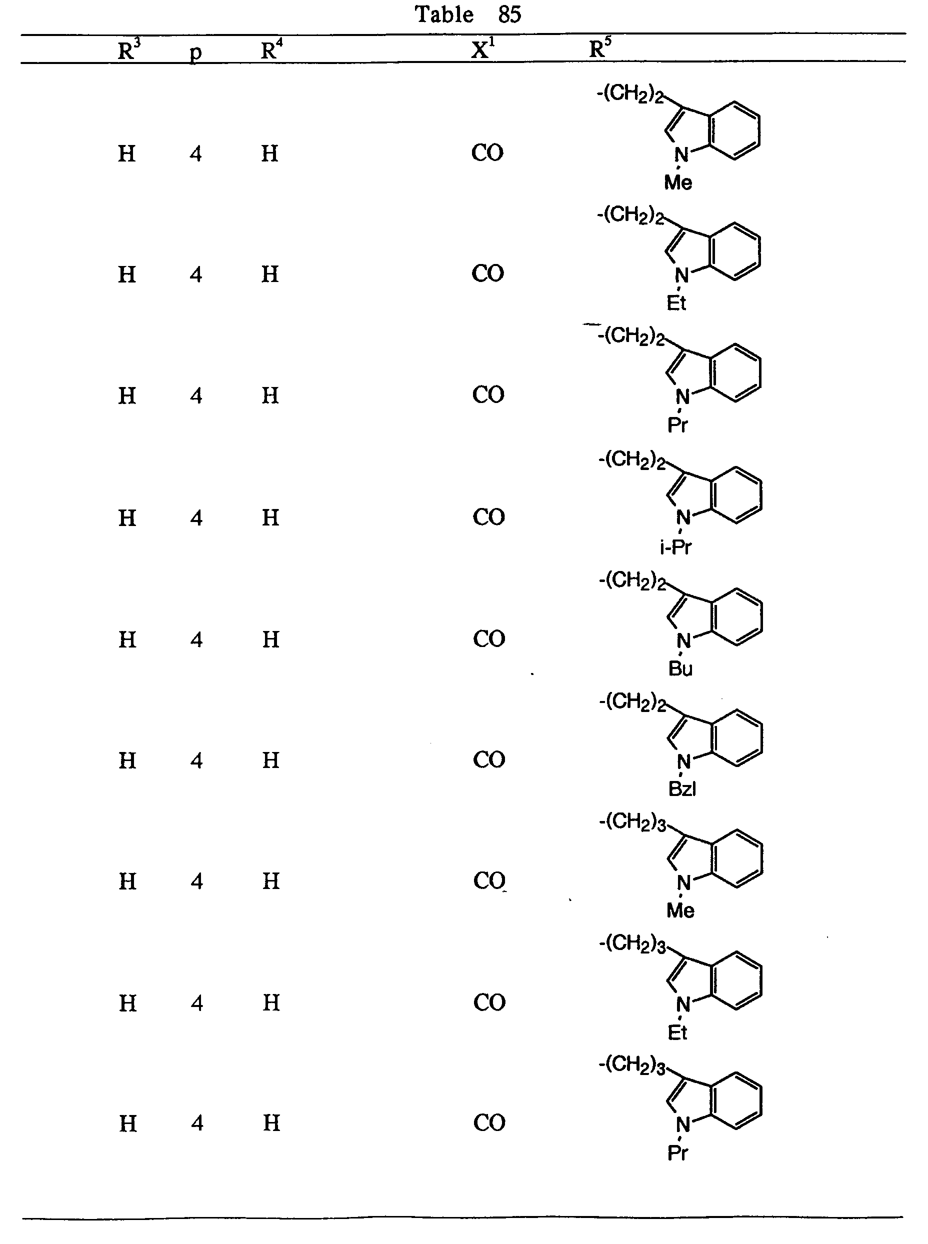

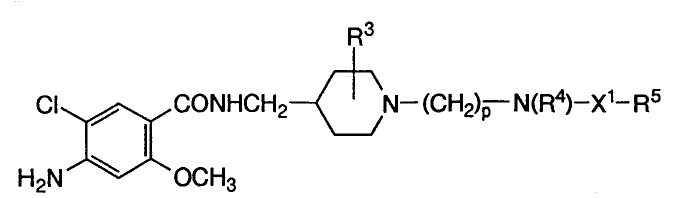

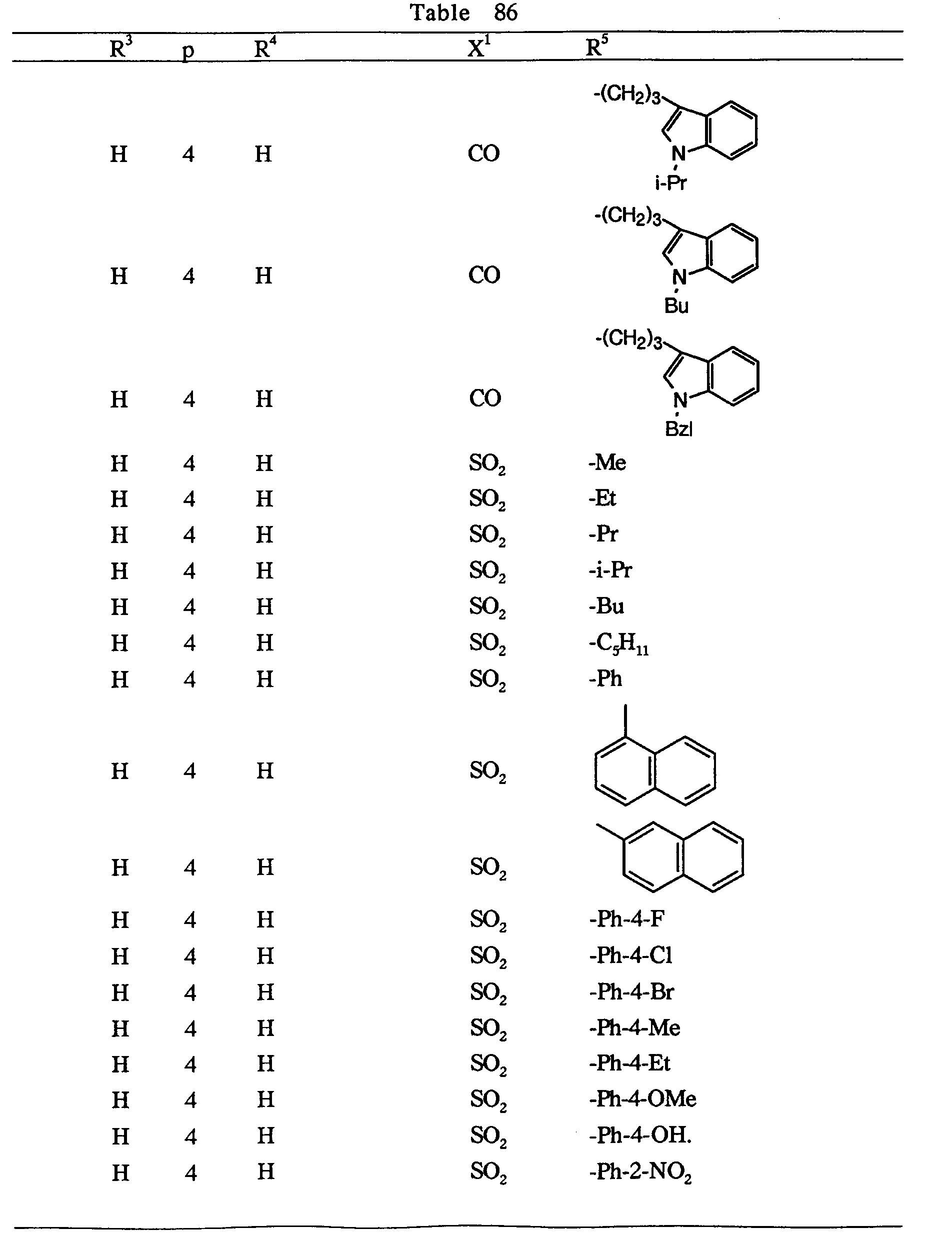

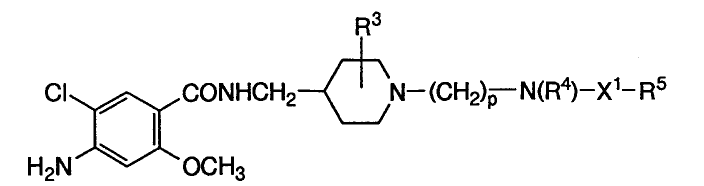

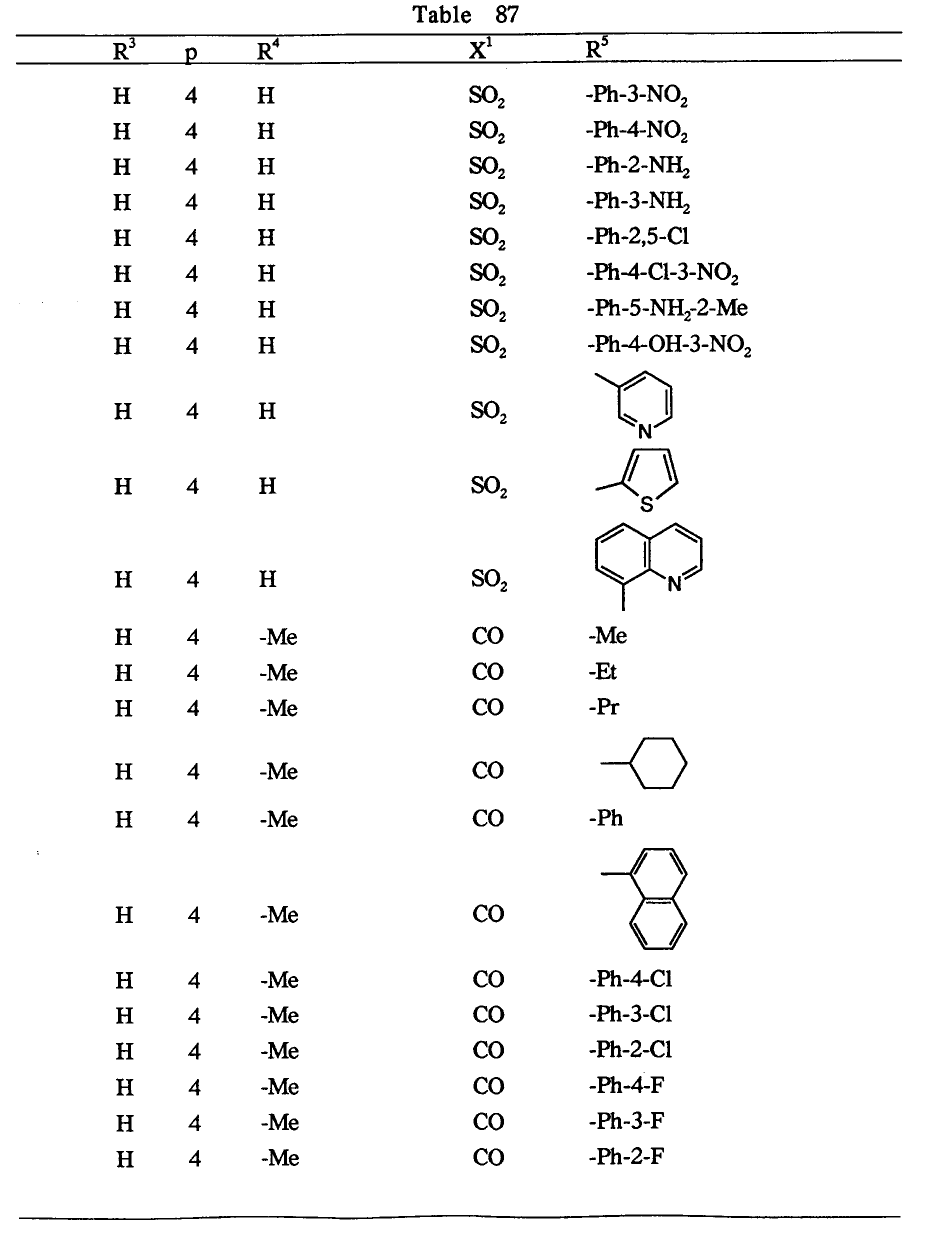

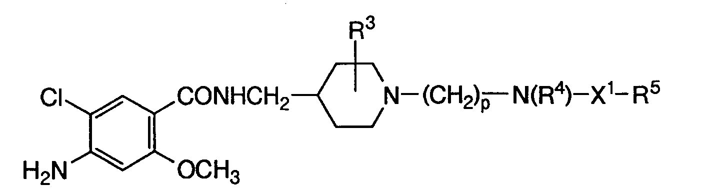

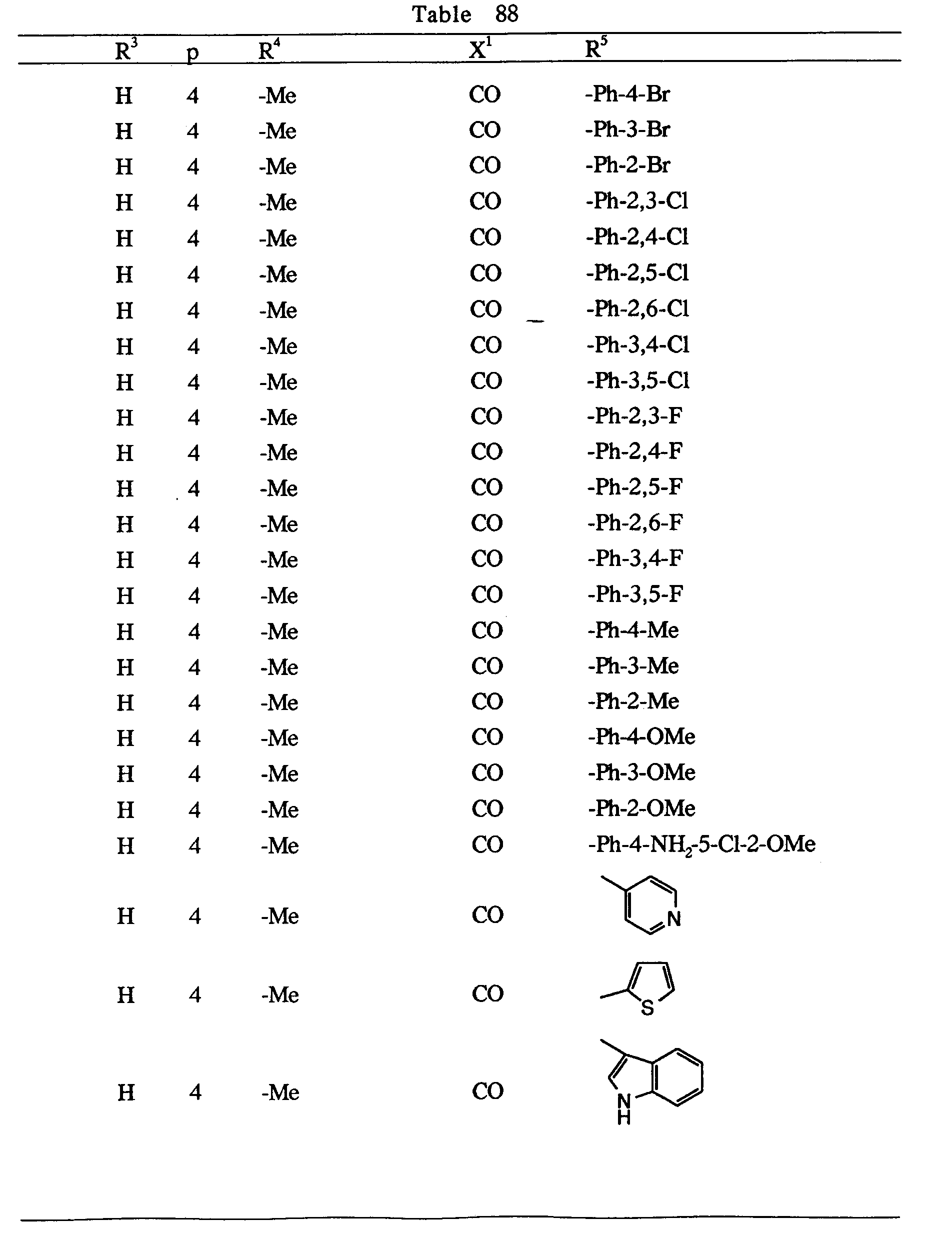

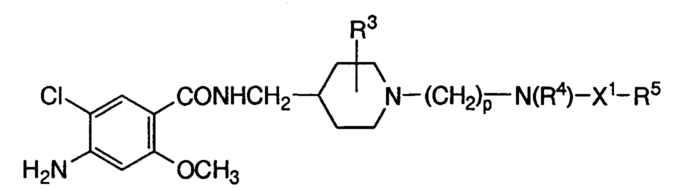

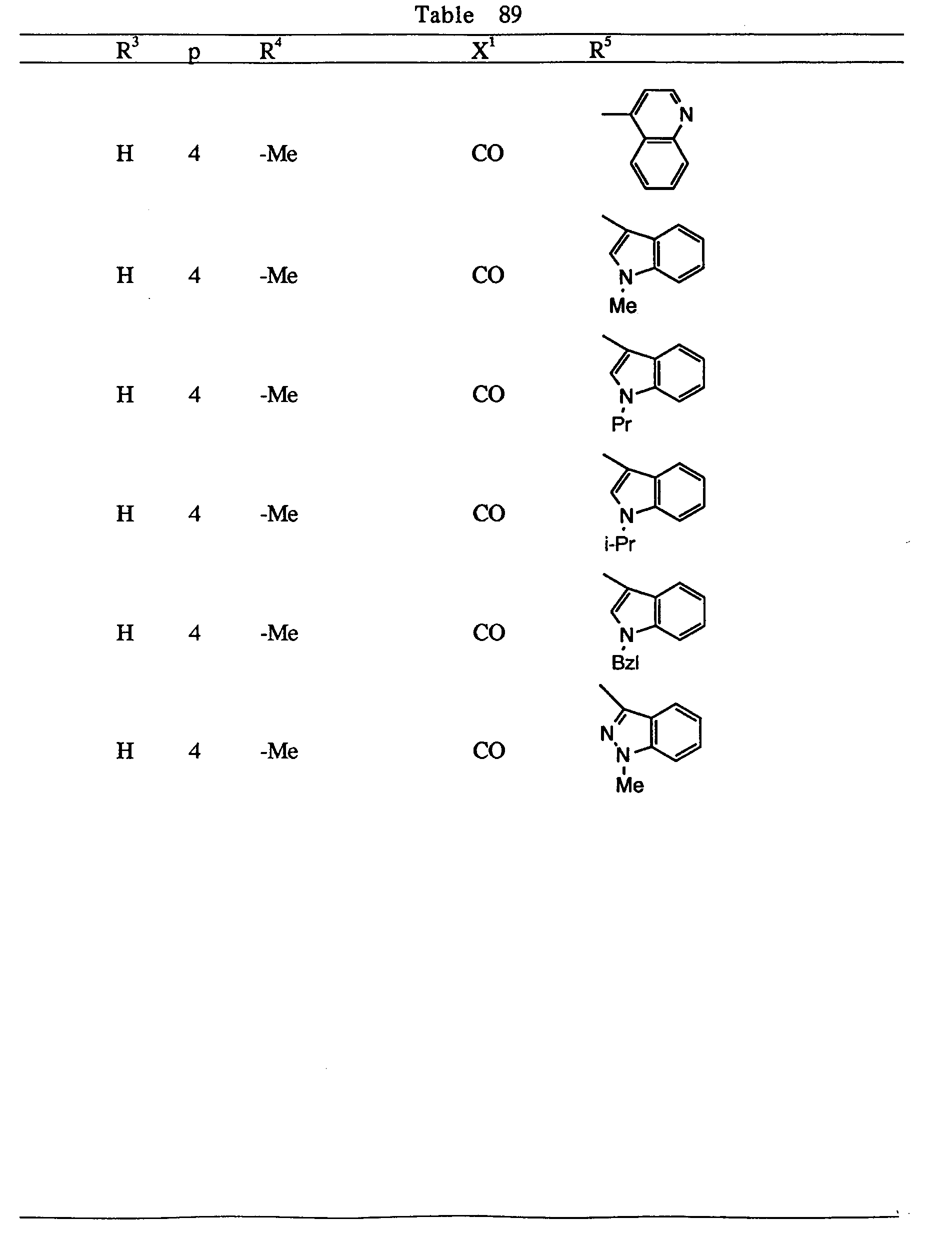

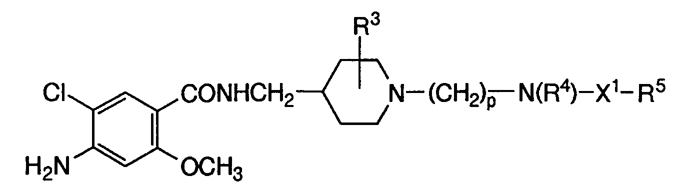

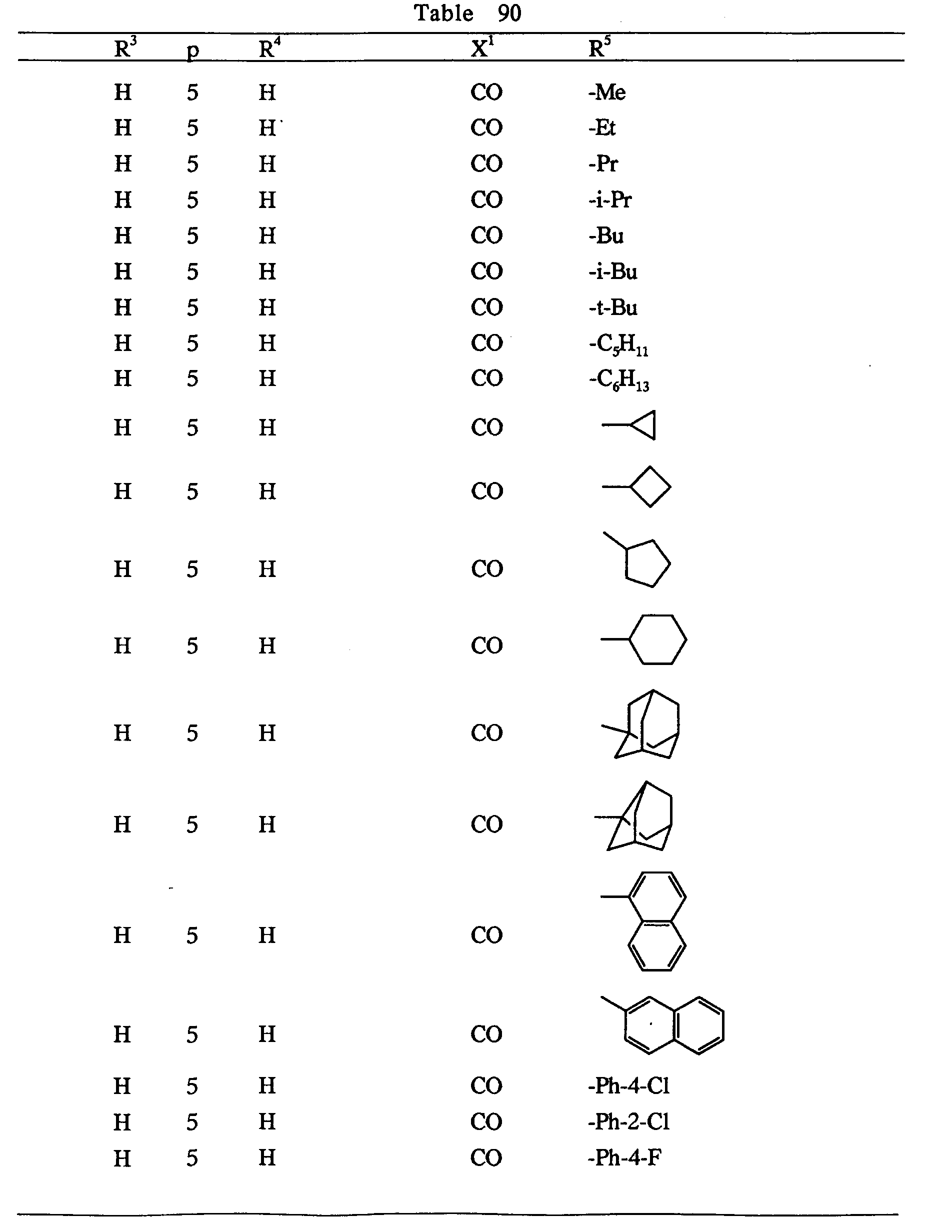

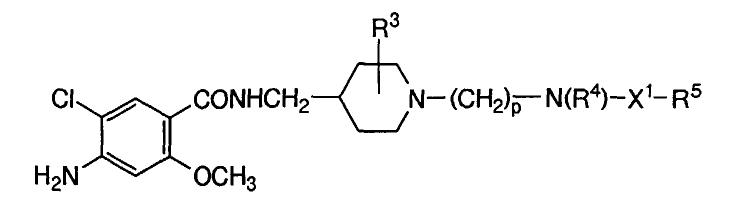

**Table 91**

| R³ | p | R⁴ | X¹ | R⁵ |
|---|---|---|---|---|
| H | 5 | H | CO | -Ph-3-F |
| H | 5 | H | CO | -Ph-2-F |
| H | 5 | H | CO | -Ph-4-Br |
| H | 5 | H | CO | -Ph-3-Br |
| H | 5 | H | CO | -Ph-2-Br |
| H | 5 | H | CO | -Ph-4-I |
| H | 5 | H | CO | -Ph-3-I |
| H | 5 | H | CO | -Ph-2-I |
| H | 5 | H | CO | -Ph-2,3-Cl |
| H | 5 | H | CO | -Ph-2,4-Cl |
| H | 5 | H | CO | -Ph-2,5-Cl |
| H | 5 | H | CO | -Ph-2,6-Cl |
| H | 5 | H | CO | -Ph-3,4-Cl |
| H | 5 | H | CO | -Ph-3,5-Cl |
| H | 5 | H | CO | -Ph-2,3,5-Cl |
| H | 5 | H | CO | -Ph-2,4,6-Cl |
| H | 5 | H | CO | -Ph-2,3-F |
| H | 5 | H | CO | -Ph-2,4-F |
| H | 5 | H | CO | -Ph-2,5-F |
| H | 5 | H | CO | -Ph-2,6-F |
| H | 5 | H | CO | -Ph-3,4-F |
| H | 5 | H | CO | -Ph-3,5-F |
| H | 5 | H | CO | -Ph-2,3,4-F |
| H | 5 | H | CO | -Ph-2,3,6-F |
| H | 5 | H | CO | -Ph-2,4,5-F |
| H | 5 | H | CO | -Ph-2,4,6-F |
| H | 5 | H | CO | -Ph-3,4,5-F |
| H | 5 | H | CO | -Ph-3,5-Br |
| H | 5 | H | CO | -Ph-3-Me |
| H | 5 | H | CO | -Ph-2-Me |
| H | 5 | H | CO | -Ph-4-Et |

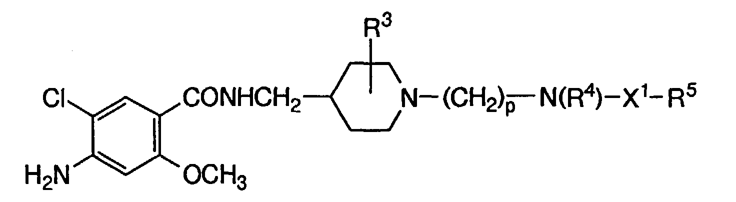

**Table 92**

| R³ | p | R⁴ | X¹ | R⁵ |
|---|---|---|---|---|
| H | 5 | H | CO | -Ph-4-Pr |
| H | 5 | H | CO | -Ph-4-i-Pr |
| H | 5 | H | CO | -Ph-4-Bu |
| H | 5 | H | CO | -Ph-4-t-Bu |
| H | 5 | H | CO | -Ph-2,3-Me |
| H | 5 | H | CO | -Ph-2,4-Me |
| H | 5 | H | CO | -Ph-2,5-Me |
| H | 5 | H | CO | -Ph-2,6-Me |
| H | 5 | H | CO | -Ph-3,4-Me |
| H | 5 | H | CO | -Ph-3,5-Me |
| H | 5 | H | CO | -Ph-3,4,5-Me |
| H | 5 | H | CO | -Ph-4-OMe |
| H | 5 | H | CO | -Ph-3-OMe |
| H | 5 | H | CO | -Ph-2-OMe |
| H | 5 | H | CO | -Ph-4-OEt |
| H | 5 | H | CO | -Ph-2-OEt |
| H | 5 | H | CO | -Ph-4-OPr |
| H | 5 | H | CO | -Ph-4-O-i-Pr |
| H | 5 | H | CO | -Ph-4-OBu |
| H | 5 | H | CO | -Ph-2,3-OMe |
| H | 5 | H | CO | -Ph-2,4-OMe |
| H | 5 | H | CO | -Ph-2,5-OMe |
| H | 5 | H | CO | -Ph-2,6-OMe |
| H | 5 | H | CO | -Ph-3,4-OMe |
| H | 5 | H | CO | -Ph-3,5-OMe |
| H | 5 | H | CO | -Ph-2,3,4-OMe |
| H | 5 | H | CO | -Ph-2,4,5-OMe |
| H | 5 | H | CO | -Ph-3,4,5-OMe |
| H | 5 | H | CO | -Ph-4-CH₂Ph |
| H | 5 | H | CO | -Ph-3-CH₂Ph |
| H | 5 | H | CO | -Ph-2-CH₂Ph |

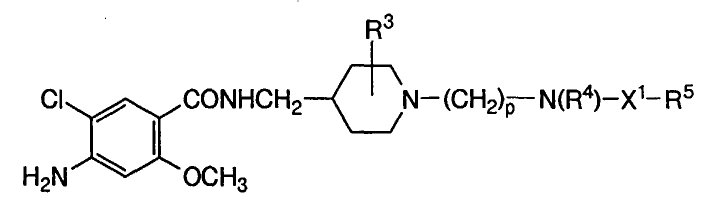

**Table 93**

| R³ | p | R⁴ | X¹ | R⁵ |
|---|---|---|---|---|
| H | 5 | H | CO | -Ph-4-OH |
| H | 5 | H | CO | -Ph-3-OH |
| H | 5 | H | CO | -Ph-2,3-OH |
| H | 5 | H | CO | -Ph-2,4-OH |
| H | 5 | H | CO | -Ph-2,5-OH |
| H | 5 | H | CO | -Ph-2,6-OH |
| H | 5 | H | CO | -Ph-3,4-OH |
| H | 5 | H | CO | -Ph-3,5-OH |
| H | 5 | H | CO | -Ph-2,3,4-OH |
| H | 5 | H | CO | -Ph-2,4,6-OH |
| H | 5 | H | CO | -Ph-4-NO₂ |
| H | 5 | H | CO | -Ph-3-NO₂ |
| H | 5 | H | CO | -Ph-2-NO₂ |
| H | 5 | H | CO | -Ph-2,4-NO₂ |
| H | 5 | H | CO | -Ph-3,5-NO₂ |
| H | 5 | H | CO | -Ph-4-NH₂ |
| H | 5 | H | CO | -Ph-3-NH₂ |
| H | 5 | H | CO | -Ph-3,4-NH₂ |
| H | 5 | H | CO | -Ph-3,5-NH₂ |
| H | 5 | H | CO | -Ph-2-F-5-Me |
| H | 5 | H | CO | -Ph-3-F-2-Me |
| H | 5 | H | CO | -Ph-3-F-4-Me |
| H | 5 | H | CO | -Ph-5-F-2-Me |
| H | 5 | H | CO | -Ph-3-Br-4-Me |
| H | 5 | H | CO | -Ph-4-Cl-2-OMe |
| H | 5 | H | CO | -Ph-5-Cl-2-OMe |
| H | 5 | H | CO | -Ph-3-F-4-OMe |
| H | 5 | H | CO | -Ph-2-Br-5-OMe |
| H | 5 | H | CO | -Ph-2-NH₂-3-OMe |
| H | 5 | H | CO | -Ph-3-NH₂-4-OMe |
| H | 5 | H | CO | -Ph-4-NH₂-3-OMe |

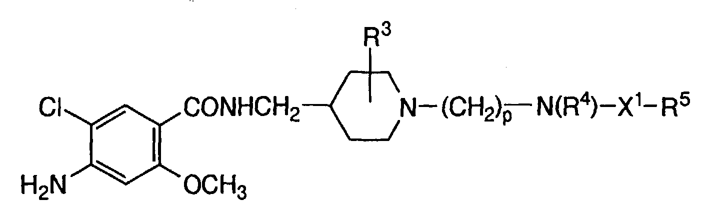

**Table 94**

| R³ | p | R⁴ | X¹ | R⁵ |
|---|---|---|---|---|
| H | 5 | H | CO | -Ph-3-NH₂-4-OH |
| H | 5 | H | CO | -Ph-4-NH₂-3-OH |
| H | 5 | H | CO | -Ph-3-NH₂-2-OH |
| H | 5 | H | CO | -Ph-4-NH₂-2-OH |
| H | 5 | H | CO | -Ph-5-NH₂-2-OH |
| H | 5 | H | CO | -Ph-3-OH-4-Me |
| H | 5 | H | CO | -Ph-2-OH-3-Me |
| H | 5 | H | CO | -Ph-2-OH-4-Me |
| H | 5 | H | CO | -Ph-2-OH-5-Me |
| H | 5 | H | CO | -Ph-2-OH-3-i-Pr |
| H | 5 | H | CO | -Ph-3-OMe-4-Me |
| H | 5 | H | CO | -Ph-2-NH₂-3-Cl |
| H | 5 | H | CO | -Ph-2-NH₂-4-Cl |
| H | 5 | H | CO | -Ph-2-NH₂-5-Cl |
| H | 5 | H | CO | -Ph-3-NH₂-4-Cl |
| H | 5 | H | CO | -Ph-4-NH₂-2-Cl |
| H | 5 | H | CO | -Ph-5-NH₂-2-Cl |
| H | 5 | H | CO | -Ph-2-NH₂-4-F |
| H | 5 | H | CO | -Ph-2-NH₂-5-F |
| H | 5 | H | CO | -Ph-2-NH₂-5-Br |
| H | 5 | H | CO | -Ph-3-Cl-4-OH |
| H | 5 | H | CO | -Ph-4-Cl-2-OH |
| H | 5 | H | CO | -Ph-5-Cl-2-OH |
| H | 5 | H | CO | -Ph-5-F-2-OH |
| H | 5 | H | CO | -Ph-5-Br-2-OH |
| H | 5 | H | CO | -Ph-2-NH₂-3-Me |
| H | 5 | H | CO | -Ph-2-NH₂-5-Me |
| H | 5 | H | CO | -Ph-2-NH₂-6-Me |
| H | 5 | H | CO | -Ph-3-NH₂-2-Me |
| H | 5 | H | CO | -Ph-3-NH₂-4-Me |
| H | 5 | H | CO | -Ph-4-NH₂-3-Me |

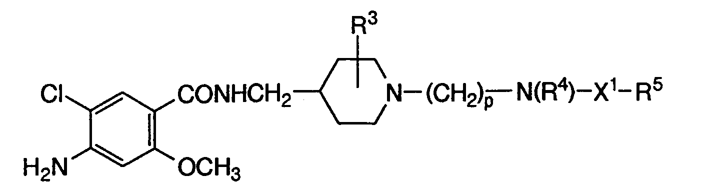

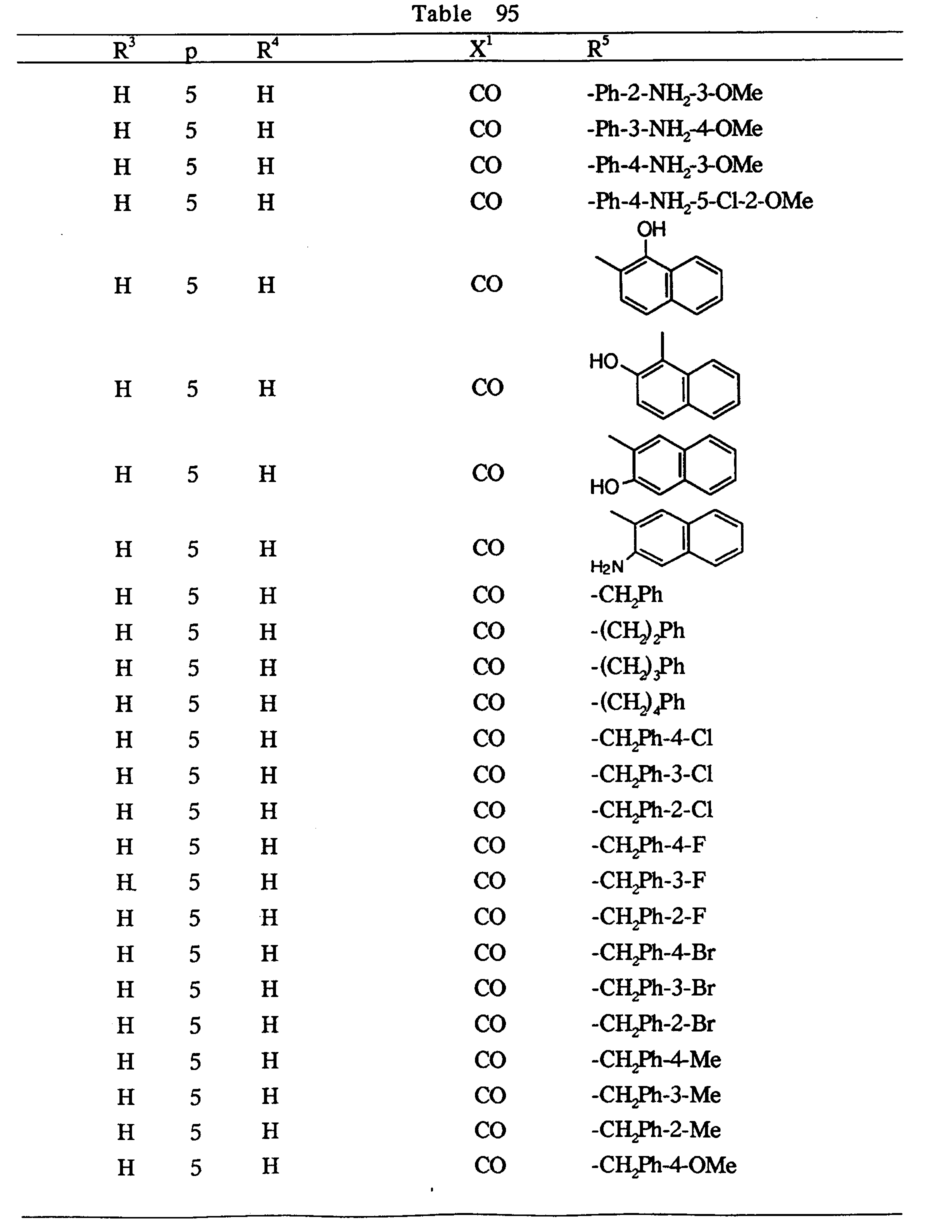

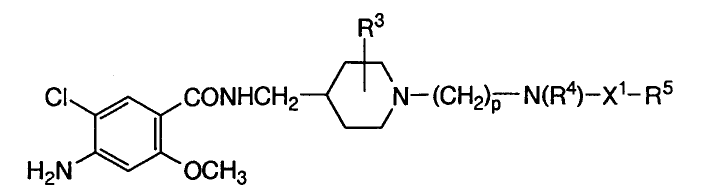

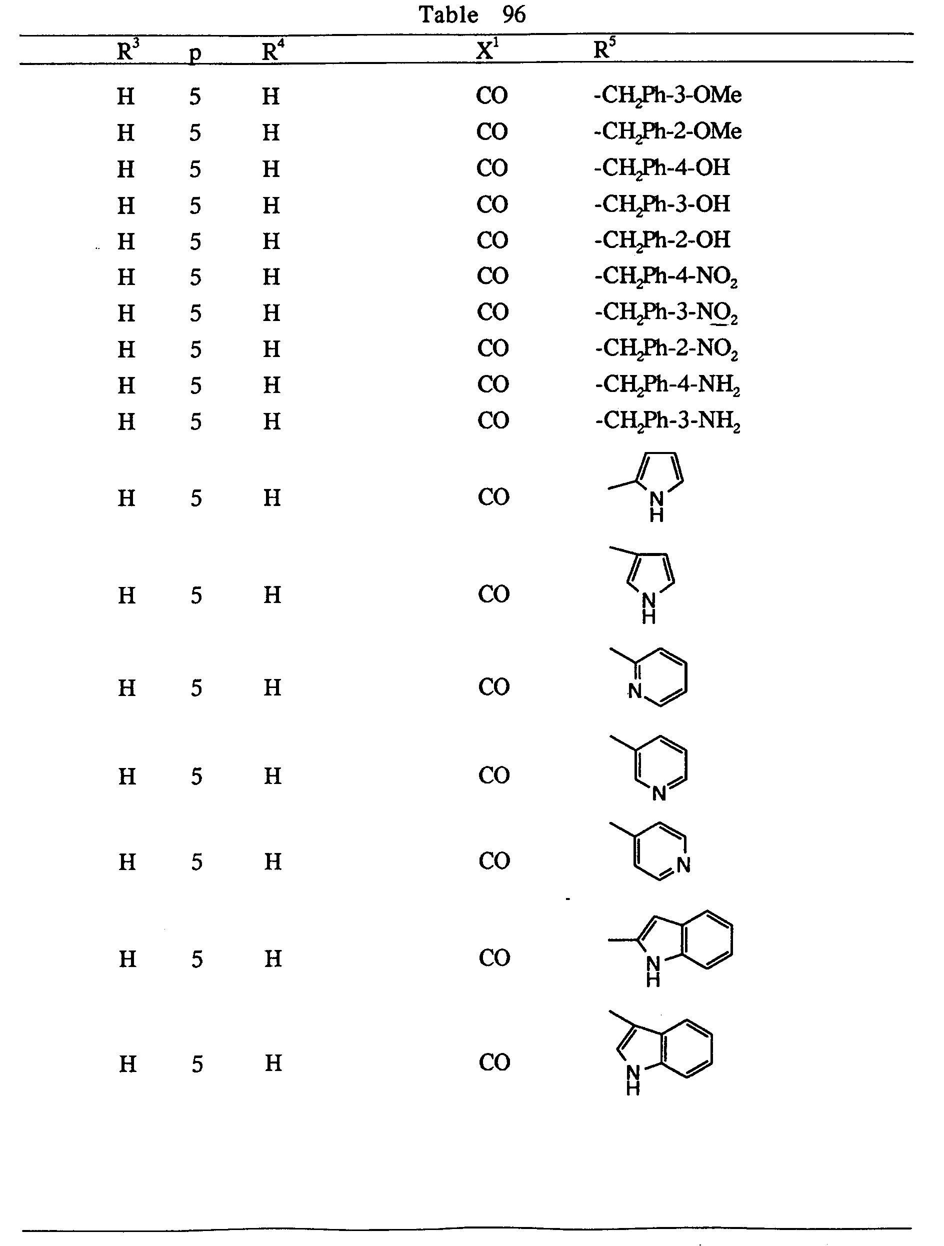

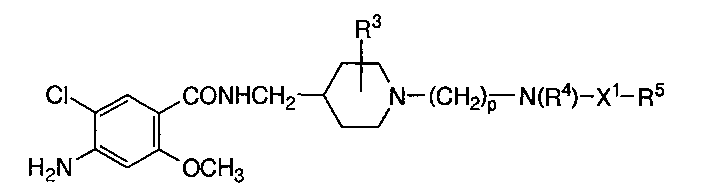

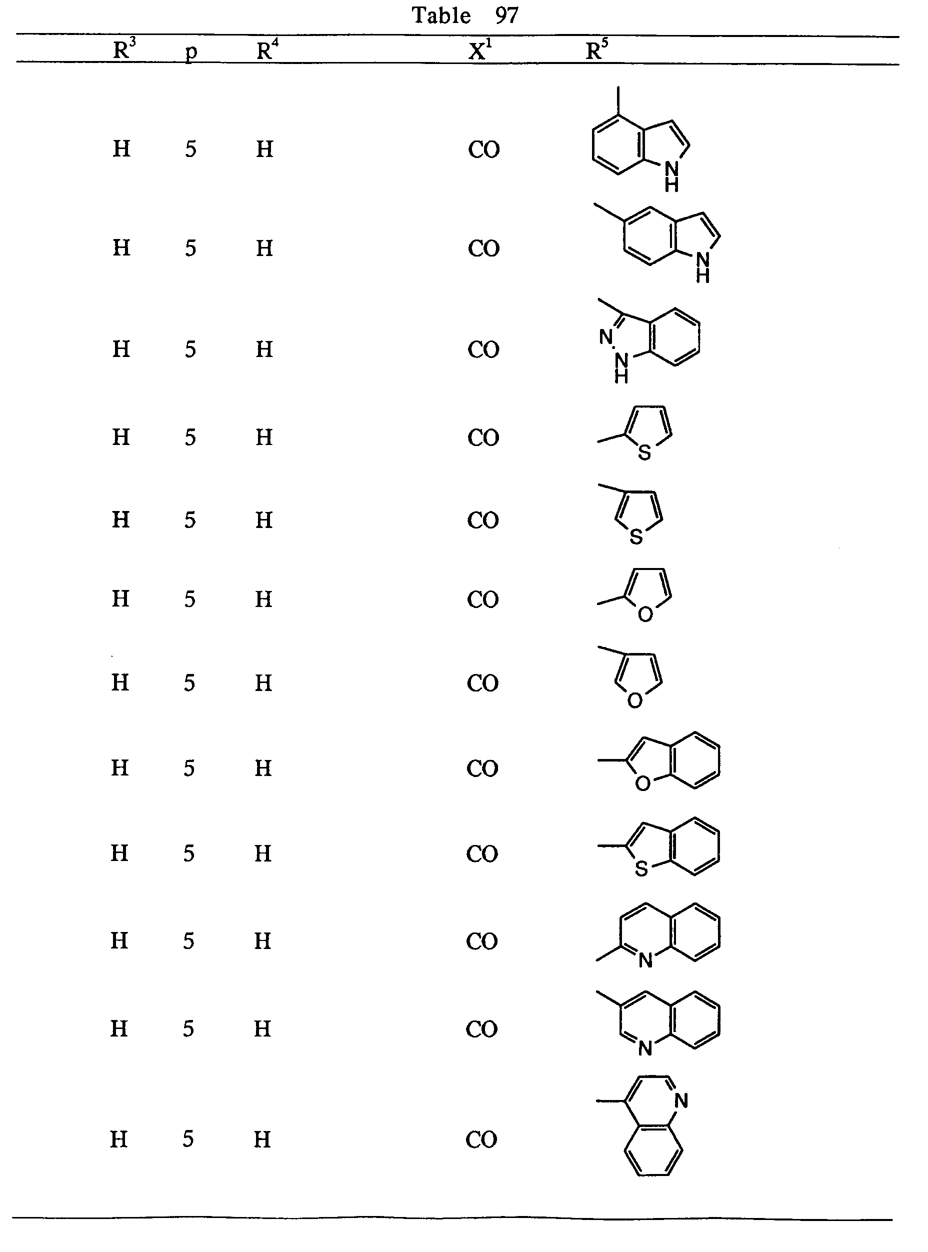

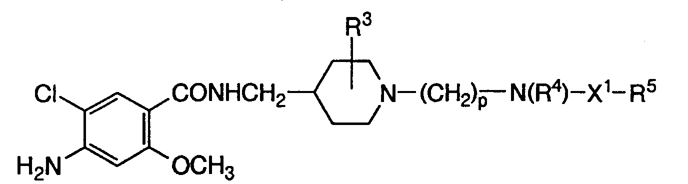

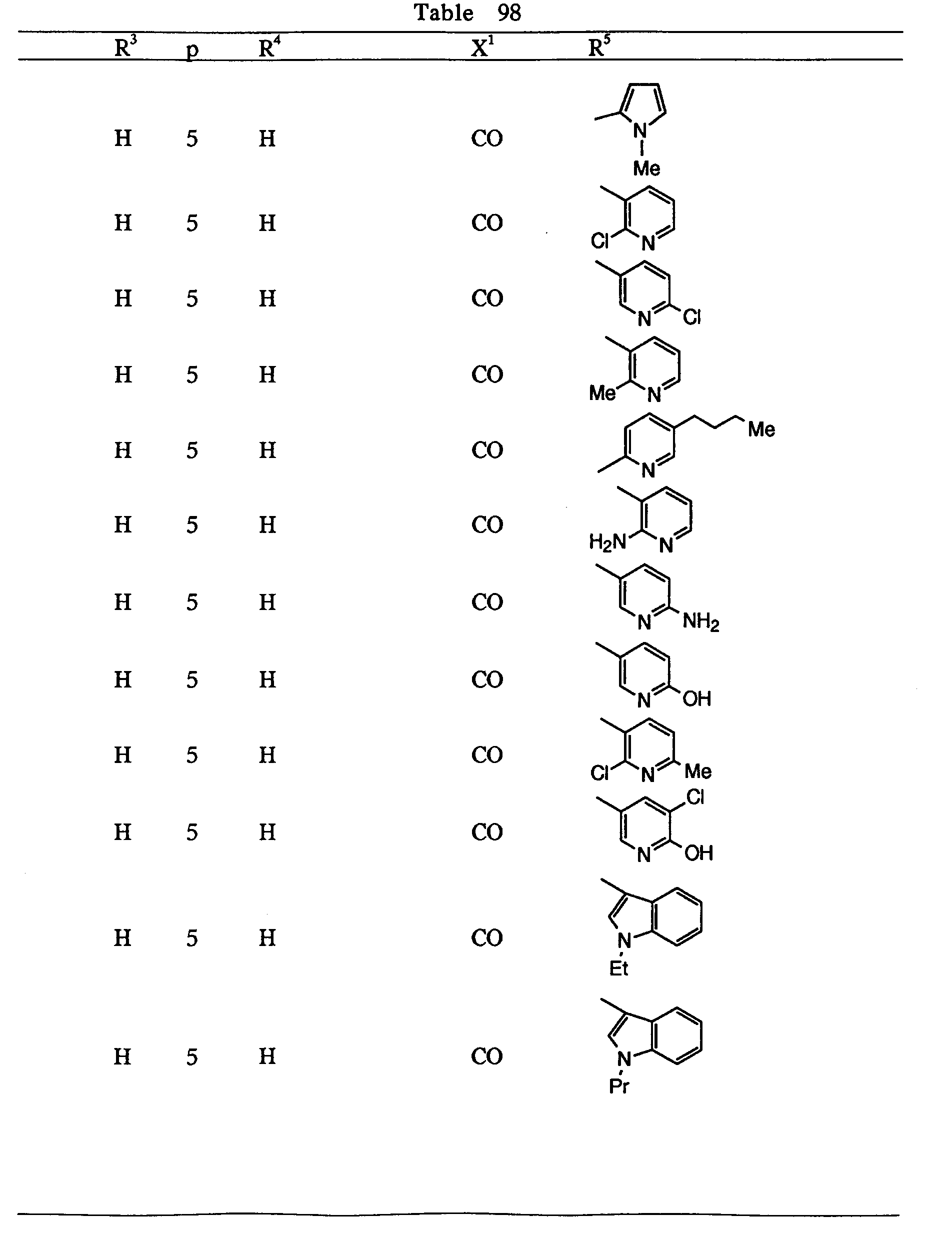

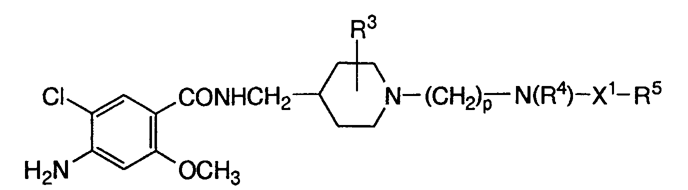

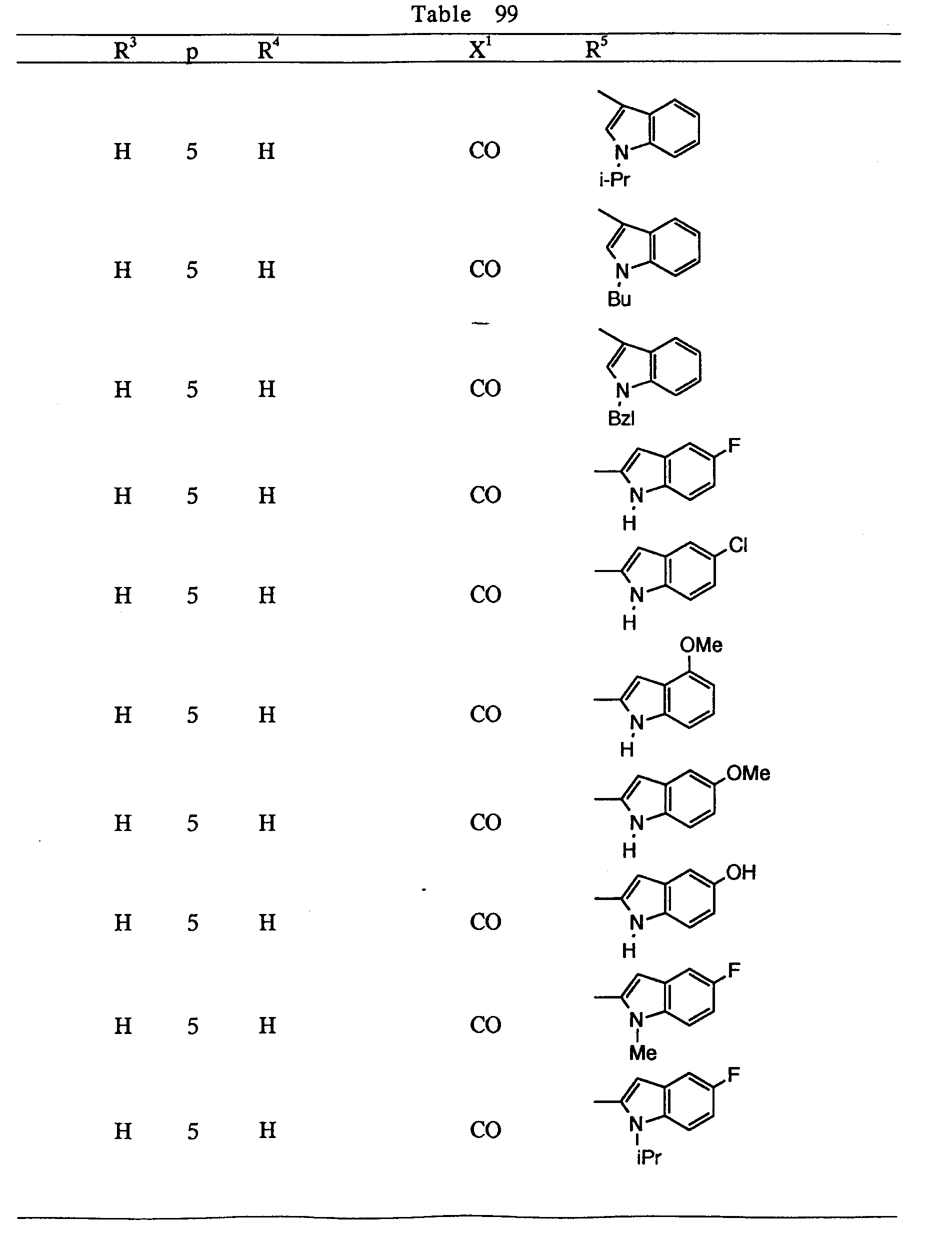

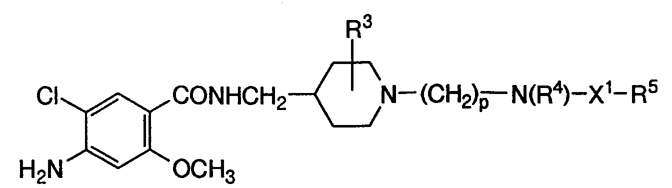

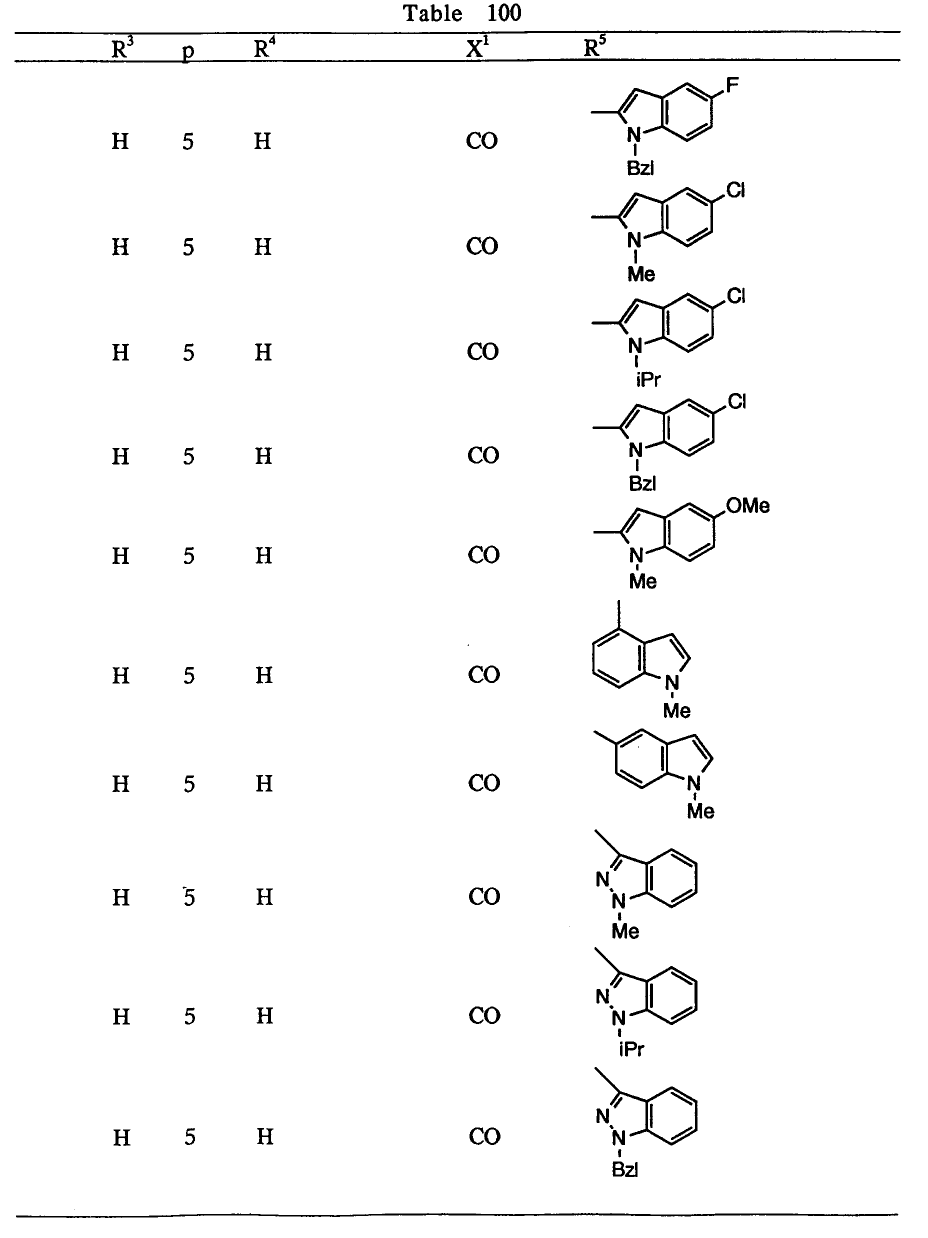

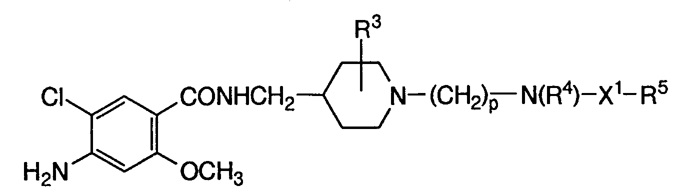

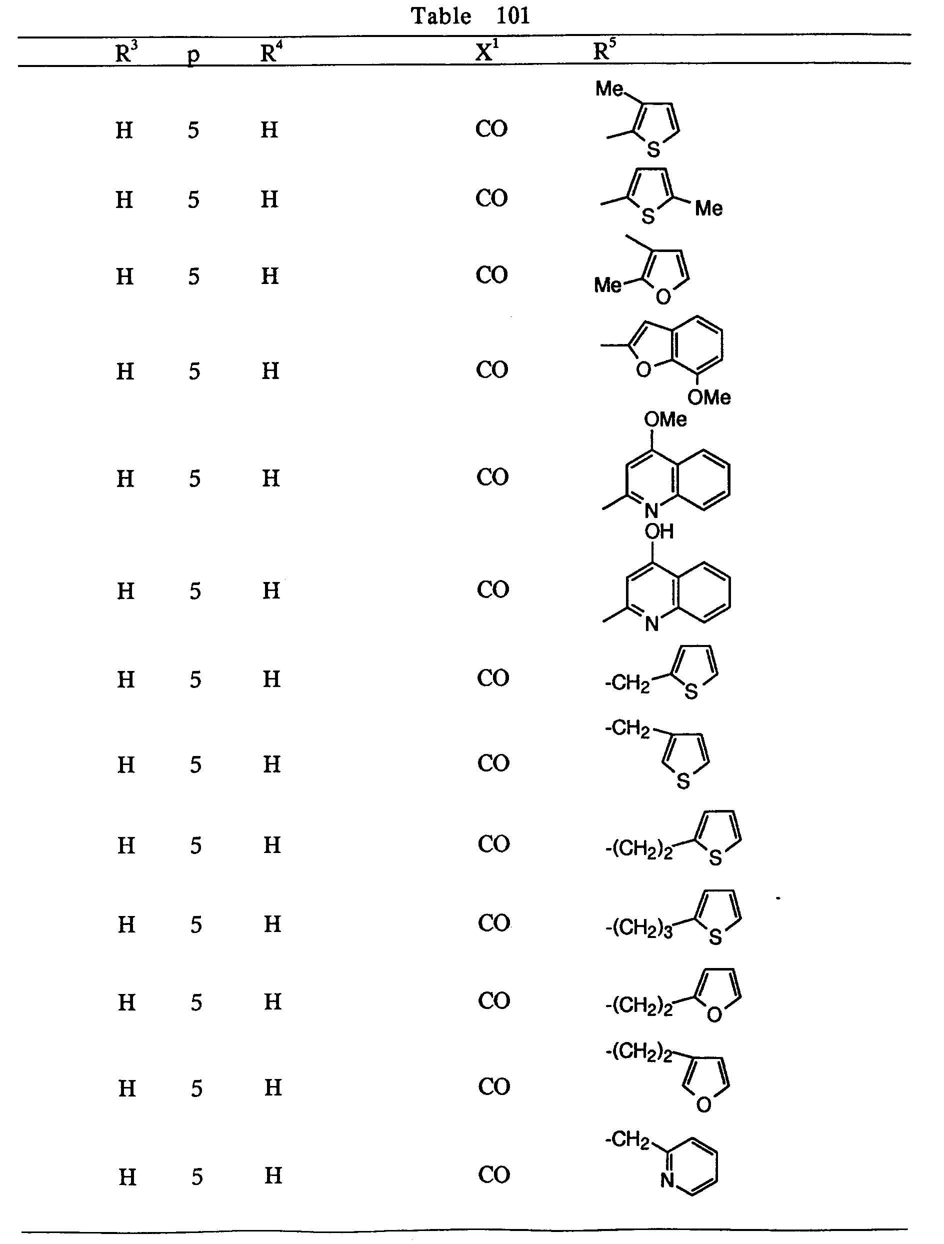

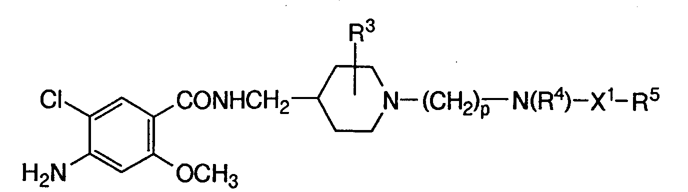

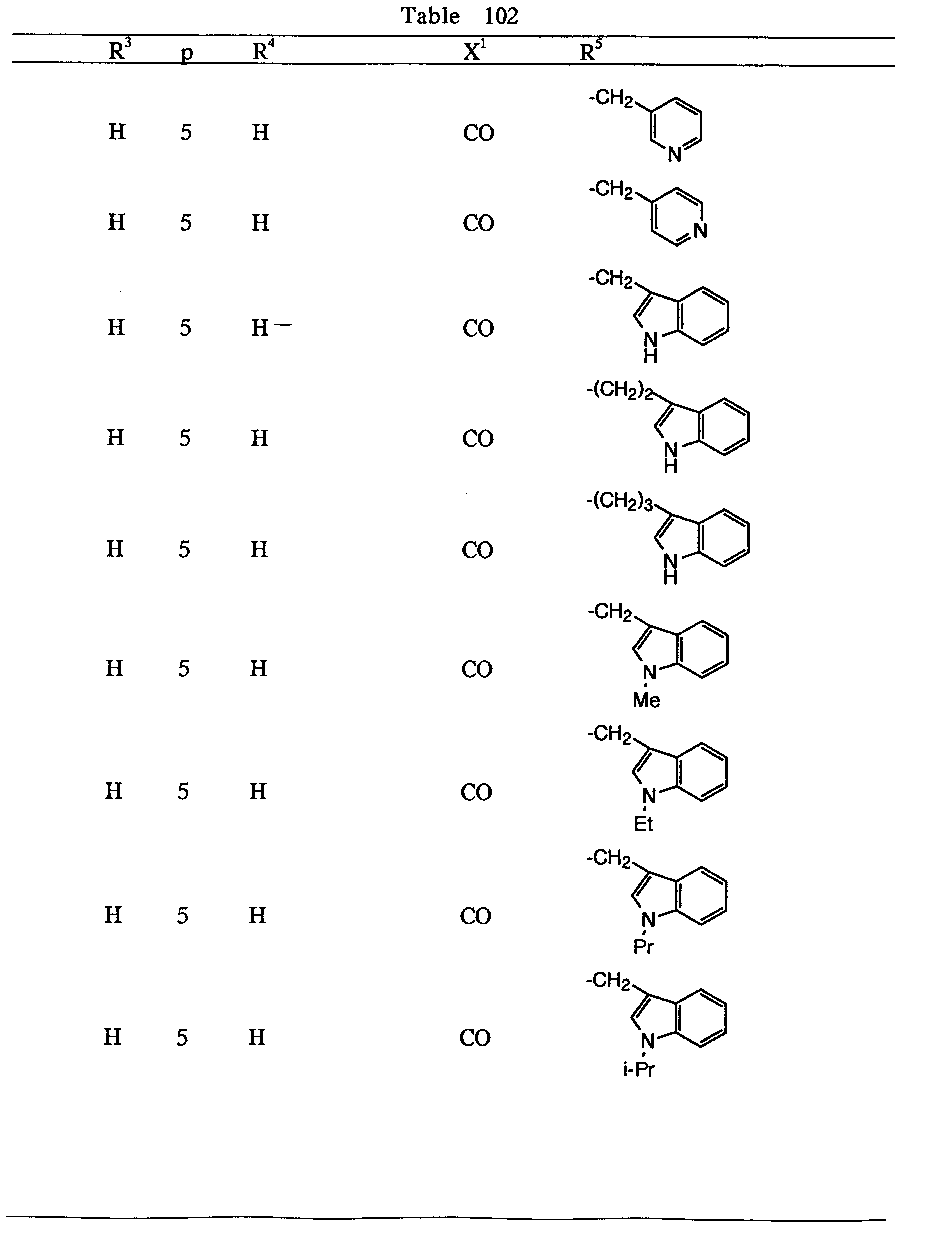

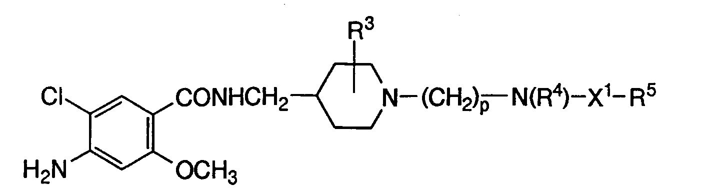

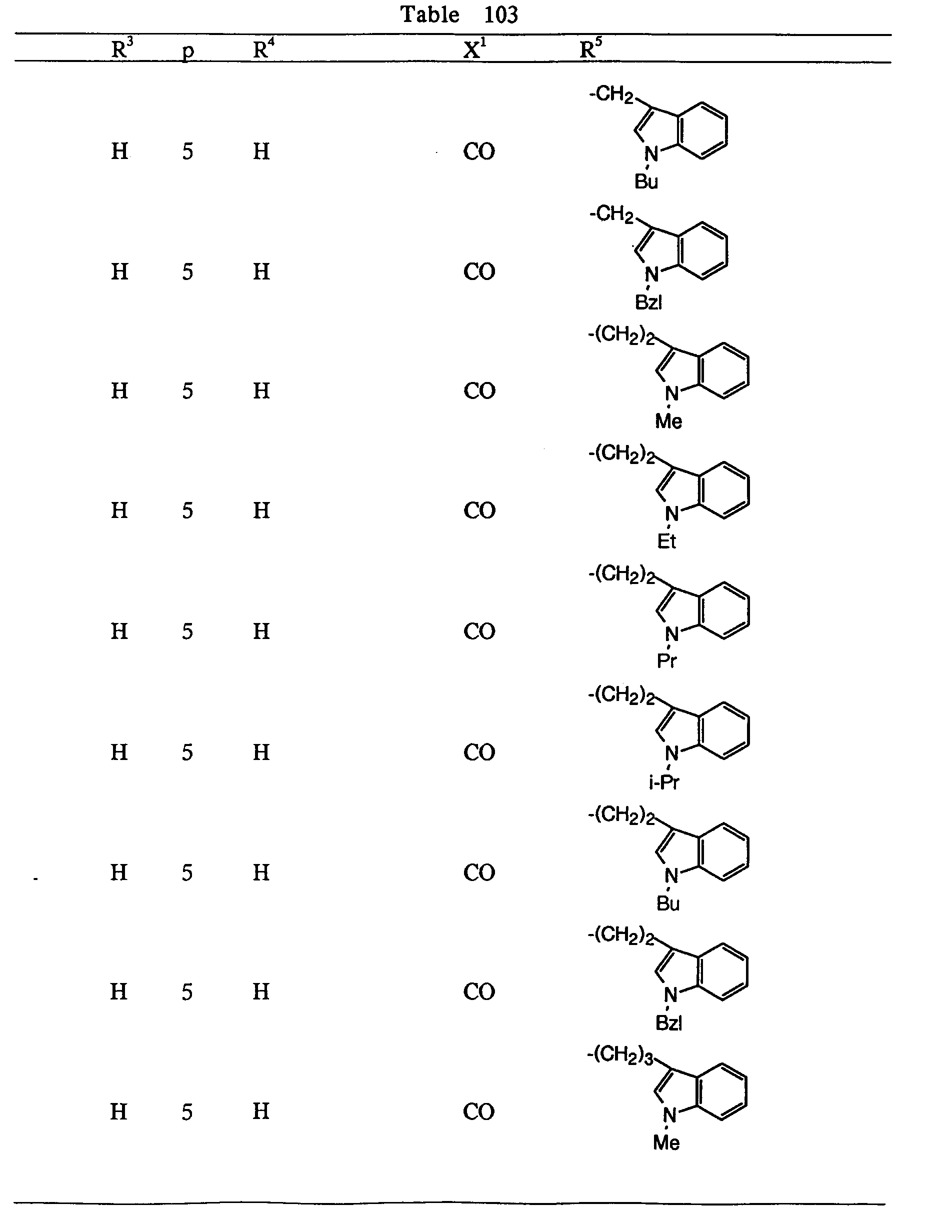

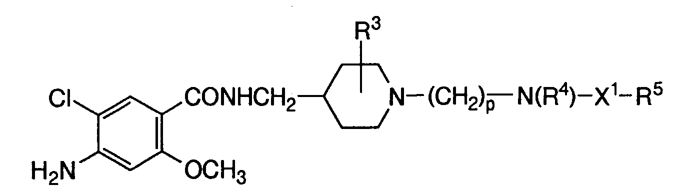

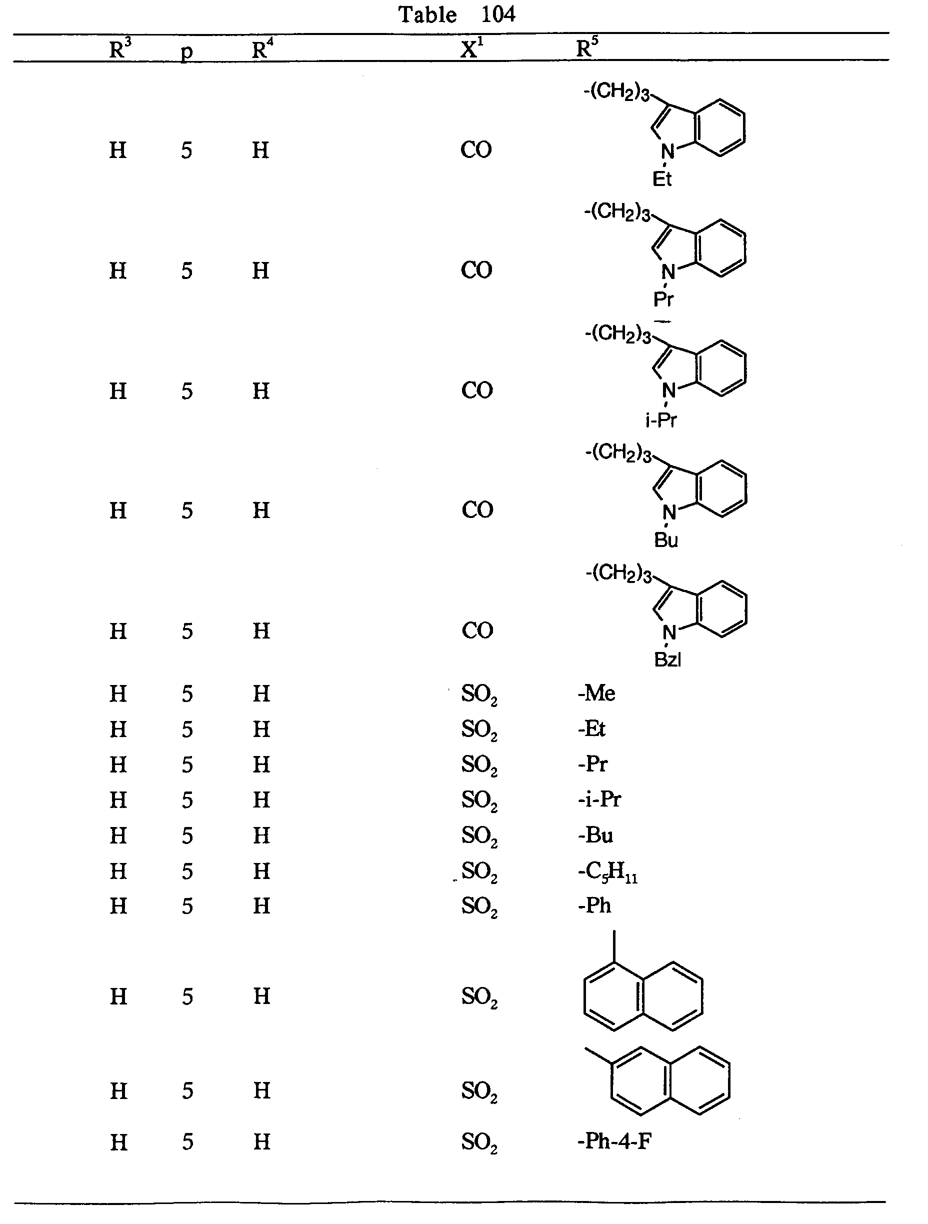

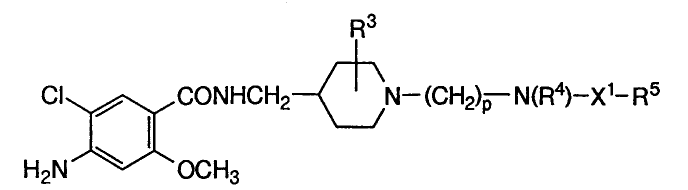

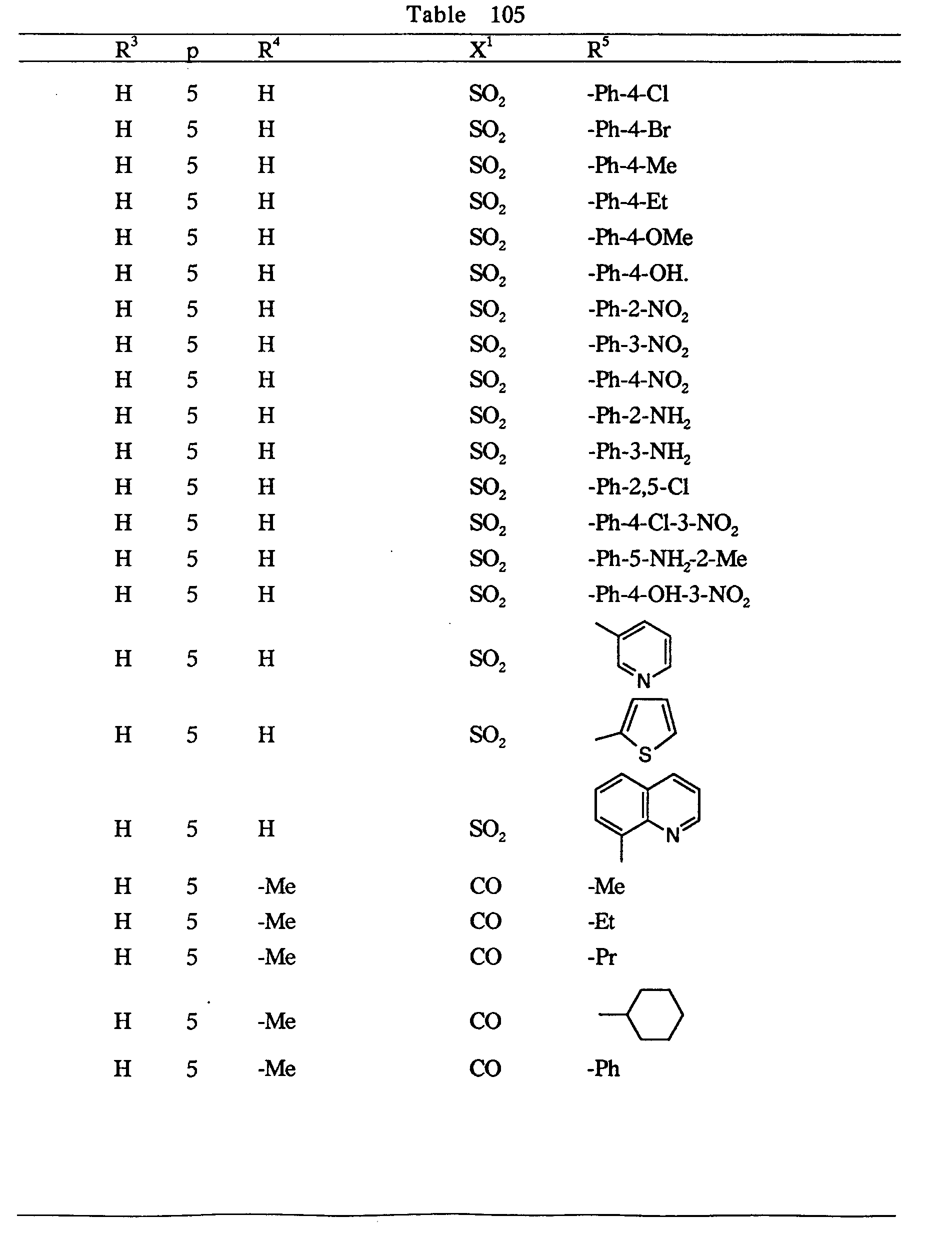

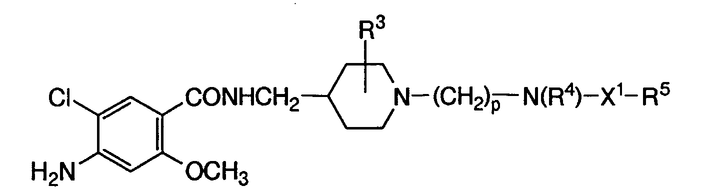

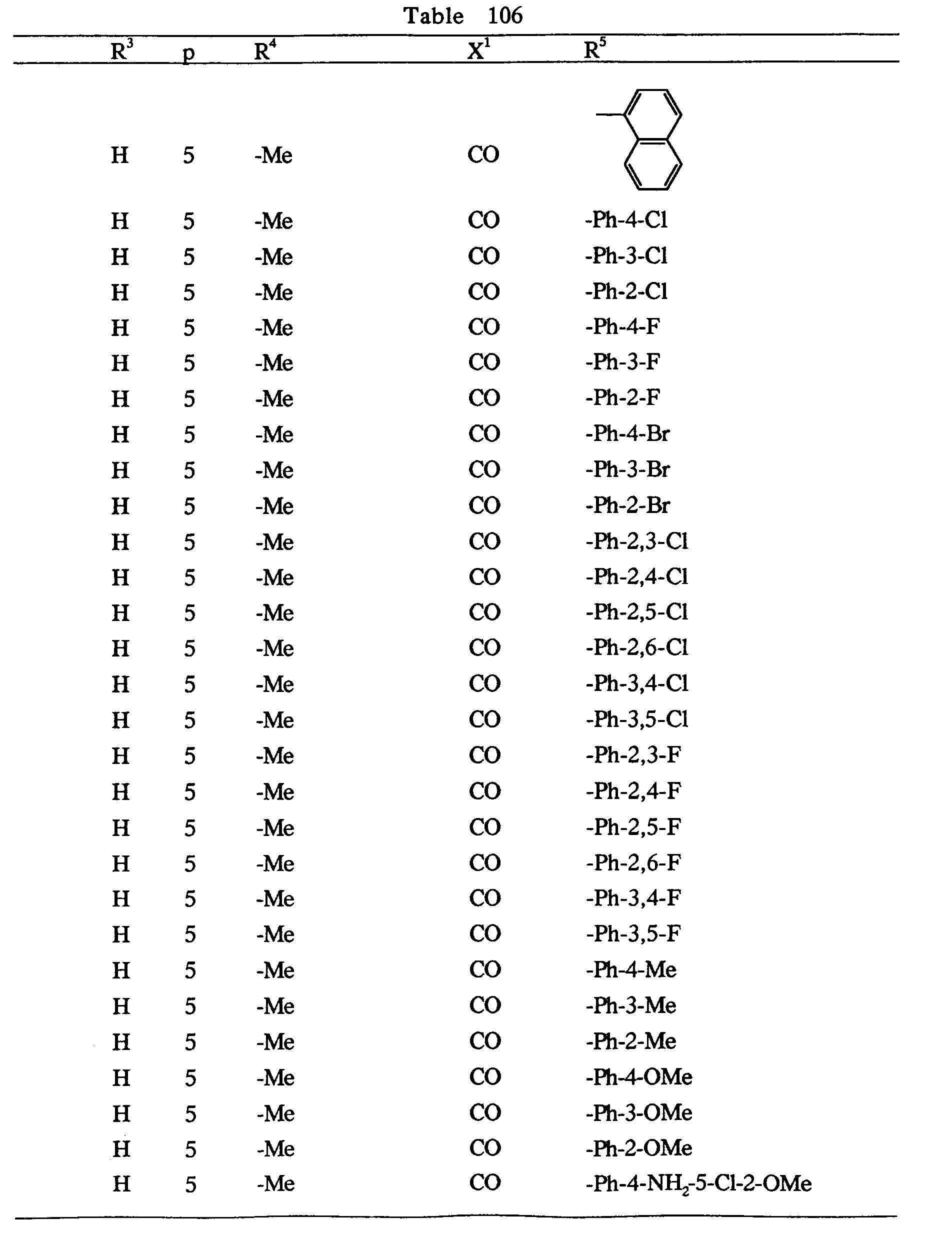

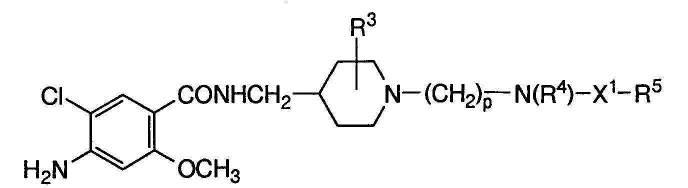

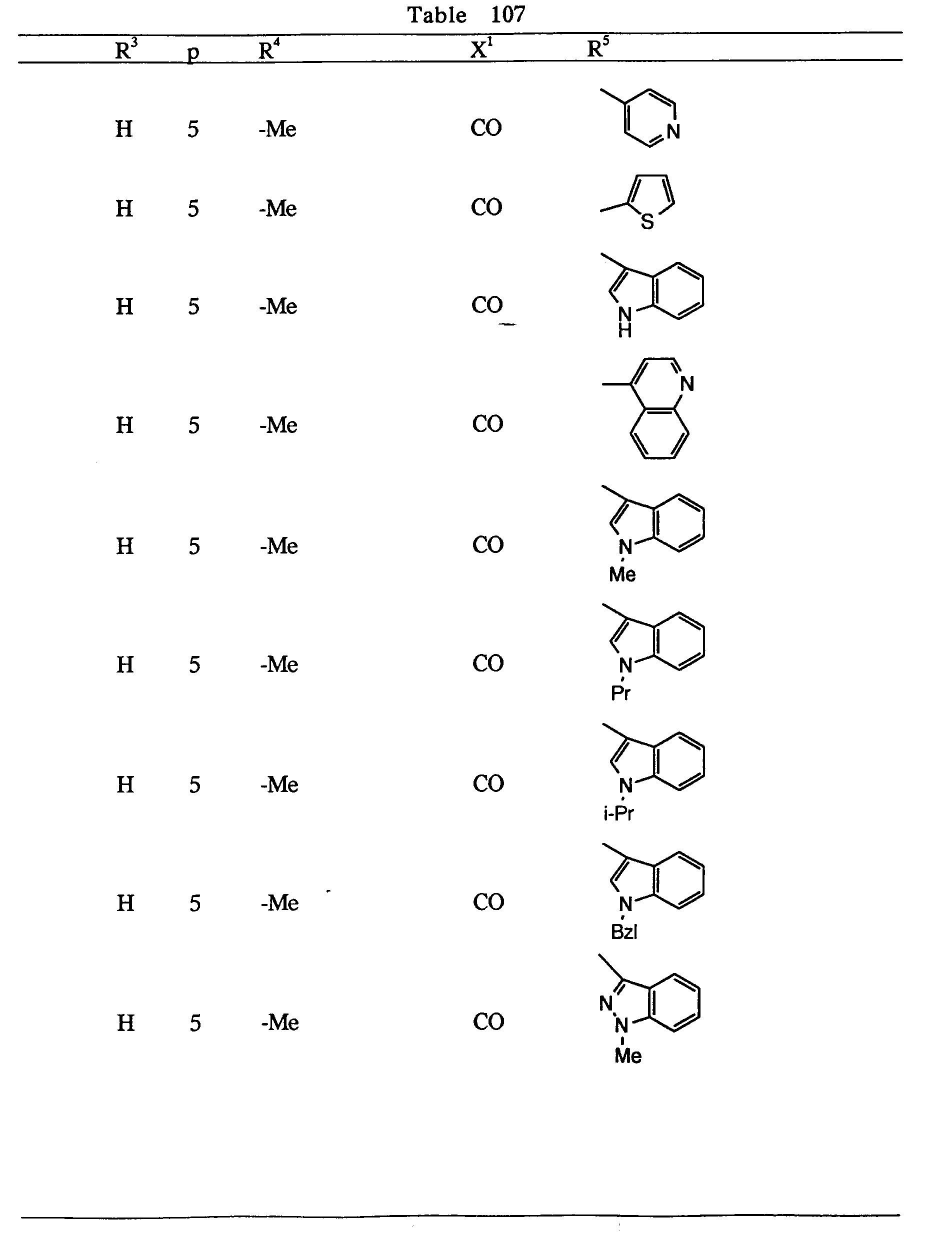

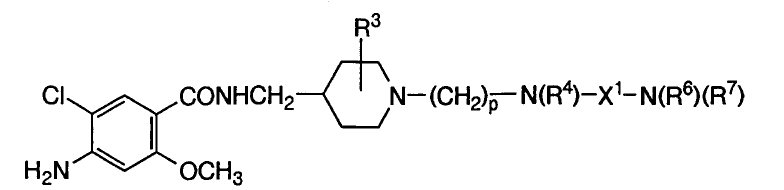

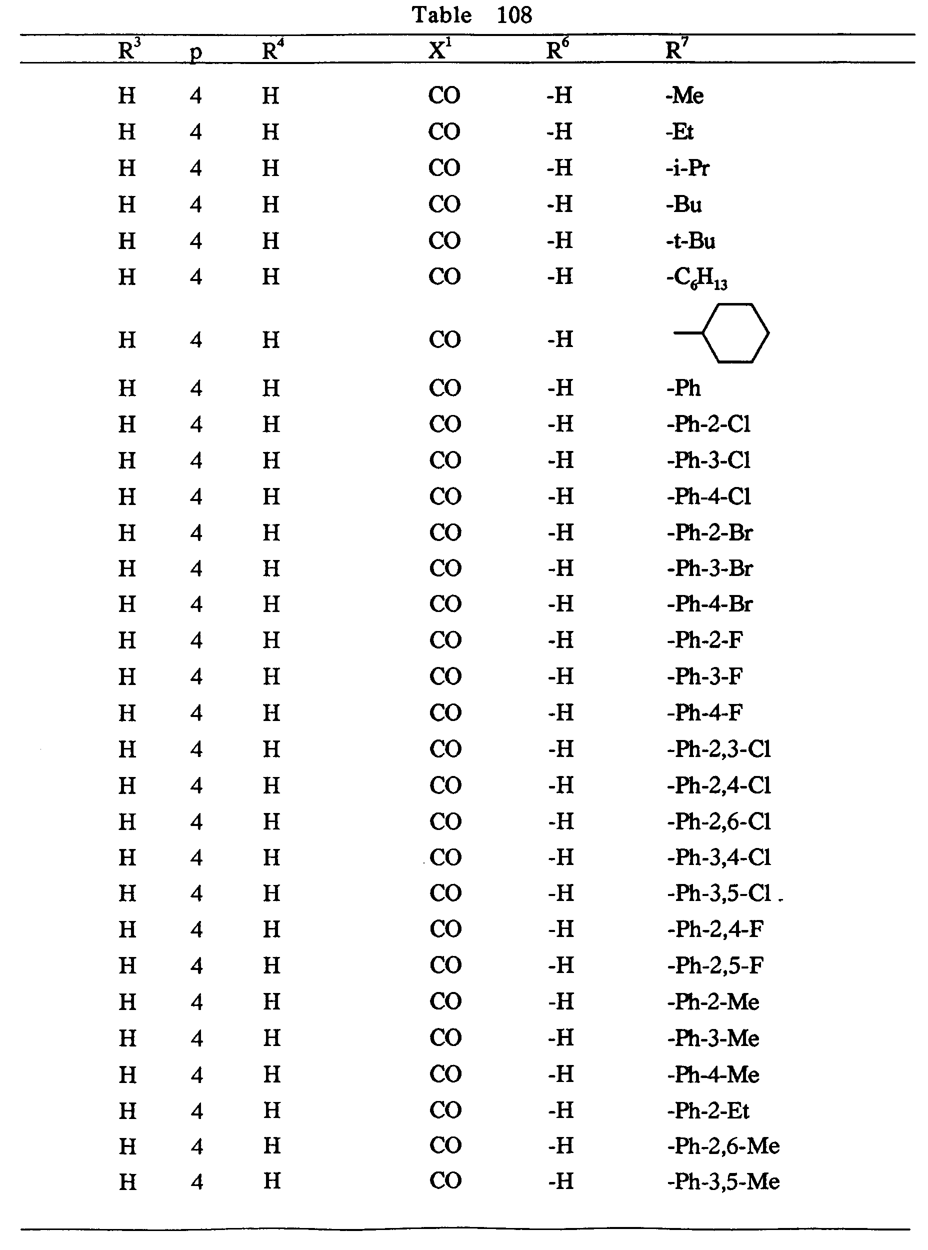

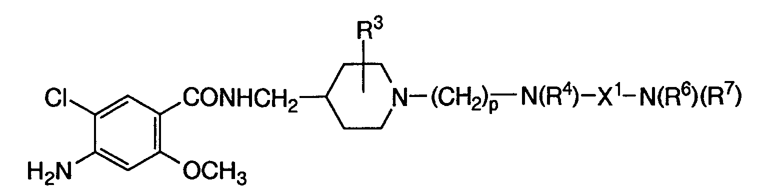

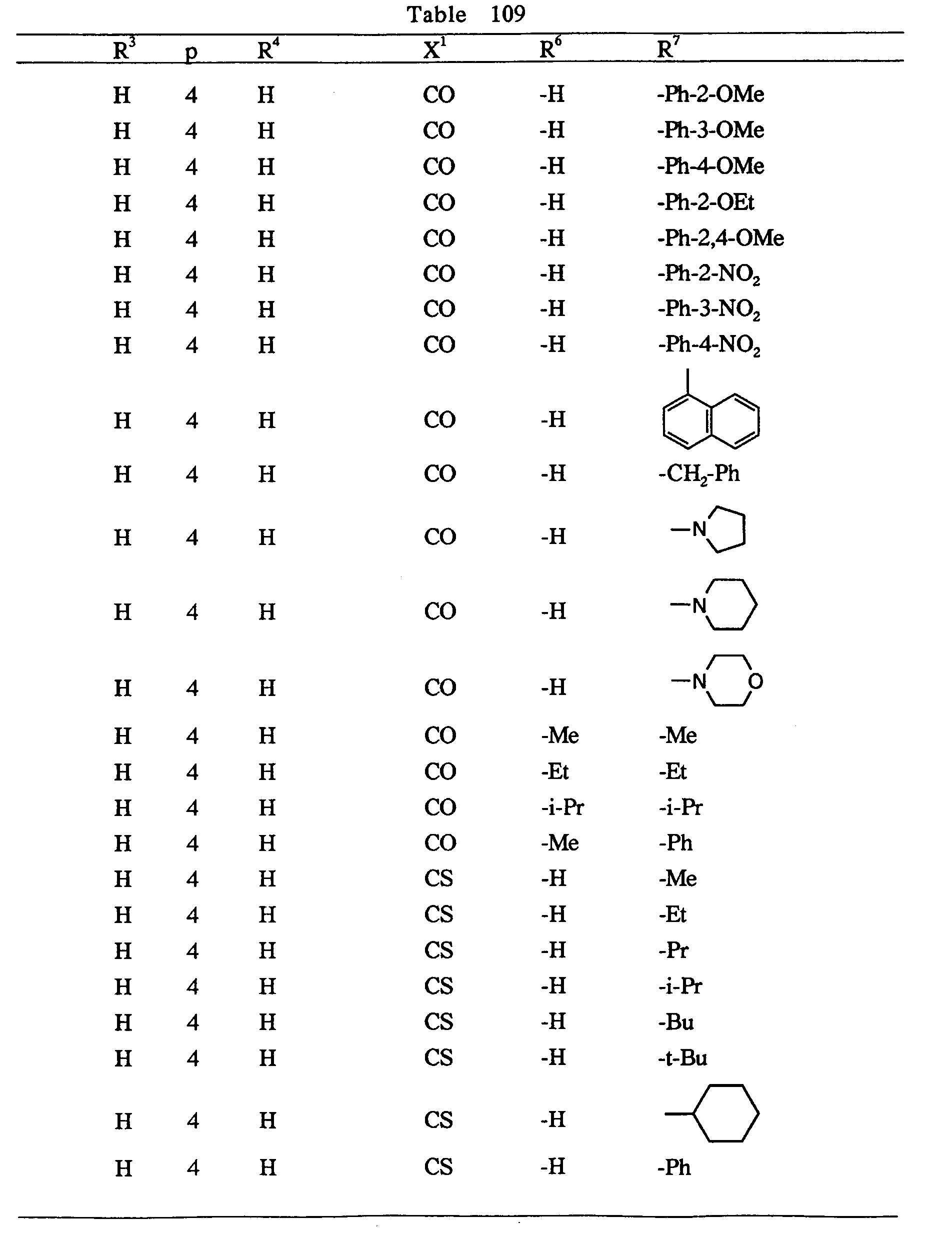

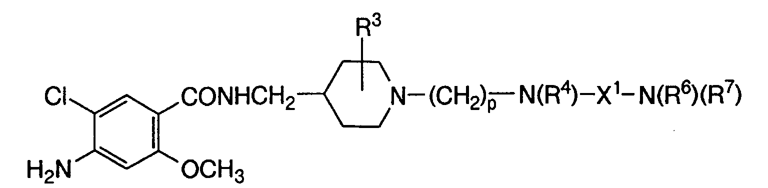

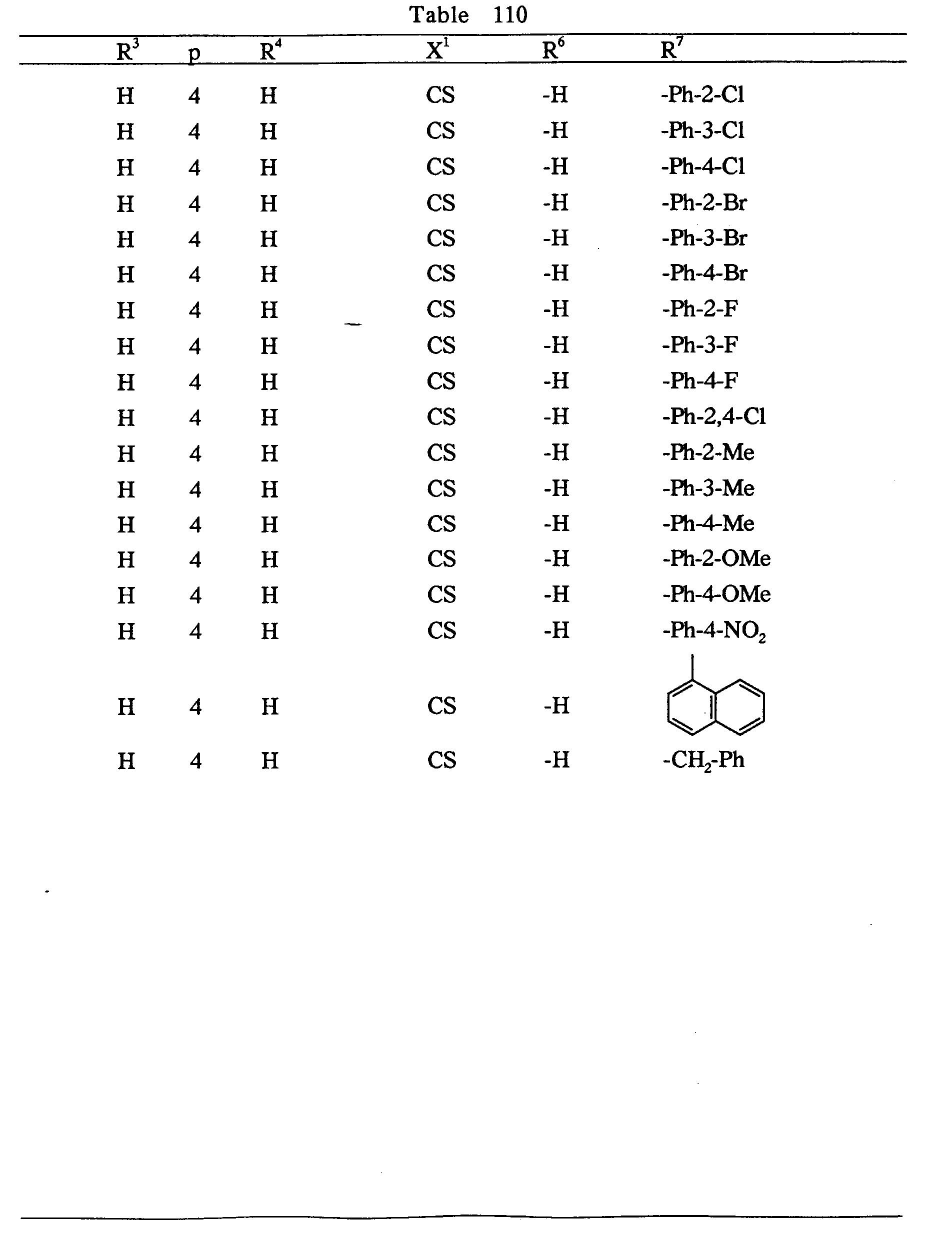

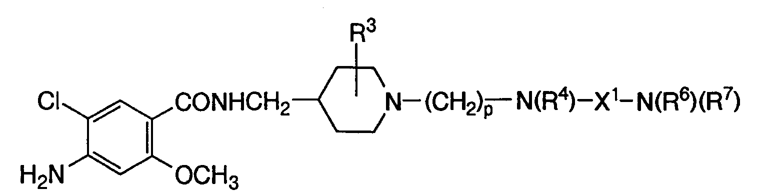

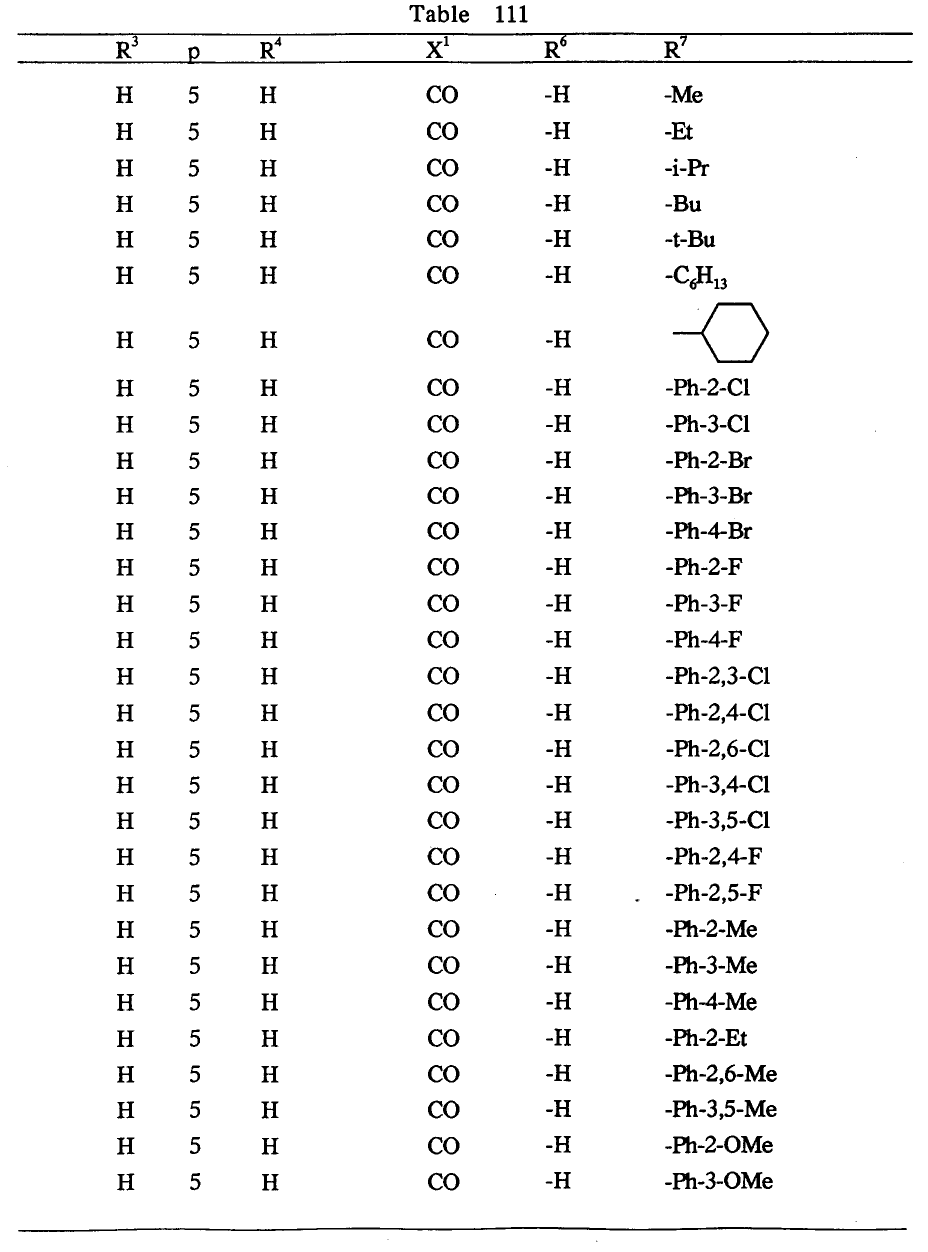

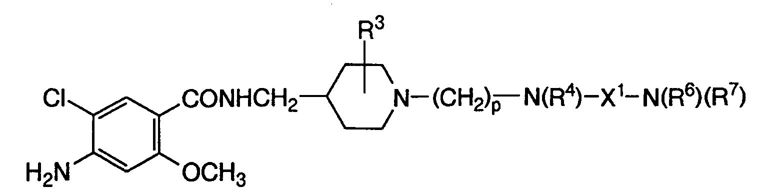

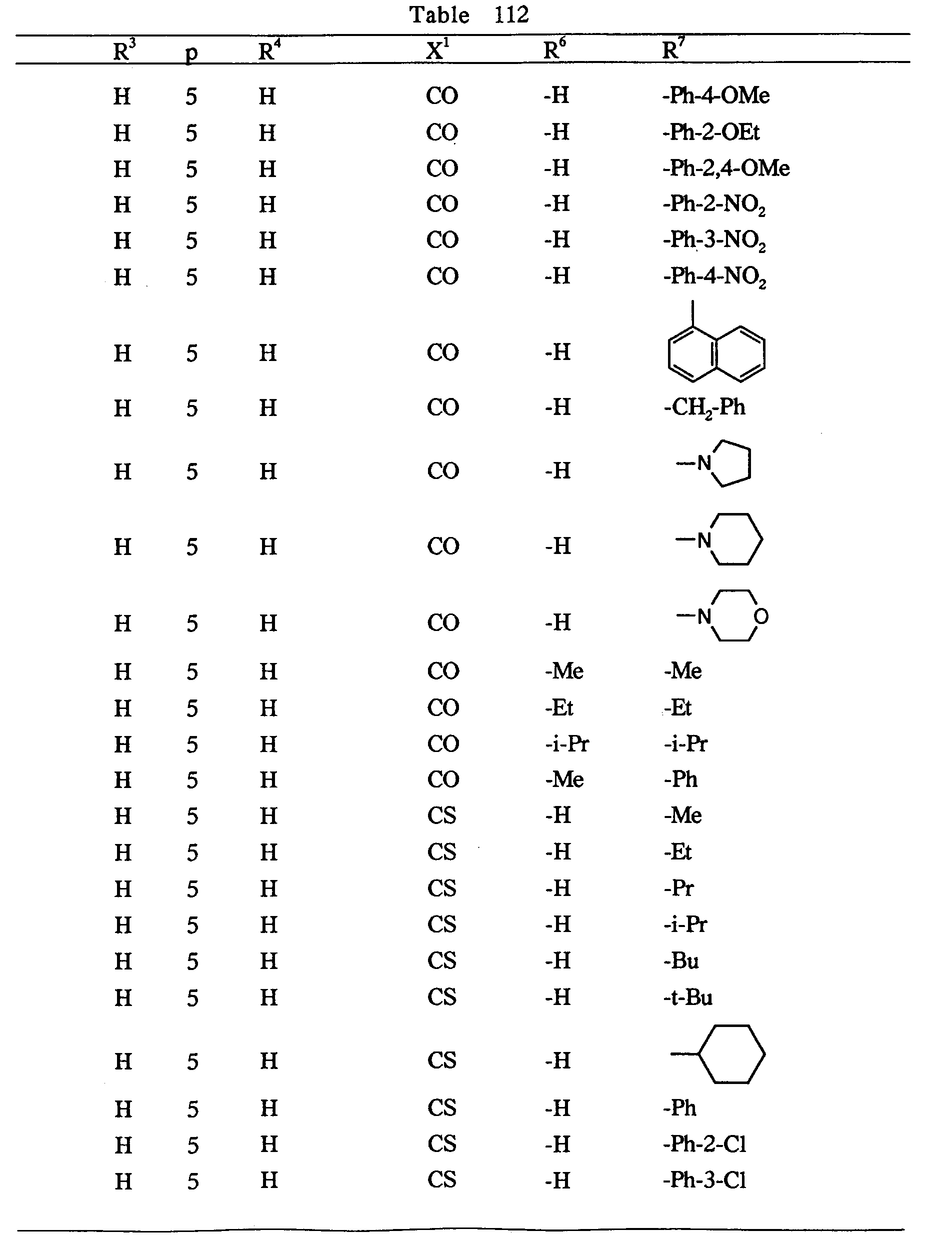

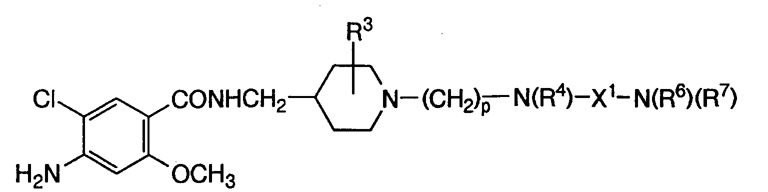

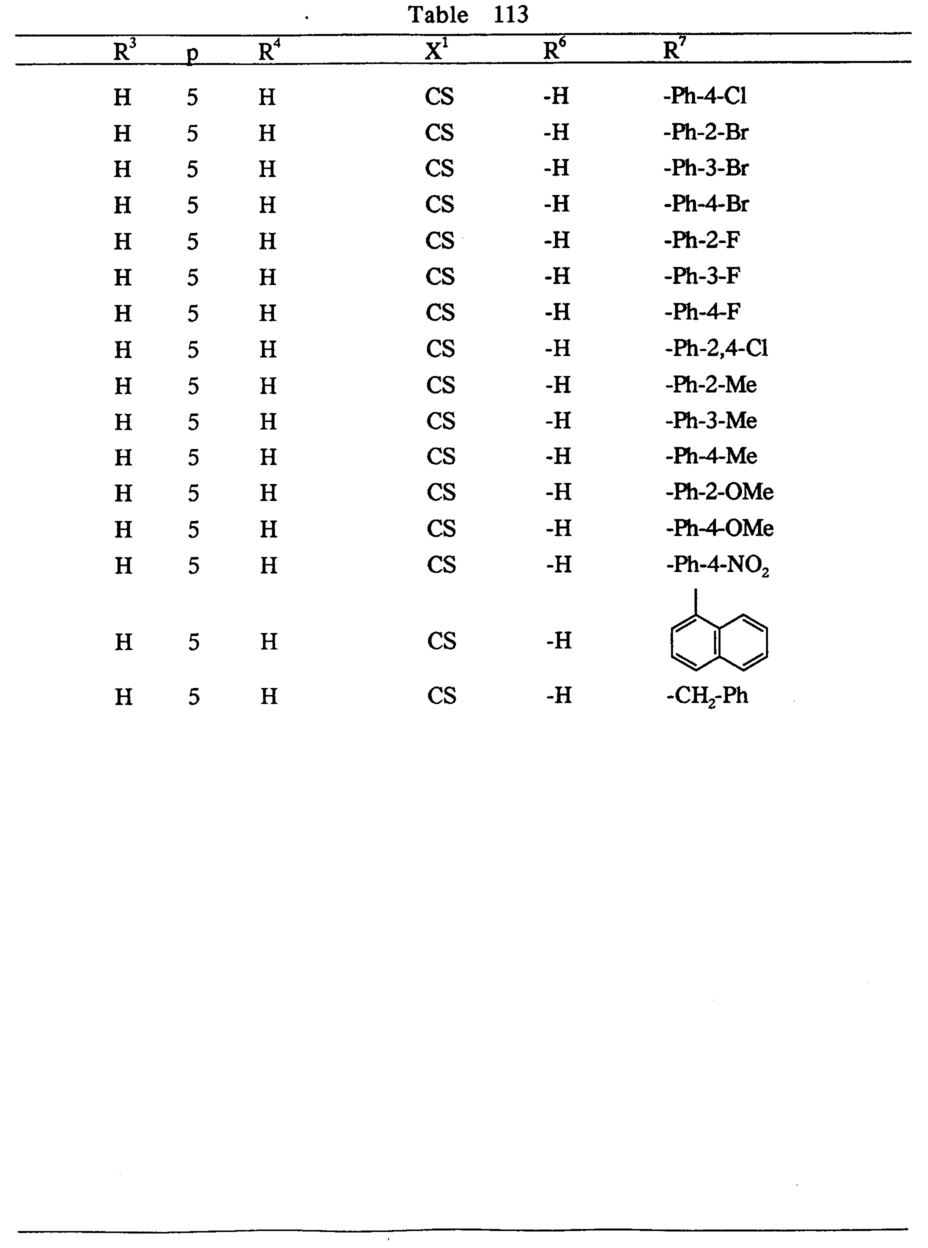

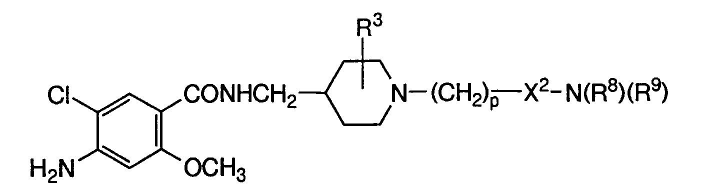

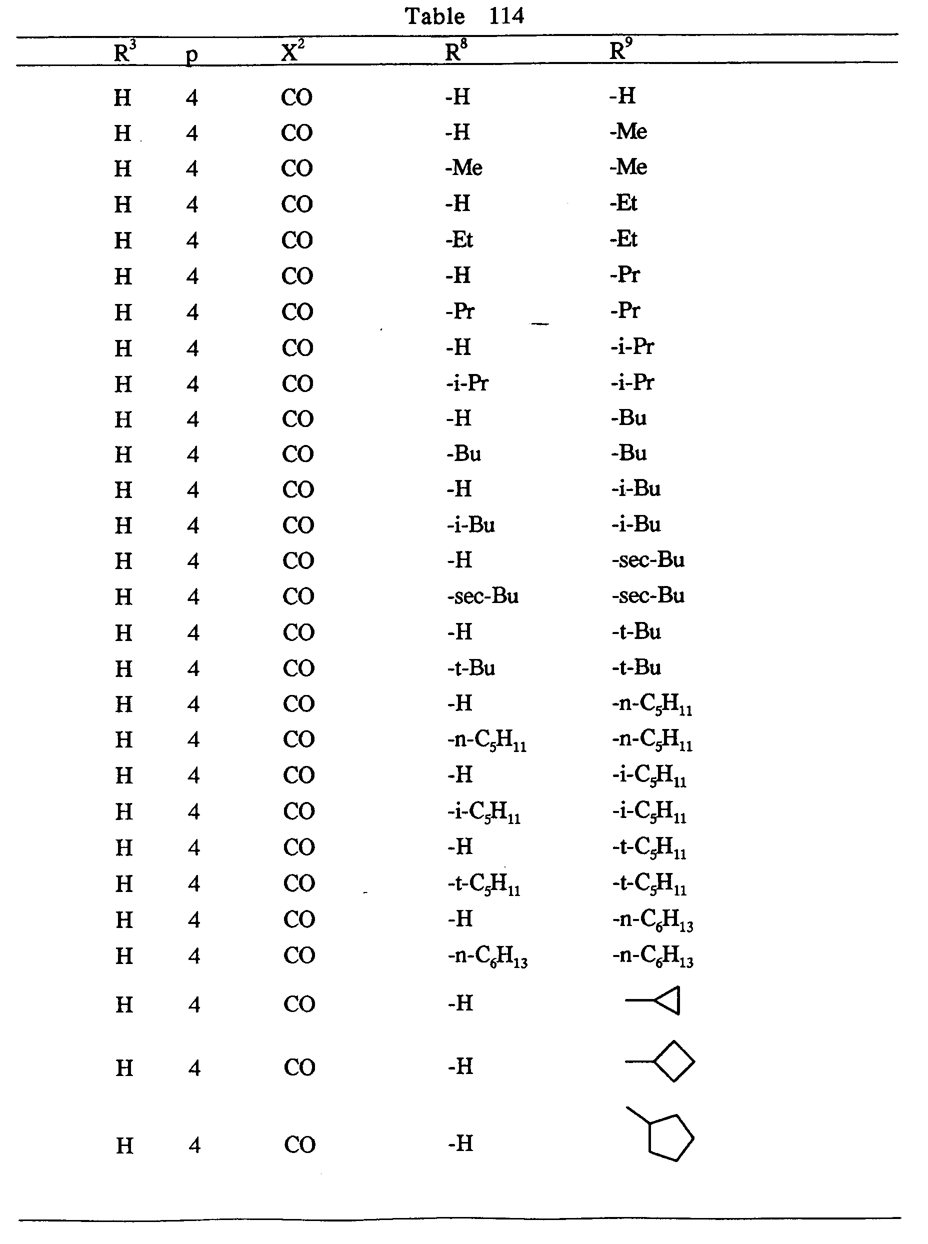

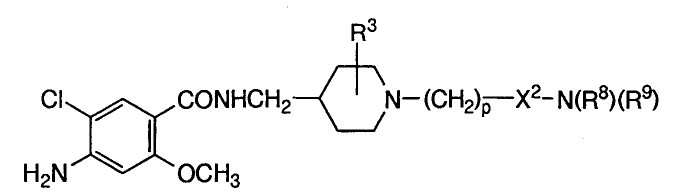

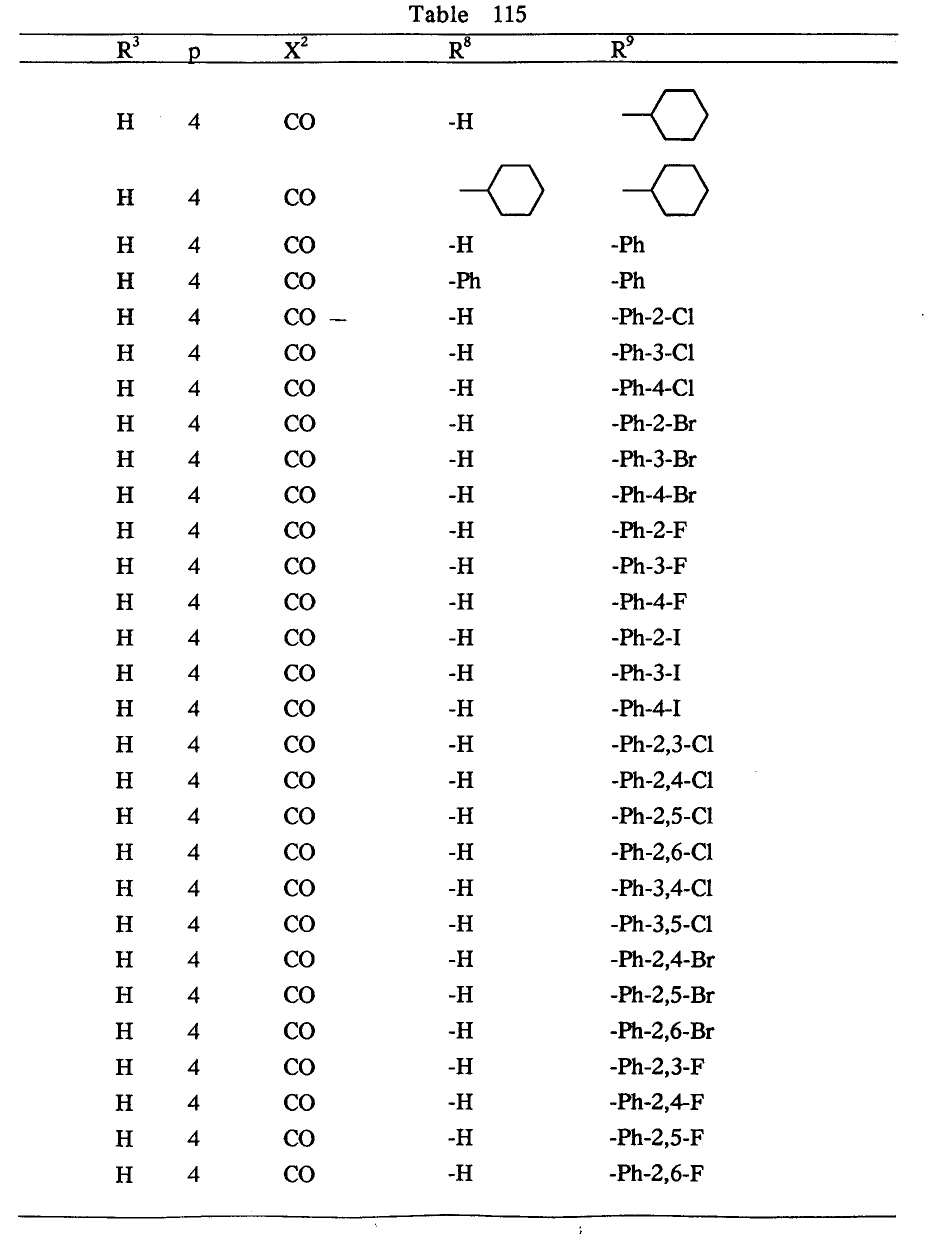

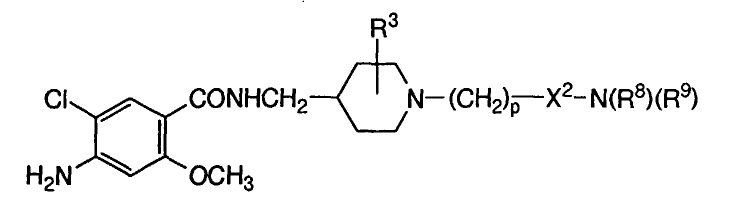

**Table 116**

| R³ | p | X² | R⁸ | R⁹ |
|---|---|---|---|---|
| H | 4 | CO | -H | -Ph-3,4-F |
| H | 4 | CO | -H | -Ph-3,5-F |
| H | 4 | CO | -H | -Ph-2-Cl-4-F |
| H | 4 | CO | -H | -Ph-3-Cl-4-F |
| H | 4 | CO | -H | -Ph-4-Cl-2-F |
| H | 4 | CO | -H | -Ph-2-Br-4-F |
| H | 4 | CO | -H | -Ph-4-Br-2-F |
| H | 4 | CO | -H | -Ph-2-Cl-4-Br |
| H | 4 | CO | -H | -Ph-2-F-4-I |
| H | 4 | CO | -H | -Ph-2-Me |
| H | 4 | CO | -H | -Ph-3-Me |
| H | 4 | CO | -H | -Ph-4-Me |
| H | 4 | CO | -H | -Ph-2-Et |
| H | 4 | CO | -H | -Ph-3-Et |
| H | 4 | CO | -H | -Ph-4-Et |
| H | 4 | CO | -H | -Ph-2-Pr |
| H | 4 | CO | -H | -Ph-4-Pr |
| H | 4 | CO | -H | -Ph-2-i-Pr |
| H | 4 | CO | -H | -Ph-4-i-Pr |
| H | 4 | CO | -H | -Ph-4-Bu |
| H | 4 | CO | -H | -Ph-2-t-Bu |
| H | 4 | CO | -H | -Ph-4-t-Bu |
| H | 4 | CO | -H | -Ph-4-sec-Bu |
| H | 4 | CO | -H | -Ph-2,3-Me |
| H | 4 | CO | -H | -Ph-2,4-Me |
| H | 4 | CO | -H | -Ph-2,5-Me |
| H | 4 | CO | -H | -Ph-2,6-Me |
| H | 4 | CO | -H | -Ph-3,4-Me |
| H | 4 | CO | -H | -Ph-3,5-Me |
| H | 4 | CO | -H | -Ph-2,4,6-Me |
| H | 4 | CO | -H | -Ph-2,6-Et |

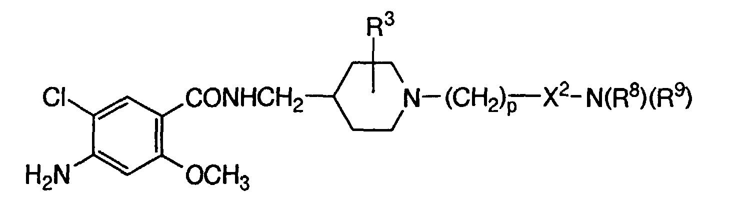

**Table 117**

| R³ | p | X² | R⁸ | R⁹ |
|---|---|---|---|---|
| H | 4 | CO | -H | -Ph-2-Me-6-Et |
| H | 4 | CO | -H | -Ph-2-Me-6-i-Pr |
| H | 4 | CO | -H | -Ph-2-OMe |
| H | 4 | CO | -H | -Ph-3-OMe |
| H | 4 | CO | -H | -Ph-4-OMe |
| H | 4 | CO | -H | -Ph-2-OEt |
| H | 4 | CO | -H | -Ph-3-OEt |
| H | 4 | CO | -H | -Ph-4-OEt |
| H | 4 | CO | -H | -Ph-2-OPr |
| H | 4 | CO | -H | -Ph-3-OPr |
| H | 4 | CO | -H | -Ph-4-OPr |
| H | 4 | CO | -H | -Ph-2-O-i-Pr |
| H | 4 | CO | -H | -Ph-3-O-i-Pr |
| H | 4 | CO | -H | -Ph-4-O-i-Pr |
| H | 4 | CO | -H | -Ph-4-O-n-Bu |
| H | 4 | CO | -H | -Ph-2-CH₂Ph |
| H | 4 | CO | -H | -Ph-2-OH |
| H | 4 | CO | -H | -Ph-3-OH |
| H | 4 | CO | -H | -Ph-4-OH |
| H | 4 | CO | -H | -Ph-2-NO₂ |
| H | 4 | CO | -H | -Ph-3-NO₂ |
| H | 4 | CO | -H | -Ph-4-NO₂ |
| H | 4 | CO | -H | -Ph-2-NH₂ |
| H | 4 | CO | -H | -Ph-3-NH₂ |
| H | 4 | CO | -H | -Ph-4-NH₂ |
| H | 4 | CO | -H | -Ph-2-OH-6-Me |
| H | 4 | CO | -H | -Ph-2-OH-5-Me |
| H | 4 | CO | -H | -Ph-3-OH-2-Me |
| H | 4 | CO | -H | -Ph-4-OH-2-Me |
| H | 4 | CO | -H | -Ph-2-OH-4-Me |
| H | 4 | CO | -H | -Ph-2-OMe-5-Me |

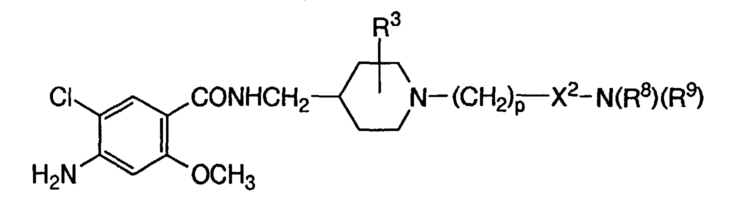

**Table 118**

| R³ | p | X² | R⁸ | R⁹ |
|---|---|---|---|---|
| H | 4 | CO | -H | -Ph-2-OMe-6-Me |
| H | 4 | CO | -H | -Ph-4-OMe-2-Me |
| H | 4 | CO | -H | -Ph-5-OMe-2-Me |
| H | 4 | CO | -H | -Ph-5-t-Bu-2-OH |
| H | 4 | CO | -H | -Ph-3-Cl-4-Me |
| H | 4 | CO | -H | -Ph-2-Cl-4-Me |
| H | 4 | CO | -H | -Ph-2-Cl-5-Me |
| H | 4 | CO | -H | -Ph-2-Cl-6-Me |
| H | 4 | CO | -H | -Ph-3-Cl-2-Me |
| H | 4 | CO | -H | -Ph-4-Cl-2-Me |
| H | 4 | CO | -H | -Ph-5-Cl-2-Me |
| H | 4 | CO | -H | -Ph-2-Br-4-Me |
| H | 4 | CO | -H | -Ph-3-Br-4-Me |
| H | 4 | CO | -H | -Ph-4-Br-2-Me |
| H | 4 | CO | -H | -Ph-4-Br-3-Me |
| H | 4 | CO | -H | -Ph-2-F-4-Me |
| H | 4 | CO | -H | -Ph-2-F-5-Me |
| H | 4 | CO | -H | -Ph-3-F-2-Me |
| H | 4 | CO | -H | -Ph-3-F-4-Me |
| H | 4 | CO | -H | -Ph-4-F-2-Me |
| H | 4 | CO | -H | -Ph-5-F-2-Me |
| H | 4 | CO | -H | -Ph-3-Cl-4-OMe |
| H | 4 | CO | -H | -Ph-5-Cl-2-OMe |
| H | 4 | CO | -H | -Ph-2-Cl-5-OMe |
| H | 4 | CO | -H | -Ph-3-F-2-OMe |
| H | 4 | CO | -H | -Ph-3-F-4-OMe |
| H | 4 | CO | -H | -Ph-5-Cl-2-OH |
| H | 4 | CO | -H | -Ph-2-Cl-4-OH |
| H | 4 | CO | -H | -Ph-2-Cl-4-NO₂ |
| H | 4 | CO | -H | -Ph-2-Cl-5-NO₂ |
| H | 4 | CO | -H | -Ph-4-Cl-2-NO₂ |

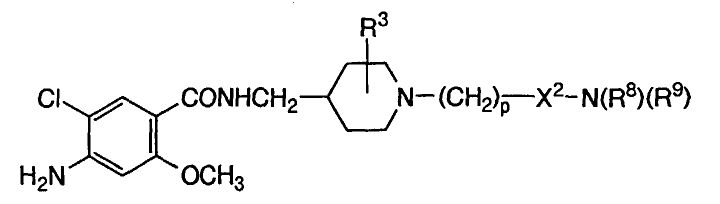

**Table 119**

| R³ | p | X² | R⁸ | R⁹ |
|---|---|---|---|---|
| H | 4 | CO | -H | -Ph-4-Cl-3-NO₂ |
| H | 4 | CO | -H | -Ph-5-Cl-2-NO₂ |
| H | 4 | CO | -H | -Ph-2-F-5-NO₂ |
| H | 4 | CO | -H | -Ph-4-F-2-NO₂ |
| H | 4 | CO | -H | -Ph-4-F-3-NO₂ |
| H | 4 | CO | -H | -Ph-4-OH-2,5-Me |
| H | 4 | CO | -H | -Ph-2-OH-3,5-Me |
| H | 4 | CO | -H | -Ph-4-OH-2,5-Me |
| H | 4 | CO | -H | -Ph-2-Cl-4,6-Me |
| H | 4 | CO | -H | -Ph-4-Br-2,6-Me |
| H | 4 | CO | -H | -CH₂Ph |
| H | 4 | CO | -CH₂Ph | -CH₂Ph |
| H | 4 | CO | -H | -CHPh₂ |
| H | 4 | CO | -H | -(CH₂)₂Ph |
| H | 4 | CO | -H | -CH(CH₃)Ph |
| H | 4 | CO | -H | -(CH₂)₃Ph |
| H | 4 | CO | -H | -CH₂CH(CH₃)Ph |
| H | 4 | CO | -H | -(CH₂)₄Ph |
| H | 4 | CO | -H | -CH(CH₃)-(CH₂)₂Ph |
| H | 4 | CO | -H | -CH₂Ph-2-Cl |
| H | 4 | CO | -H | -CH₂Ph-3-Cl |
| H | 4 | CO | -H | -CH₂Ph-4-Cl |
| H | 4 | CO | -H | -CH₂Ph-2-Br |
| H | 4 | CO | -H | -CH₂Ph-3-Br |
| H | 4 | CO | -H | -CH₂Ph-4-Br |
| H | 4 | CO | -H | -CH₂Ph-2-F |
| H | 4 | CO | -H | -CH₂Ph-3-F |
| H | 4 | CO | -H | -CH₂Ph-4-F |
| H | 4 | CO | -H | -CH₂Ph-3-I |
| H | 4 | CO | -H | -CH₂Ph-2-Me |
| H | 4 | CO | -H | -CH₂Ph-3-Me |

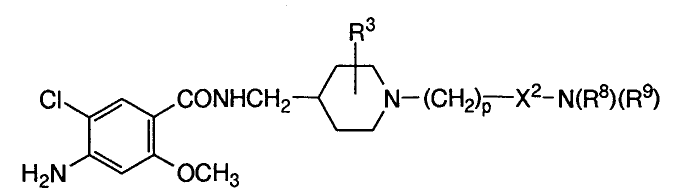

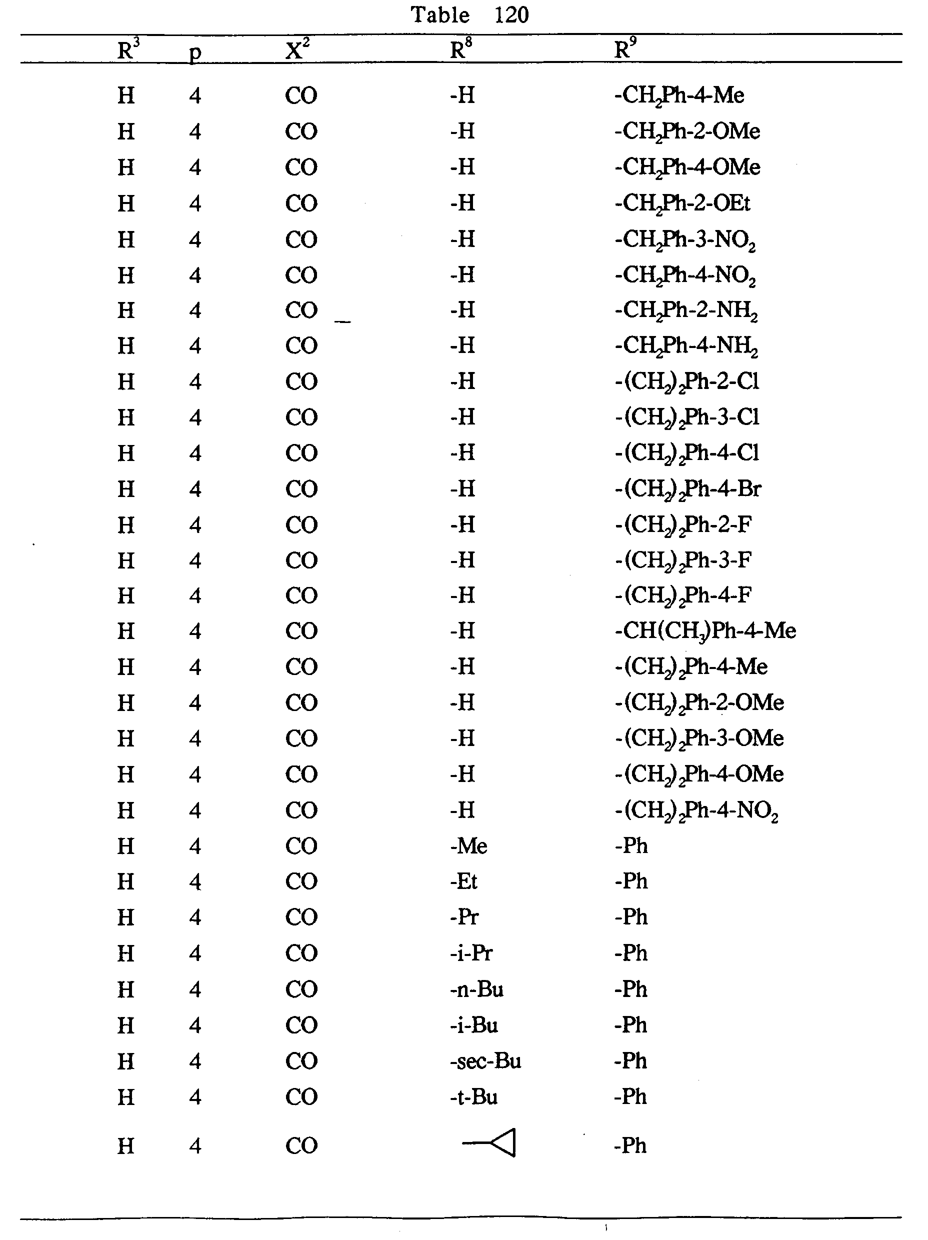

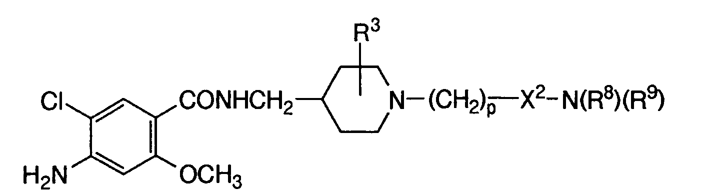

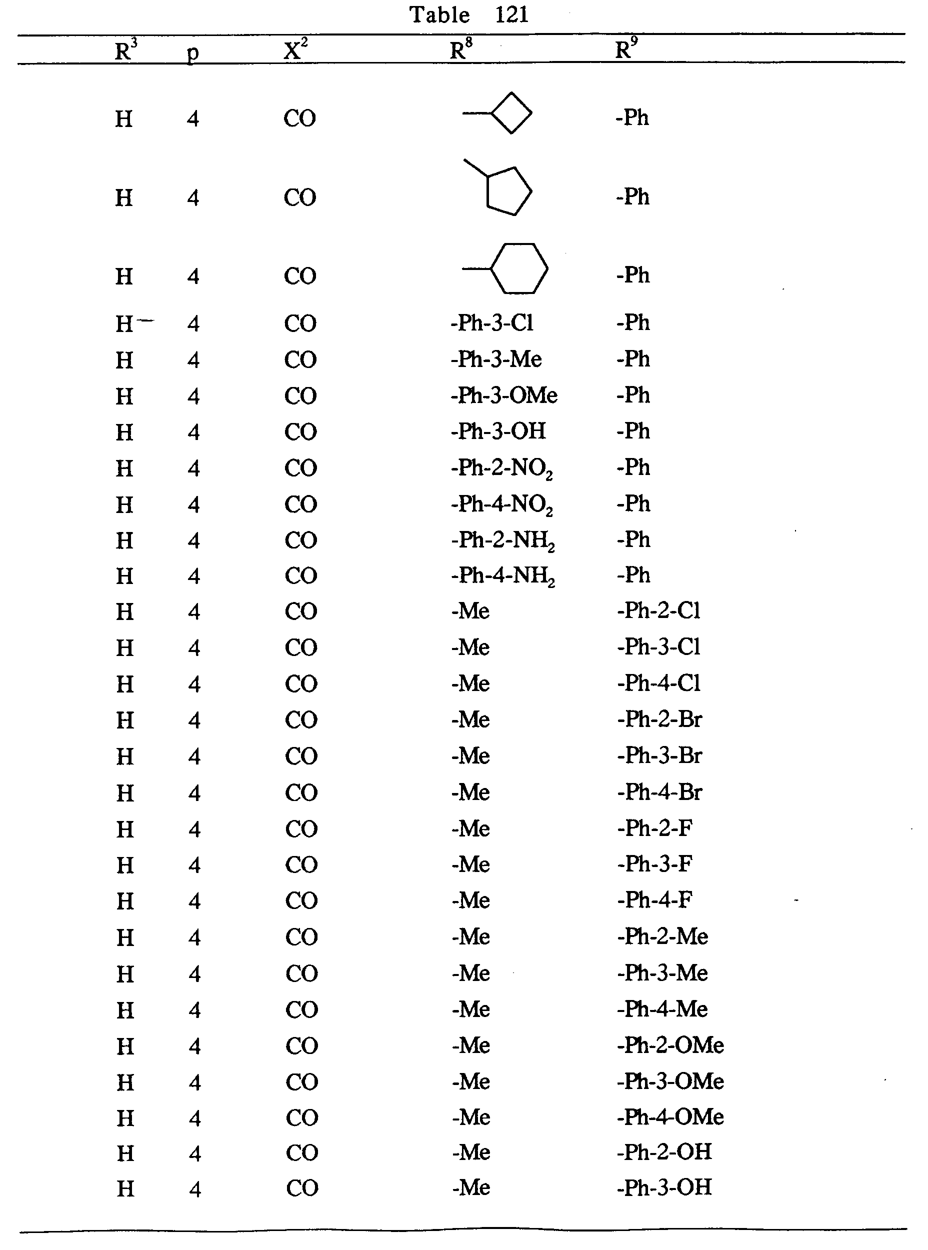

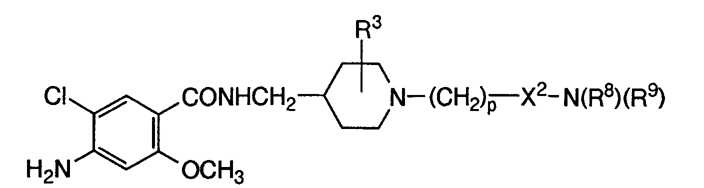

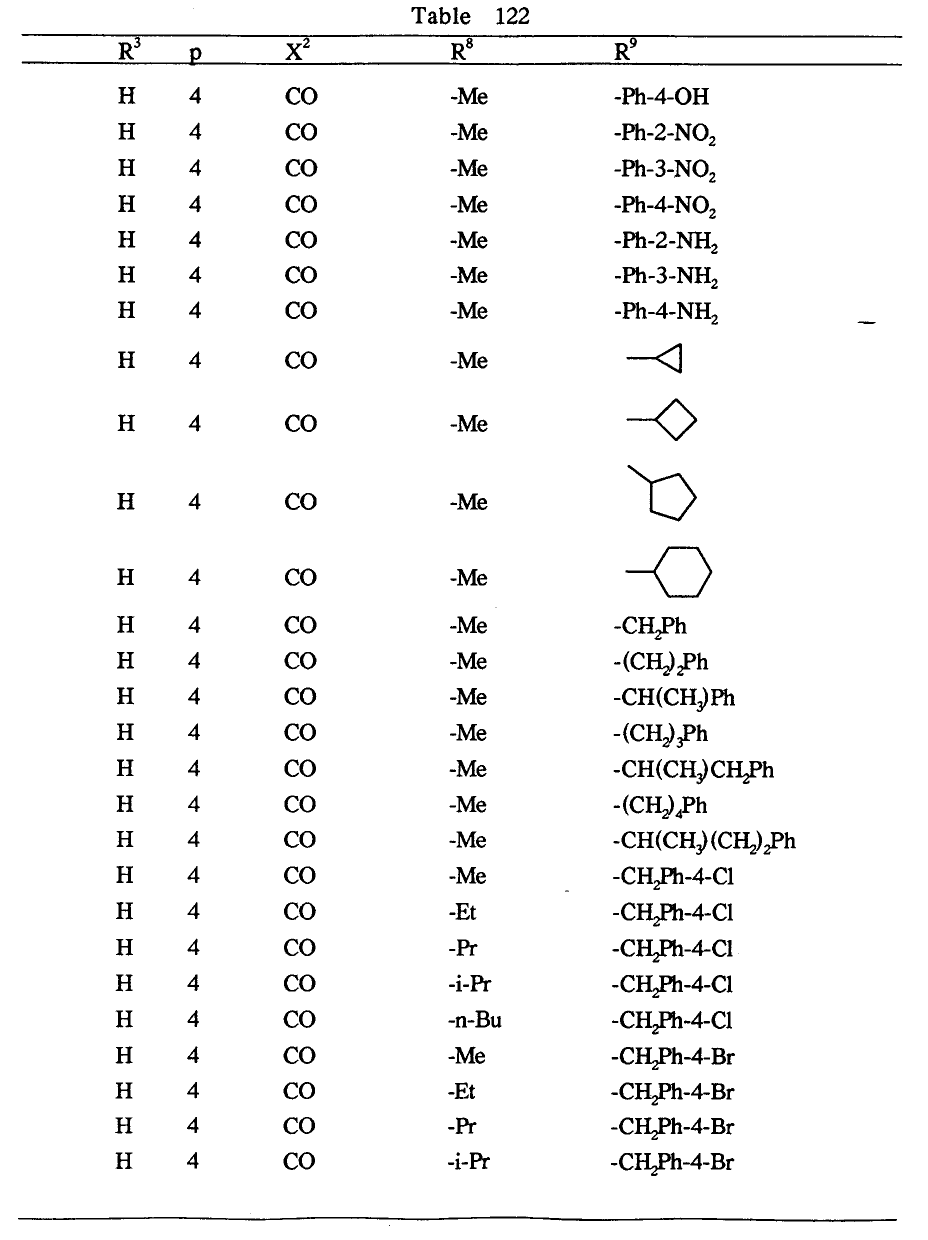

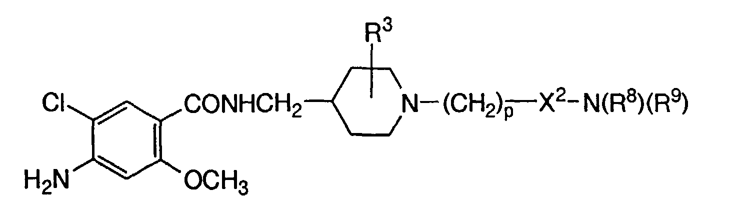

**Table 123**

| R³ | p | X² | R⁸ | R⁹ |
|---|---|---|---|---|
| H | 4 | CO | -n-Bu | -CH₂Ph-4-Br |
| H | 4 | CO | -Me | -CH₂Ph-4-F |
| H | 4 | CO | -Et | -CH₂Ph-4-F |
| H | 4 | CO | -Pr | -CH₂Ph-4-F |
| H | 4 | CO | -i-Pr | -CH₂Ph-4-F |
| H | 4 | CO | -n-Bu | -CH₂Ph-4-F |
| H | 4 | CO | -Me | -CH₂Ph-4-Me |
| H | 4 | CO | -Et | -CH₂Ph-4-Me |
| H | 4 | CO | -Pr | -CH₂Ph-4-Me |
| H | 4 | CO | -i-Pr | -CH₂Ph-4-Me |
| H | 4 | CO | -n-Bu | -CH₂Ph-4-Me |
| H | 4 | CO | -Me | -CH₂Ph-4-OMe |
| H | 4 | CO | -Et | -CH₂Ph-4-OMe |
| H | 4 | CO | -Pr | -CH₂Ph-4-OMe |
| H | 4 | CO | -i-Pr | -CH₂Ph-4-OMe |
| H | 4 | CO | -n-Bu | -CH₂Ph-4-OMe |
| H | 4 | CO | -Me | -CH₂Ph-4-NO₂ |
| H | 4 | CO | -Et | -CH₂Ph-4-NO₂ |
| H | 4 | CO | -Pr | -CH₂Ph-4-NO₂ |
| H | 4 | CO | -i-Pr | -CH₂Ph-4-NO₂ |
| H | 4 | CO | -n-Bu | -CH₂Ph-4-NO₂ |
| H | 4 | CO | -Me | -CH₂Ph-4-NH₂ |
| H | 4 | CO | -Et | -CH₂Ph-4-NH₂ |
| H | 4 | CO | -Pr | -CH₂Ph-4-NH₂ |
| H | 4 | CO | -i-Pr | -CH₂Ph-4-NH₂ |
| H | 4 | CO | -n-Bu | -CH₂Ph-4-NH₂ |
| H | 4 | CO | -Me | -(CH₂)₂Ph-4-Cl |
| H | 4 | CO | -Et | -(CH₂)₂Ph-4-Cl |
| H | 4 | CO | -Pr | -(CH₂)₂Ph-4-Cl |
| H | 4 | CO | -i-Pr | -(CH₂)₂Ph-4-Cl |
| H | 4 | CO | -Bu | -(CH₂)₂Ph-4-Cl |

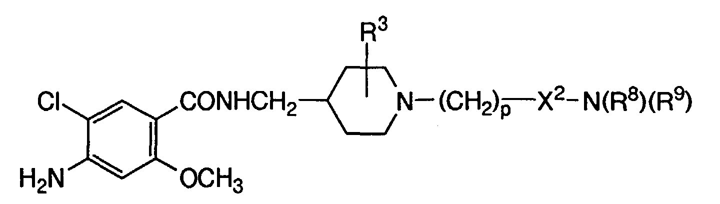

**Table 124**

| R³ | p | X² | R⁸ | R⁹ |
|---|---|---|---|---|
| H | 4 | CO | -Me | -(CH₂)₂Ph-4-Br |
| H | 4 | CO | -Et | -(CH₂)₂Ph-4-Br |
| H | 4 | CO | -Pr | -(CH₂)₂Ph-4-Br |
| H | 4 | CO | -i-Pr | -(CH₂)₂Ph-4-Br |
| H | 4 | CO | -Bu | -(CH₂)₂Ph-4-Br |
| H | 4 | CO | -Me | -(CH₂)₂Ph-4-F |
| H | 4 | CO | -Et | -(CH₂)₂Ph-4-F |
| H | 4 | CO | -Pr | -(CH₂)₂Ph-4-F |
| H | 4 | CO | -i-Pr | -(CH₂)₂Ph-4-F |
| H | 4 | CO | -Bu | -(CH₂)₂Ph-4-F |
| H | 4 | CO | -Me | -(CH₂)₂Ph-4-Me |
| H | 4 | CO | -Et | -(CH₂)₂Ph-4-Me |
| H | 4 | CO | -Pr | -(CH₂)₂Ph-4-Me |
| H | 4 | CO | -i-Pr | -(CH₂)₂Ph-4-Me |
| H | 4 | CO | -Bu | -(CH₂)₂Ph-4-Me |
| H | 4 | CO | -Me | -(CH₂)₂Ph-4-OMe |
| H | 4 | CO | -Et | -(CH₂)₂Ph-4-OMe |
| H | 4 | CO | -Pr | -(CH₂)₂Ph-4-OMe |
| H | 4 | CO | -i-Pr | -(CH₂)₂Ph-4-OMe |
| H | 4 | CO | -Bu | -(CH₂)₂Ph-4-OMe |
| H | 4 | CO | -Me | -(CH₂)₂Ph-4-NO₂ |
| H | 4 | CO | -Et | -(CH₂)₂Ph-4-NO₂ |
| H | 4 | CO | -Pr | -(CH₂)₂Ph-4-NO₂ |
| H | 4 | CO | -i-Pr | -(CH₂)₂Ph-4-NO₂ |
| H | 4 | CO | -Bu | -(CH₂)₂Ph-4-NO₂ |
| H | 4 | CO | -(CH₂)₄- | |
| H | 4 | CO | -(CH₂)₅- | |
| H | 4 | CO | -(CH₂)₂O(CH₂)₂- | |
| H | 4 | CO | -(CH₂)₂S(CH₂)₂- | |
| H | 4 | CO | -(CH₂)₂N(CH₃)(CH₂)₂- | |

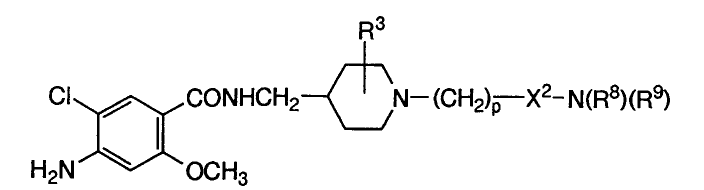

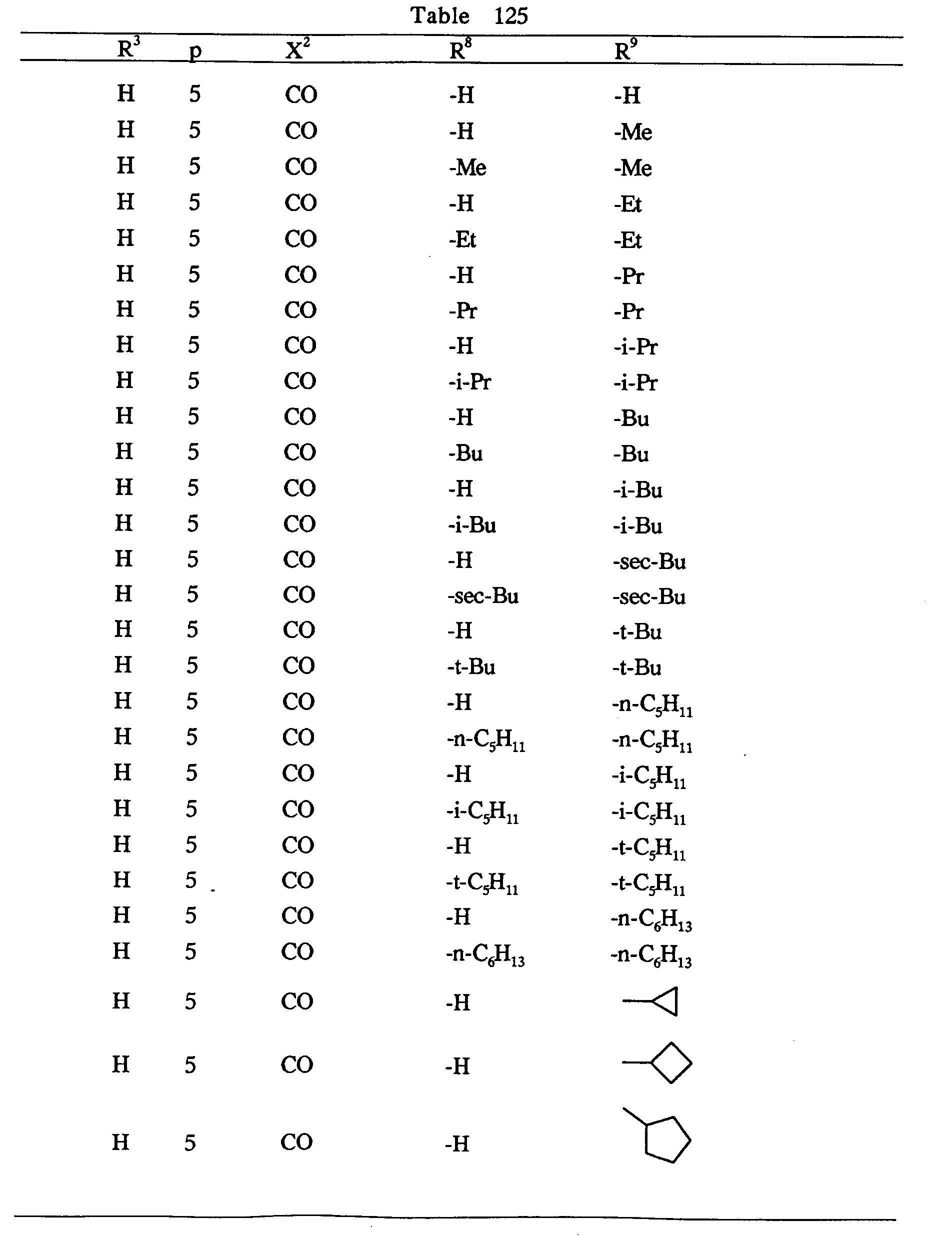

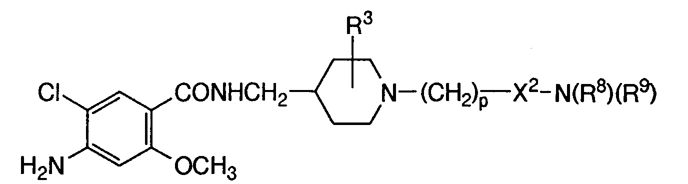

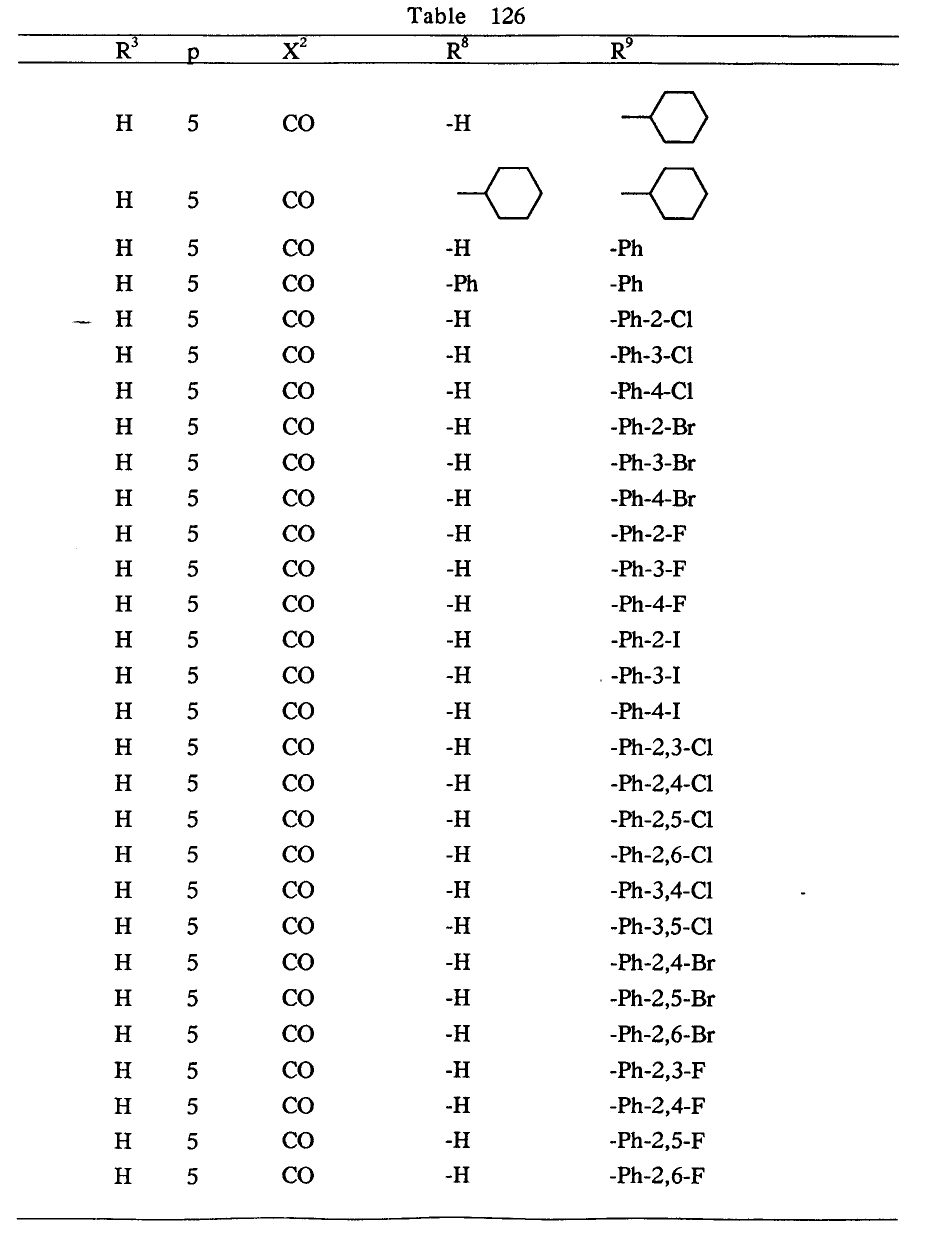

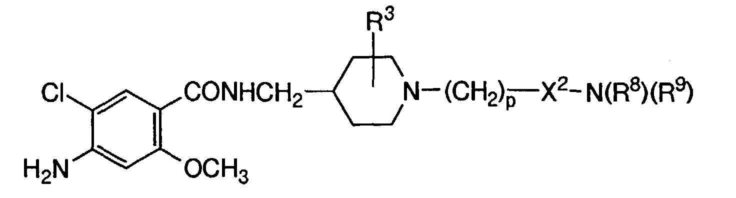

**Table 127**

| R³ | p | X² | R⁸ | R⁹ |
|---|---|---|---|---|
| H | 5 | CO | -H | -Ph-3,4-F |
| H | 5 | CO | -H | -Ph-3,5-F |
| H | 5 | CO | -H | -Ph-2-Cl-4-F |
| H | 5 | CO | -H | -Ph-3-Cl-4-F |
| H | 5 | CO | -H | -Ph-4-Cl-2-F |
| H | 5 | CO | -H | -Ph-2-Br-4-F |
| H | 5 | CO | -H | -Ph-4-Br-2-F |
| H | 5 | CO | -H | -Ph-2-Cl-4-Br |
| H | 5 | CO | -H | -Ph-2-F-4-I |
| H | 5 | CO | -H | -Ph-2-Me |
| H | 5 | CO | -H | -Ph-3-Me |
| H | 5 | CO | -H | -Ph-4-Me |
| H | 5 | CO | -H | -Ph-2-Et |
| H | 5 | CO | -H | -Ph-3-Et |
| H | 5 | CO | -H | -Ph-4-Et |
| H | 5 | CO | -H | -Ph-2-Pr |
| H | 5 | CO | -H | -Ph-4-Pr |
| H | 5 | CO | -H | -Ph-2-i-Pr |
| H | 5 | CO | -H | -Ph-4-i-Pr |
| H | 5 | CO | -H | -Ph-4-Bu |
| H | 5 | CO | -H | -Ph-2-t-Bu |
| H | 5 | CO | -H | -Ph-4-t-Bu |
| H | 5 | CO | -H | -Ph-4-sec-Bu |
| H | 5 | CO | -H | -Ph-2,3-Me |
| H | 5 | CO | -H | -Ph-2,4-Me |
| H | 5 | CO | -H | -Ph-2,5-Me |
| H | 5 | CO | -H | -Ph-2,6-Me |
| H | 5 | CO | -H | -Ph-3,4-Me |
| H | 5 | CO | -H | -Ph-3,5-Me |
| H | 5 | CO | -H | -Ph-2,4,6-Me |
| H | 5 | CO | -H | -Ph-2,6-Et |

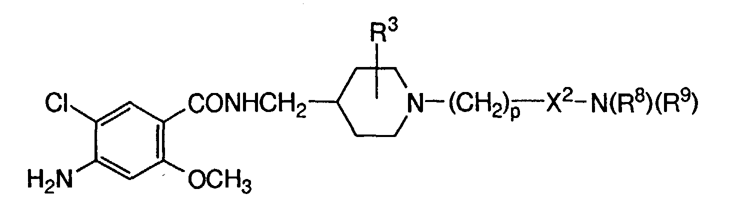

**Table 128**

| R³ | p | X² | R⁸ | R⁹ |
|---|---|---|---|---|
| H | 5 | CO | -H | -Ph-2-Me-6-Et |
| H | 5 | CO | -H | -Ph-2-Me-6-i-Pr |
| H | 5 | CO | -H | -Ph-2-OMe |
| H | 5 | CO | -H | -Ph-3-OMe |
| H | 5 | CO | -H | -Ph-4-OMe |
| H | 5 | CO | -H | -Ph-2-OEt |
| H | 5 | CO | -H | -Ph-3-OEt |
| H | 5 | CO | -H | -Ph-4-OEt |
| H | 5 | CO | -H | -Ph-2-OPr |
| H | 5 | CO | -H | -Ph-3-OPr |
| H | 5 | CO | -H | -Ph-4-OPr |
| H | 5 | CO | -H | -Ph-2-O-i-Pr |
| H | 5 | CO | -H | -Ph-3-O-i-Pr |
| H | 5 | CO | -H | -Ph-4-O-i-Pr |
| H | 5 | CO | -H | -Ph-4-O-n-Bu |
| H | 5 | CO | -H | -Ph-2-CH₂Ph |
| H | 5 | CO | -H | -Ph-2-OH |
| H | 5 | CO | -H | -Ph-3-OH |
| H | 5 | CO | -H | -Ph-4-OH |
| H | 5 | CO | -H | -Ph-2-NO₂ |
| H | 5 | CO | -H | -Ph-3-NO₂ |
| H | 5 | CO | -H | -Ph-4-NO₂ |
| H | 5 | CO | -H | -Ph-2-NH₂ |
| H | 5 | CO | -H | -Ph-3-NH₂ |
| H | 5 | CO | -H | -Ph-4-NH₂ |
| H | 5 | CO | -H | -Ph-2-OH-6-Me |
| H | 5 | CO | -H | -Ph-2-OH-5-Me |
| H | 5 | CO | -H | -Ph-3-OH-2-Me |
| H | 5 | CO | -H | -Ph-4-OH-2-Me |
| H | 5 | CO | -H | -Ph-2-OH-4-Me |
| H | 5 | CO | -H | -Ph-2-OMe-5-Me |

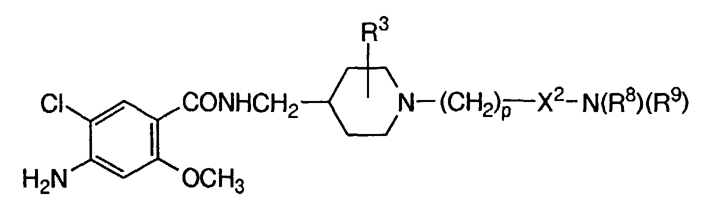

**Table 129**

| R³ | p | X² | R⁸ | R⁹ |
|---|---|---|---|---|
| H | 5 | CO | -H | -Ph-2-OMe-6-Me |
| H | 5 | CO | -H | -Ph-4-OMe-2-Me |
| H | 5 | CO | -H | -Ph-5-OMe-2-Me |
| H | 5 | CO | -H | -Ph-5-t-Bu-2-OH |
| H | 5 | CO | -H | -Ph-3-Cl-4-Me |
| H | 5 | CO | -H | -Ph-2-Cl-4-Me |
| H | 5 | CO | -H | -Ph-2-Cl-5-Me |
| H | 5 | CO | -H | -Ph-2-Cl-6-Me |
| H | 5 | CO | -H | -Ph-3-Cl-2-Me |
| H | 5 | CO | -H | -Ph-4-Cl-2-Me |
| H | 5 | CO | -H | -Ph-5-Cl-2-Me |
| H | 5 | CO | -H | -Ph-2-Br-4-Me |
| H | 5 | CO | -H | -Ph-3-Br-4-Me |
| H | 5 | CO | -H | -Ph-4-Br-2-Me |
| H | 5 | CO | -H | -Ph-4-Br-3-Me |
| H | 5 | CO | -H | -Ph-2-F-4-Me |
| H | 5 | CO | -H | -Ph-2-F-5-Me |
| H | 5 | CO | -H | -Ph-3-F-2-Me |
| H | 5 | CO | -H | -Ph-3-F-4-Me |
| H | 5 | CO | -H | -Ph-4-F-2-Me |
| H | 5 | CO | -H | -Ph-5-F-2-Me |
| H | 5 | CO | -H | -Ph-3-Cl-4-OMe |
| H | 5 | CO | -H | -Ph-5-Cl-2-OMe |
| H | 5 | CO | -H | -Ph-2-Cl-5-OMe |
| H | 5 | CO | -H | -Ph-3-F-2-OMe |
| H | 5 | CO | -H | -Ph-3-F-4-OMe |
| H | 5 | CO | -H | -Ph-5-Cl-2-OH |
| H | 5 | CO | -H | -Ph-2-Cl-4-OH |
| H | 5 | CO | -H | -Ph-2-Cl-4-NO₂ |
| H | 5 | CO | -H | -Ph-2-Cl-5-NO₂ |
| H | 5 | CO | -H | -Ph-4-Cl-2-NO₂ |

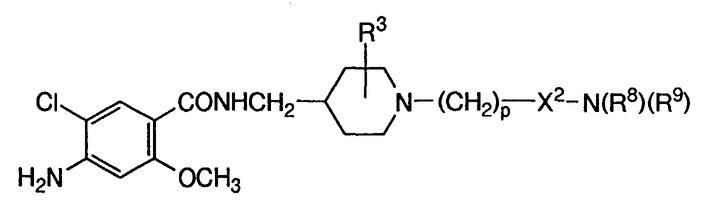

**Table 130**

| R³ | p | X² | R⁸ | R⁹ |
|---|---|---|---|---|
| H | 5 | CO | -H | -Ph-4-Cl-3-NO₂ |
| H | 5 | CO | -H | -Ph-5-Cl-2-NO₂ |
| H | 5 | CO | -H | -Ph-2-F-5-NO₂ |
| H | 5 | CO | -H | -Ph-4-F-2-NO₂ |
| H | 5 | CO | -H | -Ph-4-F-3-NO₂ |
| H | 5 | CO | -H | -Ph-4-OH-2,5-Me |
| H | 5 | CO | -H | -Ph-2-OH-3,5-Me |
| H | 5 | CO | -H | -Ph-4-OH-2,5-Me |
| H | 5 | CO | -H | -Ph-2-Cl-4,6-Me |
| H | 5 | CO | -H | -Ph-4-Br-2,6-Me |
| H | 5 | CO | -H | -CH₂Ph |
| H | 5 | CO | -CH₂Ph | -CH₂Ph |
| H | 5 | CO | -H | -CHPh₂ |
| H | 5 | CO | -H | -(CH₂)₂Ph |
| H | 5 | CO | -H | -CH(CH₃)Ph |
| H | 5 | CO | -H | -(CH₂)₃Ph |
| H | 5 | CO | -H | -CH₂CH(CH₃)Ph |
| H | 5 | CO | -H | -(CH₂)₄Ph |
| H | 5 | CO | -H | -CH(CH₃)-(CH₂)₂Ph |
| H | 5 | CO | -H | -CH₂Ph-2-Cl |
| H | 5 | CO | -H | -CH₂Ph-3-Cl |
| H | 5 | CO | -H | -CH₂Ph-4-Cl |
| H | 5 | CO | -H | -CH₂Ph-2-Br |
| H | 5 | CO | -H | -CH₂Ph-3-Br |
| H | 5 | CO | -H | -CH₂Ph-4-Br |
| H | 5 | CO | -H | -CH₂Ph-2-F |
| H | 5 | CO | -H | -CH₂Ph-3-F |
| H | 5 | CO | -H | -CH₂Ph-4-F |
| H | 5 | CO | -H | -CH₂Ph-3-I |
| H | 5 | CO | -H | -CH₂Ph-2-Me |
| H | 5 | CO | -H | -CH₂Ph-3-Me |

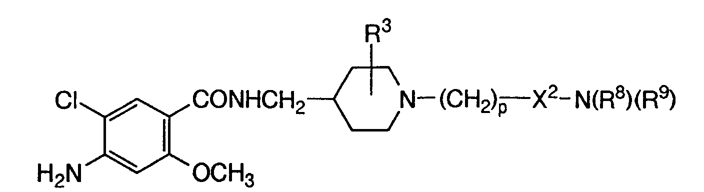

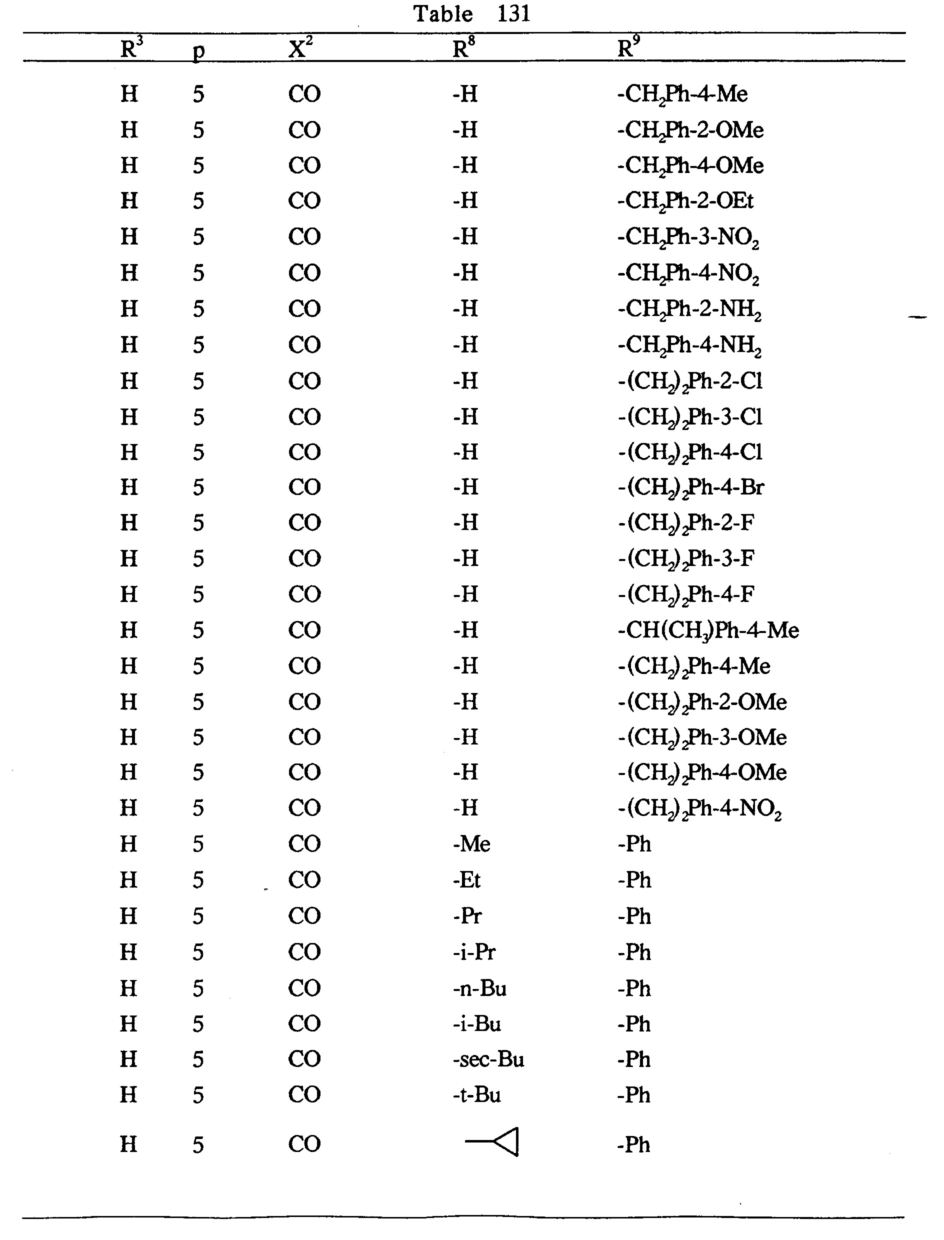

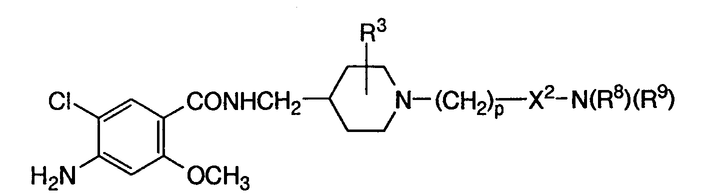

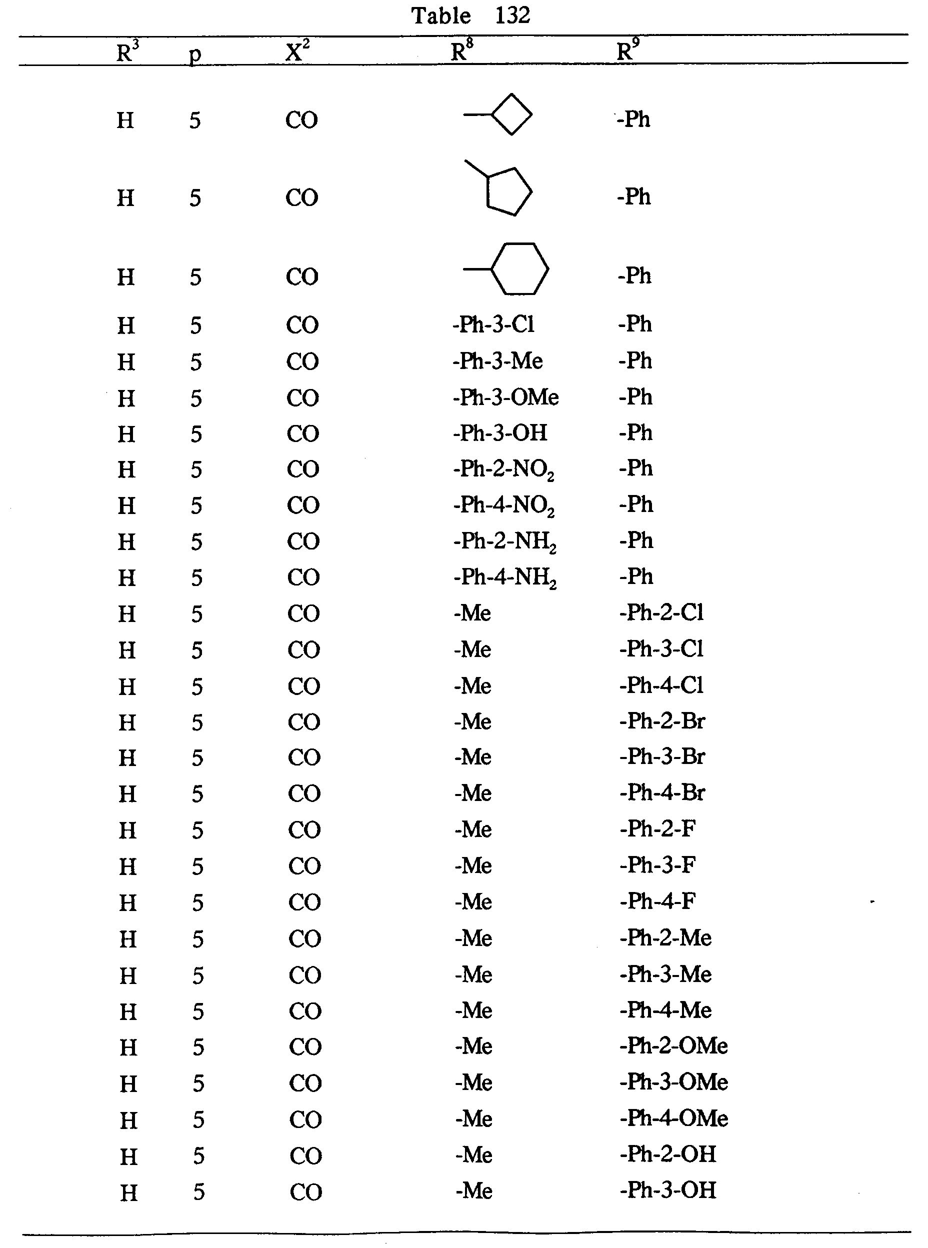

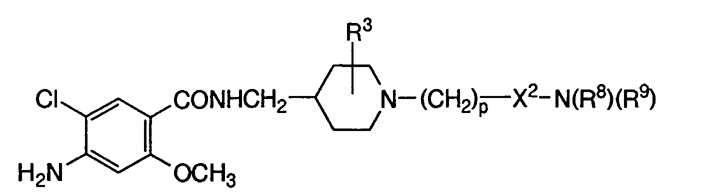

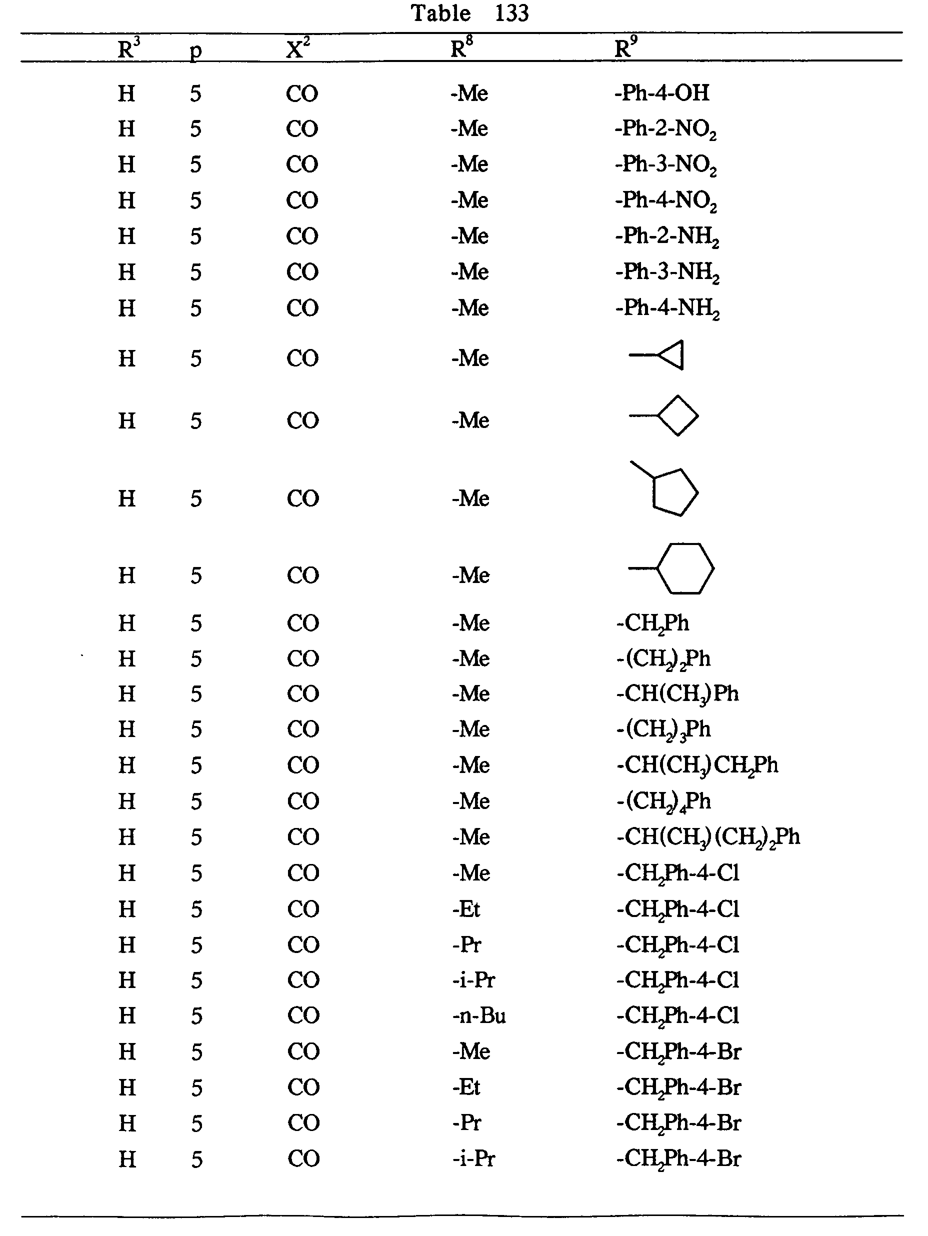

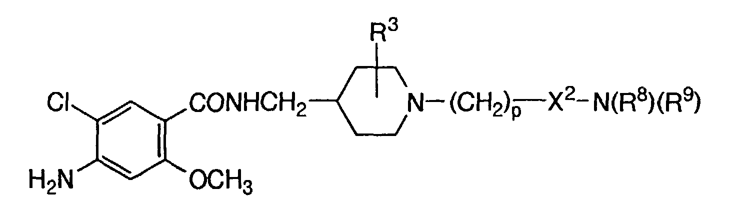

**Table 134**

| R³ | p | X² | R⁸ | R⁹ |
|---|---|---|---|---|
| H | 5 | CO | -n-Bu | -CH₂Ph-4-Br |
| H | 5 | CO | -Me | -CH₂Ph-4-F |
| H | 5 | CO | -Et | -CH₂Ph-4-F |
| H | 5 | CO | -Pr | -CH₂Ph-4-F |
| H | 5 | CO | -i-Pr | -CH₂Ph-4-F |
| H | 5 | CO | -n-Bu | -CH₂Ph-4-F |
| H | 5 | CO | -Me | -CH₂Ph-4-Me |
| H | 5 | CO | -Et | -CH₂Ph-4-Me |
| H | 5 | CO | -Pr | -CH₂Ph-4-Me |
| H | 5 | CO | -i-Pr | -CH₂Ph-4-Me |
| H | 5 | CO | -n-Bu | -CH₂Ph-4-Me |
| H | 5 | CO | -Me | -CH₂Ph-4-OMe |
| H | 5 | CO | -Et | -CH₂Ph-4-OMe |
| H | 5 | CO | -Pr | -CH₂Ph-4-OMe |
| H | 5 | CO | -i-Pr | -CH₂Ph-4-OMe |
| H | 5 | CO | -n-Bu | -CH₂Ph-4-OMe |
| H | 5 | CO | -Me | -CH₂Ph-4-NO₂ |
| H | 5 | CO | -Et | -CH₂Ph-4-NO₂ |
| H | 5 | CO | -Pr | -CH₂Ph-4-NO₂ |
| H | 5 | CO | -i-Pr | -CH₂Ph-4-NO₂ |
| H | 5 | CO | -n-Bu | -CH₂Ph-4-NO₂ |
| H | 5 | CO | -Me | -CH₂Ph-4-NH₂ |
| H | 5 | CO | -Et | -CH₂Ph-4-NH₂ |
| H | 5 | CO | -Pr | -CH₂Ph-4-NH₂ |
| H | 5 | CO | -i-Pr | -CH₂Ph-4-NH₂ |
| H | 5 | CO | -n-Bu | -CH₂Ph-4-NH₂ |
| H | 5 | CO | -Me | -(CH₂)₂Ph-4-Cl |
| H | 5 | CO | -Et | -(CH₂)₂Ph-4-Cl |
| H | 5 | CO | -Pr | -(CH₂)₂Ph-4-Cl |
| H | 5 | CO | -i-Pr | -(CH₂)₂Ph-4-Cl |
| H | 5 | CO | -Bu | -(CH₂)₂Ph-4-Cl |

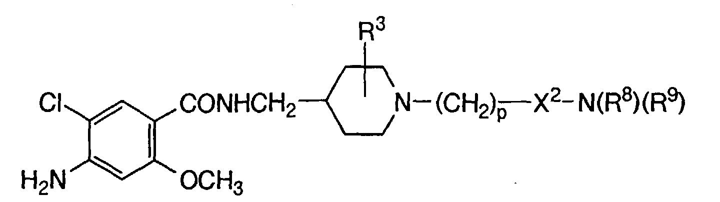

**Table 135**

| R³ | p | X² | R⁸ | R⁹ |
|---|---|---|---|---|
| H | 5 | CO | -Me | -(CH₂)₂Ph-4-Br |
| H | 5 | CO | -Et | -(CH₂)₂Ph-4-Br |
| H | 5 | CO | -Pr | -(CH₂)₂Ph-4-Br |
| H | 5 | CO | -i-Pr | -(CH₂)₂Ph-4-Br |
| H | 5 | CO | -Bu | -(CH₂)₂Ph-4-Br |
| H | 5 | CO | -Me | -(CH₂)₂Ph-4-F |
| H | 5 | CO | -Et | -(CH₂)₂Ph-4-F |
| H | 5 | CO | -Pr | -(CH₂)₂Ph-4-F |
| H | 5 | CO | -i-Pr | -(CH₂)₂Ph-4-F |
| H | 5 | CO | -Bu | -(CH₂)₂Ph-4-F |
| H | 5 | CO | -Me | -(CH₂)₂Ph-4-Me |
| H | 5 | CO | -Et | -(CH₂)₂Ph-4-Me |
| H | 5 | CO | -Pr | -(CH₂)₂Ph-4-Me |
| H | 5 | CO | -i-Pr | -(CH₂)₂Ph-4-Me |
| H | 5 | CO | -Bu | -(CH₂)₂Ph-4-Me |
| H | 5 | CO | -Me | -(CH₂)₂Ph-4-OMe |
| H | 5 | CO | -Et | -(CH₂)₂Ph-4-OMe |
| H | 5 | CO | -Pr | -(CH₂)₂Ph-4-OMe |
| H | 5 | CO | -i-Pr | -(CH₂)₂Ph-4-OMe |
| H | 5 | CO | -Bu | -(CH₂)₂Ph-4-OMe |
| H | 5 | CO | -Me | -(CH₂)₂Ph-4-NO₂ |
| H | 5 | CO | -Et | -(CH₂)₂Ph-4-NO₂ |
| H | 5 | CO | -Pr | -(CH₂)₂Ph-4-NO₂ |
| H | 5 | CO | -i-Pr | -(CH₂)₂Ph-4-NO₂ |
| H | 5 | CO | -Bu | -(CH₂)₂Ph-4-NO₂ |
| H | 5 | CO | -(CH₂)₄- | |
| H | 5 | CO | -(CH₂)₅- | |
| H | 5 | CO | -(CH₂)₂O(CH₂)₂- | |
| H | 5 | CO | -(CH₂)₂S(CH₂)₂- | |
| H | 5 | CO | -(CH₂)₂N(CH₃)(CH₂)₂- | |

## Claims

1. A benzoic acid compound of the formula 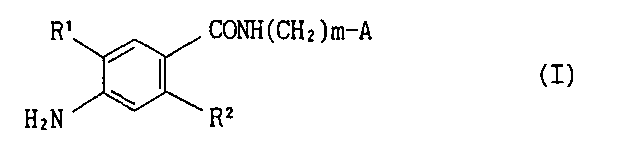 wherein
R¹ is a halogen;
R² is a lower alkoxy, a substituted lower alkoxy, a cycloalkyloxy or a cycloalkylalkoxy;
m is 1 or 2; and
A is 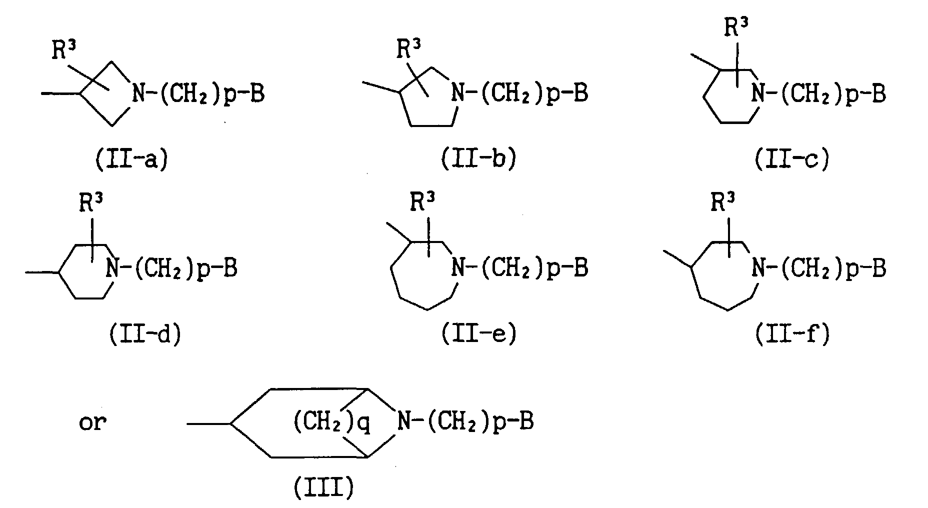
wherein
R³ is hydrogen, hydroxy, lower alkyl or lower alkoxy, p is an integer of 1-6, q is 2 or 3, and B is a group of the formula
-N(R⁴)-X¹-R⁵,
-N(R⁴)-X²-N(R⁶)(R⁷),
-X¹-N(R⁸)(R⁹) or
-Het
wherein
X¹ is CO, CS or SO₂, X² is CO or CS, R⁴ is hydrogen, lower alkyl, phenyl, substituted phenyl, aralkyl or substituted aralkyl, R⁵ is lower alkyl, cycloalkyl, crosslinked cycloalkyl, aryl, substituted aryl, aralkyl, substituted aralkyl, heteroaryl, substituted heteroaryl, heteroarylalkyl, substituted heteroarylalkyl or 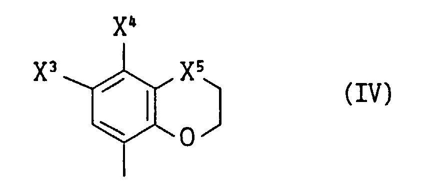 wherein X³ is halogen, X⁴ is hydrogen or amino, and X⁵ is a direct bond, methylene, oxygen atom, NH or N-CH₃,
R⁶ and R⁷ are the same or different and each is hydrogen, lower alkyl, cycloalkyl, aryl, substituted aryl, aralkyl or substituted aralkyl, or R⁶ and R⁷ optionally form a ring together with the adjacent nitrogen atom, R⁸ and R⁹ are the same or different and each is hydrogen, lower alkyl, cycloalkyl, aryl, substituted aryl, aralkyl or substituted aralkyl or R⁸ and R⁹ optionally form a ring together with the adjacent nitrogen atom, and Het is a 5- or 6-membered mono- or bicyclic heterocycle having amide or urea in the ring and having 1 to 5 hetero atom(s) selected from the group consisting of oxygen atom, sulfur atom and nitrogen atom,
an optical isomer thereof or a pharmaceutically acceptable salt thereof.

2. A benzoic acid compound of the formula 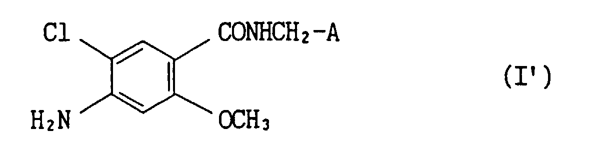 wherein A is as defined in claim 1, an optical isomer thereof or a pharmaceutically acceptable salt thereof.

3. A benzoic acid compound of the formula 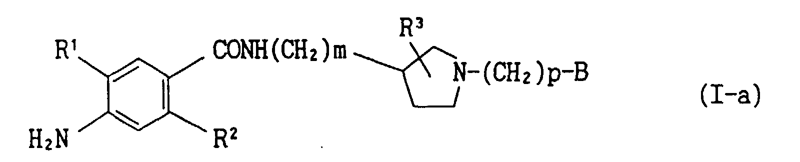 wherein R¹, R², R³, m, p and B are as defined in claim 1, an optical isomer thereof or a pharmaceutically acceptable salt thereof.

4. A benzoic acid compound of the formula 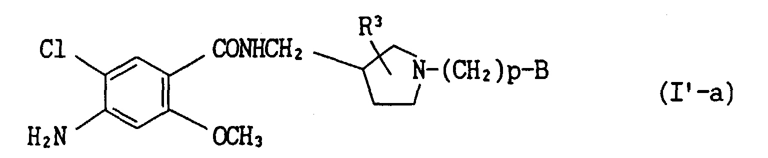 wherein R³, p and B are as defined in claim 1, an optical isomer thereof or a pharmaceutically acceptable salt thereof.

5. A benzoic acid compound of the formula 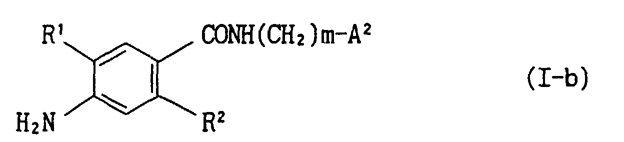 wherein R¹, R² and m are as defined in claim 1, and A² is 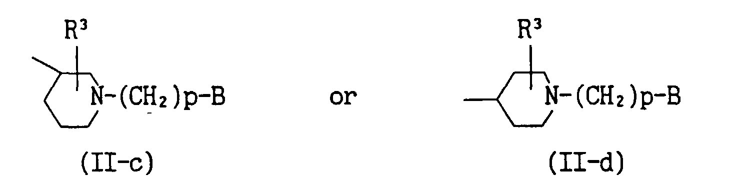 wherein R³, p and B are as defined in claim 1, an optical isomer thereof or a pharmaceutically acceptable salt thereof.

6. A benzoic acid compound of the formula 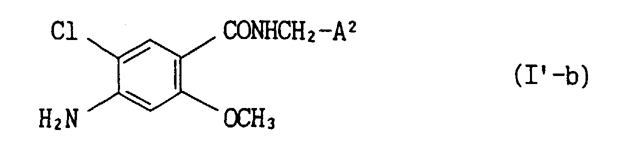 wherein A² is as defined in claim 5, an optical isomer thereof or a pharmaceutically acceptable salt thereof.

7. The benzoic acid compound of claim 1 or claim 2, wherein B is a group of the formula
-N(R⁴)-CO-R⁵,
-N(R⁴)-CO-N(R⁶)(R⁷),
-CO-N(R⁸)(R⁹) or 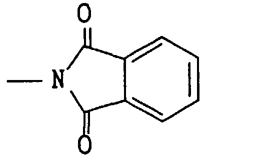 wherein R⁴, R⁵, R⁶, R⁷, R⁸ and R⁹ are as defined in claim 1, an optical isomer thereof or a pharmaceutically acceptable salt thereof.

8. The benzoic acid compound of claim 3 or claim 4, wherein B is a group of the formula
-N(R⁴)-CO-R⁵,
-N(R⁴)-CO-N(R⁶)(R⁷),
-CO-N(R⁸)(R⁹) or 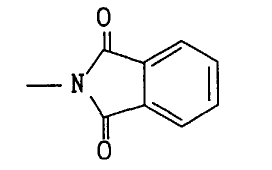 wherein R⁴, R⁵, R⁶, R⁷, R⁸ and R⁹ are as defined in claim 1, an optical isomer thereof or a pharmaceutically acceptable salt thereof.

9. The benzoic acid compound of claim 5 or claim 6, wherein B is a group of the formula
-N(R⁴)-CO-R⁵,
-N(R⁴)-CO-N(R⁶)(R⁷),
-CO-N(R⁸)(R⁹) or 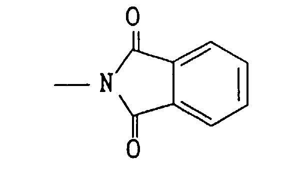 wherein R⁴, R⁵, R⁶, R⁷, R⁸ and R⁹ are as defined in claim 1, an optical isomer thereof or a pharmaceutically acceptable salt thereof.

10. The benzoic acid compound of any one of claims 1 to 9, wherein B is a group of the formula
-N(R⁴)-CO-R⁵ or
-N(R⁴)-CO-N(R⁶)(R⁷)
wherein R⁴, R⁵, R⁶ and R⁷ are as defined in claim 1, an optical isomer thereof or a pharmaceutically acceptable salt thereof.

11. The benzoic acid compound of any one of claims 1 to 10, wherein B is a group of the formula
-NHCOR⁵
wherein R⁵ is as defined in claim 1, an optical isomer thereof or a pharmaceutically acceptable salt thereof.

12. The benzoic acid compound of any one of claims 1 to 10, wherein B is a group of the formula
-NHCONHR^{6a}
wherein R^{6a} is hydrogen, lower alkyl, cycloalkyl, aryl, substituted aryl, aralkyl or substituted aralkyl, an optical isomer thereof or a pharmaceutically acceptable salt thereof.

13. The benzoic acid compound of any one of claims 1 to 9, wherein B is a group of the formula
-CONHR^{8a}
wherein R^{8a} is hydrogen, lower alkyl, cycloalkyl, aryl, substituted aryl, aralkyl or substituted aralkyl, an optical isomer thereof or a pharmaceutically acceptable salt thereof.

14. The benzoic acid compound of any one of claims 1 to 9, wherein B is a group of the formula 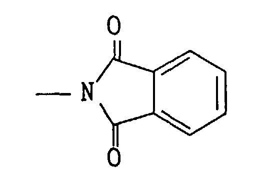 an optical isomer thereof or a pharmaceutically acceptable salt thereof.

15. The benzoic acid compound of any one of claims 1 to 11, wherein R⁵ is aryl, substituted aryl, aralkyl, heteroaryl or substituted heteroaryl, an optical isomer thereof or a pharmaceutically acceptable salt thereof.

16. The benzoic acid compound of any one of claims 1 to 10 and 12, wherein R⁶ is lower alkyl, aryl or substituted aryl, an optical isomer thereof or a pharmaceutically acceptable salt thereof.

17. The benzoic acid compound of any one of claims 1 to 9 and 13, wherein R⁸ is aryl or substituted aryl, an optical isomer thereof or a pharmaceutically acceptable salt thereof.

18. The benzoic acid compound of any one of claims 1 to 11, wherein R⁵ is 1-methyl-3-indolyl, 1-isopropyl-3-indolyl, 1-benzyl-3-indolyl, 1-naphthyl, 2-naphthyl, phenyl, 2-chlorophenyl, 3-chlorophenyl, 4-chlorophenyl, 4-methylphenyl, 4-nitrophenyl, 4-amino-5-chloro-2-methoxyphenyl, 2-thienyl or 3-phenylpropyl, an optical isomer thereof or a pharmaceutically acceptable salt thereof.

19. The benzoic acid compound of any one of claims 1 to 10 and 12, wherein R⁶ is ethyl, propyl, phenyl or 4-chlorophenyl, an optical isomer thereof or a pharmaceutically acceptable salt thereof.

20. The benzoic acid compound of any one of claims 1 to 9 and 13, wherein R⁸ is phenyl, an optical isomer thereof or a pharmaceutically acceptable salt thereof.

21. The benzoic acid compound of any one of claims 1 to 20, wherein p is an integer of 3-6, an optical isomer thereof or a pharmaceutically acceptable salt thereof.

22. The benzoic acid compound of any one of claims 1 to 20, wherein p is 4 or 5, an optical isomer thereof or a pharmaceutically acceptable salt thereof.

23. The benzoic acid compound of claim 4, wherein R³ is hydrogen, p is an integer of 2-5, and B is a group of the formula
-NHCOR^{5a},
-NHCONHR^{6b},
-CONHR^{8b} or 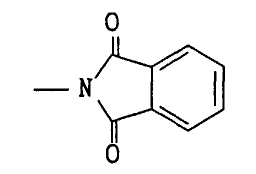 wherein R^{5a} is aryl, substituted aryl, aralkyl, heteroaryl or substituted heteroaryl, R^{6b} is lower alkyl, aryl or substituted aryl, and R^{8b} is aryl or substituted aryl,
an optical isomer thereof or a pharmaceutically acceptable salt thereof.

24. The benzoic acid compound of any one of claims 1 to 4, 7, 8, 10, 11, 15, 18, 21, 22 and 23, which is a member selected from the group consisting of:
4-amino-N-((3R)-1-(3-benzoylaminopropyl)pyrrolidin-3-ylmethyl)-5-chloro-2-methoxybenzamide,
4-amino-5-chloro-2-methoxy-N-((3R)-1-(4-(1-naphthoylamino)butyl)pyrrolidin-3-ylmethyl)benzamide,
4-amino-N-((3R)-1-(5-benzoylaminopentyl)pyrrolidin-3-ylmethyl)-5-chloro-2-methoxybenzamide,
4-amino-N-((3R)-1-(5-(4-amino-5-chloro-2-methoxybenzoylamino)pentyl)pyrrolidin-3-ylmethyl)-5-chloro-2-methoxybenzamide,
N-(4-((3R)-3-(4-amino-5-chloro-2-methoxybenzoylaminomethyl)pyrrolidin-1-yl)butyl)-1-methyl-1H-indole-3-carboxamide,
N-(5-((3R)-3-(4-amino-5-chloro-2-methoxybenzoylaminomethyl)pyrrolidln-1-yl)pentyl)-1-methyl-1H-indole-3-carboxamide,
N-(4-((3R)-3-(4-amino-5-chloro-2-methoxybenzoylaminomethyl)pyrrolidin-1-yl)butyl)-1-isopropyl-1H-indole-3-carboxamide,
N-(4-((3R)-3-(4-amino-5-chloro-2-methoxybenzoylaminomethyl)pyrrolidin-1-yl)butyl)-1-benzyl-1H-indole-3-carboxamide,
4-amino-5-chloro-2-methoxy-N-((3R)-1-(4-(2-naphthoylamlno)butyl)pyrrolidin-3-ylmethyl)benzamide,
4-amino-5-chloro-N-((3R)-1-(5-(4-chlorobenzoylamlno)pentyl)pyrrolidin-3-ylmethyl)-2-methoxybenzamide,
4-amino-5-chloro-N-(1-(3-(3-chlorobenzoylamino)propyl)pyrrolidin-3-ylmethyl)-2-methoxybenzamide,
4-amino-5-chloro-N-(1-(3-(2-chlorobenzoylamino)propyl)pyrrolidin-3-ylmethyl)-2-methoxybenzamide,
4-amino-5-chloro-2-methoxy-N-(1-(3-(4-nitrobenzoylamino)propyl)pyrrolidin-3-ylmethyl)benzamide,
4-amino-5-chloro-2-methoxy-N-((3R)-1-(2-(4-phenylbutyrylamino)ethyl)pyrrolidin-3-ylmethyl)benzamide,
4-amino-5-chloro-N-(1-(3-(4-chlorobenzoylamino)propyl)pyrrolidin-3-ylmethyl)-2-methoxybenzamide,
4-amino-5-chloro-2-methoxy-N-(1-(3-(4-methylbenzoylamino)propyl)pyrrolidin-3-ylmethyl)benzamide and
4-amino-5-chloro-2-methoxy-N-(1-(3-(2-thiophenecarbonylamino)propyl)pyrrolidin-3-ylmethyl)benzamide,
an optical isomer thereof or a pharmaceutically acceptable salt thereof.

25. The benzoic acid compound of any one of claims 1 to 4, 7, 8, 10, 12, 16, 19, 21, 22 and 23, which is a member selected from the group consisting of:
4-amino-5-chloro-2-methoxy-N-((3R)-1-(5-(3-n-propylureido)pentyl)pyrrolidin-3-ylmethyl)benzamide,
4-amino-5-chloro-2-methoxy-N-((3R)-1-(5-(3-phenylureido)pentyl)pyrrolidin-3-ylmethyl)benzamide and
4-amino-5-chloro-N-((3R)-1-(5-(3-ethylureido)pentyl)pyrrolidin-3-ylmethyl)-2-methoxybenzamide,
an optical isomer thereof or a pharmaceutically acceptable salt thereof.

26. The benzoic acid compound of any one of claims 1 to 4, 7, 8, 13, 17, 20, 21 and 23, which is 4-amino-5-chloro-2-methoxy-N-(1-(3-phenylcarbamoylpropyl)pyrrolidin-3-ylmethyl)benzamide, an optical isomer thereof or a pharmaceutically acceptable salt thereof.

27. The benzoic acid compound of any one of claims 1 to 4, 7, 8, 14, 21, 22 and 23, which is 4-amino-5-chloro-N-(1-(5-(2,3-dihydro-1,3-dioxo-1H-isoindol-2-yl)pentyl)pyrrolidin-3-ylmethyl)-2-methoxybenzamide,
an optical isomer thereof or a pharmaceutically acceptable salt thereof.

28. The benzoic acid compound of claim 6, wherein A² is a group of the formula 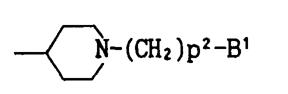 wherein p² is 4 or 5, and B¹ is a group of the formula
-NHCOR^{5a} or
-NHCONHR^{6b}
wherein R^{5a} is aryl, substituted aryl, heteroaryl or substituted heteroaryl, and R^{6b} is lower alkyl, aryl or substituted aryl, or a pharmaceutically acceptable salt thereof.

29. The benzoic acid compound of any one of claims 1, 2, 5 to 7, 9, 10, 11, 15, 18, 21, 22 and 28, which is a member selected from the group consiting of:
N-(4-(4-(4-amino-5-chloro-2-methoxybenzoylaminomethyl)piperidin-1-yl)butyl)-1-methyl-1H-indole-3-carboxamide,
4-amino-5-chloro-2-methoxy-N-(1-(4-(1-naphthoylamino)butyl)piperidin-4-ylmethyl)benzamide,
4-amino-5-chloro-2-methoxy-N-(1-(4-(2-naphthoylamino)butyl)piperidin-4-ylmethyl)benzamide,
4-amino-N-(1-(5-benzoylaminopentyl)piperidin-4-ylmethyl)-5-chloro-2-methoxybenzamide,
4-amino-5-chloro-N-(1-(5-(3-chlorobenzoylamino)pentyl)piperidin-4-ylmethyl)-2-methoxybenzamide,
4-amino-5-chloro-N-(1-(5-(4-methylbenzoylamino)pentyl)piperidin-4-ylmethyl)-2-methoxybenzamide and
N-(5-(4-(4-amino-5-chloro-2-methoxybenzoylaminomethyl)piperidin-1-yl)pentyl)-1-methyl-1H-indole-3-carboxamide, or a pharmaceutically acceptable salt thereof.

30. The benzoic acid compound of any one of claims 1, 2, 5 to 7, 9, 10, 12, 16, 19, 21, 22 and 28, which is a member selected from the group consisting of:
4-amino-5-chloro-2-methoxy-N-(1-(4-(3-n-propylureido)butyl)piperidin-4-ylmethyl)benzamide,
4-amino-5-chloro-2-methoxy-N-(1-(5-(3-n-propylureido)pentyl)piperidin-4-ylmethyl)benzamide, and
4-amino-5-chloro-N-(1-(5-(3-(4-chlorophenyl)ureido)pentyl)piperidin-4-ylmethyl)-2-methoxybenzamide, or a pharmaceutically acceptable salt thereof.

31. A pharmaceutical composition comprising a benzoic acid compound of any one of claims 1 to 30, an optical isomer thereof or a pharmaceutically acceptable salt thereof, and a pharmaceutically acceptable additive.

32. A serotonin 4 receptor agonist comprising a benzoic acid compound of any one of claims 1 to 30, an optical isomer thereof or a pharmaceutically acceptable salt thereof as an active ingredient.

33. A gastrointestinal prokinetic agent comprising a benzoic acid compound of any one of claims 1 to 30, an optical isomer thereof or a pharmaceutically acceptable salt thereof as an active ingredient.

34. A therapeutic agent for various gastrointestinal diseases selected from the group consisting of delayed gastric emptying, indigestion, meteorism, reflux esophagitis, abdominal indefinite complaint, intestinal pseudoileus, constipation, acute or chronic gastritis, gastric or duodenal ulcer, Crohn's disease, non-ulcer dyspepsia, ulcerative colitis, postgastrectomy syndrome, postoperative digestive function failure, gastrointestinal injury due to gastric neurosis, gastroptosis or diabetes, and irritable bowel syndrome, which comprises a benzoic acid compound of any one of claims 1 to 30, an optical isomer thereof or a pharmaceutically acceptable salt thereof as an active ingredient.
